# EUROPEAN PATENT APPLICATION

(11) **EP 2 332 984 A2**
(43) Date of publication of application: **15.06.2011**
(21) Application number: 10191171.7
(22) Date of filing: 29.09.2006
(51) Int. Cl.: C07K 14/82, C12Q 1/68, C12N 15/10

(54) **Tumor angiogenesis associated genes and a method for their identification**

(30) Priority: 30.09.2005 EP 05447220
(62) Divisional of application: 06818246.8
(71) Applicant: Universiteit Maastricht, NL-6229 ER Maastricht (NL)
(72) Inventor: Griffioen, Arjan Willem, 2103 XB Heemstede (NL); van Beijnum, Judith Rosina, 6228 DP, Maastricht (NL)
(74) Representative: Swinkels, Bart Willem

(57) **Abstract**

Crucial to designing anti-angiogenic and vascular targeting approaches is the identification of specific target molecules. We compared transcriptional profiles of tumor endothelial cells with that of normal resting endothelial cells, normal but angiogenically activated placental endothelial cells, and cultured endothelial cells. Although the majority of transcripts were classified as general angiogenesis markers, we identified 17 genes that show specific overexpression in tumor endothelium. Antibody targeting of four cell-surface expressed or secreted products (vimentin, CD59, HMGB1 and IGFBP7) inhibited angiogenesis *in vitro* and *in vivo.* Finally, targeting endothelial vimentin in a mouse tumor model significantly inhibited tumor growth and reduced microvessel density. Our results demonstrate the utility of the identification and subsequent targeting of specific tumor endothelial markers for anticancer therapy.

## Description

### BACKGROUND OF THE INVENTION

Tumor progression and the development of distant metastases require the presence of an extensive vasculature. Active angiogenesis is a hallmark of most malignancies and inhibition of this process is considered to be a promising strategy for the treatment of tumors. In order to develop the most specific and effective anti-angiogenic therapies for treating cancer, it is of importance to have a fundamental understanding of the molecular differences between tumor endothelial cells and their normal counterparts. Since angiogenesis is not limited to pathological conditions, careful evaluation of the putative targets is necessitated to prevent side effects associated with impaired physiological angiogenesis.

### Angiogenesis

Angiogenesis occurs in the healthy body for healing wounds and for restoring blood flow to tissues after injury. In females, angiogenesis also occurs during the monthly reproductive cycle, e.g. to rebuild the uterus lining and to mature the egg during ovulation, and during pregnancy, e.g. to build the placenta and the circulation between mother and fetus. The healthy body controls angiogenesis through a series of angiogenesis-stimulating growth factors and angiogenesis inhibitors. When angiogenic growth factors are produced in excess of angiogenesis inhibitors, the balance is tipped in favor of blood vessel growth. When inhibitors are present in excess of stimulators, angiogenesis is stopped. The normal, healthy body maintains a perfect balance of angiogenesis modulators. In general, angiogenesis is "turned off" when more inhibitors being produced than stimulators. In general it is believed that tumors produce large amounts of angiogenic growth factors, overwhelming natural inhibitors, to recruit their own blood supply.

Angiogenesis not only allows solid tumors to grow, it also makes them more dangerous because they are more likely to metastasize, i.e. spread elsewhere in the body through the bloodstream. The new blood vessels in the tumor increase the chance of cancer cells getting into the blood, especially since the tumor's blood vessels are often imperfectly formed. Moreover, it is reported that human breast cancers which became metastatic had many more blood vessels than those which did not. In order to grow larger than about two cubic millimetres, metastases require their own system of newly formed blood vessels. It is believed that if you could stop the said vascularization, it would be possible to cut the supply line to primary tumors as well as the tumor's metastases, causing them to starve.

### Gene expression

Gene expression profiling techniques are widely used to detect changes in transcript expression levels and provide the tools to study molecular events in biological processes or to identify tissue or tumor endothelial specific markers. Different cell culture models have been developed to study angiogenesis, but the temporal and spatial complex actions of all factors exerting effect on endothelial cells *in vivo* may not be accurately reflected *in vitro.* Gene expression analysis of tumor endothelial cells (TECs) encounters difficulties related to the fact that endothelial cells (ECs) are embedded in complex tissues and comprise only a small fraction of the cells present in these tissues.

### Cultured endothelial cells

Several laboratories have reported gene expression profiles of cultured endothelial cells that were subject to pro-angiogenic growth factor stimulation. In cell culture conditions, however, cells reside in an artificial microenvironment and might respond aberrantly to certain stimuli, giving a false representation of *the in vivo* situation. In fact, genes induced in these studies are highly biased to metabolic function, protein turnover and cell turnover (Abe and Sato, 2001; Dell'Era et *al.,* 2002; Gerritsen *et al.,* 2003b; Van Beijnum and Crriffioen, 2005; Wang *et al.,* 2003; Zhang *et al.,* 1999). This "cell-cycle signature" can be related to the transition from quiescent to proliferative endothelium, which is an early event in angiogenesis.

An alternative *in vitro* approach uses the three-dimensional culture of endothelial cells in matrix components such as collagen. Endothelial cell tube formation *in vitro* is mainly associated with changes in the expression of genes that mediate cell-cell contact and cell-matrix interactions, such as adhesion molecules and matrix metalloproteinases (Van Beijnum and Griffioen, 2005). Nevertheless, the complex microenvironment of angiogenic endothelial cells in tissues is extremely difficult to mimic adequately *in vitro.* In addition, when regarding angiogenesis in cancer, tumor endothelial cells have resided in the tumor microenvironments for months to years, whereas culture systems only cover a time period of days, which in addition contributes to discrepancies in observed gene expression profiles of endothelial cells in vitro vs in vivo.

In conclusion, it appears difficult to accurately mimic *in vitro* the complex temporal and spatial actions of all microenvironmental factors exerting an effect on endothelial cells *in vivo.* Therefore, extrapolation of data generated by *in vitro* experiments to the *in vivo* situation is limited, stressing the importance of approaches that make use of more relevant cell sources such as tissue derived cells.

### Freshly isolated tumor ECs

To date, only a limited number of studies have characterized the gene expression profile of freshly isolated tumor ECs (*Madden et al.,* 2004; Parker *et al.,* 2004; St Croix *et al.,* 2000). For instance, SAGE tag repertoires were generated from ECs isolated from both tumor and normal tissues and compared to identify differentially expressed genes. Notably, an extensive bias towards genes functioning in extracellular matrix remodelling among the Tumor endothelial markers (TEMs) was evident in published SAGE data sets of isolated tumor endothelial cells that were compared to, solely, normal endothelial cells (Parker *et al.,* 2004; St Croix *et al.,* 2000). The same was true for glioma endothelial markers (GEMs) (*Madden et al.,* 2004). Apparently, genes thought to play a role in the initiation of angiogenesis are only rarely identified in gene expression profiling of endothelial cells derived from tumors (*Madden et al.,* 2004; Parker *et al.,* 2004; St Croix *et al.,* 2000).

In these studies using freshly isolated tumor ECs, however, gene expression associated with physiological processes never was taken into account.

### Summary of the invention

A most crucial element in designing anti-angiogenic and vascular targeting approaches is the identification of specific target molecules.

Although it appears that some tumor endothelial cell associated markers have been identified, translation to the clinic remains a hurdle to be taken, predominantly since the prior art TAG-like molecules were not evaluated vis a vis physiological angiogenesis (TAG = tumor angiogenesis associated gene).

In the present study, the identification of markers of tumor EC is described that are also overexpressed as compared to physiologically activated placenta EC. Specifically, gene expression profiles of isolated EC from malignant colon carcinoma tissues, non-malignant angiogenic placenta tissues, as well as from non-angiogenic normal resting tissues were evaluated by using suppression subtractive hybridisation (SSH). In addition, these gene expression profiles were compared with an *in vitro* model of tumor-conditioned EC activation.

A large overlap in the expression of markers of tumor endothelium and physiologically angiogenic endothelium was observed. Hence, the present invention demonstrated that gene expression profiles of tumor derived and placenta derived endothelial cells reflect the later stages of angiogenesis, and though most upregulated genes are representative of physiological angiogenesis, a number of genes contribute specifically to a tumor endothelium specific phenotype. In addition, it was shown that *in vitro* EC activation is only to a very limited extent representative of tumor angiogenesis.

In the present invention, 17 genes were identified in detail that were specifically overexpressed in tumor endothelium, among which a number of genes coding for surface expressed or secreted protein products, e.g., vimentin, CD59, HMGB1 and IGFBP7. Antibodies targeting these proteins inhibited angiogenesis both in *in vitro* and *in vivo* assays. Targeting endothelial proteins in tumor models significantly and dose-dependently inhibited tumor growth and reduced microvessel density, with minimal effects on physiological angiogenesis.

This is the first report to investigate gene expression in endothelial cells, in which a direct distinction is made between pathological and physiological angiogenesis in comparison with quiescent endothelium, using both *in vitro* and *in vivo* sources. These findings have crucial impact on the design and improvement of angiogenesis interfering strategies for treatment of human disease.

### DETAILED DESCRIPTION OF THE INVENTION

Active angiogenesis is a hallmark of most malignancies and processes in which tissue growth is essential. Identification of tumor angiogenesis associated genes (TAGs) within the present invention, provides primary targets for the development of molecular imaging approaches and therapeutic modalities for combating cancer. However, the present invention also contemplates the identification of (general and/or specific) angiogenesis associated genes which, in addition, may provide targets for other angiogenesis dependent proliferative diseases. The present invention thus provides a method for identifying tumor angiogenesis associated genes, wherein said tumor is primarily malignant (cancer) but may also be benign.

The term "cancer" within the present specification refers to any disease characterized by uncontrolled cell division leading to a malignant (cancerous) tumor (or neoplasm, abnormal growth of tissue). Malignant tumors can invade other organs, spread to distant locations (metastasize) and become life threatening. The term "proliferative disease" refers to the rapid proliferation of cells which may either lead to a benign (not cancerous) tumor (or neoplasm) that does not spread to other parts of the body or invades other tissues - they are rarely a threat to life, or which may lead to a malignant (cancerous) tumor (or neoplasm).

Since angiogenesis is not limited to pathologies or disease, careful evaluation of putative therapeutic targets is necessary to prevent side effects associated with impaired physiological angiogenesis. In contrast to the prior art, within the present invention, transcriptional profiles of angiogenic endothelial cells isolated from both malignant and non-malignant tissues were compared with resting endothelial cells to identify tumor-specific angiogenesis markers and to distinguish these from general angiogenesis markers. Targeting TAG proteins with antibodies inhibited angiogenesis *in vitro* and *in vivo,* confirming their active contribution to the process and confirms therapeutic applications. Accordingly, the present invention relates to a method for identifying tumor angiogenesis associated genes, and the use thereof in diagnosis, therapy, and identification of modulators of angiogenesis.

### A. General Techniques

The practice of the present invention will employ, unless otherwise indicated, conventional techniques of organic chemistry, pharmacology, molecular biology (including recombinant techniques), cell biology, biochemistry, and immunology, which are within the skill of the art. Such techniques are explained fully in the literature, such as, "Molecular Cloning: A Laboratory Manual", Second Edition (Sambrook et al., 1989); "Oligonucleotide Synthesis" (M. J. Gait, ed., 1984); "Animal Cell Culture" (R. I. Freshney, ed., 1987); the series "Methods in Enzymology" (Academic Press,Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987, and periodicals); "Polymerase Chain Reaction" (Mullis et al., eds., 1994); and "Current Protocols in Immunology" (J. E. Coligan et al., eds., 1991).

### B. Definitions

As used herein, certain terms may have the following defined meanings. As used in the specification and claims, the singular form "a," "an" and "the" include plural references unless the context clearly dictates otherwise. For example, the term "a cell" includes a plurality of cells, including mixtures thereof. Similarly, use of "a compound" for treatment or preparation of medicaments as described herein contemplates using one or more compounds of this invention for such treatment or preparation unless the context clearly dictates otherwise.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention.

Throughout this disclosure, various publications, patents and published patent specifications are referenced by an identifying citation. The disclosures of these publications, patents and published patent specifications are hereby incorporated by reference into the present disclosure to describe more fully the state of the art to which this invention pertains.

Embodiments defined by each of these transition terms are within the scope of this invention.

### C. Method for identifying TAGs

The present invention relates particularly to a method for identifying specific tumor angiogenesis associated genes (TAGs). In contrast to the prior art, the present invention compared the expression profiles of tumor endothelial cells with resting endothelial cells from normal tissue, endothelial cells from placenta and cultured resting and stimulated endothelial cells.

The term "expression profiles" is well known in the art, and relates to the determination of spatial and temporal expression of genes. In particular, expression profiling may include determining the spatial and temporal amount of mRNAs, relative to metabolic conditions, genotypes, or physiopathological states of analysed tissues, and subsequent bioinformatics analysis, to characterize gene involvement in angiogenesis. Expression profiling may help elucidating what genes show different expression levels in different samples; elucidating the patterns of expression of the genes; elucidating the function of a particular gene; and elucidating the relationship with other information about these genes. The person skilled in the art is knowledgeable about algorithms and tools of bioinformatics used in expression profiling.

Techniques to differentiate between expression in different tissues are well known in the art, and include techniques such as SAGE, Suppression Subtractive Hybridization (SSH), differential display, microarray analysis, and oligonucleotide array analysis (e.g., Affymetrix).

SSH is a subtraction technique, creating a cDNA repertoire of sequences overexpressed in one tester cDNA population compared to the other driver cDNA population.

Compared to SAGE, SSH is much less labour intensive on a technical as well as a logistic level. Furthermore, unlike SAGE, SSH provides cDNA repertoires comprising individual partial cDNAs having a characteristic overexpression in tester compared to driver cDNA. These individual cDNAs may then be used as a starting point for multiple purposes including their use in immobilisation of target molecules in cDNA arrays, use as labelled probes for hybridisation experiments such as e.g., Northern blotting etc, use in the expression of partial proteins in functional studies, and use as template molecule for generating siRNA.

SSH consists of 2 hybridization steps, followed by suppression PCR to reduce the redundancy of overexpressed cDNAs. In a first step, 2 tester cDNA populations - ligated to different adaptor sequences - are hybridized in separate reactions to an excess of driver cDNA to subtract common sequences in tester and driver cDNA populations *i.e.* the non-differentially expressed genes. The cDNA is amplified, (using for instance Clontech SMART™ cDNA amplification kit) generating sufficient starting material for tester and driver, whereby the original transcript distribution is maintained. In the second hybridization the two primary hybridization samples are mixed and here create the template for the subsequent suppression PCR. During this reaction, inverted terminal repeats prevent amplification of highly abundant molecules and the amplification of differentially expressed genes is favoured. The final cDNA repertoire generated by PCR consists of cDNA fragments that are overexpressed in the tester as compared to the driver population (Clontech protocol # PT1117-1). Amplification of the target genes is dependent upon the template, such as the length, the GC content, and/or presence of inverted repeats. Therefore, the number of amplification cycles in either step is of crucial importance. As such, the method of the present invention limits the number of cycles, and preferably adapts the number of cycles to e.g. 2, 3, 4, 5, 6, 7, 8, 9 or 10 cycles, in the amplification step(s) of the SSH to ensure effective subtraction and suppression. Optimization of the number of amplification cycles ensures proper suppression and reduction of redundancy. The person skilled in the art may use routine trial and error to establish the optimum or near-optimum number of cycles to satisfy the specific needs, e.g. the provision of the original representation of transcripts.

Another main advantage of using SSH is that it is independent on previously cloned genes. Although the DNA microarray technique is considered one of the keys for deciphering the information content of the genome, *i.e.* measuring the expression levels of single genes or thousands of genes simultaneously, microarray technologies have an extremely important drawback in that only previously known and cloned genes are considered, e.g. commercially available array systems are available with gene sets biased to a particular disease. Depending on the research question and experimental setup this gene set may or may not be relevant to screen for differentially expressed genes. However, since the density of DNA sequences on a given glass slides has limitations, certain genes will not be represented.

Nevertheless, microarrays can be custom-made, and the commercially available array systems aim to cover more and more of the (human) expressed genome. As such, microarrays may find their use in combination with SSH. SSH is biased to genes of interest in the experimental setup because of the subtraction and suppression. Since it will not always be 100% effective and does not give direct information about the extent of overexpression, it may be advisable to perform cDNA array screening of the SSH repertoires.

In a first embodiment, the present invention provides a method for identifying specific tumor angiogenesis associated genes (TAGs), said method comprising:
(a) producing a cDNA library from tumor endothelial cells (TEC), producing a cDNA library from normal endothelial cells (NEC), and producing a cDNA library from active endothelial cells (AEC),
(b) performing suppression subtractive hybridisation (SSH) of TEC subtracted with NEC and TEC subtracted with AEC, and
(c) identifying the cDNAs that are overexpressed in TEC relative to NEC and/or AEC as TAGs.

In a second embodiment, the present invention provides a method for identifying specific tumor angiogenesis associated genes (TAGs), said method comprising:
(a) producing a cDNA library from tumor endothelial cells (TEC), producing a cDNA library from normal endothelial cells (NEC), and producing a cDNA library from active endothelial cells (AEC),
(b) performing suppression subtractive hybridisation (SSH) of NEC subtracted with TEC and AEC subtracted with TEC, and
(c) identifying the cDNAs that are underexpressed in TEC relative to NEC and/or AEC as TAGs.

The term "endothelial cell" is well-known to the person skilled in the art and relates to a thin, flattened cell, of which a layer lines the inside surfaces of e.g., body cavities, blood vessels, and lymph vessels, making up the endothelium. Endothelial cells perform several functions, including acting as a selective barrier to the passage of molecules and cells between the blood and the surrounding bodily tissue; they play an essential role in summoning and capturing white blood cells (leukocytes) to the site of an infection; they regulate coagulation of the blood at the site of a trauma; they regulate the growth of the vascular muscular cells; and they secrete and modify several vascular signaling molecules. A "normal endothelial cell" relates to a resting or quiescent endothelial cell, *i.e.* an endothelial cell which is not activated to an angiogenic state. As used herein, "active endothelial cell" (AEC) relates to endothelial cells which are activated to an angiogenic state, such as tissues with enhanced angiogenesis but which are not related to malignant tissues, such as, for instance, endothelial cells involved in the female productive processes and revascularization in wound healing (*i.e.,* physiological angiogenesis). Accordingly, AEC includes, but is not limited to "placental endothelial cell" (PLEC). PLEC relates to endothelial cell derived from a placenta. In the present invention, the term "tumor endothelial cell" (TEC) relates to an endothelial cell which is activated to an angiogenic state, and which is related to a malignant tissue. For instance, TECs can be derived from colorectal tumor endothelial cells or tumorigenic endothelial cells induced by malignant gliomas, e.g., glioma-endothelial cells (GECs). For comparison purposes, and evaluating the similarity with endothelial cells derived from tissues, cultured endothelial cells can be used. In this regard, human umbilical vein endothelial cells (HUVEC) are ubiquitously used. HUVECs can be freshly isolated and cultured for one or a few passages. Alternatively, established HUVEC cell lines can be used, such as the EC line EVLC2, which is a cell line derived from human umbilical vein ECs by immortalization with simian virus 40 large T antigen (Leeuwen *et al.,* 2001). Cultured endothelial cells can be activated by agents to differentiate, migrate, etc (HUVEC+). These activated cultured endothelial cells are preferably used to identify differential expression patterns resulting from activation by a particular agent, such as TPA (12-O-tetra-decanoylphorbol-13-acetate). Within the present invention, the term "HUVEC+" refers to activated cultured HUVEC cells. The term "HUVEC-" refers to non-activated, quiescent, cultured or primary HUVEC cells.

The terms "angiogenesis" and "activated to an angiogenic state" are well known in the art and relate to the formation of new branches from pre-existing blood vessels. Angiogenesis occurs, for instance, in the female reproductive tract during the formation of the corpus luteum, during endometrial development and during embryo implantation and placentation. This type of vessel growth also occurs during pathologic conditions, such as retinopathies, arthropathies, wound healing, tumor growth and metastases.

It is believed that in wound healing, hypoxic macrophages release angiogenic substances at the edges or outer surfaces of wounds that initiate revascularization. Solid tumors require their own system of newly formed blood vessels in order to grow larger than about two cubic millimeters. Beyond the critical volume of 2 cubic millimeters, oxygen and nutrients have difficulty diffusing to the cells in the center of the tumor, causing a state of cellular hypoxia that marks the onset of tumoral angiogenesis. In addition, tumors need vasculature to dispose of their metabolic waste products. New blood vessel development is an important process in tumor progression. It favors the transition from hyperplasia to neoplasia *i.e.* the passage from a state of cellular multiplication to a state of uncontrolled proliferation characteristic of tumor cells. Neovascularization also influences the dissemination of cancer cells throughout the entire body eventually leading to metastasis formation. The vascularization level of a solid tumor is thought to be an indicator of its metastatic potential.

The TAGs identifiable by the method of the invention are over- or under-expressed in tumor endothelial cells relative to normal endothelial cells and/or AEC, such as PLEC. In order to obtain generally useful TAGs and to rule out individual expression differences, the method according to the invention preferably evades source related differences. As such, the method may incorporate patient matched endothelial cells derived from normal tissue (NEC), and malignant tissue (TEC). The advantage of patient-matched endothelial cells as described above is that artefacts in TEC and NEC are allowed to be levelled out against one another. In order to further evade individual expression differences or MHC (Major Histocompatibility Complex) class differences, the endothelial cells for producing cDNA libraries may be pooled from at least two different patients, and preferably from more patients, such as, for instance, from 3, 4, 5 or even more patients.

The endothelial cells are embedded in other cell types, which obscure endothelial cell specific expression. Therefore, in a further embodiment, the present invention relates to endothelial cells for producing cDNA libraries which are isolated to at least 90% purity, and preferably, to at least 95%, 96%, 97%, 98%, 99% or 100% purity. Methods for purifying cells are known in the art, and are described for instance in the examples section. For instance, endothelial cells may be isolated by using endothelial specific cell markers, e.g. CD31 and/or antibodies directed thereto, e.g. anti-CD31, and cell sorting, e.g. FACS.

The term "purified" as applied herein, refers to a composition wherein the desired component, such as a polypeptide, nucleic acid, antibody, cell, etc comprises at least 50%, 60%, 70%, 80%, 90% and preferably at least 95%, 96%, 97%, 98%, 99% or 100% of the desired component in the composition. The composition may contain other compounds, such as carbohydrates, salts, solvents, lipids, and the like, without affecting the determination of percentage purity as used herein.

### D. Isolated targets

The method according to the invention allows the identification of differentially expressed genes by pair-wise comparisons, such as in TEC relative to AEC, or NEC, or cultured endothelial cells (freshly isolated and cultured EC or established EC cell lines). Specifically, the present invention relates to the identification of genes which are over- or under-expressed in TEC relative to NEC; TEC relative to AEC, such as PLEC; AEC such as PLEC relative to NEC; and HUVEC+ relative to HUVEC-. In particular, the present invention relates to the identification of differentially expressed genes in TEC relative to AEC, such as PLEC, and TEC relative to NEC.

### D1. TAGs

Accordingly, the present invention relates to tumor angiogenesis associated genes (TAGs) identifiable by the method of the invention (Table 2). The group of TAGs includes the nucleic acids as depicted in Table 2, *i.e*. characterized by the GenBank accession numbers: NM_152862.1, NM_000611, NM_004642.2, NM_000088.2, NM_001845.2, NM_002128.3, BC003378, BC041913, NM_014571, NM_017994.1, X02160, NM_001553, NM_002300, CV337080, AJ320486, NM 003118.1, AF077200 and X56134, which are included herein specifically by reference. Particularly, the present invention relates to isolated polynucleotides comprising or consisting of nucleic acids characterized by any of SEQ ID NO:s 1, 3, 5, 7, 9, 11, 13, 15, 17, 19, 21, 23, 25, 27, 29, 31 or 33, or a part thereof, the complement thereof, or a variant of said nucleic acids. It will be appreciated that the present invention also relates to parts and complements of said variants. The connoted parts are preferably unique parts (*i.e.*, non-repetitive sequence parts and/or not present in other genes). Using routine techniques, the person skilled in the art is able to establish the percentage identity. The present invention is also directed to variants of the nucleotide sequence of the nucleic acid disclosed in the invention, and preferably any of SEQ ID NO:s 1, 3, 5, 7, 9, 11, 13, 15, 17, 19, 21, 23, 25, 27, 29, 31 or 33, or the corresponding complementary strand.

The nucleotide and amino acid sequences of the TAG genes of the invention are depicted in Figure 9A. The nucleotide sequences of SSH identified TAG inserts are depicted in Figure 9B.

The term "variant" relates to a nucleic acid molecule which is at least 65%, 70%, 75%, 80%, 85%, 90%, 95%, 96%, 97%, 97,5%, 98%, 98,5%, 99% or 99,5% identical to the nucleotide sequences of the invention, and preferably as represented in SEQ ID NO:s 1, 3, 5, 7, 9, 11, 13, 15, 17, 19, 21, 23, 25, 27, 29, 31 or 33, or the corresponding complementary strand, or parts thereof.

By a nucleic acid having a nucleotide sequence of at least, for example, 95% "identity" to a reference nucleotide sequence of the present invention, it is intended that the nucleotide sequence of said nucleic acid is identical to the reference sequence except that the nucleotide sequence may include up to five point mutations per each 100 nucleotides of the reference nucleotide sequence. In other words, to obtain a nucleic acid having a nucleotide sequence of at least 95% identity to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. As a practical matter, whether any particular nucleic acid molecule is at least 65%, 70%, 75%, 80%, 85%, 90%, 95%, 96%, 97%, 97,5%, 98%, 98,5%, 99% or 99,5% identical to a nucleotide sequence of the present invention can be determined using known algorithms. A preferred method for determining the best overall match between a query sequence (a sequence of the present invention) and a subject sequence can be determined using a Blast search (Altschul *et al.,* 1997). It will be appreciated that the terms "nucleic acids" and "nucleotide sequence" are used interchangeably herein.

By using the method of the invention, it may be possible that, inherent to molecular biology techniques, only part of the transcript corresponding to the differentially expressed gene is isolated. Nevertheless, even part of the transcript, and also the corresponding cDNA, allows determining the identity of the gene. For instance, after establishing the sequence of the (partial) transcript or cDNA, the identity of the corresponding gene can be established by a sequence comparison with commonly available sequences, such as present in the GenBank. Alternatively, in case the corresponding gene is not known, the complete sequence of the gene can be revealed by routine molecular biological techniques, such as for instance screening cDNA libraries, preferably derived from endothelial cells, including but not limited to endothelial tumor tissue such as malignant endothelial cell derived tumors e.g. angiomas, and gene-walking. Accordingly, the nucleotide sequences presented in the present invention may be extended starting from a partial nucleotide sequence and employing various methods known in the art to detect the full sequence in case said sequence would only be a part of a coding region as well as upstream sequences such as promoters and regulatory elements.

The identification of the differentially expressed genes by the method according to the invention facilitates the identification of the corresponding amino acid sequence. Accordingly, the present invention relates to isolated polypeptides comprising, or alternatively consisting of, an amino acid sequence according to the invention, and preferably characterized by any of SEQ ID NO:s 2, 4, 6, 8, 10, 12, 14, 16, 18, 20, 22, 24, 26, 28, 30, 32 or 34, or a part thereof, or comprising or consisting of a variant thereof, or an immunologically active and/or functional fragment thereof.

A variant peptide is characterized by an amino acid sequence which is at least 65%, 70%, 75%, 80%, 85%, 90%, 95%, 96%, 97%, 97,5%, 98%, 98,5%, 99%, 99,5% or 100% identical to an amino acid sequence according to the invention, and preferably characterized by any of SEQ ID NO:s 2, 4, 6, 8, 10, 12, 14, 16, 18, 20, 22, 24, 26, 28, 30, 32 or 34, or a part thereof. Using routine techniques, the person skilled in the art is able to establish the percentage identity.

It will be appreciated that the present invention relates to an isolated polypeptide encodable by a nucleic acid according to the invention, or a variant or a derivative thereof, or an immunologically active and/or functional fragment thereof. More preferably, a polypeptide comprising or consisting of an amino acid sequence as given in SEQ ID NO:s 2, 4, 6, 8, 10, 12, 14, 16, 18, 20, 22, 24, 26, 28, 30, 32 or 34, or a variant or a derivative thereof, or an immunologically active and/or functional fragment thereof. Specifically the present invention relates to an isolated nucleic acid comprising a member selected from a group of nucleic acids identifiable as a tumor angiogenesis associated gene (TAG) according to the method of the invention, said group consisting of:
(a) a nucleic acid comprising a DNA sequence as given in SEQ ID NO 1, 3, 5, 7, 9, 11, 13, 15, 17, 19, 21, 23, 25, 27, 29, 31 or 33, or a part thereof, or the complement thereof,
(b) a nucleic acid comprising the RNA sequences corresponding to SEQ ID NO 1,3, 5, 7, 9, 11, 13, 15, 17, 19, 21, 23, 25, 27, 29, 31 or 33, or a part thereof, or the complement thereof,
(c) a nucleic acid specifically hybridizing to the nucleotide sequence as defined in (a) or (b),
(d) a nucleic acid comprising of a nucleotide sequence, which is at least 65% identical to the sequence defined in (a),
(e) a nucleic acid encoding a protein with an amino acid sequence, which is at least 65% identical to the amino acid sequence as given in SEQ ID NO 2, 4, 6, 8, 10, 12, 14, 16, 18, 20, 22, 24, 26, 28, 30, 32 or 34, or a part thereof,
(f) a nucleic acid encoding a protein comprising the amino acid sequence as given in any of SEQ ID NO 2, 4, 6, 8, 10, 12, 14, 16, 18, 20, 22, 24, 26, 28, 30, 32 or 34, or a part thereof,
(g) a nucleic acid which is degenerated as a result of the genetic code to a nucleotide sequence of a nucleic acid as given in SEQ ID NO 1, 3, 5, 7, 9, 11, 13, 15, 17, 19, 21, 23, 25, 27, 29, 31 or 33, or a part thereof or as defined in (a) to (f),
(h) a nucleic acid which is diverging due to the differences in codon usage between the organisms to a nucleotide sequence encoding a protein as given in SEQ ID NO 2, 4, 6, 8, 10, 12, 14, 16, 18, 20, 22, 24, 26, 28, 30, 32 or 34 or as defined in (a) to (g),
(i) a nucleic acid which is diverging due to the differences between alleles encoding a protein as given in SEQ ID NO 2, 4, 6, 8, 10, 12, 14, 16, 18, 20, 22, 24, 26, 28, 30, 32 or 34, or as defined in (a) to (h),
(j) a nucleic acid encoding an immunologically active and/or functional fragment of a protein encoded by a DNA sequence as given in SEQ ID NO 1, 3, 5, 7, 9, 11, 13, 15, 17, 19, 21, 23, 25, 27, 29, 31 or 33,
(k) a nucleic acid encoding a gene family member of the nucleic acid as given in SEQ ID NO 1, 3, 5, 7, 9, 11, 13, 15, 17, 19, 21, 23, 25, 27, 29, 31 or 33, and,
(l) a nucleic acid encoding a protein as defined in SEQ ID NO 2, 4, 6, 8, 10, 12, 14, 16, 18, 20, 22, 24, 26, 28, 30, 32 or 34, or a nucleic acid as defined in any one of (a) to (k) characterized in that said sequence is DNA, cDNA, genomic DNA or synthetic DNA.

In the present invention, the term "immunologically active" fragment relates to a fragment of the polypeptide according to the invention which comprises an epitope (T-cell and/or B-cell epitope). The minimal length of an epitope will be about 5 amino acids, but is preferably longer, such as, for instance, 6, 7, 8, 9, 10 or even more amino acids.

In the present invention, the term "functional fragment" relates to a fragment of the polypeptide according to the invention, and said functional fragment comprises still at least 60% activity of the protein from which it is derived. The activity of a protein may be determined by functional assays applicable to the particular protein at issue and well known in the art.

### D2. GAGs

In contrast to the prior art, the present invention was able to distinguish between differential expression of genes upregulated and downregulated in TEC compared to the expression of genes involved in NEC and physiological angiogenesis such as female reproductive processes (PLEC) and wound healing, by comparison of the expression patterns of tumor endothelial cells; normal, *i.e.* resting, endothelial cells; and active but non-malignant endothelial cells. As such, the present invention relates to the identification of differentially expressed genes in physiological angiogenesis of AEC, and preferably PLEC, relative to NEC. Even more preferably, the present invention relates to the identification of differentially expressed genes in AEC, such as PLEC relative to NEC, and TEC relative to NEC (defined as general angiogenesis genes A or GAG/A). Hence, the present invention relates to an isolated nucleic acid comprising a member selected from a group of nucleic acids identifiable as general angiogenesis genes GAG/A according to the method of the invention, or a part thereof, or comprising or consisting of a variant thereof, or an immunologically active and/or functional fragment thereof. The group of GAG/A includes the nucleic acids as depicted in Table 3, *i.e.* characterized by the GenBank accession numbers: NM_007200, NM_001575, NM_147783.1, NM_005348, NM_001753, BX115183, NM_001921.1, NM_001344, NM_006304, BC047664, NM_007036, AW269823, NM_003107, NM_004280.2, NM_000801, AK056761, BC003394, NM_145058, NM_002211, NM_006479, NM_170705.1, BC011818, NM_033480, NM_032186, NM_002421, NM_002425, NM_001416, BC025278, NM_014959, M15887, AI793182, BC032350, NM_002982, NM_002422, NM_021109, BC018163, AA296386, NM_003347, AI422919, NM_004339.2, BC050637, AY117690.1, NM_015987.2, AK094809.1, NM_000983 and NM_175862, which are included herein specifically by reference.

In order to determine the usefulness of cultured cells in resembling *in vivo* processes, the expression profiles of cultured endothelial cells, possibly treated with tumor promoting agents and/or agents that activate angiogenesis, may be compared with the expression profiles of AEC, such as PLEC; NEC; and/or TEC. Accordingly, the present invention relates to the identification of differentially expressed genes, such as overexpressed genes, in tumor conditioned HUVEC+ relative to AEC, NEC, and/or TEC. Even more preferably, the present invention relates to the identification of differentially expressed genes in TEC relative to NEC and HUVEC+ relative to HUVEC- (defined as general angiogenesis genes B or GAG/B). As such, the present invention relates to an isolated nucleic acid comprising a member selected from a group of nucleic acids identifiable as general angiogenesis genes B (GAG/B) according to the method of the invention, or a part thereof, or comprising or consisting of a variant thereof, or an immunologically active and/or functional fragment thereof. The group of GAG/B includes the nucleic acids as depicted in Table 3, *i.e.* characterized by the GenBank accession numbers: NM_001575, NM_005348, BX115183, NM_006304, BC047664, NM_007036, NM_003107, NM_004280.2, BC003394, BC011818, NM_033480, NM_032186, NM_002425, BC025278, NM_014959, M15887, NM_021109, NM_003347, NM_000442.2 and NM_000982.2, which are included herein specifically by reference.

It will be appreciated that there is an overlap between GAG/A and GAG/B.

### D3. General

The present invention relates also to a nucleic acid molecule of at least 12, or more preferably 13, 14, 15, 16, 17, 18, 19, 20, 25, 30, 40, 50 or even more nucleotides in length specifically hybridizing with a nucleic acid according to the invention. Longer nucleotides are also contemplated, e.g. of about 75, 100, 200 or even more nucleotides. Different types of hybridisation techniques and formats are well known in the art. The said nucleic acid molecule may be labeled, thereby allowing the detection of the hybrid. In this regard, the present invention provides methods for detecting the nucleic acids of the present invention. The term "label" as used in present specification refers to a molecule propagating a signal to aid in detection and quantification. Said signal may be detected either visually (e.g., because it has color, or generates a colored product, or emits fluorescence) or by use of a detector that detects properties of the reporter molecule (e.g., radioactivity, magnetic field, etc.). Labeling systems are well known in the art and include, without limitation, the use of a variety of stains or the incorporation of fluorescent, luminescent, radioactive or otherwise chemically modified nucleotides such as e.g., labeled streptavidin conjugate, digoxigenin, anti-digoxigenin, luciferase, P-galactosidase, antigens, enzymes and enzyme conjugates, (e.g. horseradish peroxidase, alkaline phosphatase and others).

In a further embodiment, the present invention relates to an amplification primer, preferably a nucleic acid molecule of at least 12, or more preferably 13, 14, 15, 16, 17, 18, 19, 20, 25 or even more nucleotides in length specifically amplifying a nucleic acid according to the invention. As such, the nucleic acid is liable to act as a primer for specifically amplifying a nucleic acid of the present invention, or a part thereof.

The primers may be used in any well described amplification technique known in the art such as, for instance, Polymerase Chain Reaction (PCR), TMA (transcripition mediated amplification) or NASBA (nucleic acid sequence based amplification) techniques, thereby allowing the amplification and subsequent detection of the nucleic acid of the present invention. Preferably, said primers may also be used to specifically amplify the nucleic acids of the present invention. As such, the present invention provides methods for detecting the nucleic acids of the present invention.

The primers of the invention provide for specifically amplifying the target sequence. In the present invention, the term "specifically amplifying" relates to the preferred amplification of the target sequence, while non-target sequences are not or less well amplified, because of which the ratio between target sequence versus the non-target sequence is increased. Hybridisation conditions for the primer binding to the target sequence are at least co-decisive for specifically amplifying. In other words, temperature, salt concentration, etc., determine the hybridisation specificity. Preferably, the present invention provides the amplification primers for TAGs as depicted in Table 4, *i.e.* SEQ ID NO:s 75 - 108.

It will be appreciated by the person skilled in the art that the term "specifically" within the context of "specifically hybridising" and "specifically amplifying" relates to the stringent hybridisation of a nucleic acid with a target sequence. It is clear to the skilled person that a specific hybridisation event, in case of an amplification primer, results in a specific amplification.

Nucleic acids which specifically hybridise to any of the strands of the nucleic acid molecules of the present invention, such as characterized by SEQ ID NO:s 1, 3, 5, 7, 9, 11, 13, 15, 17, 19, 21, 23, 25, 27, 29, 31 or 33 under stringent hybridisation conditions or lower stringency conditions are also particularly encompassed by the present invention.

"Stringent hybridisation conditions" are dependent upon the composition of the probe, including length and GC-content, and can be determined by appropriate computer programmes. Hybridisation under high and low stringency conditions are principles which are well understood by the person skilled in the art (see, for instance, Sambrook et al. Molecular Cloning: A laboratory manual. Cold Spring Harbor laboratory press 1989). For instance, in hybridisation experiments, stringent hybridisation conditions refer in general to an overnight incubation at 68°C in a solution comprising 5xSSC (750 mM NaC1, 75 mM trisodium citrate), 50 mM sodium phosphate (pH 7.6), 5x Denhardt's solution, 10% dextran sulfate and 20 µg/ml denatured sheared salmon sperm DNA, followed by washing the filters in 0.1xSSC at about 65°C. Changes in the stringency of hybridisation are primarily accomplished through the manipulation of the SSC dilution in the washing steps (higher concentration SSC in washing buffer results in lower stringency) and the temperature (lower washing temperature results in lower stringency). For example, lower stringency conditions include washes performed at 1xSSC and at 55-60°C.

### D4. Expression vectors

Methods which are well known to those skilled in the art may be used to construct expression vectors containing at least a fragment of any of the nucleic acids of the present invention together with appropriate transcriptional and translational control elements. These methods include *in vitro* recombinant DNA techniques, synthetic techniques, and *in vivo* genetic recombination. Such techniques are described, for example, in Sambrook et al. Molecular Cloning: A laboratory manual. Cold Spring Harbor laboratory press 1989. Correspondingly, the present invention relates also to vectors comprising a nucleic acid of the present invention, or a fragment thereof. This nucleic acid may be a member selected from a group of nucleic acids identifiable as TAG, GAG/A and/or GAG/B. Preferably, said nucleic acid is a member selected from a group represented by SEQ ID NOa 1, 3, 5, 7, 9, 11, 13, 15, 17, 19, 21, 23, 25, 27, 29, 31 and 33, including variants, fragments or homologues thereof.

The present invention particularly contemplates recombinant expression vectors, preferably said vectors comprising a vector sequence, an appropriate prokaryotic, eukaryotic or viral or synthetic promoter sequence followed by the nucleic acid of the present invention or a fragment thereof. Preferably, the vector used for expressing the nucleic acid according to the present invention can be a vector for expression in *E. coli,* a yeast shuttle vector, or a yeast two-hybrid vector, a plant vector, an insect vector, a mammalian expression vector, including but not limited to, a herpes virus vector, a baculovirus vector, a lentivirus vector, a retrovirus vector, an alphavirus vector, an adenoviral vector or any combination thereof. Accordingly, in a preferred embodiment said vector is an expression vector, wherein the nucleotide sequence is operably linked to one or more control sequences allowing the expression of said sequence in prokaryotic and/or eukaryotic host cells.

In a further embodiment, the vectors of the invention are present in a host cell. The host cell is preferably a yeast, bacterial, insect, fungal, plant, fish, avian, reptilian or mammalian cell. It will be appreciated that the host cell may comprise an integrated or episomal copy of a nucleic acid according to the invention or a vector according to the invention.

In addition, the present invention provides a method for producing a polypeptide according to the invention, comprising culturing a host cell as described supra under conditions allowing the expression of the polypeptide.

It will be understood that the present invention relates also to a transgenic non-human animal comprising one or more copies of a nucleic acid of the present invention stably integrated in the genome, or an animal comprising regulatory elements that modulate the expression of a nucleic acid of the present invention.

In addition to transgenic animals, a gene may be knocked out, for instance to study effects thereof. A gene can be knocked-out by various means. Therefore, a preferred embodiment of the present invention pertains to a knock-out non-human animal comprising a deletion of one or two alleles encoding a nucleic acid according to the invention, or a animal comprising a targeted mutation in the genomic region, including regulatory sequences, comprising any of the nucleic acid sequences according to the invention. In general, a knock-out will result in the ablation of the function of the particular gene.

In an even more preferred embodiment, the present invention relates to the use of a transgenic or knock-out non-human animal according to the present invention as a model system for studying angiogenesis, and in particular proliferative diseases.

### E. Antibodies

In a preferred embodiment, the invention provides an antibody specifically recognising the polypeptides of the present invention, or a specific epitope of said polypeptide. The term "epitope" refers to portions of a polypeptide having antigenic or immunogenic activity in an animal, preferably a mammal, and most preferably in a human. Epitope-bearing polypeptides of the present invention may be used to induce antibodies according to methods well known in the art including, but not limited to, *in vivo* immunisation, *in vitro* immunisation, phage display methods or ribosome display.

The antibody of the present invention relates to any polyclonal or monoclonal antibody binding to a protein of the present invention. The term "monoclonal antibody" used herein refers to an antibody composition having a homogeneous antibody population. The term is not limiting regarding the species or source of the antibody, nor is it intended to be limited by the manner in which it is made. Hence, the term "antibody" contemplates also antibodies derived from camels (Arabian and Bactrian), or the genus lama. Thus, the term "antibody" also refers to antibodies derived from phage display technology or drug screening programs. In addition, the term "antibody" also refers to humanised antibodies in which at least a portion of the framework regions of an immunoglobulin are derived from human immunoglobulin sequences and single chain antibodies as described in U.S. patent No 4,946,778 and to fragments of antibodies such as F_{ab}, F_{'}(_{ab})₂, F_{v,} and other fragments which retain the antigen binding function and specificity of the parent antibody. The term "antibody" also refers to diabodies, triabodies or multimeric (mono-, bi -, tetra- or polyvalent/ mono-, bi- or polyspecific) antibodies, as well as enzybodies, i.e. artificial antibodies with enzyme activity. Combinations of antibodies with any other molecule that increases affinity or specificity, are also contemplated within the term "antibody". Antibodies also include modified forms (e.g. mPEGylated or polysialylated form (Fernandes & Gregoriadis, 1997) as well as covalently or non-covalently polymer bound forms. In addition, the term "antibody" also pertains to antibody-mimicking compounds of any nature, such as, for example, derived from lipids, carbohydrates, nucleic acids or analogues e.g. PNA, aptamers (see Jayasena, 1999).

In specific embodiments, antibodies of the present invention cross-react with murine, goat, rat and/or rabbit homologues of human proteins and the corresponding epitopes thereof. As such, the present invention provides a method for detecting the polypeptides of the present invention, the method comprising the use of the antibodies in immunoassays for qualitatively or quantitatively measuring levels of the polypeptides of the present invention in biological samples.

In particular, the present invention relates to an antibody specifically recognising a polypeptide encoded by a nucleic acid according to the present invention, or a specific epitope of said polypeptide.

Antibodies of the present invention may act as inhibitors, agonists or antagonists of the polypeptides of the present invention.

Antibodies of the present invention may be used, for example, but not limited to, to purify, detect, target, and/or inhibit the activity of the polypeptides of the present invention, in TEC, but also PLEC, AEC or NEC, including both *in vitro* and *in vivo* diagnostic and therapeutic methods, as well as in drug screens.

### F. Diagnosis

As described in the introduction, a large number of diseases including solid tumor formation are caused by a disturbance of the fine-tuned balance between signals regulating angiogenesis. The pathologies caused by disturbances in angiogenic processes include proliferative disorders including malignancies, diabetic retinopathy, rheumatoid arthritis, psoriasis, restenosis, endometriosis, impaired wound healing, and atherosclerosis. Methods which can be used for diagnosis are also further detailed in the examples section. Accordingly, the present invention relates to diagnosing a pathological condition, wherein said pathological condition is chosen from the group consisting of proliferative disorders, including tumors, diabetic retinopathy, rheumatoid arthritis, psoriasis, restenosis, endometriosis, impaired wound healing, and atherosclerosis. Correct diagnosis of a pathological condition would be beneficial for treatment of and medication to a patient suffering from said pathological condition. Furthermore, diagnosis may aid in determining a predisposition or susceptibility to a pathological condition, e.g. before onset of the pathological condition. Correspondingly, the present invention relates to a polynucleotide, polypeptide or antibody according to the invention for diagnosing a pathological condition or a susceptibility to a pathological condition. The present invention also provides the use of a polynucleotide according to the invention, such as TAG, GAG/A and/or GAG/B polynucleotides and preferably characterized by any of SEQ ID NO:s 1, 3, 5, 7, 9, 11, 13, 15, 17, 19, 21, 23, 25, 27, 29, 31 or 33, or a part thereof, for diagnosing angiogenesis, and preferably tumor endothelial cells. In a further embodiment, the present invention provides the use of an antibody specifically directed against a polypeptide according to the invention, such as TAG, GAG/A and/or GAG/B polypeptide, and preferably characterized by any of SEQ ID NO:s 2, 4, 6, 8, 10, 12, 14, 16, 18, 20, 22, 24, 26, 28, 30, 32 or 34, or a part thereof, for diagnosing a pathological condition such as a proliferative disorders and/or impaired angiogenesis.

In a preferred embodiment, the present invention relates to a method of diagnosing a pathological condition or a susceptibility to a pathological condition in a subject comprising the steps of:
(a) determining the over- or under-expression of a polynucleotide or a polypeptide according to the invention in a biological sample relative to the expression in a control sample, and,
(b) diagnosing a pathological condition or a susceptibility to a pathological condition based on the over- or under-expression of said polynucleotide or said polypeptide in said biological sample relative to the expression in a control sample.

The term "biological sample" refers to a sample that is tested for the presence, abundance, quality or an activity of a molecule of interest, such as a polypeptide according to the invention, a polynucleotide encoding a polypeptide according to the invention, or an agent or compound that modifies or modulates the activity of a polypeptide according to the invention. A sample containing a molecule of interest, may be obtained in numerous ways known in the art. Virtually any sample may be analysed using the method according to the present specification including cell lysates, purified genomic DNA, body fluids such as from a human or animal, clinical samples, etc. Thus, a "biological sample" contemplates a sample obtained from an organism or from components (e.g., cells) of an organism. The sample may be of any biological tissue or fluid. Usually, the sample is a biological or a biochemical sample. Frequently the sample will be a "clinical sample" which is a sample derived from a patient. Such samples include, but are not limited to, sputum, cerebrospinal fluid, blood, blood fractions such as serum including foetal serum (e.g., SFC) and plasma, blood cells (e.g., white cells), tissue or fine needle biopsy samples, urine, peritoneal fluid, and pleural fluid, or cells there from. Biological samples may also include sections of tissues such as frozen sections taken for histological purposes. The sample can be, for example, also a physiological sample. The term "tissue" as used herein refers to cellular material from a particular physiological region. The cells in a particular tissue can comprise several different cell types. A non-limiting example of this would be tumor tissue that comprises capillary endothelial cells and blood cells, all contained in a given tissue section or sample. It will be appreciated from the invention that in addition to solid tissues, the term "tissue" is also intended to encompass non-solid tissues, such as blood. A "control sample" or "standard" relates to a sample of which the expression level, amount and/or abundance of a polynucleotide, nucleic acid, polypeptide and/or activity of a polypeptide is known, or has been determined previously. As such, the control sample may be derived from a "healthy" person, i.e. a person diagnosed previously as not suffering or predisposed from the pathological condition(s) at issue. Alternatively, the control sample may be derived from a "diseased" person, i.e. a person diagnosed previously as suffering or predisposed from the pathological condition(s) at issue. The sample may be spiked with a known amount of molecules. In a further alternative, the control sample may be synthetic, i.e. not derived from a person, but comprising a known amount of molecules.

In a preferred embodiment, the present invention relates to a method of diagnosing a pathological condition or a susceptibility to a pathological condition, said method comprising:
(a) contacting a biological sample with a probe specific for any of the nucleic acids according to the invention, such as TAG, GAG/A and/or GAG/B nucleic acids, and preferably SEQ ID NO:s 1, 3, 5, 7, 9, 11, 13, 15, 17, 19, 21, 23, 25, 27, 29, 31 or 33, or a part thereof;
(b) detecting binding of said probe to said nucleic acids according to the invention, such as TAG, GAG/A and/or GAG/B nucleic acids, and preferably SEQ ID NOa 1, 3, 5, 7, 9, 11, 13, 15, 17, 19, 21, 23, 25, 27, 29, 31 or 33, or a part thereof present in said biological sample;
(c) comparing the binding detected in step (b) with a standard,
wherein a difference in binding relative to the standard is diagnostic of a pathological condition or a susceptibility to a pathological condition.

In another embodiment, the present invention relates to a method for targeting a diagnostic agent to tumor-associated vasculature in an animal, preferably a human, having a vascularized tumor, comprising: administering a diagnostic agent to the animal, wherein the diagnostic agent comprises an operatively attached targeting compound, and wherein the targeting compound recognizes and binds to a TAG, said TAG preferably being chosen from the group characterized by any of SEQ ID NOa 1 to 34.

As used herein, the "diagnostic agent" relates to an agent comprising two functional moieties, *i.e.* a first moiety enabling detection (detection compound) and a second moiety enabling binding to the molecule to be diagnosed (targeting compound). In a preferred embodiment, the present invention relates to a method as described herein, wherein said targeting compound is an antibody and the detection compound is a paramagnetic, radioactive or fluorogenic molecule that is detectable upon imaging.

In another embodiment, the present invention relates to a method of identifying regions of (neo)angiogenesis in an animal, preferably a human, comprising:
- administering to an animal a diagnostic agent comprising an antibody variable region which specifically binds to a polypeptide according to the invention, such as TAG, GAG/A and/or GAG/B polypeptide, or a part thereof, said polypeptide preferably selected from the group consisting of SEQ ID NO:s 2, 4, 6, 8, 10, 12, 14, 16, 18, 20, 22, 24, 26, 28, 30, 32 and 34, including parts thereof;
- detecting the diagnostic agent in the patient; and
thereby identifying regions of (neo)angiogenesis in the patient.

In a further embodiment, the present invention relates to a method of screening for (neo)angiogenesis in a patient, comprising:
(a) contacting a biological sample with a molecule comprising an antibody variable region which specifically binds to a polypeptide according to the invention, such as TAG, GAG/A and/or GAG/B polypeptide, or a part thereof, said polypeptide preferably selected from the group consisting of SEQ ID NO : 2, 4, 6, 8, 10, 12, 14, 16, 18, 20, 22, 24, 26, 28, 30, 32 and 34, including parts thereof; and
(b) detecting material in the biological sample that is cross-reactive with the molecule, and
wherein detection of cross-reactive material indicates neo-angiogenesis in the patient. The invention also provides a method of screening for neo-angiogenesis in a patient, comprising:
(a) detecting an expression product of at least one gene according to the invention, such as TAG, GAG/A and/or GAG/B genes, in a first tissue sample of a patient, wherein said at least one gene is preferably selected from the group consisting of SEQ ID NO:s 1, 3, 5, 7, 9, 11, 13, 15, 17, 19, 21, 23, 25, 27, 29, 31 and 33, including parts thereof; and
(b) comparing expression of the expression product of said at least one gene in the first tissue sample with expression of the expression product of the at least one gene in a second tissue sample which is normal,
wherein an increased expression of the expression product of the at least one gene in the first tissue sample relative to the second tissue sample identifies the first tissue sample as likely to be neo-angiogenic.

### F.1 Diagnosing nucleic acids

Also, the present invention relates to a method for diagnosing a pathological condition or a susceptibility to a pathological condition, comprising the steps of :
(a) detecting an expression product of at least one gene according to the invention in a first biological sample suspected of a pathological condition, wherein said at least one gene characterized by a polynucleotide according to the invention, such as TAG, GAG/A and/or GAG/B polynucleotides, and preferably selected from the group consisting of SEQ ID NO:s 1, 3, 5, 7, 9, 11, 13, 15, 17, 19, 21, 23, 25, 27, 29, 31 and 33, including parts thereof; and
(b) comparing expression of the expression product of at least one gene in the first biological sample with expression of the expression product of the at least one gene in a second biological sample which is normal,
wherein a difference in expression of the expression product of the at least one gene in the first biological sample relative to the second biological sample identifies the first biological sample as likely to be pathological or susceptible to a pathological condition. In a preferred embodiment, the present invention relates to a method for diagnosing a biological sample as likely to be neoplastic or vascularized tumors, comprising the steps of :
(a) detecting an expression product of at least one gene in a first biological sample suspected of being neoplastic wherein said expression product of at least one gene is characterized by a polynucleotide according to the invention, such as TAG, GAG/A and/or GAG/B polynucleotides, and preferably selected from the group consisting of SEQ ID NO:s 1, 3, 5, 7, 9, 11, 13, 15, 17, 19, 21, 23, 25, 27, 29, 31 and 33, including parts thereof; and
(b) comparing expression of the at least one gene in the first biological sample with expression of the at least one gene in a second biological sample which is normal, wherein increased expression of the at least one gene in the first biological sample relative to the second biological sample identifies the first biological sample as likely to be neoplastic.

In another preferred embodiment, the present invention relates to a method for diagnosing impaired wound healing, comprising the steps of :
(a) detecting an expression product of at least one gene in a first biological sample suspected of having impaired wound healing, wherein said at least one gene is characterized by a polynucleotide according to the invention, such as TAG, GAG/A and/or GAG/B polynucleotides, and preferably selected from the group consisting of GAG/A or GAG/B, SEQ ID NO:s 1, 3, 5, 7, 9, 11, 13, 15, 17, 19, 21, 23, 25, 27, 29, 31 and 33, or a part thereof; and
(b) comparing expression of the at least one gene in the first biological sample with expression of the at least one gene in a second biological sample which is normal, wherein differential, e.g. decreased or increased, expression of the at least one gene in the first biological sample relative to the second biological sample identifies the first biological sample as likely to be impaired in wound healing.

Difference in expression levels of genes can be determined by any method known in the art, such as for instance quantitative PCR or hybridisation techniques. The difference in expression qualifying a first biological sample as likely to be pathogenic, e.g. neoplastic or impaired in wound healing is at least 2-fold, relative to the expression level in a second biological sample which is normal. Accordingly, the present invention relates to a method as described herein, wherein the difference in expression, the increased expression or the decreased expression of the at least one gene in the first biological sample relative to the second biological sample is at least 2-fold, and preferably 5-fold or even more, such as 10-fold. Preferably, the expression product for which the expression level is determined, is RNA, e.g. mRNA, preferably encoding for SEQ ID NO 2, 4, 6, 8, 10, 12, 14, 16, 18, 20, 22, 24, 26, 28, 30, 32 or 34, or GAG/A or GAG/B or a part thereof.

In a further preferred embodiment, the present invention relates to the use of a nucleic acid characterized by any of SEQ ID NO 11, or a part thereof, for diagnosing angiogenesis, and preferably tumor endothelial cells.

### F.2 Diagnosing polypeptides

In a preferred embodiment, the present invention relates to a method of diagnosing a pathological condition or a susceptibility to a pathological condition, said method comprising:
(a) contacting a biological sample with an antibody specific for a polypeptide according to the invention, such as TAG, GAG/A and/or GAG/B polypeptides, and preferably chosen from the group consisting of SEQ ID NO:s 2, 4, 6, 8, 10, 12, 14, 16, 18, 20, 22, 24, 26, 28, 30, 32 and 34, including parts thereof;
(b) detecting binding of said antibody to said polypeptide, or a part thereof, present in said biological sample;
(c) comparing the binding detected in step (b) with a standard,
wherein a difference in binding relative to the standard is diagnostic of a pathological condition or a susceptibility to a pathological condition. The method of diagnosing a pathological condition according to the invention may comprise FACS analysis, e.g. the detection step is performed by using FACS, or the use of protein or antibody arrays, ELISA, or immunoblotting.

The present invention also relates to a method for diagnosing a pathological condition or a susceptibility to a pathological condition, comprising the steps of :
(a) detecting an expression product of at least one gene in a first tissue sample suspected of pathological, wherein said expression product of at least one gene is selected from the genes according to the invention, such as TAG, GAG/A and/or GAG/B genes, and preferably selected from the group consisting of SEQ ID NO:s 2, 4, 6, 8, 10, 12, 14, 16, 18, 20, 22, 24, 26, 28, 30, 32 and 34, including parts thereof; and
(b) comparing expression of the expression product of at least one gene in the first tissue sample with expression of the expression product of the at least one gene in a second tissue sample which is normal,
wherein a difference in expression of the expression product of the at least one gene in the first tissue sample relative to the second tissue sample identifies the first tissue sample as likely to be pathological or susceptible to a pathology.

In a further embodiment, the present invention relates to a method for diagnosing vascularized tumors, comprising the steps of :
(a) detecting an expression product of at least one gene in a first biological sample suspected of being neoplastic, wherein said expression product of at least one gene is characterized by a polypeptide according to the invention, such as TAG, GAG/A and/or GAG/B polypeptides, and preferably chosen from the group consisting of SEQ ID NO:s 2, 4, 6, 8, 10, 12, 14, 16, 18, 20, 22, 24, 26, 28, 30, 32 and 34, including a part thereof; and
(b) comparing expression of the at least one gene in the first biological sample with expression of the at least one gene in a second biological sample which is normal, wherein increased expression of the at least one gene in the first biological sample relative to the second biological sample identifies the first biological sample as likely to be neoplastic.

In another embodiment, the present invention relates to a method for diagnosing impaired wound healing, comprising the steps of :
(a) detecting an expression product of at least one gene in a first biological sample suspected of having impaired wound healing, wherein said expression product of at least one gene is characterized by a polynucleotide according to the invention, such as TAG, GAG/A and/or GAG/B polynucleotides, and preferably selected from the group consisting of SEQ ID NO:s 2, 4, 6, 8, 10, 12, 14, 16, 18, 20, 22, 24, 26, 28, 30, 32 and 34, including parts thereof; and
(b) comparing expression of the at least one gene in the first biological sample with expression of the at least one gene in a second biological sample which is normal, wherein decreased expression of the at least one gene in the first biological sample relative to the second biological sample identifies the first biological sample as likely to be impaired in wound healing.

It will be appreciated that the first and second biological samples are preferably derived from human. Furthermore, the first and second biological samples may be derived from the same human, e.g. the first biological sample is derived from a tissue suspected of being neoplastic, while the second biological sample is derived from another, non-malignant tissue.

In the diagnostic methods of the invention, the step of detecting may be performed by any diagnostic technique, known by the person skilled in the art, and preferably using immunoassays, which may include the use of antibodies, such as Western blot, ELISA, RIA, immuno(histo)chemical assay, and/or hybridisation assays such as Southern /Northern / Virtual Northern blotting techniques and/or oligonucleotide arrays and microarrays, and/or specific amplification techniques, such as PCR, NASBA or TMA technologies, and any combination of the above.

In another preferred embodiment, the present invention relates to the use of an antibody specifically directed against a protein characterized by SEQ ID NO: 12, or a part thereof, for diagnosing proliferative disorders and/or angiogenesis.

### F.3 Detecting endothelial cells

The molecules identified in the present invention may support the detection of endothelial cells. Accordingly, the present invention also relates to a method for identifying endothelial cells, comprising:
(a) contacting a population of cells with at least one molecule comprising a variable region which binds specifically to a polypeptide according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, or to any other polypeptide identified in the present invention as endothelial cell specific, or a part thereof;
(b) detecting cells in the population which have bound to said molecules; and
(c) identifying cells which are bound to said one or more molecules as endothelial cells.

Also, the present invention relates to a method for identifying endothelial cells, comprising:
(a) contacting cDNA or mRNA of a population of cells with one or more nucleic acid hybridization probes which are complementary to a cDNA or mRNA for a gene characterized by a polynucleotide according to the invention, such as TAG, GAG/A and/or GAG/B polynucleotides, and preferably selected from the group consisting of SEQ ID NOs 1, 3, 5, 7, 9, 11, 13, 15, 17, 19, 21, 23, 25, 27, 29, 31 and 33, or GAG/A or GAG/B, including parts thereof,
(b) detecting cDNA or mRNA which have specifically hybridized to said nucleic acid hybridization probes; and
identifying cells whose nucleic acids specifically hybridized to said nucleic acid hybridization probes as endothelial cells.

### F.4 Selection of endothelial cells

The staining or selection of endothelial cells may be accomplished by staining with anti-CD31 and anti-CD34 antibodies; and isolated by positive selection e.g., by using goat anti-mouse IgG coated paramagnetic beads. Hence, in one embodiment, the present invention also provides for the selection of endothelial cells from human tissues for the purpose of gene expression by using the combination of anti-CD31 and anti-CD34 antibodies.

### G. Treatment and medicaments

The terms "treatment", "treating", and the like, as used herein include amelioration or elimination of a developed disease or condition once it has been established or alleviation of the characteristic symptoms of such disease or condition. As used herein these terms also encompass, depending on the condition of the patient, preventing the onset of a disease or condition or of symptoms associated with a disease or condition, including reducing the severity of a disease or condition or symptoms associated therewith prior to affliction with said disease or condition. Such prevention or reduction prior to affliction refers to administration of the compound or composition of the invention to a patient that is not at the time of administration afflicted with the disease or condition. "Preventing" also encompasses preventing the recurrence or relapse-prevention of a disease or condition or of symptoms associated therewith, for instance after a period of improvement.

As used herein, the term "medicament" also encompasses the terms "drug", "therapeutic", "potion" or other terms which are used in the field of medicine to indicate a preparation with a therapeutic or prophylactic effect.

To prepare the pharmaceutical compositions, comprising the compounds, described herein, such as nucleic acids, polypeptides, antisense oligonucleotides, siRNA, antibodies and the like, an effective amount of the active ingredients, in acid or base addition salt form or base form, may be combined in admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously, transdermally, by parenteral, intramuscular, intravascular injection or intrathecal administration. The pharmaceutical compounds for treatment are intended for parenteral, topical, oral or local administration and generally comprise a pharmaceutically acceptable carrier and an amount of the active ingredient sufficient to reverse or prevent the adverse effects of pathological conditions connected with impaired angiogenesis or proliferative diseases. The carrier may be any of those conventionally used and is limited only by chemico-physical considerations, such as solubility and lack of reactivity with the compound, and by the route of administration. Hence, the present invention relates to the use of a nucleic acid, polypeptide, antibody, siRNA, or antisense oligonucleotide according to the invention for the preparation of a medicament for treating a pathological condition, e.g. preventing, treating and/or alleviating proliferative disorders, or for stimulating angiogenesis. In addition, the present invention relates to a method for the production of a composition comprising the steps of admixing a nucleic acid, polypeptide, antibody, siRNA, or antisense oligonucleotide according to the invention with a pharmaceutically acceptable carrier. The present invention relates specifically to the use of an inhibitor of HMGB1 for the preparation of a medicament for preventing, treating and/or alleviating proliferative disorders. In particular, the present invention relates to the use as described above, wherein said inhibitor is an anti-HMGB1 antibody. In an alternative embodiment, the present invention relates to the use as described herein, wherein said inhibitor is siRNA duplex, said siRNA duplex complexes with a nucleic acid comprising a nucleotide sequence which is at least 90% identical to SEQ ID NO: 11 or a part thereof.

### G.1 Treating proliferative diseases

### Antibodies

The present invention demonstrated that the selected TAG markers are related to the process of angiogenesis. *In vitro* as well as *in vivo* bioassays proved that therapeutic agents directed against the TAG markers showed inhibitory effects. In particular, antibodies inhibited endothelial tube formation in an *in vitro* collagen-gel-based sprout-formation assay. Also, antibodies directed against the polypeptides of the invention specifically inhibited the developing chorioallantoic membrane (CAM) of the chick embryo. Furthermore, antibodies inhibited tumor growth in a mouse model.

Accordingly, the present invention relates to an antibody specifically recognizing a polypeptide of the invention for use as a medicament. The present invention also contemplates a method as described herein, wherein the therapeutic agent is an antibody directed specifically against any of the polypeptides according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, including parts thereof.

As used in the present invention, a "therapeutic agent" is a compound which is able to interfere with the expression, whether up or down, of a gene according to the invention. The term "therapeutic agent" also contemplates a compound which is able to interfere with the activity an expression product from a gene according to the invention. The therapeutic agent according to the invention may comprise an anticellular moiety capable of killing or suppressing the growth or cell division of targeted endothelial cells. The anti-cellular agent moiety may be chosen from the group consisting of a chemotherapeutic agent, a radioisotope, a cytotoxin, a steroid, an antimetabolite, an anthracycline, a vinca alkaloid, an antibiotic, an alkylating agent, or an epipodophyllotoxin, or a plant-, fungus- or bacteria-derived toxin. The therapeutic agent may be antibodies directed against the polypeptides according to the invention, or parts thereof, and said antibodies are coupled to anti-cellular agents. A therapeutic agent is intended to treat or alleviate a pathological condition, such as proliferative diseases or disorders, including cancer, arthritis, diabetes, psoriasis and endometriosis or ischemia, heart failure, infertility, ulcer formation and impaired wound healing.

The term "expression" according to the present invention comprises the activity of gene and its gene product, including transcription into mRNA and/or translation of the mRNA into protein. It will be appreciated that an "expression product" of a gene encompasses the mRNA but also the protein derived therefrom, as well as the activity, function and mode of action of said protein.

In a preferred embodiment, the present invention relates to a method for inhibiting a pathological condition, such as proliferative diseases or disorders in a subject comprising such a pathological condition, e.g. a proliferative disease or disorder, comprising: administering to the subject an effective amount of a composition comprising an antibody which specifically binds to an epitope of any of the polypeptides according to invention, such as TAG, GAG/A and/or GAG/B polypeptides, and preferably comprising or consisting of SEQ ID NO:s 2, 4, 6, 8, 10, 12, 14, 16, 18, 20, 22, 24, 26, 28, 30, 32 or 34, or a part thereof.

In a further preferred embodiment, the present invention relates to a method for inducing an immune response to a polypeptide according to the invention in a mammal, such as TAG, GAG/A and/or GAG/B polypeptides, comprising: administering to a subject who has or is at risk of developing a proliferative disease or disorder a protein according to the invention, or a nucleic acid encoding a protein according to the invention, wherein said protein is preferably selected from the group consisting of TAG, GAG/A and/or GAG/B polypeptides, and preferably comprising or consisting of SEQ ID NO:s 2, 4, 6, 8, 10, 12, 14, 16, 18, 20, 22, 24, 26, 28, 30, 32 and 34, including parts thereof; whereby a humoral or cellular immune response to the protein according to the invention is raised in the human subject. The therapeutic methods according to the invention may further comprise administering to the subject an immune adjuvant to augment the immune response.

Preferably, the present invention relates to therapeutic methods according to the invention, wherein the proliferative disorders are vascularized tumors possibly comprising enhanced angiogenesis and/or tumor endothelial cells. As such, the present invention relates to a method of treating a vascularized tumor, comprising the step of : contacting cells of the vascularized tumor with an antibody, wherein the antibody specifically binds to an extracellular epitope of a polypeptide according to invention, such as TAG, GAG/A and/or GAG/B polypeptides, and said polypeptide preferably comprising or consisting of any of SEQ ID NO:s 2, 4, 6, 8, 10, 12, 14, 16, 18, 20, 22, 24, 26, 28, 30, 32 or 34, or a part thereof; whereby immune destruction of cells of the vascularized tumor is triggered.

Also, the present invention relates to a method for targeting a therapeutic agent to tumor-associated vasculature in an animal having a vascularized tumor, comprising: administering a therapeutic agent to the animal, wherein the therapeutic agent compound comprises a targeting compound, and wherein the targeting compound recognizes and binds to a polypeptide or polynucleotide according to the invention, preferably a TAG according to the invention, such as a TAG being chosen from the group characterized by any of SEQ ID NOa 1 - 34. Preferably, the targeting compound is an antibody. Said antibody may recognize and bind to a TAG which is present on the surface of the tumor-associated endothelial cell, preferably at a higher concentration than on the surface of normal, non-tumor associated endothelial cells.

### Antisense - ribozyme - siRNA technology

In a further preferred embodiment, the present invention relates to a method for treating or alleviating proliferative diseases or disorders, comprising the use of a therapeutic agent which allows interfering with the expression of a nucleic acid or a polypeptide according to the invention, in a patient.

Antisense technology can be used to control gene expression, for example for inhibition of gene expression, i.e. transcription, as described in the art. As such, antisense nucleic acids can be used as antagonist compounds, and may be employed to regulate the effects of the polypeptides of the present invention on the modulation of angiogenesis, and in particular the onset of angiogenesis in malignancies, both *in vitro* and *in vivo.* Thus, in a further embodiment, the present invention provides an antisense nucleic acid directed against the nucleic acid according to the present invention, or a part thereof. Such antisense nucleic acids can be constructed by recombinant DNA technology methods standard in the art. In a preferred embodiment, the present invention provides a vector comprising a polynucleotide sequence as described herein encoding an antisense nucleic acid. In a more preferred embodiment, said vector is an expression vector wherein the antisense polynucleotide sequence is operably linked to one or more control sequences allowing the expression, i.e. transcription, of said sequence in prokaryotic and/or eukaryotic host cells.

Potential antagonists according to the invention also include catalytic RNA, or a ribozyme. Ribozymes cleave mRNA at site-specific recognition sequences and can be used to destroy mRNAs corresponding to the nucleic acids of the present invention. The construction and production of ribozymes is well known in the art. As in the antisense approach, ribozymes of the invention can be used as antagonist compounds, and can be delivered to cells to, for example, inhibit *in vitro* or *in vivo* angiogenesis or stimulate the induction of endothelial activation effects of the polypeptides of the present invention. Similarly, the nucleic acids of the present invention, the RNA molecules derived thereof, functional equivalent parts or fragments thereof can contain enzymatic activity or can squelch RNA binding polypeptides or can exert effects as antisense agents by binding the endogenous sense strand of mRNA, all of which can modulate angiogenesis, preferably the down regulation of TEC specific genes.

The invention further provides the nucleic acid sequences for controlling gene expression using RNA interference (*i.e.* siRNA, formerly known as double stranded RNA or dsRNA). It has been described in the art (WO 99/32169) that providing siRNA to a target cell can result in the down regulation of the translation/ expression of any desired RNA sequence that may be present in said cell. As such, the nucleic acids of the present invention can be used as antagonistic or agonistic compounds, and may be employed to regulate the effects of the polypeptides of the present invention on the modulation of angiogenesis and in particular the down regulation of TEC overexpressed genes (i.e., overexpressed in TEC relative to NEC and PLEC), both *in nitro* and *in vivo.* Moreover, the present invention relates to siRNA for use as a medicament, characterised that said siRNA agonises or antagonises angiogenesis by said polynucleotide sequences. Accordingly, the present invention relates to a cell, in which the polynucleotide sequences comprising the nucleic acids sequences as described herein have been introduced.

The present invention also contemplates a method as described herein, wherein the therapeutic agent is an antisense molecule, a ribozyme or an siRNA directed specifically against a polynucleotide according to the invention, such as TAG, GAG/A and/or GAG/B polynucleotides, and preferably any of SEQ ID NOa 1, 3, 5, 7, 9, 11, 13, 15, 17, 19, 21, 23, 25, 27, 29, 31 or 33, or a part thereof.

### G.2 Method for treating disorders connected with insufficient angiogenesis

The methods of the invention identified genes and gene products involved in disproportionate angiogenesis, but also clarified the role of various genes and gene products in normal physiological angiogenic processes, e.g. active angiogenesis in wound healing. This in turn elucidated the role of these genes and gene products in cases of insufficient angiogenesis. Therefore, the present invention also relates to the therapeutic agents to stimulate angiogenesis e.g. vascular proliferation. This may be beneficial to patients having wounds, impaired wound healing, ischemia, heart failure, infertility, or ulcer formation. Accordingly, the present invention encompasses nucleic acids or polypeptides according to the invention for use as a medicament. The present invention further encompasses a method for treating or alleviating a pathological condition resulting or connected with insufficient angiogenesis, such as impaired wound healing, ischemia, heart failure, infertility, ulcer formation, comprising the use of a therapeutic agent which allows to interfere with, preferably increase the expression of a nucleic acid or a polypeptide according to the invention, in a patient. Hence, the present invention relates to a method for stimulating vascular proliferation comprising: administering to a subject with a insufficient angiogenesis a protein according to the invention, such as TAG, GAG/A and/or GAG/B protein, or a polynucleotide or nucleic acid encoding a protein according to the invention, or a functional fragment thereof, wherein said protein according to the invention is characterized by a TAG, GAG/A and/or GAG/B polypeptide according to the invention, and preferably chosen from the group consisting of SEQ ID NO:s 2, 4, 6, 8, 10, 12, 14, 16, 18, 20, 22, 24, 26, 28, 30, 32 and 34, including parts thereof; whereby vascular proliferation is promoted.

The present invention also contemplates the use of the polynucleotides or polypeptides of the present inventions in persons having wounds or scar tissue in order to stimulate vascular proliferation. As such, the present invention relates to a method for stimulating vascular proliferation comprising: administering to a subject with a wound or scar tissue a protein according to the invention or nucleic acid encoding a protein according to the invention, wherein the protein according to the invention is preferably characterized by a TAG, GAG/A and/or GAG/B protein, and preferably chosen from the group consisting of SEQ ID NO:s 2, 4, 6, 8, 10, 12, 14, 16, 18, 20, 22, 24, 26, 28, 30, 32 and 34, including parts thereof; whereby wound healing and the break down of scar tissue is promoted.

In another embodiment, the present invention contemplates a method for regulating or modulating angiogenesis, and in particular inducing angiogenesis comprising:
(a) introducing a nucleic acid or an expression vector comprising a nucleic acid according to the present invention in a desired target cell, *in vitro* or *in vivo,*
(b) expressing said nucleic acid, and,
(c) regulating angiogenesis by the products expressed by said nucleic acid or the product of said expression vector.

In a preferred embodiment, the invention provides polypeptides, including protein fusions, or fragments thereof, for regulating angiogenesis, and in particular induction of endothelial cell activity, *in vitro* or *in vivo.* For example, the induction of endothelial cell activity may occur as a direct result of administering polypeptides to mammalian, preferably human, cells. Delivering compositions containing the polypeptide of the invention to target cells, may occur via association via heterologous polypeptides, heterologous nucleic acids, toxins, or pro-drugs via hydrophobic, hydrophilic, ionic and/or covalent interactions.

In another preferred embodiment the present invention provides a gene therapy method for treating, alleviating or preventing disorders and diseases involving pathological disturbance of angiogenesis. The gene therapy methods relate to the introduction of nucleic acid sequences into an animal to achieve expression of a polypeptide of the present invention. This method requires a nucleic acid, which codes for a polypeptide of the invention that is operatively linked to a promoter or any other genetic element necessary for the expression of the polypeptide in the target tissue. Such gene therapy and delivery techniques are known in the art, see, for example, EP-A-0 707 071.

In a further embodiment, the nucleic acid of the invention is delivered as a naked polynucleotide. The term naked nucleic acid refers to sequences that are free from any delivery vehicle that acts to assist, promote or facilitate entry into a cell, including viral sequences, viral particles, liposome formulations, lipofectin or precipitating agents and the like. The naked nucleic acids can be delivered by any method known in the art, including, but not limited to, direct needle injection at the delivery site, intravenous injection, topical administration, catheter infusion, and so-called "gene guns".

In another embodiment, the nucleic acids of the present invention may be delivered with delivery vehicles such as viral sequences, viral particles, liposome formulations, lipofectin or precipitating agents and the like. Viral vectors that can be used for gene therapy applications include, but are not limited to, a herpes virus vector, a baculovirus vector, a lentivirus vector, a retrovirus vector, an alphavirus vector, an adeno-associated virus vector or an adenoviral vector or any combination thereof.

Delivery of the nucleic acids into a subject may be either direct, in which case the subject is directly exposed to the nucleic acid or nucleic acid-carrying vectors, or indirect, in which case cells are first transformed with the nucleic acids *in vitro,* and then transplanted into the subject. These two approaches are known, respectively, as *in vivo* or *ex vivo* gene therapy and are well described. In addition, the polypeptides according to the invention can be used to produce a biopharmaceutical. The term "biopharmaceutical" relates to a recombinantly or synthetically produced polypeptide or protein. Means to recombinantly or synthetically produce polypeptides or proteins are well known in art, such as for example described in Sambrook *et al.* (1989). Said biopharmaceutical can be applied *in vivo,* such as for example intravenously or subcutaneously. Alternatively, said biopharmaceutical can be applied in vivo, such as for example by isolating cells of patient, after which the cells are treated with said biopharmaceutical. Subsequently, said treated cells are re-introduced into said patient. In a more preferred embodiment, the present invention provides a gene therapy method for stimulating vascular proliferation comprising the use of vectors as described herein. Cells into which nucleic acids or polypeptides of the present invention can be introduced, for example for therapeutic purposes, encompass any desired available cell type, including but not limited to endothelial cells, progenitors of endothelial cells, and various stem cells, in particular endothelial stem cells.

In a preferred embodiment, the invention provides a method for treating, alleviating or preventing disorders involving pathological disturbance of angiogenesis comprising the use of a molecule, which allows interfering with the expression of a polynucleotide and/or expression and/or functional activity of a polypeptide of the present invention in a patient in need of such a treatment. The invention also provides a method for regulating cell proliferation, said method comprising introduction of a nucleic acid or an expression vector according to the invention in a desired target cell.

Accordingly, the present invention relates to a cell, in which the polynucleotide sequences comprising the nucleic acids sequences as described herein have been introduced. It will be understood that said cell could be used as a medicament, in that said cell could be introduced in a patient suffering from pathologies related to the disturbance of angiogenesis. Repopulating with said cells will be beneficial to the patient.

Hence, the present invention relates to the use of a polynucleotide encoding a polypeptide comprising an amino acid sequence which is at least 65% identical to any of the polypeptides according to the invention, such as TAG, GAG/A and/or GAG/B polypeptides, and preferably any of SEQ ID NO:s 2, 4, 6, 8, 10, 12, 14, 16, 18, 20, 22, 24, 26, 28, 30, 32 or 34, for stimulating angiogenesis.

In addition, the present invention provides the use of a polypeptide comprising an amino acid sequence which is at least 65% identical to any of the polypeptides according to the invention, such as TAG, GAG/A and/or GAG/B polypeptides, and preferably any of SEQ ID NO:s 2, 4, 6, 8, 10, 12, 14, 16, 18, 20, 22, 24, 26, 28, 30, 32 or 34, for stimulating angiogenesis.

### H. Methods for identifying modulators of angiogenesis

The present invention characterized for the first time various molecules that are involved in angiogenesis, including normal angiogenic processes, e.g. GAG/A molecules, as well as pathological angiogenesis, e.g. TAG molecules. It was furthermore shown in the present invention that inhibition of these TAG molecules inhibited or impaired angiogenesis. Hence, the present invention enables the further identification of therapeutic agents able to modulate angiogenesis.

As used herein, a "modulator" and "agent that modulates", which are used interchangeably herein, refer to any compound that "modulates", i.e. modulate, change, or interfere with angiogenesis, including excessive angiogenesis as well as insufficient angiogenesis, such as an agent that increases or decreases the expression of a gene of the invention, increases or decreases the activity of a gene product of the invention, or any compound that increases or decreases the intracellular response initiated by an active form of the gene product of the invention, or any compound that increases or decreases angiogenesis. A modulator includes an agonist, antagonist, inhibitor or inverse agonist of angiogenesis. The modulator according to the invention may aid in preventing, treating or alleviating a pathological condition. A modulator can be a protein, a nucleic acid, an antibody or fragment thereof, such as an antigen-binding fragment, a protein, a polypeptide, a peptide, a lipid, a carbohydrate, a small inorganic or organic molecule, etc. Candidate modulators can be natural or synthetic compounds, including, for example, small molecules, compounds contained in extracts of animal, plant, bacterial or fungal cells, as well as conditioned medium from such cells. In this respect, it will be understood that either the nucleic acid itself or the product encoded by said nucleic acid, e.g. the mRNA or the polypeptide, can interfere with the mechanisms involved in angiogenesis. Methods to be used in screening for modulators are further detailed in the examples section. Preferably, the candidate modulator inhibits the expression or activity of any of said genes or proteins according to the invention, such as TAG, GAG/A and/or GAG/B genes or proteins, and preferably characterized by SEQ ID NOa 1 to 34. The modulators of angiogenesis may be used as drugs to treat pathological conditions linked with perturbed angiogenesis, e.g. impaired or excessive angiogenesis.

Accordingly, the present invention provides a method of identifying an agent that modulates a pathological condition, such as proliferative diseases or disorders, said method comprising :
(a) contacting a cell line expressing, and preferably over-expressing, a polynucleotide comprising any of the polynucleotides according to the invention, such as TAG, GAG/A and/or GAG/B polynucleotides, and preferably any of the polynucleotides characterized by SEQ ID NO:s 1, 3, 5, 7, 9, 11, 13, 15, 17, 19, 21, 23, 25, 27, 29, 31 or 33 in the presence and absence of a candidate modulator under conditions permitting the interaction of said candidate modulator with said cell; and,
(b) measuring the expression of said polynucleotide,
wherein a modulation in expression of said polynucleotide, in the presence of said candidate modulator, relative to the expression in the absence of said candidate modulator identifies said candidate modulator as an agent that modulates a pathological condition such as proliferative diseases or disorders.

In a further embodiment, the present invention provides a method of identifying an agent that modulates a pathological condition, such as proliferative diseases or disorders, said method comprising :
(a) contacting a cell line expressing, and preferably over-expressing any of the polypeptides according to the invention, such as TAG, GAG/A and/or GAG/B polypeptides, and preferably any of the polypeptides comprising the amino acid sequence characterized by any of SEQ ID NOa 2, 4, 6, 8, 10, 12, 14, 16, 18, 20, 22, 24, 26, 28, 30, 32 or 34 in the presence and absence of a candidate modulator under conditions permitting the interaction of said candidate modulator with said cell; and
(b) measuring the expression of said polypeptide,
wherein a modulation in expression of said polypeptide, in the presence of said candidate modulator, relative to the expression in the absence of said candidate modulator identifies said candidate modulator as an agent that modulates pathological condition, such as proliferative diseases or disorders.

In an even further embodiment, the present invention provides a method for screening agents for preventing, treating or alleviating pathological condition, such as proliferative diseases or disorders comprising the steps of:
(a) contacting the agent to be screened with a polynucleotide or a polypeptide according to the invention, and,
(b) determining whether said agent affects the expression activity of said polynucleotide or said polypeptide.

In a preferred embodiment, the present invention provides a method for screening agents that interact with the polypeptide according to the invention, or a variant or a derivative thereof, or an immunologically active and/or functional fragment thereof, comprising :
(a) combining the polypeptide according to the invention, or a variant or a derivative thereof, or an immunologically active and/or functional fragment thereof, with an agent, to form a complex, and,
(b) detecting the formation of a complex,
wherein the ability of the agent to interact with said polypeptide, or a variant or a derivative thereof, or an immunologically active and/or functional fragment thereof, is indicated by the presence of the agent in the complex.

In a further preferred embodiment, the present invention provides a method to identify candidate drugs for treating a pathological condition or a susceptibility to a pathological condition, such as tumors or wounds, comprising: contacting a test agent with cells which express one or more genes of the invention, characterized by the polynucleotides of the invention, such as TAG, GAG/A and/or GAG/B polynucleotides, and preferably selected from the group consisting of SEQ ID NO:s 1, 3, 5, 7, 9, 11, 13, 15, 17, 19, 21, 23, 25, 27, 29, 31 and 33, including parts thereof, determining the amount of expression of said one or more genes by hybridization of mRNA of said cells or cDNA or cRNA copied from said mRNA to a nucleic acid probe which is complementary to an mRNA of said one or more genes; identifying a test agent as a candidate drug for treating a pathological condition or a susceptibility to a pathological condition if it modulates the expression of said one or more genes. Preferably, the present invention relates to a method as described supra, wherein said a pathological condition or a susceptibility to a pathological condition is a tumor, and wherein said test agent is identified as a candidate drug for treating said tumor if it decreases expression of said one or more genes.

In an another embodiment, the present invention relates to a method as described above, wherein said a pathological condition or a susceptibility to a pathological condition is impaired wound healing, and wherein said test agent is identified as a candidate drug for treating said impaired wound healing if it increases expression of said one or more genes.

In an even further preferred embodiment, the present invention provides a method to identify candidate drugs for treating a pathological condition or a susceptibility to a pathological condition, such as tumors or wounds, comprising: contacting a test agent with cells which express one or more polypeptides according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, including parts thereof; determining the amount of said one or more of said polypeptides in said cells; identifying a test agent as a candidate drug for treating a pathological condition or a susceptibility to a pathological condition if it modulates the amount of one or more of said polypeptides in said cells. Preferably, the present invention provides a method described above, wherein said pathological condition or a susceptibility to a pathological condition is a tumor, and wherein said test agent is identified as a candidate drug for treating said tumor if it decreases the amount of one or more of said proteins in said cells. The present invention also contemplates a method as described supra, wherein said pathological condition or a susceptibility to a pathological condition is impaired wound healing, and wherein said test agent is identified as a candidate drug for treating said impaired wound healing if it increases the amount of one or more of said proteins in said cells.

Also, the present invention provides a method for identifying candidate drugs for treating a pathological condition or a susceptibility to a pathological condition, such as tumors or wounds, comprising: contacting a test agent with cells which express one or more polypeptides according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, including parts thereof; determining activity of said one or more polypeptides in said cells; identifying a test agent as a candidate drug for treating a pathological condition or a susceptibility to a pathological condition if it modulates the activity of one or more of said polypeptides in said cells. As such, the present invention particularly relates to a method as described herein, wherein said pathological condition or a susceptibility to a pathological condition is a tumor, and wherein said test agent is identified as a candidate drug for treating said tumor if it decreases the activity of one or more of said proteins in said cells.

According to a preferred embodiment, the present invention relates to a method as described herein, wherein said pathological condition or a susceptibility to a pathological condition is impaired wound healing, and wherein said test agent is identified as a candidate drug for treating said impaired wound healing if it increases the activity of one or more of said proteins in said cells.

In another embodiment, the present invention provides a method to identify candidate drugs for treating patients having pathological conditions or a susceptibility to a pathological condition, such as bearing tumors or for treating wounds, comprising: contacting a test agent with recombinant host cells which are transfected with an expression construct which encodes one or more polypeptides according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, including parts thereof; determining the amount of proliferation of said cells; identifying a test agent as a candidate drug for treating patients having a pathological condition or a susceptibility to a pathological condition if it modulates proliferation of said cells. Accordingly, the present invention relates to a method of above, wherein said pathological condition or a susceptibility to a pathological condition is a tumor, and wherein said test agent is identified as a candidate drug for treating said tumor if it inhibits proliferation of said cells. Accordingly, the present invention relates to a method as described above, wherein said pathological condition or a susceptibility to a pathological condition is impaired wound healing, and wherein said test agent is identified as a candidate drug for treating said impaired wound healing if it stimulates proliferation of said cells.

It will be appreciated by the person skilled in the art, that the present invention also relates to any of the methods described herein, wherein said pathological condition or a susceptibility to a pathological condition is chosen from the group consisting of proliferative disorders, including tumors, diabetic retinopathy, rheumatoid arthritis, psoriasis, restenosis, endometriosis, impaired wound healing, and atherosclerosis. In addition, the present invention also relates to any of the methods described herein, wherein said pathological condition relates to enhancing wound healing.

The present invention also provides a method to identify a ligand involved in endothelial cell regulation, comprising: contacting an isolated and purified human polypeptide according to the invention, such as TAG, GAG/A and/or GAG/B polypeptides, and preferably selected from the group consisting of SEQ ID NO:s 2, 4, 6, 8, 10, 12, 14, 16, 18, 20, 22, 24, 26, 28, 30, 32 and 34, including parts thereof, and preferably a transmembrane polypeptide with a test compound and a molecule comprising an antibody variable region which specifically binds to said polypeptide, preferably to an extracellular domain of said transmembrane polypeptide, or a part thereof; determining the amount of binding of the molecule comprising an antibody variable region to the polypeptide, preferably a human transmembrane polypeptide; whereby a test compound which diminishes the binding of the molecule comprising an antibody variable region to said polypeptide, such as a human transmembrane polypeptide is identified as a ligand involved in endothelial cell regulation. Preferably, the method further comprises contacting the test compound with endothelial cells and determining if the test compound inhibits growth of said cells.

In the methods according to the invention the used cells may be any mammalian cell, including cultured cells, cell lines, or primary cultures such as HUVEC. Preferably, the cells are endothelial cells, including resting and activated cells. The cells may be recombinant host cells which are transfected with an expression construct encoding one or more of the polypeptides according to the invention, or the cells may be in a mammal.

It will be appreciated that the present invention relates also to the agent identified by the method as described herein, as well as a method for the production of a composition comprising the steps of admixing an agent identifiable by a method according to the invention with a pharmaceutically acceptable carrier.

### I. Kits and compositions

The present invention provides kits for the diagnosis of a pathological condition related to aberrant angiogenesis in a patient, such as impaired or excessive angiogenesis.

Accordingly, in an embodiment, the present invention provides a kit for the diagnosis of a pathological condition in a patient comprising a nucleic acid or an antibody according to the invention, and possibly a manual for use. Preferably, the pathological condition to be diagnosed is a proliferative disease or disorder or impaired wound healing. As such, the present invention also pertains to the use of a nucleic acid, polypeptide or antibody, according to the invention for the preparation of a diagnostic kit, which may include a manual, for detecting a pathological condition, such as a proliferative disease or disorder and/or impaired wound healing.

It is another object of the present invention to provide a composition comprising an therapeutic agent that binds to a marker which is expressed, accessible or localized on intratumoral blood vessels of a vascularized tumor, possibly comprising an anti-cellular moiety, wherein said marker is chosen from the group consisting of a polypeptide according to the invention, such as TAG, GAG/A and/or GAG/B polypeptides, and preferably chosen from any of SEQ ID NO:s 2, 4, 6, 8, 10, 12, 14, 16, 18, 20, 22, 24, 26, 28, 30, 32 or 34, or a part thereof.

It will be understood that the following figures and examples are meant to illustrate the embodiments of the present invention and are in no way to be construed as limiting the present invention. To the contrary, the teachings of the specific examples are intended to be generalized for substantiating the embodiments. As such, the present invention may be practiced other than as particularly described and still be within the scope of the accompanying claims.

### SHORT DESCRIPTION OF THE FIGURES and TABLES

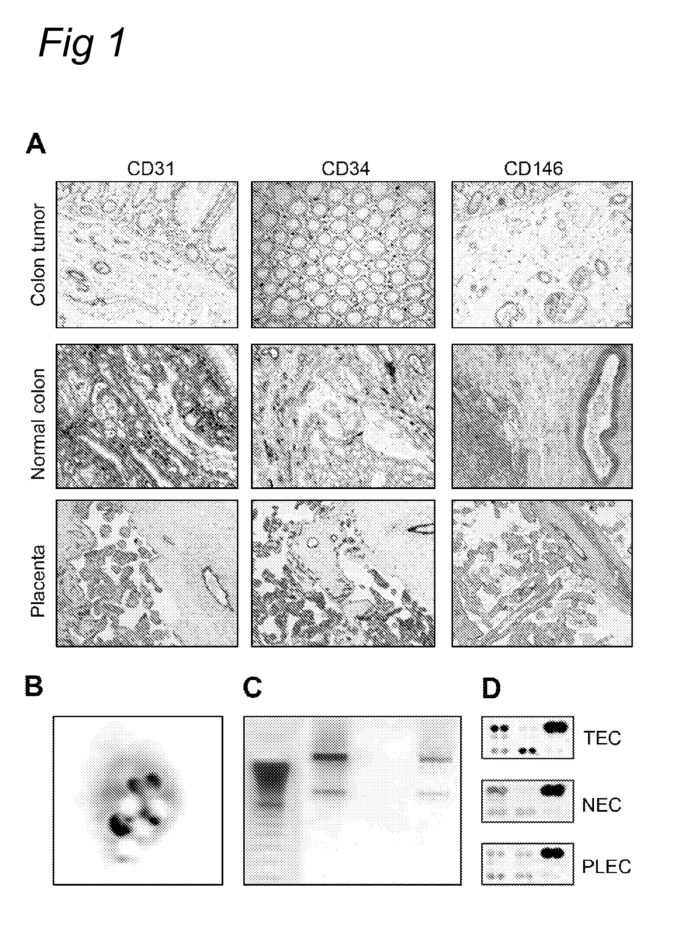

**Figure 1****: Endothelial cell selection for gene expression profiling by SSH**
   **A)** Immunohistochemical staining of normal colon, colon tumor and placenta tissues for CD31, CD34 and CD146 to determine which antigens are most specific for endothelial cell selection. Anti-CD31 and anti-CD34 antibodies specifically stain endothelial cells in all three tissues.
   **B)** Endothelial cells selected from fresh tissues using antibodies and magnetic beads (black spots) were stained for von Willebrand factor (vWF; green) to determine purity of the selected cell population. Nuclei were counterstained with DAPI (blue).
   **C)** RNA isolated from the selected endothelial cells shows very good integrity.
   **D)** Section of the SSH filters hybridized with TEC, NEC and PLEC, showing several spots with overexpression in TEC.

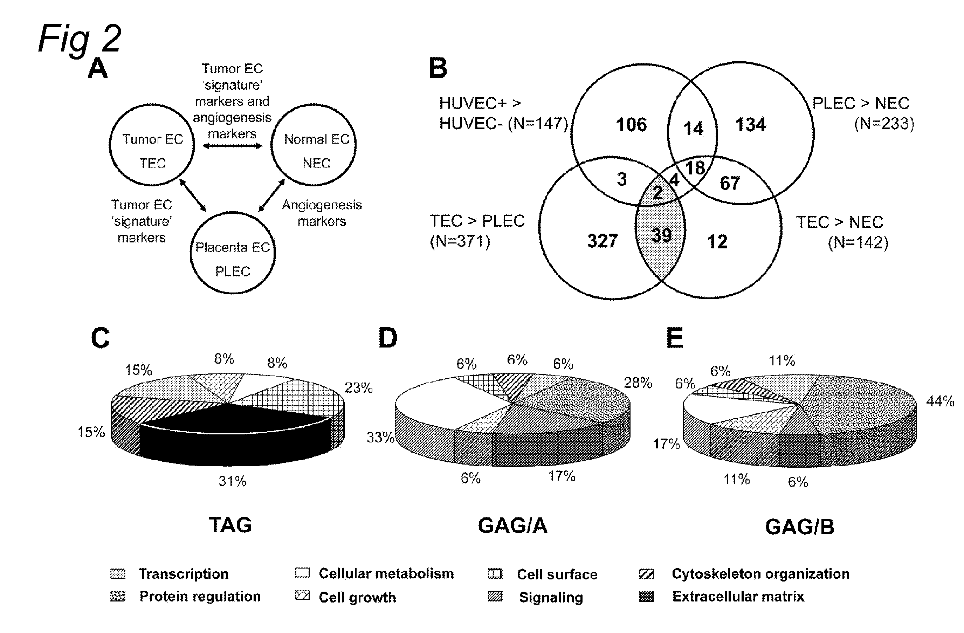

**Figure 2****: Endothelial gene expression and tissue environment.**
   **A)** Interrelationship between endothelial cells of different sources and their gene expression signatures. EC from a malignant and pro-angiogenic environment (TEC) are compared with EC from organ matched and patient matched non-malignant sources (NEC), and with non-malignant pro-angiogenic microenvironment derived EC (PLEC) to identify the subset of genes that show expression induced by the tumor microenvironment specifically (tumor EC 'signature' markers').
   **B)** Venn diagram representation of upregulated genes in different types of endothelial cells. Four pair-wise comparisons were performed by cDNA array screening of SSH repertoires: tumor conditioned (HUVEC+) vs quiescent HUVEC (HUVEC-), colorectal tumor endothelial cells vs normal colon endothelial cells (TEC vs NEC), colorectal tumor endothelial cells vs placenta endothelial cells (TEC vs PLEC) and placenta endothelial cells vs normal colon endothelial cells (PLEC vs NEC). Included are spots that showed at least a 2-fold difference in expression.
   **C)** TAG markers classified as being overexpressed in TEC vs NEC and in TEC vs PLEC (tumor EC 'signature' markers) are strongly biased towards genes associated with extracellular matrix remodeling.
   **D)** GAG/A markers, classified as overexpressed in both TEC and PLEC vs NEC (angiogenesis markers) show a diverse functional profile.
   **E)** GAG/B markers, classified as overexpressed in TEC and in activated HUVEC, are biased to protein turnover and transcriptional activity.

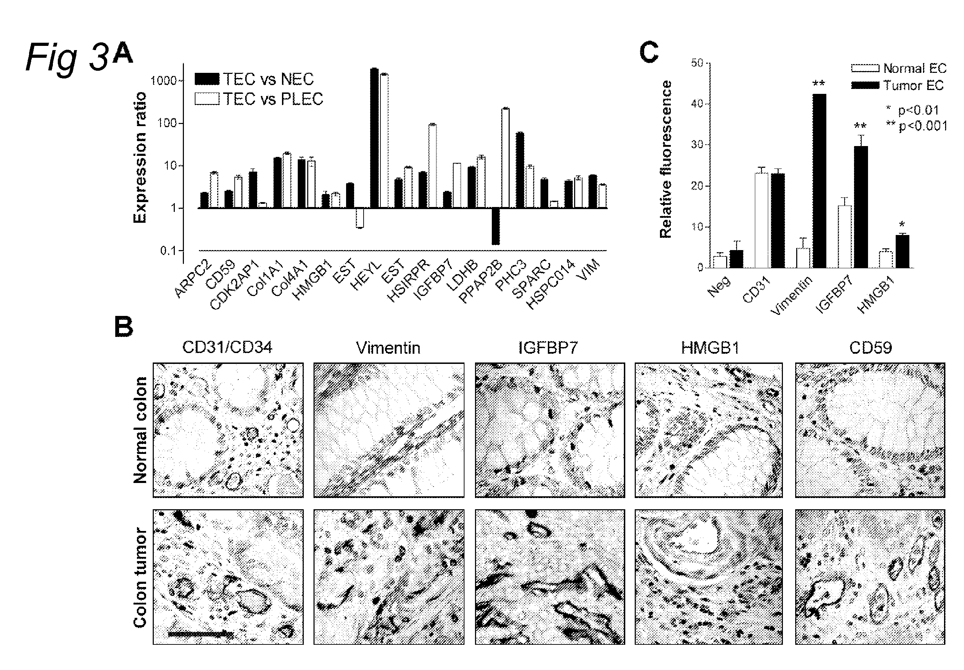

**Figure 3****: Expression validation of TAGS.**
   **A)** Transcriptional validation of TAG markers. Shown are expression ratios in TEC vs NEC (black bars) and TEC vs PLEC (grey bars), normalized for cyclophilin A, by quantitative real-time PCR.
   **B)** Immunohistochemical staining of different TAGs in colorectal tumor and normal colon tissue sections.
   **C)** Relative protein expression levels of HMGB1, IGFBP7 and vimentin on tumor endothelial cells compared to normal endothelial cells, assessed by flow-cytometry.

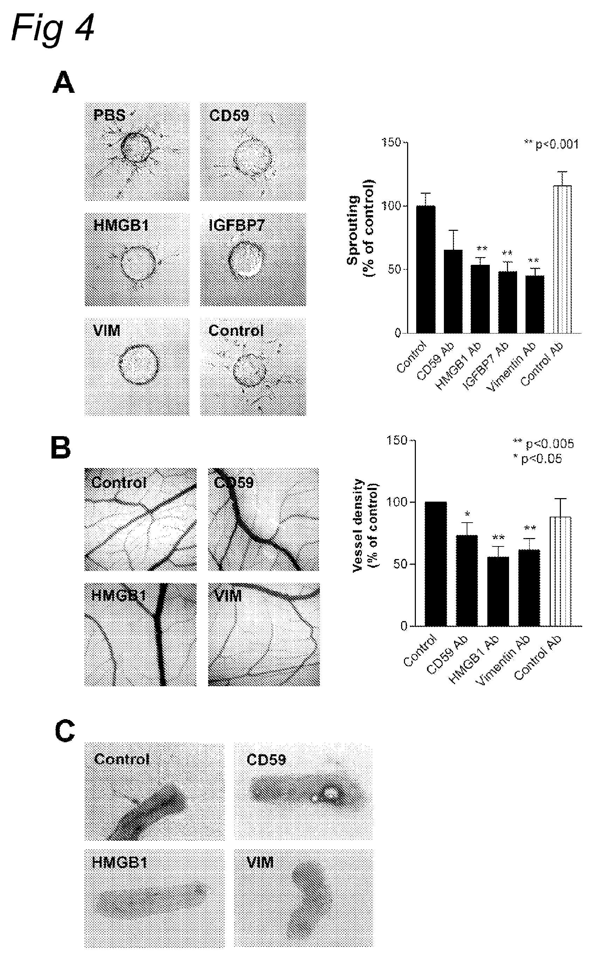

**Figure 4****: Inhibition of *in vitro* and *in vivo* angiogenesis by antibody-mediated targeting of TAG proteins.**
   **A)** Sprout formation of bovine capillary endothelial cells (BCEs) in collagen gel is inhibited by the addition of antibodies directed against different cell surface and secreted TAGs.
   **B)** Angiogenesis in the chick chorioallantoic membrane is inhibited by treatment with antibodies directed at the indicated TAGs.
   **C)** Capillary sprouting of tumor vessels embedded in collagen gel is inhibited by antibodies directed at the indicated TAGs.

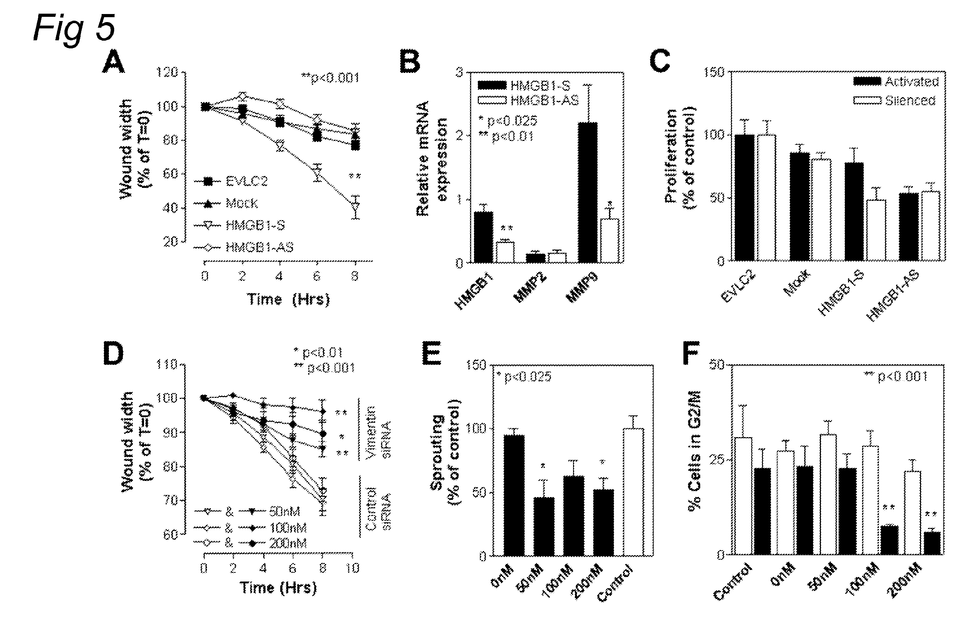

**Figure 5****: Modulation of TAG expression affects endothelial cell function**
   **A-C)** EVLC2 cells were transfected with expression constructs containing HMGB 1 ORF in the sense (HMGB1-S) or in the antisense (HMGB1-AS) orientation, to study the influence of over-expression and down-regulation of HMGB1 on endothelial cell biology. **A)** HMGB1-S cells have an increased ability to migrate into a wounded area as compared to controls and HMGB1-AS cells. **B)** RTQ PCR analysis indicates MMP9 expression is increased in HMGB1-S cells as compared to HMGB1-AS cells. **C)** HMGB1-AS cells have an impaired ability to respond to growth factor activation, **D-F)** Effects of siRNA mediated down-regulation of vimentin on endothelial cell biology. Down-regulation of vimentin by siRNA duplexes significantly inhibits endothelial cell migration (D) and sprouting (E), where only high concentrations of siRNA duplex inhibit cell proliferation (F).

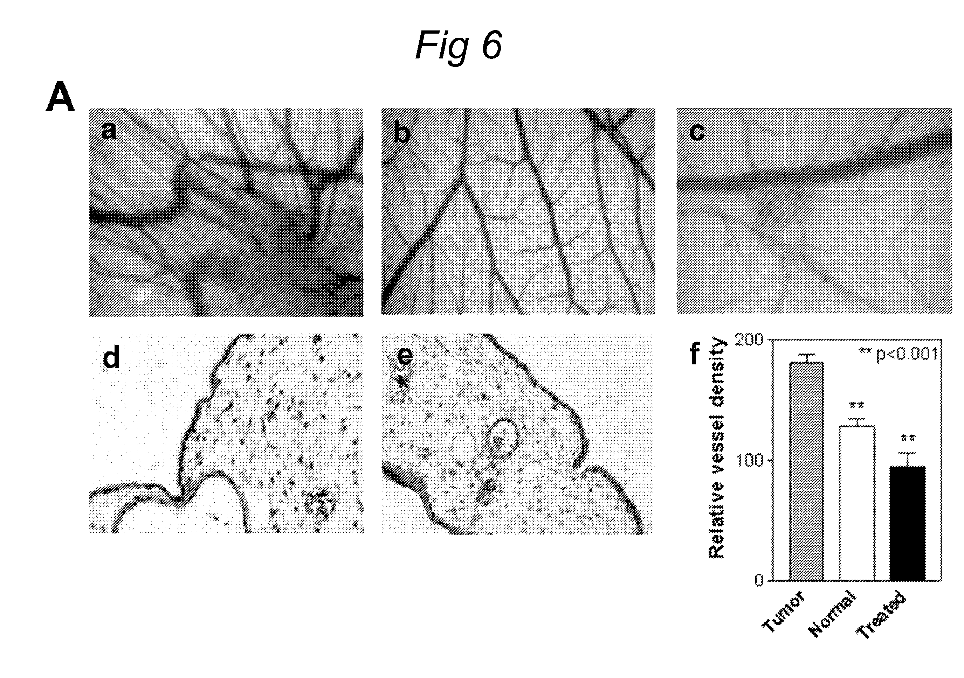

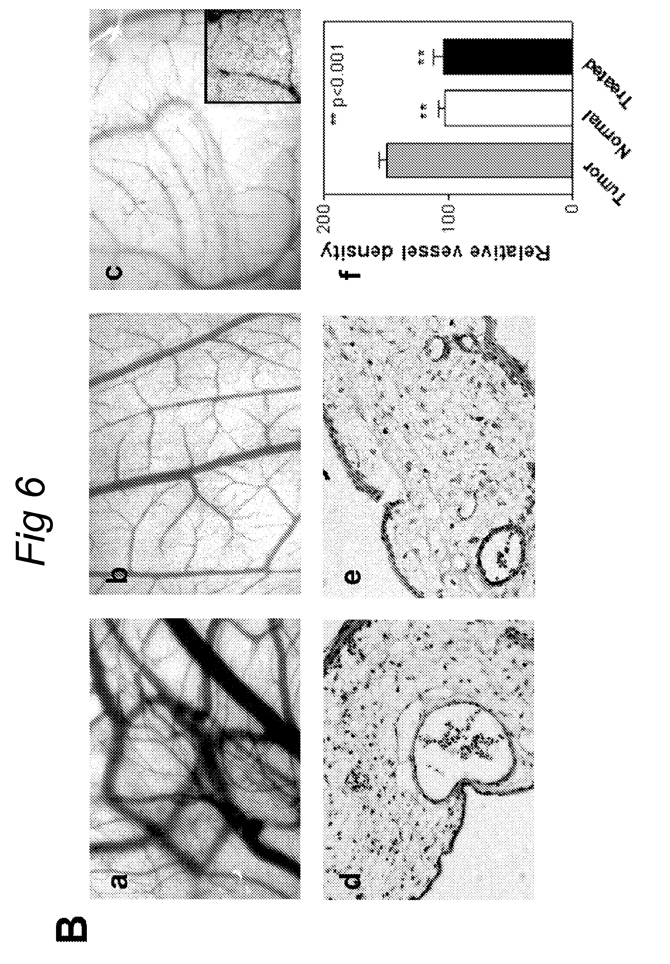

**Figure 6****: Inhibition of tumor angiogenesis on CAM by targeting TAGs**
   LS174T tumor cell spheroids were transplanted onto the CAM and treated with antibodies directed against HMGB1 (A) and vimentin (B). Transplantation of tumor cell spheroids induces increased vascular density and aberrant vascular morphology in the CAMs (a) as compared to normal CAMs (b). Tumor-induced vasculature was reduced by treatment with antibodies (c, f). Chicken endothelial cell reactivity of the antibodies was confirmed by immunohistochemistry using the treatment antibodies (e) and non-relevant control antibodies (d).

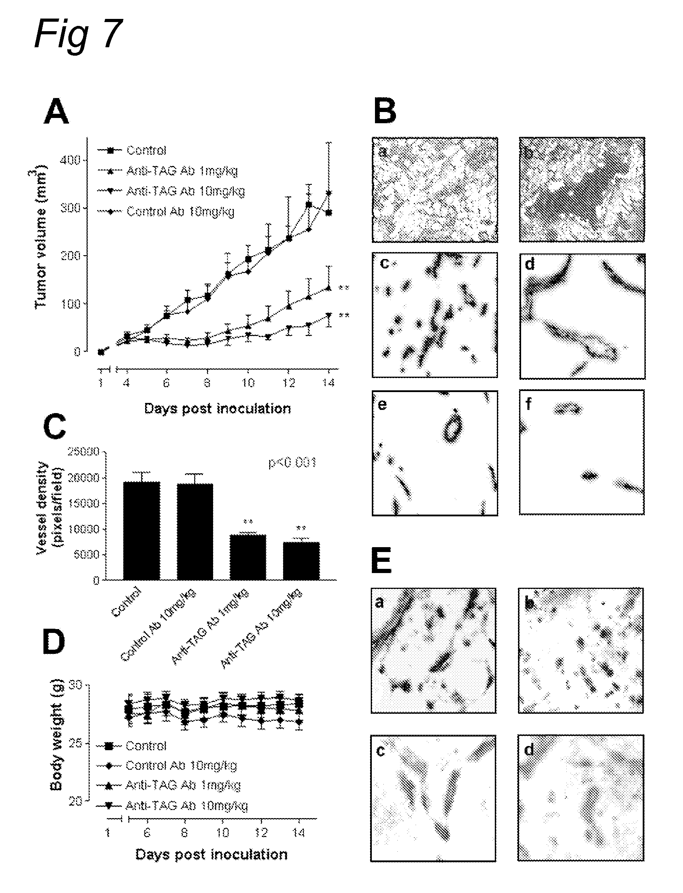

**Figure 7****: Inhibition of tumor growth and tumor angiogenesis antibody-mediated targeting of TAGs**
   **A)** Tumor growth curves of LS174T xenografts in nude mice, treated with vehicle, isotype control antibody (10 mg/kg) or anti-vimentin antibody (10 mg/kg and 1 mg/kg). Antibodies were administered every 3 days i.p. for a period of 12 days. A dose-dependent inhibition of tumor growth is evident in mice treated with anti-vimentin antibody 1 mg/kg, **p<0.0001; 10 mg/kg, **p<0.0001; Two-way ANOVA), whereas treatment with the isotype control antibody did not show inhibition of tumor growth (IIB5 10 mg/kg, p=0.661).
   **B)** Immunohistochemical staining of LS174T tumor xenografts in mice with CD31 (a) and anti-vimentin antibody (b) show that vimentin expression is restricted to the endothelium. Microvessel density of treated LS174T tumors was assessed by the number of pixels representative of immunoreactivity for CD31 in control mice (c), isotype control antibody treated mice (10 mg/kg/treatment) (d), anti vimentin antibody (1 mg/kg/treatment) (e) and anti vimentin antibody (1 mg/kg/treatment) treated mice (f).
   **C)** Quantification of microvessel density (** p<0.001, Student's T-test).
   **D)** Body weight of mice during treatment as an indicator of possible toxicity.
   **E)** Detection of treatment antibodies targeted to the tumor endothelium. Mouse antibodies were detected (green fluorescence) in mice treated with saline (a), isotype control antibody (b), anti-vimentin antibody (1mg/kg/treatment) (c), and anti-vimentin antibody (1 0mg/kg/treatment). Endothelial cells are stained with PE-labeled anti-CD31 antibody (red fluorescence). Localization of injected antibody to the tumor vasculature is indicated by yellow fluorescence.

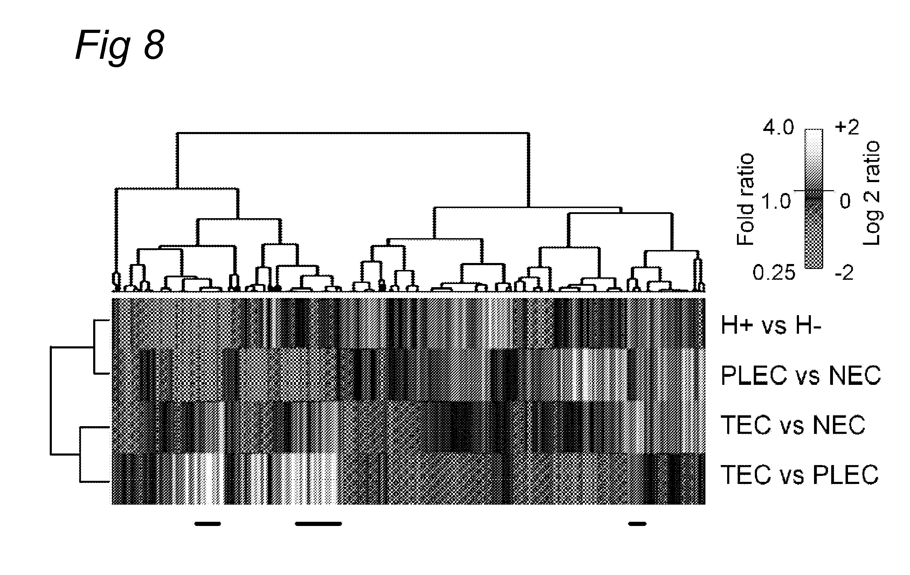

**Figure 8****: Diversity of expression patterns in TEC, NEC and PLEC.**
   Hierarchical clustering analysis of expression ratios of the entire SSH repertoire. The dendrogram represents the results of hierarchical clustering analysis based on similarities in gene expression patterns of the different comparisons indicated to the right of the clustered image maps. Expression ratios are color-coded as indicated on the far right and shown for the indicated comparisons. Bars at the bottom indicate clustered regions containing the genes that confer a tumor 'signature' to EC (TAGs). The dendrogram at the left is an indicator of overall correlation between the comparisons shown in the rows. Note that differential gene expression during physiological angiogesis (PLEC vs NEC) is most closely related to differential gene expression in activated vs quiescent HUVEC (H+ vs H-).
**Figure 9: TAG sequences**
   **A)** Nucleotide and amino acid sequences of TAG genes.
   **B)** Nucleotide sequences of SSH identified TAG inserts
**Table 1:** Characteristics of EC gene expression identified by differential screening of SSH repertoires.
**Table 2:** Tumor angiogenesis genes (TAGs).
**Table 3:** General angiogenesis genes (GAG/A and GAG/B).
**Table 4:** Amplification primers for TAGs.

### Figure 9A

**SEQ ID NO: 1 (NM_152862.1)** 
1 ggccggctag agccgggggc tgggcgggga ccgggcttgt cggtgaagcg
gcagtggcgg
61 cggcggcggc ggctcggcag gcgggttcag gcttcggggg
ccagccgccg ccatgatcct
121 gctggaggtg aacaaccgca tcatcgagga gacgctcgcg
ctcaagttcg agaacgcggc
181 cgccggaaac aaaccggaag cagtagaagt aacatttgca
gatttcgatg ggtcctcta
241 tcatatttca aatcctaatg gagacaaaac aaaagtgatg
gtcagtattt ctttgaaatt
301 ctacaaggaa cttcaggcac atggtgctga tgagttatta
aagagggtgt acgggagttt
361 cttggtaaat ccagaatcag gatacaatgt ctctttgcta
tatgaccttg aaaatcttcc
421 ggcatccaag gattccattg tgcatcaagc tggcatgttg
aagcgaaatt gttttgcctc
481 tgtctttgaa aaatacttcc aattccaaga agagggcaag
gaaggagaga acagggcagt
541 tatccattat agggatgatg agaccatgta tgttgagtct
aaaaaggaca gagtcacagt
601 agtcttcagc acagtgttta aggatgacga cgatgtggtc
attggaaagg tgttcatgca
661 ggagttcaaa gaaggacgca gagccagcca cacagcccca
caggtcctct ttagccacag
721 ggaacctcct ctggagctga aagacacaga cgccgctgtg
ggtgacaaca ttggctacat
781 tacctttgtg ctgttccctc gtcacaccaa tgccagtgct
cgagacaaca ccatcaacct
841 gatccacacg ttccgggact acctgcacta ccacatcaag
tgctctaagg cctatattca
 
901    cacacgtatg cgggcgaaaa cgtctgactt cctcaaggtg
ctgaaccgcg cacgcccaga
961    tgccgagaaa aaagaaatga aaacaatcac ggggaagacg
ttttcatccc gctaatcttg
1021  ggaataagag gaggaagcgg ctggcaactg aaggctggaa
cacttgctac tggataatcg
1081  tagcttttaa tgttgcgcct cttcaggttc ttaagggatt
ctccgttttg gttccatttt
1141  gtacacgttt ggaaaataat ctgcagaaac gagctgtgct
tgcaaagact tcatagttcc
1201  caagaattaa aaaaaaaaaa aaaagaattc cacttgatca
acttaattcc ttttctttat
1261  cttccctccc tcacttccct tttctcccac cctcttttcc
aagctgtttc gctttgcaat
1321  atattactgg taatgagttg caggataatg cagtcataac
ttgttttctc ctaagtattt
1381  gagttcaaaa ctcctgtatc taaagaaata cggttggggt
cattaataaa gaaaatcttt
1441  ctatcttaaa aaaaaaaaaa aa
**SEQ ID NO: 2 (NM_152862.1) (cds 113-1015)** 
1 MILLEVNNRI IEETLALKFE NAAAGNKPEA VEVTFADFDG
VLYHISNPNG
51 DKTKVMVSIS LKFYKELQAH GADELLKRVY GSFLVNPESG
YNVSLLYDLE
101 NLPASKDSIV HQAGMLKRNC FASVFEKYFQ FQEEGKEGEN
RAVIHYRDDE
151 TMYVESKKDR VTVVFSTVFK DDDDVVIGKV FMQEFKEGRR
ASHTAPQVLF
201 SHREPPLELK DTDAAVGDNI GYITFVLFPR HTNASARDNT
INLIHTFRDY
251 LHYHIKCSKA YIHTRMRAKT SDFLKVLNRA RPDAEKKEMK
TITGKTFSSR
**SEQ ID NO: 3 (NM_000611.2)** 
1 gggccggggg gcggagcctt gcgggctgga gcgaaagaat
gcgggggctg agcgcagaag
61 cggctcgagg ctggaagagg atcttgggcg ccgccaggtt
ctgtggacaa tcacaatggg
121 aatccaagga gggtctgtcc tgttcgggct gctgctcgtc
ctggctgtct tctgccattc
181 aggtcatagc ctgcagtgct acaactgtcc taacccaact
gctgactgca aaacagccgt
241 caattgttca tctgattttg atgcgtgtct cattaccaaa
gctgggttac aagtgtataa
301 caagtgttgg aagtttgagc attgcaattt caacgacgtc
acaacccgct tgagggaaaa
361 tgagctaacg tactactgct gcaagaagga cctgtgtaac
tttaacgaac agcttgaaaa
421 tggtggacaa tccttatcag agaaaacagt tcttctgctg
gtgactccat ttctggcagc
481 agcctggagc cttcatccct aagtcaacac caggagagct
tctcccaaac tccccgttcc
541 tgcgtagtcc gctttctctt gctgccacat tctaaaggct
tgatattttc caaatggatc
601 ctgttgggaa agaataaaat tagcttgagc aacctggcta
agatagaggg gctctgggag
661 actttgaaga ccagtcctgt ttgcagggaa gccccacttg
aaggaagaag tctaagagtg
721 aagtaggtgt gacttgaact agattgcatg cttcctcctt
tgctcttggg aagaccagct
781 ttgcagtgac agcttgagtg ggttctctgc agccctcaga
ttatttttcc tctggctcct
841 tggatgtagt cagttagcat cattagtaca tctttggagg
gtggggcagg agtatatgag
901 catcctctct cacatggaac gctttcataa acttcaggga
tcccgtgttg ccatggaggc
 
961 atgccaaatg ttccatatgt gggtgtcagt cagggacaac
aagatcctta atgcagagct
**Figure 9A - continued** 
1021 agaggacttc tggcagggaa gtggggaagt gttccagata
gcagggcatg aaaacttaga
1081 gaggtacaag tggctgaaaa tcgagttttt cctctgtctt
taaattttat atgggctttg
1141 ttatcttcca ctggaaaagt gtaatagcat acatcaatgg
tgtgttaaag ctatttcctt
1201 gccttttttt tattggaatg gtaggatatc ttggctttgc
cacacacagt tacagagtga
1261 acactctact acatgtgact ggcagtatta agtgtgctta
ttttaaatgt tactggtaga
1321 aaggcagttc aggtatgtgt gtatatagta tgaatgcagt
ggggacaccc tttgtggtta
1381 cagtttgaga cttccaaagg tcatccttaa taacaacaga
tctgcagggg tatgttttac
1441 catctgcatc cagcctcctg ctaactccta gctgactcag
catagattgt ataaaatacc
1501 tttgtaacgg ctcttagcac actcacagat gtttgaggct
ttcagaagct cttctaaaaa
1561 atgatacaca cctttcacaa gggcaaactt tttccttttc
cctgtgtatt ctagtgaatg
1621 aatctcaaga ttcagtagac ctaatgacat ttgtatttta
tgatcttggc tgtatttaat
1681 ggcataggct gacttttgca gatggaggaa tttcttgatt
aatgttgaaa aaaaaccctt
1741 gattatactc tgttggacaa accgagtgca atgaatgatg
cttttctgaa aatgaaatat
1801 aacaagtggg tgaatgtggt tatggccgaa aaggatatgc
agtatgctta atggtagcaa
 
1861 ctgaaagaag acatcctgag cagtgccagc tttcttctgt
tgatgccgtt ccctgaacat
1921 aggaaaatag aaacttgctt atcaaaactt agcattacct
tggtgctctg tgttctctgt
1981 tagctcagtg tctttcctta catcaatagg tttttttttt
tttttttggc ctgaggaagt
2041 actgaccatg cccacagcca ccggctgagc aaagaagctc
atttcatgtg agttctaagg
2101 aatgagaaac aattttgatg aatttaagca gaaaatgaat
ttctgggaac tttttggggg
2161 gcggggggtt ggggaattca gccacactcc agaaagccag
gagtcgacag ttttggaagc
2221 ctctctcagg attgagattc taggatgaga ttggcttact
gctatcttgt gtcatgtacc
2281 cactttttgg ccagactaca ctgggaagaa ggtagtcctc
taaagcaaaa tctgagtgcc
2341 actaaatggg gagatggggc tgttaagctg tccaaatcaa
caagggtcat ataaatggcc
2401 ttaaactttg gggttgcttt ctgcaaaaag ttgctgtgac
tcatgccata gacaaggttg
2461 agtgcctgga cccaaaggca atactgtaat gtaaagacat
ttatagtact aggcaaacag
2521 caccccaggt actccaggcc ctcctggctg gagagggctg
tggcaataga aaattagtgc
2581 caactgcagt gagtcagcct aggttaaata gagagtgtaa
gagtgctgga caggaacctc
2641 caccctcatg tcacatttct tcaatgtgac ccttctggcc
cctctcctcc tgacagcgga
2701 acaatgactg ccccgatagg tgaggctgga ggaagaatca
gtcctgtcct tggcaagctc
2761 ttcactatga cagtaaaggc tctctgcctg ctgccaaggc
ctgtgacttt ctaacctggc
2821 ctcacgctgg gtaagcttaa ggtagaggtg caggattagc
aagcccacct ggctaccagg
 
2881 ccgacagcta catcctccaa ctgaccctga tcaacgaaga
gggattcatg tgtctgtctc
2941 agttggttcc aaatgaaacc agggagcagg ggagttagga
atcgaacacc agtcatgcct
3001 actggctctc tgctcgagag ccaataccct gtgccctcca
ctcatctgga tttacaggaa
3061 ctgtcatagt gttcagtatt gggtggtgat aagcccattg
gattgtcccc ttgggggat
3121 gagctagggg tgcaaggaac acctgatgag tagataagtg
gagctcatgg tatttcctga
3181 aagatgctaa tctatttgcc aaacttggtc ttgaatgtac
tgggggcttc aaggtatggg
3241 tatatttttc ttgtgtcctt gcagttagcc cccatgtctt
atgtgtgtcc tgaaaaaata
3301 agagcctgcc caagactttg ggcctcttga cagaattaac
cacttttata catctgagtt
3361 ctcttggtaa gttctttagc agtgttcaaa gtctactagc
tcgcattagt ttctgttgct
3421 gccaacagat ctgaactaat gctaacagat ccccctgagg
gattcttgat gggctgagca
3481 gctggctgga gctagtactg actgacattc attgtgatga
gggcagcttt ctggtacagg
3541 attctaagct ctatgtttta tatacatttt catctgtact
tgcacctcac tttacacaag
3601 aggaaactat gcaaagttag ctggatcgct caaggtcact
taggtaagtt ggcaagtcca
3661 tgcttcccac tcagctcctc aggtcagcaa gtctacttct
ctgcctattt tgtatactct
3721 ctttaatatg tgcctagctt tggaaagtct agaatgggtc
cctggtgcct ttttactttg
3781 aagaaatcag tttctgcctc tttttggaaa agaaaacaaa
gtgcaattgt tttttactgg
3841 aaagttaccc aatagcatga ggtgaacagg acgtagttag
gccttcctgt aaacagaaaa
 
3901 tcatatcaaa acactatctt cccatctgtt tctcaatgcc
tgctacttct tgtagatatt
3961 tcatttcagg agagcagcag ttaaacccgt ggattttgta
gttaggaacc tgggttcaaa
4021 ccctcttcca ctaattggct atgtctctgg acaagttttt
tttttttttt tttttaaac
4081 cctttctgaa ctttcacttt ctatgtctac ctcaaagaat
tgttgtgagg cttgagataa
4141 tgcatttgta aagggtctgc cagataggaa gatgctagtt
atggatttac aaggttgtta
4201 aggctgtaag agtctaaaac ctacagtgaa tcacaatgca
tttacccccca ctgacttgga
**Figure 9A - continued** 
4261 cataagtgaa aactagccag aagtctcttt ttcaaattac
ttacaggtta ttcaatataa
4321 aatttttgta atggataatc ttatttatct aaactaaagc
ttcctgttta tacacactcc
4381 tgttattctg ggataagata aatgaccaca gtaccttaat
ttctaggtgg gtgcctgtga
4441 tggttcattg taggtaagga cattttctct tttcagcag
ctgtgtaggt ccagagcctc
4501 tgggagagga ggggggtagc atgcacccag caggggactg
aactgggaaa ctcaaggttc
4561 tttttactgt ggggtagtga gctgcctttc tgtgatcggt
ttccctaggg atgttgctgt
4621 tcccctcctt gctattcgca gctacataca acgtggccaa
ccccagtagg ctgatcctat
4681 atatgatcag tgctggtgct gactctcaat agccccaccc
aagctggcta taggtttaca
4741 gatacattaa ttaggcaacc taaaatattg atgctggtgt
tggtgtgaca taatgctatg
 
4801 gccagaactg aaacttagag ttataattca tgtattaggg
ttctccagag ggacagaatt
4861 agtaggatat atgtatatat gaaagggagg ttattaggga
gaactggctc ccacagttag
4921 aaggcgaagt cgcacaatag gccgtctgca agctgggtta
gagagaagcc agtagtggct
4981 cagcctgagt tcaaaaacct caaaactggg gaagctgaca
gtgcagccag ccttcagtct
5041 gtggccaaag gcccaagagc ccctggcaac caacccactg
gtgcaagtcc tagattccaa
5101 aggctgaaga acctggagtc tgatgtccaa gagcaggaag
agtggaagaa agccagaaga
5161 ctcagcaaac aaggtagaca gtgtctacca ccatagtggc
catacccaaag aggctaccga
5221 ttccttcctg ctacctggat ccctgaagtt gccctggtct
ctgcaccttc taaacctagt
5281 tcttaagagc tttccattac atgagctgtc tcaaagccct
ccaataaatt ctcagtgtaa
5341 gcttctgttg cttgtggaca gaaaattctg acagacctac
cctataagtg ttactgtcag
5401 gataacatga gaacgcacaa cagtaagtgg tcactaagtg
ttagctacgg ttattttgcc
5461 caaggtagca tggctagttg atgccggttg atgggcttta
aacccagctc cctcatcttc
5521 caggcctctg tactccctat tccactaaac tacctctcag
gtttattttt ttaaattctt
5581 actctgcaag tacataggac cacatttacc tgggaaaaca
agaataaagg ctgctctgca
5641 ttttttagaa acttttttga aagggagatg ggaatgcctg
cacccccaag tccagaccaa
5701 cacaatggtt aattgagatg aataataaag gaaagactgt
tctgggcttc ccagaatagc
5761 ttggtcctta aattgtggca caaacaacct cctgtcagag
ccagcctcct gccaggaaga
 
5821 ggggtaggag actagaggcc gtgtgtgcag ccttgccctg
aaggctaggg tgacaatttg
5881 gaggctgtcc aaacaccctg gcctctagag ctggcctgtc
tatttgaaat gccggctctg
5941 atgctaatcg gcgaccctca ggcaagttac ttaaccttac
atgcctcagt tttctcatct
6001 ggaaaatgag aaccctaggt ttagggttgt tagaaaagtt
aaatgagtta agacaagtgc
6061 ctgggacaca gtagcctctt gtgtgtgttt atcattatgt
cctcagcagg tcgtagaagc
6121 agcttctcag gtgtgaggct ggcgcattat ctggagtggg
ttgggttttc taggatggac
6181 cccctgctgc attttcctca ttcatccacc agggcttaat
ggggaatcaa ggaatccatg
6241 tgtaactgta taataactgt agccacactc caatgaccac
ctactagttg tccctggcac
6301 tgcttataca tatgtccatc aaatcaatcc tatgaagtag
atactgtctt cattttatag
6361 atcagagaca attggggttc agagagctga tgtgattttc
ccagggtcac agagagtccc
6421 agattcaggc acaactcttg tattccaaga cacaaccact
acatgtccaa aggctgccca
6481 gagccaccgg gcacggcaaa ttgtgacata tccctaaaga
ggctgagcac ctggtcagga
6541 tctgatggct gacagtgtgt ccagatgcag agctggagtg
ggggagggga agggggctc
6601 cttgggacag agaaggcttt ctgtgctttc tctgaaggga
gcagtctgag gaccaaggga
6661 acccggcaaa cagcacctca ggtactccag gccctcctgg
ctggagaggg ctgtggcaat
6721 ggaaaattag tgccaactgc aatgagtcag cctcggttaa
atagagagtg aagaatgctg
6781 gacaggaacc tccaccctca tgtcacattt cttcagtgtg
acccttctgg cccctctcct
 
6841 cctgacagcg gaacaatgac tgccccgata ggtgaggctg
gaggaagaat cagtcctgtc
6901 cttggcaagc tcttcactat gacagtaaag gctctctgcc
tgctgccaag gcctgtgact
6961 ttctaacctg gcctcacgct gggtaagctt aaggtagagg
tgcaggatta gcaagcccac
7021 ctggctacca ggccgacagc tacatctttc aactgaccct
gatcaacgaa gagggacttg
7081 tgtctctcag ttggttccaa atgaaaccag ggagcagggg
cgttaggaag ctccaacagg
7141 atggtactta atggggcatt tgagtggaga ggtaggtgac
atagtgcttt ggagcccagg
7201 gagggaaagg ttctgctgaa gttgaattca agactgttct
ttcatcacaa acttgagttt
7261 cctggacatt tgtttgcaga aacaaccgta gggttttgcc
ttaacctcgt gggtttatta
7321 ttacctcata gggactttgc ctcctgacag cagtttatgg
gtgttcattg tggcacttga
7381 gttttcttgc atacttgtta gagaaaccaa gtttgtcatc
aacttcttat ttaacccct
**Figure 9A - continued** 
7441 ggctataact tcatggatta tgttataatt aagccatcca
gagtaaaatc tgtttagatt
7501 atcttggagt aagggggaaa aaatctgtaa tttttctcc
tcaactagat atatacataa
7561 aaaatgattg tattgcttca tttaaaaaat ataacgcaaa
atctcttttc cttctaaaaa
7621 aaaaaaaaa aaa
**SEQ ID NO: 4 (NM_000611.2) (cds 116-502)** 
1   MGIQGGSVLF GLLLVLAVFC HSGHSLQCYN CPNPTADCKT
AVNCSSDFDA CLITKAGLQV
61 YNKCWKFEHC NFNDVTTRLR ENELTYYCCK KDLCNFNEQL
ENGGTSLSEK TVLLLVTPFL
121 AAAWSLHP
**SEQ ID NO: 5 (NM_004642.2)** 
1 gcccccacca tcaaggggaa gaaagtgctc ttcggattcc
ggttcgccct ggcctcccgc
61 agccgccgcg ggaccggccc ccagcacacc cccggggcgc
cgggcgcggg gcagccgccc
121 ggacgcgcgc gggcctcagg cgccgccggg accccagccc
cccaaacttt ggcaagttgc
181 gggcgccgag cgcacccgga ggcgcggggc gcggccgcag
gcggagccgc cccctgacgc
241 cgggccgccc cctcccggcc ccggccgccc cgccggctcc
gcggaaagtt tgcggccgcc
301 cctgcgccgc acccggggcc tgggtgagac tgcggcggcg
gcagggcgcg gacggccata
361 tttgccggcg cggcccgagc cgccgacaac aaaaagtgcg
cgggcgctcg gcgggcgctc
421 ggacgggcgc ggggctgcag cgctaccgcc cggcctcgcc
gccgccgccg ccgccctcgc
481 ggcctggccc cgccgcgccc ggcgcgcccg ccgcccgggg
ggatgtctta caaaccgaac
541 ttggccgcgc acatgcccgc cgccgccctc aacgccgctg
ggagtgtcca ctcgccttcc
601 accagcatgg caacgtcttc acagtaccgc cagctgctca
gtgactacgg gccaccgtcc
661 ctaggctaca cccagggaac tgggaacagc caggtgcccc
aaagcaaata cgcggagctg
 
721 ctggccatca ttgaagagct ggggaaggag atcagaccca
cgtacgcagg gagcaagagt
781 gccatggaga ggctgaagcg cggcatcatt cacgctagag
gactggttcg ggagtgcttg
841 gcagaaacgg aacggaatgc cagatcctag ctgccttgtt
ggttttgaag gatttccatc
901 tttttacaag atgagaagtt acagttcatc tcccctgttc
agatgaaacc cttgttttca
961 aaatggttac agtttcgttt ttcctcccat ggttcacttg
gctctgaacc tacagtctca
1021 aagattgaga aaagattttg cagttaatta ggatttgcat
tttaagtagt taggaactgc
1081 ccaggttttt tttgtttttt aagcattgat ttaaaagatg
cacggaaagt tatcttacag
1141 caaactgtag tttgcctcca agacaccatt gtctcccttt
aatcttctct tttgtataca
1201 tttgttaccc atggtgttct ttgttccttt tcataagcta
ataccactgt agggattttg
1261 ttttgaacgc atattgacag cacgctttac ttagtagccg
gttcccattt gccatacaat
1321 gtaggttctg cttaatgtaa cttctttttt gcttaagcat
ttgcatgact attagtgctt
1381 caaagtcaat ttttaaaaat gcacaagtta taaatacaga
agaaagagca acccaccaaa
1441 cctaacaagg acccccgaac actttcatac taagactgta
agtagatctc agttctgcgt
1501 ttattgtaag ttgataaaaa catctggaag aaaatgacta
aaactgtttg catctttgta
1561 tgtatttatt acttgatgta ataaagctta ttttcattaa
caatttgtat taaaaaaaaa
1621 aaaaaaa
**SEQ ID NO: 6 (NM_004642.2) (cds 523-870)** 
1 MSYKPNLAAH MPAAALNAAG SVHSPSTSMA TSSQYRQLLS
DYGPPSLGYT QGTGNSQVPQ
61 SKYAELLAII EELGKEIRPT YAGSKSAMER LKRGIIHARG
LVRECLAETE RNARS
**SEQ ID NO: 7 (NM_000088.2)** 
1 agcagacggg agtttctcct cggggtcgga gcaggaggca
cgcggagtgt gaggccacgc
61 atgagcggac gctaaccccc tccccagcca caaagagtct
acatgtctag ggtctagaca
121 tgttcagctt tgtggacctc cggctcctgc tcctcttagc
ggccaccgcc ctcctgacgc
181 acggccaaga ggaaggccaa gtcgagggcc aagacgaaga
catcccacca atcacctgcg
241 tacagaacgg cctcaggtac catgaccgag acgtgtggaa
acccgagccc tgccggatct
**Figure 9A - continued** 
301 gcgtctgcga caacggcaag gtgttgtgcg atgacgtgat
ctgtgacgag accaagaact
361 gccccggcgc cgaagtcccc gagggcgagt gctgtcccgt
ctgccccgac ggctcagagt
421 cacccaccga ccaagaaacc accggcgtcg agggacccaa
gggagacact ggcccccgag
481 gcccaagggg acccgcaggc ccccctggcc gagatggcat
ccctggacag cctggacttc
541 ccggaccccc cggacccccc ggacctcccg gacccctgg
cctcggagga aactttgctc
601 cccagctgtc ttatggctat gatgagaaat caaccggagg
aatttccgtg cctggcccca
661 tgggtccctc tggtcctcgt ggtctccctg gccccctgg
tgcacctggt ccccaaggct
 
721 tccaaggtcc ccctggtgag cctggcgagc ctggagcttc
aggtcccatg ggtccccgag
781 gtcccccagg tccccctgga aagaatggag atgatgggga
agctggaaaa cctggtcgtc
841 ctggtgagcg tgggcctcct gggcctcagg gtgctcgagg
attgcccgga acagctggcc
901 tccctggaat gaagggacac agaggtttca gtggtttgga
tggtgccaag ggagatgctg
961 gtcctgctgg tcctaagggt gagcctggca gccctggtga
aaatggagct cctggtcaga
1021 tgggcccccg tggcctgcct ggtgagagag gtcgccctgg
agcccctggc cctgctggtg
1081 ctcgtggaaa tgatggtgct actggtgctg ccgggccccc
tggtcccacc ggccccgctg
1141 gtcctcctgg cttccctggt gctgttggtg ctaagggtga
agctggtccc caagggcccc
1201 gaggctctga aggtccccag ggtgtgcgtg gtgagcctgg
ccccctggc cctgctggtg
1261 ctgctggccc tgctggaaac cctggtgctg atggacagcc
tggtgctaaa ggtgccaatg
1321 gtgctcctgg tattgctggt gctcctggct tccctggtgc
ccgaggcccc tctggacccc
1381 agggccccgg cggccctcct ggtcccaagg gtaacagcgg
tgaacctggt gctcctggca
1441 gcaaaggaga cactggtgct aagggagagc ctggccctgt
tggtgttcaa ggacccctg
1501 gccctgctgg agaggaagga aagcgaggag ctcgaggtga
acccggaccc actggcctgc
1561 ccggaccccc tggcgagcgt ggtggacctg gtagccgtgg
tttccctggc gcagatggtg
1621 ttgctggtcc caagggtccc gctggtgaac gtggttctcc
tggccccgct ggccccaaag
1681 gatctcctgg tgaagctggt cgtcccggtg aagctggtct
gcctggtgcc aagggtctga
 
1741 ctggaagccc tggcagccct ggtcctgatg gcaaaactgg
cccccctggt cccgccggtc
1801 aagatggtcg ccccggaccc ccaggcccac ctggtgcccg
tggtcaggct ggtgtgatgg
1861 gattccctgg acctaaaggt gctgctggag agcccggcaa
ggctggagag cgaggtgttc
1921 ccggaccccc tggcgctgtc ggtcctgctg gcaaagatgg
agaggctgga gctcagggac
1981 cccctggccc tgctggtccc gctggcgaga gaggtgaaca
aggccctgct ggctcccccg
2041 gattccaggg tctccctggt cctgctggtc ctccaggtga
agcaggcaaa cctggtgaac
2101 agggtgttcc tggagacctt ggcgcccctg gcccctctgg
agcaagaggc gagagaggtt
2161 tccctggcga gcgtggtgtg caaggtcccc ctggtcctgc
tggaccccga ggggccaacg
2221 gtgctcccgg caacgatggt gctaagggtg atgctggtgc
ccctggagct cccggtagcc
2281 agggcgcccc tggccttcag ggaatgcctg gtgaacgtgg
tgcagctggt cttccagggc
2341 ctaagggtga cagaggtgat gctggtccca aaggtgctga
tggctctcct ggcaaagatg
2401 gcgtccgtgg tctgaccggc cccattggtc ctcctggccc
tgctggtgcc cctggtgaca
2461 agggtgaaag tggtcccagc ggccctgctg gtcccactgg
agctcgtggt gcccccggag
2521 accgtggtga gcctggtccc cccggccctg ctggctttgc
tggccccct ggtgctgacg
2581 gccaacctgg tgctaaaggc gaacctggtg atgctggtgc
caaaggcgat gctggtcccc
2641 ctgggcctgc cggacccgct ggaccccctg gccccattgg
taatgttggt gctcctggag
2701 ccaaaggtgc tcgcggcagc gctggtcccc ctggtgctac
tggtttccct ggtgctgctg
 
2761 gccgagtcgg tcctcctggc ccctctggaa atgctggacc
ccctggccct cctggtcctg
2821 ctggcaaaga aggcggcaaa ggtccccgtg gtgagactgg
ccctgctgga cgtcctggtg
2881 aagttggtcc ccctggtccc cctggccctg ctggcgagaa
aggatcccct ggtgctgatg
2941 gtcctgctgg tgctcctggt actcccgggc ctcaaggtat
tgctggacag cgtggtgtgg
3001 tcggcctgcc tggtcagaga ggagagagag gcttccctgg
tcttcctggc ccctctggtg
3061 aacctggcaa acaaggtccc tctggagcaa gtggtgaacg
tggtcccccc ggtcccatgg
3121 gcccccctgg attggctgga ccccctggtg aatctggacg
tgagggggct cctgctgccg
3181 aaggttcccc tggacgagac ggttctcctg gcgccaaggg
tgaccgtggt gagaccggcc
3241 ccgctggacc ccctggtgct cctggtgctc ctggtgcccc
tggccccgtt ggccctgctg
3301 gcaagagtgg tgatcgtggt gagactggtc ctgctggtcc
cgccggtccc gtcggccccg
3361 tcggcgcccg tggccccgcc ggaccccaag gcccccgtgg
tgacaagggt gagacaggcg
3421 aacaggcgga cagaggcata aagggtcacc gtggcttctc
tggcctccag ggtccccctg
**Figure 9A - continued** 
3481 gccctcctgg ctctcctggt gaacaaggtc cctctggagc
ctctggtcct gctggtcccc
3541 gaggtccccc tggctctgct ggtgctcctg gcaaagatgg
actcaacggt ctccctggcc
3601 ccattgggcc ccctggtcct cgcggtcgca ctggtgatgc
tggtcctgtt ggtccccccg
 
3661 gccctcctgg acctcctggt ccccctggtc ctcccagcgc
tggtttcgac ttcagcttcc
3721 tgccccagcc acctcaagag aaggctcacg atggtggccg
ctactaccgg gctgatgatg
3781 ccaatgtggt tcgtgaccgt gacctcgagg tggacaccac
cctcaagagc ctgagccagc
3841 agatcgagaa catccggagc ccagagggaa gccgcaagaa
ccccgcccgc acctgccgtg
3901 acctcaagat gtgccactct gactggaaga gtggagagta
ctggattgac cccaaccaag
3961 gctgcaacct ggatgccatc aaagtcttct gcaacatgga
gactggtgag acctgcgtgt
4021 accccactca gcccagtgtg gcccagaaga actggtacat
cagcaagaac cccaaggaca
4081 agaggcatgt ctggttcggc gagagcatga ccgatggatt
ccagttcgag tatggcggcc
4141 agggctccga ccctgccgat gtggccatcc agctgacctt
cctgcgcctg atgtccaccg
4201 aggcctccca gaacatcacc taccactgca agaacagcgt
ggcctacatg gaccagcaga
4261 ctggcaacct caagaaggcc ctgctcctca agggctccaa
cgagatcgag atccgcgccg
4321 agggcaacag ccgcttcacc tacagcgtca ctgtcgatgg
ctgcacgagt cacaccggag
4381 cctggggcaa gacagtgatt gaatacaaaa ccaccaagtc
ctcccgcctg cccatcatcg
4441 atgtggcccc cttggacgtt ggtgccccag accaggaatt
cggcttcgac gttggccctg
4501 tctgcttcct gtaaactccc tccatcccaa cctggctccc
tcccacccaa ccaactttcc
4561 ccccaacccg gaaacagaca agcaacccaa actgaacccc
cccaaaagcc aaaaaatggg
4621 agacaatttc acatggactt tggaaaatat ttttttcctt
tgcattcatc tctcaaactt
 
4681 agtttttatc tttgaccaac cgaacatgac caaaaaccaa
aagtgcattc aaccttacca
4741 aaaaaaaaaa aaaaaaaaaa agaataaata aataagtttt
taaaaaagga agcttggtcc
4801 acttgcttga agacccatgc gggggtaagt ccctttctgc
ccgttgggtt atgaaacccc
4861 aatgctgccc tttctgctcc tttctccaca ccccccttgg
cctcccctcc actccttccc
4921 aaatctgtct ccccagaaga cacaggaaac aatgtattgt
ctgcccagca atcaaaggca
4981 atgctcaaac acccaagtgg cccccaccct cagcccgctc
ctgcccgccc agcaccccca
5041 ggccctgggg acctggggtt ctcagactgc caaagaagcc
ttgccatctg gcgctcccat
5101 ggctcttgca acatctcccc ttcgtttttg aggggtcat
gccgggggag ccaccagccc
5161 ctcactgggt tcggaggaga gtcaggaagg gccacgacaa
agcagaaaca tcggatttgg
5221 ggaacgcgtg tcatcccttg tgccgcaggc tgggcgggag
agactgttct gttctgttcc
5281 ttgtgtaact gtgttgctga aagactacct cgttcttgtc
ttgatgtgtc accggggcaa
5341 ctgcctgggg gcggggatgg gggcagggtg gaagcggctc
cccattttta taccaaaggt
5401 gctacatcta tgtgatgggt ggggtgggga gggaatcact
ggtgctatag aaattgagat
5461 gcccccccag gccagcaaat gttccttttt gttcaaagtc
tatttttatt ccttgatatt
5521 ttttctttct tttttttttt ttttgtggat ggggacttgt
gaatttttct aaaggtgcta
5581 tttaacatgg gaggagagcg tgtgcgctcc agcccagccc
gctgctcact ttccaccctc
5641 tctccacctg cctctggctt ctcaggcctc tgctctccga
cctctctcct ctgaaaccct
 
5701 cctccacagc tgcagcccat cctcccggct ccctcctagt
ctgtcctgcg tcctctgtcc
5761 ccgggtttca gagacaactt cccaaagcac aaagcagttt
ttccctaggg gtgggaggaa
5821 gcaaaagact ctgtacctat tttgtatgtg tataataatt
tgagatgttt ttaattattt
5881 tgattgctgg aataaagcat gtggaaatga cccaaacata a
**SEQ ID NO: 8 (NM_000088.2) cds 120..4514** 
1 MFSFVDLRLL LLLAATALLT HGQEEGQVEG QDEDIPPITC
VQNGLRYHDR DVWKPEPCRI
61 CVCDNGKVLC DDVICDETKN CPGAEVPEGE CCPVCPDGSE
SPTDQETTGV EGPKGDTGPR
121 GPRGPAGPPG RDGIPGQPGL PGPPGPPGPP GPPGLGGNFA
PQLSYGYDEK STGGISVPGP
181 MGPSGPRGLP GPPGAPGPQG FQGPPGEPGE PGASGPMGPR
GPPGPPGKNG DDGEAGKPGR
241 PGERGPPGPQ GARGLPGTAG LPGMKGHRGF SGLDGAKGDA
GPAGPKGEPG SPGENGAPGQ
301 MGPRGLPGER GRPGAPGPAG ARGNDGATGA AGPPGPTGPA
GPPGFPGAVG AKGEAGPQGP
361 RGSEGPQGVR GEPGPPGPAG AAGPAGNPGA DGQPGAKGAN
GAPGIAGAPG FPGARGPSGP
421 QGPGGPPGPK GNSGEPGAPG SKGDTGAKGE PGPVGVQGPP
GPAGEEGKRG ARGEPGPTGL
481 PGPPGERGGP GSRGFPGADG VAGPKGPAGE RGSPGPAGPK
GSPGEAGRPG EAGLPGAKGL
541 TGSPGSPGPD GKTGPPGPAG QDGRPGPPGP PGARGQAGVM
GFPGPKGAAG EPGKAGERGV
**Figure 9A - continued** 
601 PGPPGAVGPA GKDGEAGAQG PPGPAGPAGE RGEQGPAGSP
GFQGLPGPAG PPGEAGKPGE
 
661 QGVPGDLGAP GPSGARGERG FPGERGVQGP PGPAGPRGAN
GAPGNDGAKG DAGAPGAPGS
721 QGAPGLQGMP GERGAAGLPG PKGDRGDAGP KGADGSPGKD
GVRGLTGPIG PPGPAGAPGD
781 KGESGPSGPA GPTGARGAPG DRGEPGPPGP AGFAGPPGAD
GQPGAKGEPG DAGAKGDAGP
841 PGPAGPAGPP GPIGNVGAPG AKGARGSAGP PGATGFPGAA
GRVGPPGPSG NAGPPGPPGP
901 AGKEGGKGPR GETGPAGRPG EVGPPGPPGP AGEKGSPGAD
GPAGAPGTPG PQGIAGQRGV
961 VGLPGQRGER GFPGLPGPSG EPGKQGPSGA SGERGPPGPM
GPPGLAGPPG ESGREGAPAA
1021 EGSPGRDGSP GAKGDRGETG PAGPPGAPGA PGAPGPVGPA
GKSGDRGETG PAGPAGPVGP
1081 VGARGPAGPQ GPRGDKGETG EQGDRGIKGH RGFSGLQGPP
GPPGSPGEQG PSGASGPAGP
1141 RGPPGSAGAP GKDGLNGLPG PIGPPGPRGR TGDAGPVGPP
GPPGPPGPPG PPSAGFDFSF
1201 LPQPPQEKAH DGGRYYRADD ANVVRDRDLE VDTTLKSLSQ
QIENIRSPEG SRKNPARTCR
1261 DLKMCHSDWK SGEYWIDPNQ GCNLDAIKVF CNMETGETCV
YPTQPSVAQK NWYISKNPKD
1321 KRHVWFGESM TDGFQFEYGG QGSDPADVAI QLTFLRLMST
EASQNITYHC KNSVAYMDQQ
1381 TGNLKKALLL KGSNEIEIRA EGNSRFTYSV TVDGCTSHTG
AWGKTVIEYK TTKSSRLPII
1441 DVAPLDVGAP DQEFGFDVGP VCFL"
**SEQ ID NO: 9 (NM_001845.2)** 
1 aggtctccgc ttggagccgc cgcacccggg acggtgcgta
tcgctggaag tccggccttc
61 cgagagctag ctgtccgccg cggcccccgc acgccgggca
gccgtccctc gcgcctcggg
 
121 cgcgccacca tggggccccg gctcagcgtc tggctgctgc
tgctgcccgc cgcccttctg
181 ctccacgagg agcacagccg ggccgctgcg aagggtggct
gtgctggctc tggctgtggc
241 aaatgtgact gccatggagt gaagggacaa aagggtgaaa
gaggcctccc ggggttacaa
301 ggtgtcattg ggtttcctgg aatgcaagga cctgaggggc
cacaggacc accaggacaa
361 aagggtgata ctggagaacc aggactacct ggaacaaaag
ggacaagagg acctccggga
421 gcatctggct accctggaaa cccaggactt cccggaattc
ctggccaaga cggcccgcca
481 ggccccccag gtattccagg atgcaatggc acaaaggggg
agagagggcc gctcgggcct
541 cctggcttgc ctggtttcgc aggaaatccc ggaccaccag
gcttaccagg gatgaagggt
601 gatccaggtg agatacttgg ccatgtgccc gggatgctgt
tgaaaggtga aagaggattt
661 cccggaatcc cagggactcc aggcccacca ggactgccag
ggcttcaagg tcctgttggg
721 cctccaggat ttaccggacc accaggtccc ccaggccctc
ccggccctcc aggtgaaaag
781 ggacaaatgg gcttaagttt tcaaggacca aaaggtgaca
agggtgacca aggggtcagt
841 gggcctccag gagtaccagg acaagctcaa gttcaagaaa
aaggagactt cgccaccaag
901 ggagaaaagg gccaaaaagg tgaacctgga tttcagggga
tgccaggggt cggagagaaa
961 ggtgaacccg gaaaaccagg acccagaggc aaacccggaa
aagatggtga caaaggggaa
1021 aaagggagtc ccggttttcc tggtgaaccc gggtacccag
gactcatagg ccgccagggc
1081 ccgcagggag aaaagggtga agcaggtcct cctggcccac
ctggaattgt tataggcaca
 
1141 ggacctttgg gagaaaaagg agagaggggc taccctggaa
ctccggggcc aagaggagag
1201 ccaggcccaa aaggtttccc aggactacca ggccaacccg
gacctccagg cctccctgta
1261 cctgggcagg ctggtgcccc tggcttccct ggtgaaagag
gagaaaaagg tgaccgagga
1321 tttcctggta catctctgcc aggaccaagt ggaagagatg
ggctcccggg tcctcctggt
1381 tcccccgggc cccctgggca gcctggctac acaaatggaa
ttgtggaatg tcagcccgga
1441 cctccaggtg accagggtcc tcctggaatt ccagggcagc
caggatttat aggcgaaatt
1501 ggagagaaag gtcaaaaagg agagagttgc ctcatctgtg
atatagacgg atatcggggg
1561 cctcccgggc cacagggacc cccgggagaa ataggtttcc
cagggcagcc aggggccaag
1621 ggcgacagag gtttgcctgg cagagatggt gttgcaggag
tgccaggccc tcaaggtaca
1681 ccagggctga taggccagcc aggagccaag gggagcctg
gtgagtttta tttcgacttg
1741 cggctcaaag gtgacaaagg agacccaggc tttccaggac
agcccggcat gccagggaga
1801 gcgggttctc ctggaagaga tggccatccg ggtcttcctg
gccccaaggg ctcgccgggt
1861 tctgtaggat tgaaaggaga gcgtggcccc cctggaggag
ttggattccc aggcagtcgt
1921 ggtgacaccg gcccccctgg gcctccagga tatggtcctg
ctggtcccat tggtgacaaa
1981 ggacaagcag gctttcctgg aggccctgga tccccaggcc
tgccaggtcc aaagggtgaa
**Figure 9A - continued** 
2041 ccaggaaaaa ttgttccttt accaggcccc cctggagcag
aaggactgcc ggggtcccca
2101 ggcttcccag gtccccaagg agaccgaggc tttcccggaa
ccccaggaag gccaggcctg
2161 ccaggagaga agggcgctgt gggccagcca ggcattggat
ttccagggcc ccccggcccc
2221 aaaggtgttg acggcttacc tggagacatg gggccaccgg
ggactccagg tcgcccggga
2281 tttaatggct tacctgggaa cccaggtgtg cagggccaga
agggagagcc tggagttggt
2341 ctaccgggac tcaaaggttt gccaggtctt cccggcattc
ctggcacacc cggggagaag
2401 gggagcattg gggtaccagg cgttcctgga gaacatggag
cgatcggacc ccctgggctt
2461 caggggatca gaggtgaacc gggacctcct ggattgccag
gctccgtggg gtctccagga
2521 gttccaggaa taggcccccc tggagctagg ggtccccctg
gaggacaggg accaccgggg
2581 ttgtcaggcc ctcctggaat aaaaggagag aagggtttcc
ccggattccc tggactggac
2641 atgccgggcc ctaaaggaga taaaggggct caaggactcc
ctggcataac gggacagtcg
2701 gggctccctg gccttcctgg acagcagggg gctcctggga
ttcctgggtt tccaggttcc
2761 aagggagaaa tgggcgtcat ggggaccccc gggcagccgg
gctcaccagg accagtgggt
2821 gctcctggat taccgggtga aaaaggggac catggctttc
cgggctcctc aggacccagg
2881 ggagaccctg gcttgaaagg tgataagggg gatgtcggtc
tccctggcaa gcctggctcc
2941 atggataagg tggacatggg cagcatgaag ggccagaaag
gagaccaagg agagaaagga
3001 caaattggac caattggtga gaaggatccc cgaggagacc
ctgggacccc aggagtgcct
 
3061 ggaaaggacg ggcaggcagg acagcctggg cagccaggac
ctaaaggtga tccaggtata
3121 agtggaaccc caggtgctcc aggacttccg ggaccaaaag
gatctgttgg tggaatgggc
3181 ttgccaggaa cacctggaga gaaaggtgtg cctggcatcc
ctggcccaca aggttcacct
3241 ggcttacctg gagacaaagg tgcaaaagga gagaaagggc
aggcaggccc acctggcata
3301 ggcatcccag gactgcgtgg tgaaaaggga gatcaaggga
tagcgggttt cccaggaagc
3361 cctggagaga agggagaaaa aggaagcatt gggatcccag
gaatgccagg gtccccaggc
3421 cttaaagggt ctcccgggag tgttggctat ccaggaagtc
ctgggctacc tggagaaaaa
3481 ggtgacaaag gcctcccagg attggatggc atccctggtg
tcaaaggaga agcaggtctt
3541 cctgggactc ctggccccac aggcccagct ggccagaaag
gggagccagg cagtgatgga
3601 atcccggggt cagcaggaga gaagggtgaa ccaggtctac
caggaagagg attcccaggg
3661 tttccagggg ccaaaggaga caaaggttca aagggtgagg
tgggtttccc aggattagcc
3721 gggagcccag gaattcctgg atccaaagga gagcaaggat
tcatgggtcc tccgggcccc
3781 cagggacagc cggggttacc gggatcccca ggccatgcca
cggagggggcc caaaggagac
3841 cgcggacctc agggccagcc tggcctgcca ggacttccgg
gacccatggg gcctccaggg
3901 cttcctggga ttgatggagt taaaggtgac aaaggaaatc
caggctggcc aggagcaccc
3961 ggtgtcccag ggcccaaggg agaccctgga ttccagggca
tgcctggtat tggtggctct
4021 ccaggaatca caggctctaa gggtgatatg gggcctccag
gagttccagg atttcaaggt
 
4081 ccaaaaggtc ttcctggcct ccagggaatt aaaggtgatc
aaggcgatca aggcgtcccg
4141 ggagctaaag gtctcccggg tcctcctggc cccccaggtc
cttacgacat catcaaaggg
4201 gagcccgggc tccctggtcc tgagggcccc ccagggctga
aagggcttca gggactgcca
4261 ggcccgaaag gccagcaagg tgttacagga ttggtgggta
tacctggacc tccaggtatt
4321 cctgggtttg acggtgcccc tggccagaaa ggagagatgg
gacctgccgg gcctactggt
4381 ccaagaggat ttccaggtcc accaggcccc gatgggttgc
caggatccat ggggccccca
4441 ggcaccccat ctgttgatca cggcttcctt gtgaccaggc
atagtcaaac aatagatgac
4501 ccacagtgtc cttctgggac caaaattctt taccacgggt
actctttgct ctacgtgcaa
4561 ggcaatgaac gggcccatgg acaggacttg ggcacggccg
gcagctgcct gcgcaagttc
4621 agcacaatgc ccttcctgtt ctgcaatatt aacaacgtgt
gcaactttgc atcacgaaat
4681 gactactcgt actggctgtc cacccctgag cccatgccca
tgtcaatggc acccatcacg
4741 ggggaaaaca taagaccatt tattagtagg tgtgctgtgt
gtgaggcgcc tgccatggtg
4801 atggccgtgc acagccagac cattcagatc ccaccgtgcc
ccagcgggtg gtcctcgctg
4861 tggatcggct actcttttgt gatgcacacc agcgctggtg
cagaaggctc tggccaagcc
4921 ctggcgtccc ccggctcctg cctggaggag tttagaagtg
cgccattcat cgagtgtcac
4981 ggccgtggga cctgcaatta ctacgcaaac gcttacagct
tttggctcgc caccatagag
5041 aggagcgaga tgttcaagaa gcctacgccg tccaccttga
aggcagggga gctgcgcacg
 
5101 cacgtcagcc gctgccaagt ctgtatgaga agaacataag
aagcctgact cagctaatgt
5161 cacaacatgg tgctacttct tcttcttttt gttaacagca
acgaacccta gaaatatatc
**Figure 9A - continued** 
5221 ctgtgtacct cactgtccaa tatgaaaacc gtaaagtgcc
ttataggaat ttgcgtaact
5281 aacacaccct gcttcattga cctctacttg ctgaaggaga
aaaagacagc gataagcttc
5341 aatagtggca taccaaatgg cacttttgat gaaataaaat
atcaatattt tctgcaatcc
5401 aatgcactga tgtgtgaagt gagaactcca tcagaaaacc
aaagggtgct aggaggtgtg
5461 ggtgccttcc atactgtttg cccattttca ttcttgtatt
ataattaatt ttctaccccc
5521 agagataaat gtttgtttat atcactgtct agctgtttca
aaatttaggt cccttggtct
5581 gtacaaataa tagcaatgta aaaatggttt tttgaacctc
caaatggaat tacagactca
5641 gtagccatat cttccaaccc cccagtataa atttctgtct
ttctgctatg tgtggtactt
5701 tgcagctgct tttgcagaaa tcacaatttt cctgtggaat
aaagatggtc caaaaatagt
5761 caaaaattaa atatatatat atattagtaa tttatataga
tgtcagcaat taggcagatc
5821 aaggtttagt ttaacttcca ctgttaaaat aaagcttaca
tagttttctt cctttgaaag
5881 actgtgctgt cctttaacat aggtttttaa agactaggat
attgaatgtg aaacatccgt
5941 tttcattgtt cacttctaaa ccaaaaatta tgtgttgcca
aaaccaaacc caggttcatg
 
6001 aatatggtgt ctattatagt gaaacatgta ctttgagctt
attgttttta ttctgtatta
6061 aatattttca gggttttaaa cactaatcac aaactgaatg
acttgacttc aaaagcaaca
6121 accttaaagg ccgtcatttc attagtattc ctcattctgc
atcctggctt gaaaaacagc
6181 tctgttgaat cacagtatca gtattttcac acgtaagcac
attcgggcca tttccgtggt
6241 ttctcatgag ctgtgttcac agacctcagc agggcatcgc
atggaccgca ggagggcaga
6301 ttcggaccac taggcctgaa atgacatttc actaaaagtc
tccaaaacat ttctaagact
6361 actaaggcct tttatgtaat ttctttaaat gtgtatttct
taagaattca aatttgtaat
6421 aaaactattt gtataaaaat taagctt
**SEQ ID NO: 10 (NM_001845.2) cds 130-5139** 
1 MGPRLSVWLL LLPAALLLHE EHSRAAAKGG CAGSGCGKCD
CHGVKGQKGE RGLPGLQGVI
61 GFPGMQGPEG PQGPPGQKGD TGEPGLPGTK GTRGPPGASG
YPGNPGLPGI PGQDGPPGPP
121 GIPGCNGTKG ERGPLGPPGL PGFAGNPGPP GLPGMKGDPG
EILGHVPGML LKGERGFPGI
181 PGTPGPPGLP GLQGPVGPPG FTGPPGPPGP PGPPGEKGQM
GLSFQGPKGD KGDQGVSGPP
241 GVPGQAQVQE KGDFATKGEK GQKGEPGFQG MPGVGEKGEP
GKPGPRGKPG KDGDKGEKGS
301 PGFPGEPGYP GLIGRQGPQG EKGEAGPPGP PGIVIGTGPL
GEKGERGYPG TPGPRGEPGP
361 KGFPGLPGQP GPPGLPVPGQ AGAPGFPGER GEKGDRGFPG
TSLPGPSGRD GLPGPPGSPG
421 PPGQPGYTNG IVECQPGPPG DQGPPGIPGQ PGFIGEIGEK
GQKGESCLIC DIDGYRGPPG
 
481 PQGPPGEIGF PGQPGAKGDR GLPGRDGVAG VPGPQGTPGL
IGQPGAKGEP GEFYFDLRLK
541 GDKGDPGFPG QPGMPGRAGS PGRDGHPGLP GPKGSPGSVG
LKGERGPPGG VGFPGSRGDT
601 GPPGPPGYGP AGPIGDKGQA GFPGGPGSPG LPGPKGEPGK
IVPLPGPPGA EGLPGSPGFP
661 GPQGDRGFPG TPGRPGLPGE KGAVGQPGIG FPGPPGPKGV
DGLPGDMGPP GTPGRPGFNG
721 LPGNPGVQGQ KGEPGVGLPG LKGLPGLPGI PGTPGEKGSI
GVPGVPGEHG AIGPPGLQGI
781 RGEPGPPGLP GSVGSPGVPG IGPPGARGPP GGQGPPGLSG
PPGIKGEKGF PGFPGLDMPG
841 PKGDKGAQGL PGITGQSGLP GLPGQQGAPG IPGFPGSKGE
MGVMGTPGQP GSPGPVGAPG
901 LPGEKGDHGF PGSSGPRGDP GLKGDKGDVG LPGKPGSMDK
VDMGSMKGQK GDQGEKGQIG
961 PIGEKGSRGD PGTPGVPGKD GQAGQPGQPG PKGDPGISGT
PGAPGLPGPK GSVGGMGLPG
1021 TPGEKGVPGI PGPQGSPGLP GDKGAKGEKG QAGPPGIGIP
GLRGEKGDQG IAGFPGSPGE
1081 KGEKGSIGIP GMPGSPGLKG SPGSVGYPGS PGLPGEKGDK
GLPGLDGIPG VKGEAGLPGT
1141 PGPTGPAGQK GEPGSDGIPG SAGEKGEPGL PGRGFPGFPG
AKGDKGSKGE VGFPGLAGSP
1201 GIPGSKGEQG FMGPPGPQGQ PGLPGSPGHA TEGPKGDRGP
QGQPGLPGLP GPMGPPGLPG
1261 IDGVKGDKGN PGWPGAPGVP GPKGDPGFQG MPGIGGSPGI
TGSKGDMGPP GVPGFQGPKG
1321 LPGLQGIKGD QGDQGVPGAK GLPGPPGPPG PYDIIKGEPG
LPGPEGPPGL KGLQGLPGPK
1381 GQQGVTGLVG IPGPPGIPGF DGAPGQKGEM GPAGPTGPRG
FPGPPGPDGL PGSMGPPGTP
1441 SVDHGFLVTR HSQTIDDPQC PSGTKILYHG YSLLYVQGNE
RAHGQDLGTA GSCLRKFSTM
 
1501 PFLFCNINNV CNFASRNDYS YWLSTPEPMP MSMAPITGEN
IRPFISRCAV CEAPAMVMAV
1561 HSQTIQIPPC PSGWSSLWIG YSFVMHTSAG AEGSGQALAS
PGSCLEEFRS APFIECHGRG
1620 TCNYYANAYS FWLATIERSE MFKKPTPSTL KAGELRTHVS
RCQVCMRRT
**Figure 9A - continued**
**SEQ ID NO: 11 (BC003378)** 
1 gagacagcgc cggggcaagt gagagccgga cgggcactgg
gcgactctgt gcctcgctga
61 ggaaaaataa ctaaacatgg gcaaaggaga tcctaagaag
ccgagaggca aaatgtcatc
121 atatgcattt tttgtgcaaa cttgtcggga ggagcataag
aagaagcacc cagatgcttc
181 agtcaacttc tcagagtttt ctaagaagtg ctcagagagg
tggaagacca tgtctgctaa
241 agagaaagga aaatttgaag atatggcaaa ggcggacaag
gcccgttatg aaagagaaat
301 gaaaacctat atccctccca aaggggagac aaaaaagaag
ttcaaggatc ccaatgcacc
361 caagaggcct ccttcggcct tcttcctctt ctgctctgag
tatcgcccaa aaatcaaagg
421 agaacatcct ggcctgtcca ttggtgatgt tgcgaagaaa
ctgggagaga tgtggaataa
481 cactgctgca gatgacaagc agccttatga aaagaaggct
gcgaagctga aggaaaaata
541 cgaaaaggat attgctgcat atcgagctaa aggaaagcct
gatgcagcaa aaaagggagt
601 tgtcaaggct gaaaaaagca agaaaaagaa ggaagaggag
gaagatgagg aagatgaaga
661 ggatgaggag gaggaggaag atgaagaaga tgaagatgaa
gaagaagatg atgatgatga
 
721 ataagttggt tctagcgcag tttttttttc ttgtctataa
agcatttaac cccccctgtac
781 acaactcact ccttttaaag aaaaaaattg aaatgtaagg
ctgtgtaaga tttgttttta
841 aactgtacag tgtctttttt tgtatagtta acacactacc
gaatgtgtct ttagatagcc
901 ctgtcctggt ggtattttca atagccacta accttgcctg
gtacagtatg ggggttgtaa
961 attggcatgg aaatttaaag caggttcttg ttggtgcaca
gcacaaatta gttatatatg
1021 gggatggtag ttttttcatc ttcagttgtc tctgatgcag
cttatacgaa ataattgttg
1081 ttctgttaac tgaataccac tctgtaattg caaaaaaaaa
aaaagttgca gctgttttgt
1141 tgacattctg aatgcttcta agtaaataca atttttttta
ttaaaaaaaa aaaaaaaaaa
1201 aaaaaaa
**SEQ ID NO: 12 (BC003378)** 
1 MGKGDPKKPR GKMSSYAFFV QTCREEHKKK HPDASVNFSE
FSKKCSERWK TMSAKEKGKF
61 EDMAKADKAR YEREMKTYIP PKGETKKKFK DPNAPKRPPS
AFFLFCSEYR PKIKGEHPGL
121 SIGDVAKKLG EMWNNTAADD KQPYEKKAAK LKEKYEKDIA
AYRAKGKPDA AKKGVVKAEK
181 SKKKKEEEED EEDEEDEEEE EDEEDEDEEE DDDDE
**SEQ ID NO: 13 (BC041913) (TAG23)** 
1 taattcagaa ttgagtaaag aaatattttt tctagtcctt
catatattga aaacttgcca
61 catgacattg tatcgtcttc attttccaga agatgcgttg
gtgtgccata ggtttctaac
121 ttccttgaaa atagtttttt aagtcaattg taaatatacg
tattattgtt aaaagtaact
 
181 ttaaactgca acacatagct tcaaaacaat atagagattt
tgtaatacct tataagtgga
241 gttggctaaa ataccttatc catataaaac ttattctatt
ctttgcatgc ttattttgtg
301 tgttggttgc tagcttaaag tttgatttgt tgttactctt
tgtgtgccaa attcactagg
361 caagcggatt tttcctcaga cttcaaaaaa taattctttt
aagaaaaaat gtaaaaatgt
421 ttattctaaa aagctgcatt aaagggacaa cctataaaaa
gttttgctag ctcatcttta
481 gaaggaagaa agaatattag cttgggtgat gtttaatttg
ggtggcgata gtttctgtag
541 gctaaacttt atgagaaaag tgtacctact ctataaaggt
aataaatgta aaacctcttg
601 ctgttattga ggaagctctt caactaccct aaatttcaca
aatgtaactt ataacactat
661 gaaaagattt gaccaacaat ttacgtttgc tgtgtgcttt
agtttttgtt taagcatatt
721 cttttgcttg aatttctgtg ttcatgagag ttagggtgtt
ttatgcttct tgaactaatt
781 ttataacata tttaatatat taccagttaa gatataaaat
catttgtaca tagcgaattg
841 taaagcagct attaaagtag gtgaaataaa gtatatattt
gccggttatc catatctttt
901 agaagtcctg acagaacaac cagtttattt gcacataggt
agcttctgtt tgaaggaagg
961 taaagttata aggaaactca aatactataa gatgtgtcaa
ggtatttctc cagaattaat
1021 tgcaaagcta gtgctgaagg attttaatca gcttctaaaa
ttttcttctc aataagacat
1081 atgttttgat tacttaggga agattcctca tttttatttg
ccctttatgc atttaatcca
1141 catgatagga cattaaaaat taatataaag aaaaatcgtg
ctcatactgt acatctattt
 
1201 ctgtgcttgg aactacttgt taatagtttt tatcgaagct
gtcagcaata agggacataa
1261 aactgctgta ttatacattg tggaattgaa taaacagcct
aatttttttt tttctagtat
**Figure 9A - continued** 
1321 agggtactta agcatttcca cttttggaag aaaagtgtat
tagtatttta tattgcattt
1381 catttaaaag gacagttttt tttttttttg taaatccatt
cattgaaatg gtttctaaac
1441 tgtataatgt aatttggagc ctatttagta atagaattaa
atgtcctatg tagtgctaca
1501 atttttgaat tagaaagtga tcaaatgtaa gaaaaaaatt
taaaaattca gcccagaaaa
1561 caaaatagtg tattaaatta gtttaatgta aaaggaattt
ataagatttt tttcctcaat
1621 atagatacct cacttgaaaa gaaagcacag catacttaaa
gtagttctag taaacatgtc
1681 ctagaaaaca gttgctaaat gtaggacatc ttttgaggaa
ttagtttatg agaaataaaa
1741 ttttacttgt ttttactatc ctgttagaag tatttgttta
tcctgataat tttaagccaa
1801 catagtagtc ttaaattact tttgaatttc taatctgtga
aggcagtaaa tgaaatatct
1861 gttctgcaac tgttgaaaca aataattggc tacattgacc
ataattaaag ttaaaatttt
1921 gccaatgatg tacagtttta tggttaaagt tgctgtggtt
ggttgcatta catgacacag
1981 aaaactgtcc tctacctcac gtgaaataaa tattttatat
ggttttacta aaaataagac
2041 tcatgtatct ggtcacctag tttacaaatt ttgaattata
tttattgaaa catgacatac
 
2101 tgtgctctga gcttatacct caattgtatt ttgtgctgtt
ttccattttc atgccttgta
2161 aataacttgt atagattgtg gatcaaatac taaataaaaa
cttttaatgc caaaaaaaaa
2221 aaaaaaa
**SEQ ID NO: 14 (BC041913) (TAG23)**
**SEQ ID NO: 15 (NM_014571) (TAG25)** 
1 accaggcagc ctgcgttcgc catgaagcga cccaaggagc
cgagcggctc cgacggggag
61 tccgacggac ccatcgacgt gggccaagag ggccagctga
gccagatggc caggccgctg
121 tccaccccca gctcttcgca gatgcaagcc aggaagaaac
gcagagggat catagagaaa
181 cggcgtcgag accgcatcaa cagtagcctt tctgaattgc
gacgcttggt ccccactgcc
241 tttgagaaac agggctcttc caagctggag aaagccgagg
tcttgcagat gacggtggat
301 cacttgaaaa tgctccatgc cactggtggg acaggattct
ttgatgcccg agccctggca
361 gttgacttcc ggagcattgg ttttcgggag tgcctcactg
aggtcatcag gtacctgggg
421 gtccttgaag ggcccagcag ccgtgcagac cccgtccgga
ttcgccttct ctcccacctc
481 aacagctacg cagccgagat ggagccttcg cccacgccca
ctggcccttt ggccttccct
541 gcctggccct ggtctttctt ccatagctgt ccaggctgc
cagccctgag caaccagctc
601 gccatcctgg gaagagtgcc cagccctgtc ctccccggtg
tctcctctcc tgcttacccc
661 atcccagccc tccgaaccgc tccccttcgc agagccacag
gcatcatcct gccagcccgg
 
721 aggaatgtgc tgcccagtcg aggggcatct tccacccgga
gggcccgccc cctagagagg
781 ccagcgaccc ctgtgcctgt cgcccccagc agcagggctg
ccaggagcag ccacatcgct
841 cccctcctgc agtcttcctc cccaacaccc cctggtccta
cagggtcggc tgcttacgtg
901 gctgttccca cccccaactc atcctcccca gggccagctg
ggaggccagc gggagccatg
961 ctctaccact cctgggtctc tgaaatcact gaaatcgggg
ctttctgagc tgccccttca
1021 ccaccccgcc ccaaggaata aggaaggttc ttttaccagg
agcccaaaaa agggcactgc
1081 cttttctgct ttgcttcgtg gactggctca tatgtgaagg
cacgttctcc agccatcaga
1141 ggccccctcc tcctccaacc catctctcct tctcactgtt
atcccagctt atccacccag
1201 ctctcctgga gctgttctgg tctcagaggc ttggttccat
ttctcacctg aacagatgag
1261 tcctgggaga gaccctcaga gatccgccca gacccctctc
ctgccctctg cacaccagca
1321 gcaggcatga accttgggtc tgggaaaaag ctttaacctg
cagggcacca ggacccaagg
1381 caggctgttc cttggggcgg tcagacccca gtcaggagca
atgactgact ggctgcagcc
1441 ttcccacgcc aagaggctgg aacatagtgt ctgcctcgct
tcctggagat agtaactgag
1501 caggggctac aaagaggtct cctgggaacc ctgtctgccc
cttcccacct gtccttgggc
1561 cacaccatca cactgaacca caggacagac cctttctcca
ccacagccaa ggcctggaga
1621 ctgggggccc agcagagcct gctcccaccc tcctcccagc
agcagacacc caccctctca
1681 ctgactaaca ggtccctgca cacagctggc ctggtaaacc
cagctgggag gtttctaggc
 
1741 agcagcaaaa ctctgtgaca gggtgtcctc acaccaggcc
ttggacagct ctcccagaca
**Figure 9A - continued** 
1801 ggagccaggg ttgagcaatg gagagcccag cccccacgtc
ttacagtcgc catcctccag
1861 gcgtgtggtc cctccccatt gggtgcacag tgcagagggg
ccgtggcccc atgtgatggt
1921 gcgcagagag gaacctcttg ggattcagca ccagacgtct
gtgctgcctg gtttgcatcc
1981 ggctcacaga gcccagactg ctggaacagc caaggactgt
caggctggac aaaaataact
2041 gcaaggaggg gcaagagaaa ggatgattcg aggcaccttg
gcccttcaag gtcatgcagt
2101 gggtcgagcg cctgagatcc tgttcaccag gactccacag
agctggctct gctcagaagc
2161 catttcattc cccggctcca ccctaggcca ctttttctaa
cagaggaaac aaatggtcca
2221 gcagtcgttc ccagcagaac agcggagcct ggactgacac
ccagtgggac cagtgttgcc
2281 acaccagttg ataaaatgca gaaacccttc tgtactcgtt
ggtaaatatc tactccccca
2341 agtgactcca ggtgcccccc accgcctggc acttccccca
ggactcctac gatctggtta
2401 ctgcctggcc gatccaaggc tgtggagtcc cagagccagc
agttcactgg tgctcattcc
2461 acactggtta gatacttcag ttgtcacccc tgggaagatt
ctcccacctc ctccctttga
2521 tggaaccacc ctccccagag gctgcattga ggagactcca
cagactgaaa agtgagtttg
2581 cagaaacctt ggggaaaagg gccctttcaa agaagtggat
aagagggagg agatcattga
 
2641 gtgacccaga aagctctttt gaaaagacag actcctcaag
gagagataaa gaggaaagca
2701 cctctttcat tttttagtgt gagctaattc catcagactg
ctgtcctcct ggacccatct
2761 gagatgtgca gtagcaagga gaggggggat cattttagag
agtgggtcat tggcagggag
2821 tgctccggag ggaggcagag gggagactgt ggtagaagga
agacagaact cacacatgct
2881 cccaggattg gggacaggga cagaggaggt aacagaaggc
aaaggccagt ttccccgtta
2941 tcatgaaggg gcccactcag gacaggaaca aggacaactc
ctcctcctcc tcctcctctc
3001 ctgctgctcc tgggatacca ggtcagtgat gtagtcttgc
agtttggcaa cttcctagcc
3061 tgagaatccc tagtggggct gtgggaaaca catttccacg
ttgcaagcat gcaactccaa
3121 agaatctgtg atgccactga aatgagatgg gaatgatcca
gctctttcag catcttggtt
3181 gaacttgctt tcattgtccc tgggatattg tggaaggaaa
ggtgactgtg tgatctgatt
3241 ctgtggtcaa ggacttgcat cttgtgtttc tatccccaag
ccttcctggt gtctccaact
3301 cctaccccat tgcatgggtt gttgcggaca tccaataaag
attttttag tgcttctgga
3361 aaaaaaaaaa aaaaaaaaaa aaaaaaaaaa aaaaaaaaaa
aaaaaaaaaa aaaacc
**SEQ ID NO: 16 (NM_014571) (TAG25) (cds 22-1008)** 
1 MKRPKEPSGS DGESDGPIDV GQEGQLSQMA RPLSTPSSSQ
MQARKKRRGI IEKRRRDRIN
61 SSLSELRRLV PTAFEKQGSS KLEKAEVLQM TVDHLKMLHA
TGGTGFFDAR ALAVDFRSIG
121 FRECLTEVIR YLGVLEGPSS RADPVRIRLL SHLNSYAAEM
EPSPTPTGPL AFPAWPWSFF
 
181 HSCPGLPALS NQLAILGRVP SPVLPGVSSP AYPIPALRTA
PLRRATGIIL PARRNVLPSR
241 GASSTRRARP LERPATPVPV APSSRAARSS HIAPLLQSSS
PTPPGPTGSA AYVAVPTPNS
301 SSPGPAGRPA GAMLYHSWVS EITEIGAF
**SEQ ID NO: 17 (NM_017994.1) (TAG27)** 
1 aaaaccgcgg ttgccggagc ccgaactgag gcggcggcgg
gagcccggtt ggcgtctggt
61 cttcgcgtcg gccccgcgga gccagacgct gcccccggcg
cggggagaag atggtgccta
121 gcggcctcgg gcccgccacg cgccgccacg agtgagccca
gcgcgaccgc gggcgtccgc
181 cgagcagctg gcccggctgg gcccggggcg cgcagctgcc
cgccggggcg gggtggagct
241 gatcagaata atgttcagca tcaaccccct ggagaacctg
aaggtgtaca tcagcagtcg
301 gcctcccctg gtggtcttca tgatcagcgt aagcgccatg
gccatagctt tcctgaccct
361 gggctacttc ttcaaaatca aggagattaa atccccagaa
atggcagagg attggaatac
421 ttttctgcta cggttcaatg atttggactt gtgtgtatca
gagaatgaaa ccctcaagca
481 tctcacaaac gacaccacaa ctccggaaag tacaatgacc
agcgggcagg cccgagcttc
541 cacccagtcc ccccaggccc tggaggactc gggcccggtg
aatatctcag tctcaatcac
601 cctaaccctg gacccactga aacccttcgg agggtattcc
cgcaacgtca cccatctgta
661 ctcaaccatc ttagggcatc agattggact ttcaggcagg
gaagcccacg aggagataaa
721 catcaccttc accctgccta cagcgtggag ctcagatgac
tgcgccctcc acggtcactg
 
781 tgagcaggtg gtattcacag cctgcatgac cctcacggcc
agccctgggg tgttccccgt
841 cactgtacag ccaccgcact gtgttcctga cacgtacagc
aacgccacgc tctggtacaa
**Figure 9A - continued** 
901 gatcttcaca actgccagag atgccaacac aaaatacgcc
caagattaca atcctttctg
961 gtgttataag ggggccattg gaaaagtcta tcatgcttta
aatcccaagc ttacagtgat
1021 tgttccagat gatgaccgtt cattaataaa tttgcatctc
atgcacacca gttacttcct
1081 ctttgtgatg gtgataacaa tgttttgcta tgctgttatc
aagggcagac ctagcaaatt
1141 gcgtcagagc aatcctgaat tttgtcccga gaaggtggct
ttggctgaag cctaattcca
1201 cagctccttg ttttttgaga gagactgaga gaaccataat
ccttgcctgc tgaacccagc
1261 ctgggcctgg atgctctgtg aatacattat cttgcgatgt
tgggttattc cagccaaaga
1321 catttcaagt gcctgtaact gatttgtaca tatttataaa
aatctattca gaaattggtc
1381 caataatgca cgtgctttgc cctgggtaca gccagagccc
ttcaacccca ccttggactt
1441 gaggacctac ctgatgggac gtttccacgt gtctctagag
aaggattcct ggatctagct
1501 ggtcacgacg atgttttcac caaggtcaca ggagcattgc
gtcgctgatg gggttgaagt
1561 ttggtttggt tcttgtttca gcccaatatg tagagaacat
ttgaaacagt ctgcaccttt
1621 gatacggtat tgcatttcca aagccaccaa tccattttgt
ggattttatg tgtctgtggc
 
1681 ttaataatca tagtaacaac aataatacct tttcctccat
tttgcttgca ggaaacatac
1741 cttaagtttt ttttgttttg tttttgtttt tttgtttttt
gttttccttt atg
**SEQ ID NO: 18 (NM_017994.1) (TAG27)** 
1 MFSINPLENL KVYISSRPPL VVFMISVSAM AIAFLTLGYF
FKIKEIKSPE MAEDWNTFLL
61 RFNDLDLCVS ENETLKHLTN DTTTPESTMT SGQARASTQS
PQALEDSGPV NISVSITLTL
121 DPLKPFGGYS RNVTHLYSTI LGHQIGLSGR EAHEEINITF
TLPTAWSSDD CALHGHCEQV
181 VFTACMTLTA SPGVFPVTVQ PPHCVPDTYS NATLWYKIFT
TARDANTKYA QDYNPFWCYK
241 GAIGKVYHAL NPKLTVIVPD DDRSLINLHL MHTSYFLFVM
VITMFCYAVI KGRPSKLRQS
301 NPEFCPEKVA LAEA
**SEQ ID NO: 19 (X02160) (TAG28)** 
1 accgggagcg cgcgctctga tccgaggaga ccccgcgctc
ccgcagccat gggcaccggg
61 ggccggcggg gggcggcggc cgcgccgctg ctggtggcgg
tggccgcgct gctactgggc
121 gccgcgggcc acctgtaccc cggagaggtg tgtcccggca
tggatatccg gaacaacctc
181 actaggttgc atgagctgga gaattgctct gtcatcgaag
gacacttgca gatactcttg
241 atgttcaaaa cgaggcccga agatttccga gacctcagtt
tccccaaact catcatgatc
301 actgattact tgctgctctt ccgggtctat gggctcgaga
gcctgaagga cctgttcccc
361 aacctcacgg tcatccgggg atcacgactg ttctttaact
acgcgctggt catcttcgag
 
421 atggttcacc tcaaggaact cggcctctac aacctgatga
acatcacccg gggttctgtc
481 cgcatcgaga agaacaatga gctctgttac ttggccacta
tcgactggtc ccgtatcctg
541 gattccgtgg aggataatta catcgtgttg aacaaagatg
acaacgagga gtgtggagac
601 atctgtccgg gtaccgcgaa gggcaagacc aactgccccg
ccaccgtcat caacgggcag
661 tttgtcgaac gatgttggac tcatagtcac tgccagaaag
tttgcccgac catctgtaag
721 tcacacggct gcaccgccga aggcctctgt tgccacagcg
agtgcctggg caactgttct
781 cagcccgacg accccaccaa gtgcgtggcc tgccgcaact
tctacctgga cggcaggtgt
841 gtggagacct gcccgccccc gtactaccac ttccaggact
ggcgctgtgt gaacttcagc
901 ttctgccagg acctgcacca caaatgcaag aactcgcgga
ggcagggctg ccaccagtac
961 gtcattcaca acaacaagtg catccctgag tgtccctccg
ggtacacgat gaattccagc
1021 aacttgctgt gcaccccatg cctgggtccc tgtcccaagg
tgtgccacct cctagaaggc
1081 gagaagacca tcgactcggt gacgtctgcc caggagctcc
gaggatgcac cgtcatcaac
1141 gggagtctga tcatcaacat tcgaggaggc aacaatctgg
cagctgagct agaagccaac
1201 ctcggcctca ttgaagaaat ttcagggtat ctaaaaatcc
gccgatccta cgctctggtg
1261 tcactttcct tcttccggaa gttacgtctg attcgaggag
agaccttgga aattgggaac
1321 tactccttct atgccttgga caaccagaac ctaaggcagc
tctgggactg gagcaaacac
1381 aacctcacca tcactcaggg gaaactcttc ttccactata
accccaaact ctgcttgtca
 
1441 gaaatccaca agatggaaga agtttcagga accaaggggc
gccaggagag aaacgacatt
1501 gccctgaaga ccaatgggga ccaggcatcc tgtgaaaatg
agttacttaa attttcttac
1561 attcggacat cttttgacaa gatcttgctg agatgggagc
cgtactggcc ccccgacttc
**Figure 9A - continued** 
1621 cgagacctct tggggttcat gctgttctac aaagaggccc
cttatcagaa tgtgacggag
1681 ttcgacgggc aggatgcatg tggttccaac agttggacgg
tggtagacat tgacccaccc
1741 ctgaggtcca acgaccccaa atcacagaac cacccagggt
ggctgatgcg ggtctcaag
1801 ccctggaccc agtatgccat ctttgtgaag accctggtca
ccttttcgga tgaacgccgg
1861 acctatgggg ccaagagtga catcatttat gtccagacag
atgccaccaa cccctctgtg
1921 cccctggatc caatctcagt gtctaactca tcatcccaga
ttattctgaa gtggaaacca
1981 ccctccgacc ccaatggcaa catcacccac tacctggttt
tctgggagag gcaggcggaa
2041 gacagtgagc tgttcgagct ggattattgc ctcaaagggc
tgaagctgcc ctcgaggacc
2101 tggtctccac cattcgagtc tgaagattct cagaagcaca
accagagtga gtatgaggat
2161 tcggccggcg aatgctgctc ctgtccaaag acagactctc
agatcctgaa ggagctggag
2221 gagtcctcgt ttaggaagac gtttgaggat tacctgcaca
acgtggtttt cgtccccagg
2281 ccatctcgga aacgcaggtc ccttggcgat gttgggaatg
tgacggtggc cgtgcccacg
 
2341 gtggcagctt tccccaacac ttcctcgacc agcgtgccca
cgagtccgga ggagcacagg
2401 ccttttgaga aggtggtgaa caaggagtcg ctggtcatct
ccggcttgcg acacttcacg
2461 ggctatcgca tcgagctgca ggcttgcaac caggacaccc
ctgaggaacg gtgcagtgtg
2521 gcagcctacg tcagtgcgag gaccatgcct gaagccaagg
ctgatgacat tgttggccct
2581 gtgacgcatg aaatctttga gaacaacgtc gtccacttga
tgtggcagga gccgaaggag
2641 cccaatggtc tgatcgtgct gtatgaagtg agttatcggc
gatatggtga tgaggagctg
2701 catctctgcg tctcccgcaa gcacttcgct ctggaacggg
gctgcaggct gcgtgggctg
2761 tcaccgggga actacagcgt gcgaatccgg gccacctccc
ttgcgggcaa cggctcttgg
2821 acggaaccca cctatttcta cgtgacagac tatttagacg
tcccgtcaaa tattgcaaaa
2881 attatcatcg gccccctcat ctttgtcttt ctcttcagtg
ttgtgattgg aagtatttat
2941 ctattcctga gaaagaggca gccagatggg ccgctgggac
cgctttacgc ttcttcaaac
3001 cctgagtatc tcagtgccag tgatgtgttt ccatgctctg
tgtacgtgcc ggacgagtgg
3061 gaggtgtctc gagagaagat caccctcctt cgagagctgg
ggcagggctc cttcggcatg
3121 gtgtatgagg gcaatgccag ggacatcatc aagggtgagg
cagagacccg cgtggcggtg
3181 aagacggtca acgagtcagc cagtctccga gagcggattg
agttcctcaa tgaggcctcg
3241 gtcatgaagg gcttcacctg ccatcacgtg gtgcgcctcc
tgggagtggt gtccaagggc
3301 cagcccacgc tggtggtgat ggagctgatg gctcacggag
acctgaagag ctacctccgt
 
3361 tctctgcggc cagaggctga gaataatcct ggccgccctc
ccccctaccct tcaagagatg
3421 attcagatgg cggcagagat tgctgacggg atggcctacc
tgaacgccaa gaagtttgtg
3481 catcgggacc tggcagcgag aaactgcatg gtcgcccatg
attttactgt caaaattgga
3541 gactttggaa tgaccagaga catctatgaa acggattact
accggaaagg gggcaagggt
3601 ctgctccctg tacggtggat ggcaccggag tccctgaagg
atgggtctt caccacttct
3661 tctgacatgt ggtcctttgg cgtggtcctt tgggaaatca
ccagcttggc agaacagcct
3721 taccaaggcc tgtctaatga acaggtgttg aaatttgtca
tggatggagg gtatctggat
3781 caacccgaca actgtccaga gagagtcact gacctcatgc
gcatgtgctg gcaattcaac
3841 cccaacatga ggccaacctt cctggagatt gtcaacctgc
tcaaggacga cctgcacccc
3901 agctttccag aggtgtcgtt cttccacagc gaggagaaca
aggctcccga gagtgaggag
3961 ctggagatgg agtttgagga catggagaat gtgcccctgg
accgttcctc gcactgtcag
4021 agggaggagg cggggggccg ggatggaggg tcctcgctgg
gtttcaagcg gagctacgag
4081 gaacacatcc cttacacaca catgaacgga ggcaagaaaa
acgggcggat tctgaccttg
4141 cctcggtcca atccttccta acagtgccta ccgtggcggg
ggcgggcagg ggttcccatt
4201 ttcgctttcc tctggtttga aagcctctgg aaaactcagg
attctcacga ctctaccatg
4261 tccaatggag ttcagagatc gttcctatac atttctgttc
atcttaaggt ggactcgttt
4321 ggttaccaat ttaactagtc ctgcagagga tttaactgtg
aacctggagg gcaaggggtt
 
4381 tccacagttg ctgctccttt ggggcaacga cggtttcaaa
ccaggatttt gtgtttttc
4441 gttcccccca cccgccccca gcagatggaa agaaagcacc
tgtttttaca aattcttttt
4501 tttttttttt ttttttgctg gtgtctgagc ttcagtataa
aagacaaaac ttcctgtttg
4561 tggaacaaaa gttcgaaaga aaaaacaaaa caaaaacacc
cagccctgtt ccaggagaat
4621 ttcaagtttt acaggttgag cttcaagatg gtttttttgg
tttttttttt ttctctcatc
4681 caggctgaag gatttttttt ttctttacaa aatgagttcc
tcaaattgac caatagctgc
4741 tgctttcata ttttggataa gggtctgtgg tcccggcgtg
tgctcacgtg tgtatgcacg
4801 tgtgtgtgtc cattagacac ggctgacgtg tgtgcaaagt
atccatgcgg agttgatgct
**Figure 9A - continued** 
4861 ttgggaattg gctcatgaag gttcttctca agggtgcgag
ctcatccccc tctctccttc
4921 cttcttattg actgggagac tgtgctctcg acagattctt
cttgtgtcag aagtctagcc
4981 tcaggtttct accctccctt cacattggtg gccaagggag
gagcatttca tttggagtga
5041 ttatgaatct tttcaagacc aaaccaagct aggacattaa
aaaaaaaaaa aagaaaaaga
5101 aagaaaaaac aaaatggaaa aaggaaaaaa aaaaagaact
gagatgacag agttttgaga
5161 atatatttgt accatattta
**SEQ ID NO: 20 (X02160) (TAG28)** 
1


61


121


181


241


301


361


421


481


541


601


 
661


721


781


841


901


961


1021


1081


1141


1201


1261


1321   
**SEQ ID NO: 21 (BC017201) (TAG29)** 
1 gccgctgcca ccgcaccccg ccatggagcg gccgtcgctg
cgcgccctgc tcctcggcgc
61 cgctgggctg ctgctcctgc tcctgcccct ctcctcttcc
tcctcttcgg acacctgcgg
121 cccctgcgag ccggcctcct gcccgcccct gcccccgctg
ggctgcctgc tgggcgagac
181 ccgcgacgcg tgcggctgct gccctatgtg cgcccgcggc
gagggcgagc cgtgcggggg
241 tggcggcgcc ggcagggggt actgcgcgcc gggcatggag
tgcgtgaaga gccgcaagag
301 gcggaagggt aaagccgggg cagcagccgg cggtccgggt
gtaagcggcg tgtgcgtgtg
361 caagagccgc tacccggtgt gcggcagcga cggcaccacc
tacccgagcg gctgccagct
421 gcgcgccgcc agccagaggg ccgagagccg cggggagaag
gccatcaccc aggtcagcaa
481 gggcacctgc gagcaaggtc cttccatagt gacgcccccc
aaggacatct ggaatgtcac
541 tggtgcccag gtgtacttga gctgtgaggt catcggaatc
ccgacacctg tcctcatctg
601 gaacaaggta aaaaggggtc actatggagt tcaaaggaca
gaactcctgc ctggtgaccg
661 ggacaacctg gccattcaga cccgggtggc ccagaaaaag
catgaagtaa ctggctgggt
721 gctggtatct cctctaagta aggaagatgc tggagaatat
gagtgccatg catccaattc
781 ccaaggacag gcttcagcat cagcaaaaat tacagtggtt
gatgccttac atgaaatacc
841 agtgaaaaaa ggtgaaggtg ccgagctata aacctccaga
atattattag tctgcatggt
 
901 taaaagtagt catggataac tacattacct gttcttgcct
aataagtttc ttttaatcca
961 atccactaac actttagtta tattcactgg ttttacacag
agaaatacaa aataaagatc
1021 acacatcaag actatctaca aaaatttatt atatatttac
agaagaaaag catgcatatc
**Figure 9A - continued** 
1081 attaaacaaa taaaatactt tttatcacaa aaaaaaaaaa
aaaaaaaaaa aaaaaaaaaa
1141 aaaaaaaaaa aaaaa
**SEQ ID NO: 22 (BC017201) (TAG29)** 
1
MERPSLRALLLGAAGLLLLLLPLSSSSSSDTCGPCEPASCPPLPPLGCLLGETRDACG
CC
61
PMCARGEGEPCGGGGAGRGYCAPGMECVKSRKRRKGKAGAAAGGPGVSGVCVCKSRYP
VC
121
GSDGTTYPSGCQLRAASQRAESRGEKAITQVSKGTCEQGPSIVTPPKDIWNVTGAQVY
LS
181
CEVIGIPTPVLIWNKVKRGHYGVQRTELLPGDRDNLAIQTRGGPEKHEVTGWVLVSPL
SK
241 EDAGEYECHASNSQGQASASAKITVVDALHEIPVKKGEGAEL
**SEQ ID NO: 23 (NM_002300) (TAG-30)** 
1 ggggcttgca gagccggcgc cggaggagac gcacgcagct
gactttgtct tctccgcacg
61 actgttacag aggtctccag agccttctct ctcctgtgca
aaatggcaac tcttaaggaa
 
121 aaactcattg caccagttgc ggaagaagag gcaacagttc
caaacaataa gatcactgta
181 gtgggtgttg gacaagttgg tatggcgtgt gctatcagca
ttctgggaaa gtctctggct
241 gatgaacttg ctcttgtgga tgttttggaa gataagctta
aaggagaaat gatggatctg
301 cagcatggga gcttatttct tcagacacct aaaattgtgg
cagataaaga ttattctgtg
361 accgccaatt ctaagattgt agtggtaact gcaggagtcc
gtcagcaaga aggggagagt
421 cggctcaatc tggtgcagag aaatgttaat gtcttcaaat
tcattattcc tcagatcgtc
481 aagtacagtc ctgattgcat cataattgtg gtttccaacc
cagtggacat tcttacgtat
541 gttacctgga aactaagtgg attacccaaa caccgcgtga
ttggaagtgg atgtaatctg
601 gattctgcta gatttcgcta ccttatggct gaaaaacttg
gcattcatcc cagcagctgc
661 catggatgga ttttggggga acatggcgac tcaagtgtgg
ctgtgtggag tggtgtgaat
721 gtggcaggtg tttctctcca ggaattgaat ccagaaatgg
gaactgacaa tgatagtgaa
781 aattggaagg aagtgcataa gatggtggtt gaaagtgcct
atgaagtcat caagctaaaa
841 ggatatacca actgggctat tggattaagt gtggctgatc
ttattgaatc catgttgaaa
901 aatctatcca ggattcatcc cgtgtcaaca atggtaaagg
ggatgtatgg cattgagaat
961 gaagtcttcc tgagccttcc atgtatcctc aatgcccggg
gattaaccag cgttatcaac
1021 cagaagctaa aggatgatga ggttgctcag ctcaagaaaa
gtgcagatac cctgtgggac
1081 atccagaagg acctaaaaga cctgtgacta gtgagctcta
ggctgtagaa atttaaaaac
 
1141 tacaatgtga ttaactcgag cctttagttt tcatccatgt
acatggatca cagtttgctt
1201 tgatcttctt caatatgtga atttgggctc acagaatcaa
agcctatgct tggtttaatg
1261 cttgcaatct gagctcttga acaaataaaa ttaactattg
tagtgcgaaa aaaaaaaaaa
1321 aaaaaaaaaa aaaaaa
**SEQ ID NO: 24 (NM_002300) (TAG-30)** 
1
MATLKEKLIAPVAEEEATVPNNKITVVGVGQVGMACAISILGKSLADELALVDVLEDK
LK
61
GEMMDLQHGSLFLQTPKIVADKDYSVTANSKIVVVTAGVRQQEGESRLNLVQRNVNVF
KF
121
IIPQIVKYSPDCIIIVVSNPVDILTYVTWKLSGLPKHRVIGSGCNLDSARFRYLMAEK
LG
181
IHPSSCHGWILGEHGDSSVAVWSGVNVAGVSLQELNPEMGTDNDSENWKEVHKMVVES
AY
241
EVIKLKGYTNWAIGLSVADLIESMLKNLSRIHPVSTMVKGMYGIENEVFLSLPCILNA
RG
301 LTSVINQKLKDDEVAQLKKSADTLWDIQKDLKDL
**SEQ ID NO: 25 (CV337076) (TAG-31)** 
1 ccctccccca gagctgttat gatgagctgc aggaagctct
ggagttgggc ggggagactg
61 gactcccggg agtacccagc gctgcttttt tgttcatgca
gcctgtgatt catagcttcc
121 ctggggtgtt ggggagaatc acatttgggt cagccaggtt
tagcactgac agttttgtct
 
181 ttagaatcaa gcagatgtgg aatcaaatct ggctgtatcc
atgaccaact ctgaagccat
241 gagtgggtta catagcttta gagcctcagc atactcatct
ggaaagtgga agtgatcatg
301 tctattttgc agagttgttg ccacttttcc tctctggacc
ccactttccc catctgtcag
**Figure 9A - continued** 
361 atgaaagagt gggatgagat ggcctgtcta tttatctctc
aatcacaatg gctctatttg
421 aaaaaagttt gaactgccct aagtgctaaa aaggaagatg
gggagccatc aagacaaact
481 taggcctaca ttaccatcga gttcagagaa tggcagaccg
taagcaccag ccagcttcac
541 tcc
**SEQ ID NO: 26 (CV337076) (TAG-31)**
**SEQ ID NO: 27 (HSA320486) (TAG-32)** 
1 tggaggagag aagatggcgg aagcggagtg agtgactaga
tgatttaagg accatagtac
61 agctatggat actgaaccaa acccgggaac atcttctgtg
tcaacaacaa ccagcagtac
121 caccaccacc accatcacca cttcctcctc tcgaatgcag
cagccacaga tctctgtcta
181 cagtggttca gaccgacatg ctgtacaggt aattcaacag
gcattgcatc ggcccccag
241 ctcagctgct cagtaccttc agcaaatgta tgcagcccaa
caacagcact tgatgctgca
301 tactgcagct cttcagcagc agcatttaag cagctcccag
cttcagagcc ttgctgctgt
 
361 tcaggcaagt ttgtccagtg gaagaccatc tacatctccc
acaggaagtg tcacacagca
421 gtcaagtatg tcccaaacgt ctatcaacct ctccacttct
cctacacctg cacagttaat
481 aagccgttcc caggcttcca gttctaccag cggcagtatt
acccaacaga ctatgttact
541 agggagtact tcccctaccc taacggcaag ccaagctcaa
atgtatctcc gagctcaaat
601 gctgattttc acacccgcta ccactgtggc tgctgtacag
tctgacattc ctgttgtctc
661 gtcgtcatcg tcatcttcct gtcagtctgc agctactcag
gttcagaatt taacattacg
721 cagccagaag ttgggtgtat tatctagctc acagaatggt
ccaccaaaaa gcactagtca
781 aactcagtca ttgacaattt gtcataacaa aacaacagtg
accagttcta aaatcagcca
841 acgagatcct tctccagaaa gtaataagaa aggagagagc
ccaagcctgg aatcacgaag
901 cacagctgtc acccggacat caagtattca ccagttaata
gcaccagctt catattctcc
961 aattcagcct cattctctaa taaaacatca gcagattcct
cttcattcac caccttccaa
1021 agtttcccat catcagctga tattacaaca gcagcaacag
caaattcagc caatcacact
1081 tcagaattca actcaagacc cacccccatc ccagcactgt
ataccactcc agaaccatgg
1141 ccttcctcca gctcccagta atgcccagtc acagcattgt
tcaccgattc agagtcatcc
1201 ctctccttta acagtgtctc ctaatcagtc acagtcagca
cagcagtctg tagtggtgtc
1261 tcctccacca cctcattcac caagtcagtc tcctactata
attattcatc cacaagcact
1321 tattcagcca caccctcttg tgtcatcagc tctccagcca
gggccaaatt tgcagcagtc
 
1381 cactgctaat caggtgcaag ctacagcaca gttgaatctt
ccatcccatc ttccacttcc
1441 agcttcccct gttgtacaca ttggcccagt tcagcagtct
gccttggtat ccccaggcca
1501 gcagattgtc tctccatcac accagcaata ttcatccctg
cagtcctctc caatcccaat
1561 tgcaagtcct ccacagatgt cgacatctcc tccagctcag
attccaccac tgcccttgca
1621 gtctatgcag tctttacaag tgcagcctga aattctgtcc
cagggccagg ttttggtgca
1681 gaatgctttg gtgtcagaag aggaacttcc agctgcagaa
gctttggtcc agttgccatt
1741 tcagactctt cctcctccac agactgttgc ggtaaaccta
caagtgcaac caccagcacc
1801 tgttgatcca ccagtggttt atcaggtaga agatgtgtgt
gaagaagaaa tgccagaaga
1861 gtcagatgaa tgtgtccgga tggatagaac cccaccacca
cccactttgt ctccagcagc
1921 tataacagtg gggagaggag aagatttgac ttctgaacat
cctttgttag agcaagtgga
1981 attacctgct gtggcatcag tcagtgcttc agtaattaaa
tctccatcag atcctcaca
2041 tgtttctgtt ccaccacctc cattgttact tccagctgcc
accacaagga gtaacagtac
2101 atctatgcac agtagcattc ccagtataga gaacaaacct
ccacaggcta ttgttaaacc
2161 acagatccta acccatgtta ttgaaggctt tgtgattcag
gagggattgg agccatttcc
2221 tgtgagtcgt tcctctttgc taatagaaca gcctgtgaaa
aaacggcctc ttttggataa
2281 tcaggtgata aattcagtgt gtgttcagcc agagctacag
aataatacaa aacatgcgga
2341 taattcatct gacacagaga tggaagacat gattgctgaa
gagacattag aagaaatgga
 
2401 cagtgagttg ctcaagtgtg aattctgtgg gaaaatggga
tatgctaatg aatttttgcg
2461 gtcaaaacga ttctgcacta tgtcatgtgc caaaaggtac
aatgttagct gttctaaaaa
2521 atttgcactt agtcgttgga atcgtaagcc tgataatcaa
agtcttgggc atcgtggccg
**Figure 9A - continued** 
2581 tcgtccaagt ggccctgatg gggcagcgag agaacatatc
cttaggcagc ttccaattac
2641 ttatccatct gcagaagaag acttggcttc tcatgaagat
tctgtgccat ctgctatgac
2701 aactcgtctg cgcaggcaga gcgagcggga aagagaacgt
gagcttcggg atgtgagaat
2761 tcggaaaatg cctgagaaca gtgacttgct accagttgca
caaacagagc catctatatg
2821 gacagttgat gatgtctggg ccttcatcca ttctttgcct
ggctgccagg atatcgcaga
2881 tgaattcaga gcacaggaga ttgatggaca ggcccttctc
ttgctgaaag aagaccatct
2941 catgagtgca atgaatatca agctaggccc agccctgaag
atctgtgcac gcatcaactc
3001 tctgaaggaa tcttaacagg aacatgaagc cttgataaaa
cagcagtttt acttttctca
3061 caaaaacttg taaggtaaag gcctaacttg gtctagaata
tgacacttat tgtggtggat
3121 agccaagcac attgggatct ccacatcaaa tactgacatt
tcttctacag gtataataat
3181 tcatcatgca ttttcataat taataaacat tggtaaaatt
aattttacag gttacatgaa
3241 acattgaaag acttgttaca gagggccatg atatttttca
aagaaatgtg ttatactaga
 
3301 taattttttt aaaggtgatg tttatcatta atataaagaa
tcctttttaaa
**SEQ ID NO: 28 (HSA320486) (TAG-32)** 
1
MDTEPNPGTSSVSTTTSSTTTTTTITTSSSRMQQPQISVYSGSDRHAVQVIQQALHRPP
SS
61
AAQYLQQMYAAQQQHLMLHTAALQQQHLSSSQLQSLAAVQASLSSGRPSTSPTGSVTQ
QS
121
SMSQTSINLSTSPTPAQLISRSQASSSTSGSITQQTMLLGSTSPTLTASQAQMYLRAQ
ML
181
IFTPATTVAAVQSDIPVVSSSSSSSCQSAATQVQNLTLRSQKLGVLSSSQNGPPKSTS
QT
241
QSLTICHNKTTVTSSKISQRDPSPESNKKGESPSLESRSTAVTRTSSIHQLIAPASYS
PI
301
QPHSLIKHQQIPLHSPPSKVSHHQLILQQQQQIQPITLQNSTQDPPPSQHCIPLQNH
GL
361
PPAPSNAQSQHCSPIQSHPSPLTVSPNQSQSAQQSVVVSPPPPHSPSQSPTIIIHPQA
LI
421
QPHPLVSSALQPGPNLQQSTANQVQATAQLNLPSHLPLPASPVVHIGPVQQSALVSPG
QQ
481
IVSPSHQQYSSLQSSPIPIASPPQMSTSPPAQIPPLPLQSMQSLQVQPEILSQGQVLV
QN
541
ALVSEEELPAAEALVQLPFQTLPPPQTVAVNLQVQPPAPVDPPVVYQVEDVCEEEMPE
ES
 
601
DECVRMDRTPPPPTLSPAAITVGRGEDLTSEHPLLEQVELPAVASVSASVIKSPSDPS
HV
661
SVPPPPLLLPAATTRSNSTSMHSSIPSIENKPPQAIVKPQILTHVIEGFVIQEGLEPF
PV
721
SRSSLLIEQPVKKRPLLDNQVINSVCVQPELQNNTKHADNSSDTEMEDMIAEETLEEM
DS
781
ELLKCEFCGKMGYANEFLRSKRFCTMSCAKRYNVSCSKKFALSRWNRKPDNQSLGHRG
RR
841
PSGPDGAAREHILRQLPITYPSAEEDLASHEDSVPSAMTTRLRRQSERERERELRDVR
IR
901
KMPENSDLLPVAQTEPSIWTVDDVWAFIHSLPGCQDIADEFRAQEIDGQALLLLKEDH
LM
961  SAMNIKLGPALKICARINSLKES
**SEQ ID NO: 29 (NM_003118.1) (TAG-33)** 
1 cgggagagcg cgctctgcct gccgcctgcc tgcctgccac
tgagggttcc cagcaccatg
61 agggcctgga tcttctttct cctttgcctg gccgggaggg
ccttggcagc ccctcagcaa
121 gaagccctgc ctgatgagac agaggtggtg gaagaaactg
tggcagaggt gactgaggta
181 tctgtgggag ctaatcctgt ccaggtggaa gtaggagaat
ttgatgatgg tgcagaggaa
241 accgaagagg aggtggtggc ggaaaatccc tgccagaacc
accactgcaa acacggcaag
301 gtgtgcgagc tggatgagaa caacaccccc atgtgcgtgt
gccaggaccc caccagctgc
 
361 ccagccccca ttggcgagtt tgagaaggtg tgcagcaatg
acaacaagac cttcgactct
421 tcctgccact tctttgccac aaagtgcacc ctggagggca
ccaagaaggg ccacaagctc
481 cacctggact acatcggccc ttgcaaatac atcccccctt
gcctggactc tgagctgacc
541 gaattccccc tgcgcatgcg ggactggctc aagaacgtcc
tggtcaccct gtatgagagg
601 gatgaggaca acaaccttct gactgagaag cagaagctgc
gggtgaagaa gatccatgag
661 aatgagaagc gcctggaggc aggagaccac cccgtggagc
tgctggcccg ggacttcgag
721 aagaactata acatgtacat cttccctgta cactggcagt
tcggccagct ggaccagcac
781 cccattgacg ggtacctctc ccacaccgag ctggctccac
tgcgtgctcc cctcatcccc
841 atggagcatt gcaccacccg ctttttcgag acctgtgacc
tggacaatga caagtacatc
901 gccctggatg agtgggccgg ctgcttcggc atcaagcaga
aggatatcga caaggatctt
961 gtgatctaaa tccactcctt ccacagtacc ggattctctc
tttaaccctc cccttcgtgt
**Figure 9A - continued** 
1021 ttcccccaat gtttaaaatg tttggatggt ttgttgttct
gcctggagac aaggtgctaa
1081 catagattta agtgaataca ttaacggtgc taaaaatgaa
aattctaacc caagacatga
1141 cattcttagc tgtaacttaa ctattaaggc cttttccaca
cgcattaata gtcccatttt
1201 tctcttgcca tttgtagctt tgcccattgt cttattggca
catgggtgga cacggatctg
 
1261 ctgggctctg ccttaaacac acattgcagc ttcaactttt
ctctttagtg ttctgtttga
1321 aactaatact taccgagtca gactttgtgt tcatttcatt
tcagggtctt ggctgcctgt
1381 gggcttcccc aggtggcctg gaggtgggca aagggaagta
acagacacac gatgttgtca
1441 aggatggttt tgggactaga ggctcagtgg tgggagagat
ccctgcagaa tccaccaacc
1501 agaacgtggt ttgcctgagg ctgtaactga gagaaagatt
ctggggctgt cttatgaaaa
1561 tatagacatt ctcacataag cccagttcat caccatttcc
tcctttacct ttcagtgcag
1621 tttcttttca cattaggctg ttggttcaaa cttttgggag
cacggactgt cagttctctg
1681 ggaagtggtc agcgcatcct gcagggcttc tcctcctctg
tcttttggag aaccagggct
1741 cttctcaggg gctctaggga ctgccaggct gtttcagcca
ggaaggccaa aatcaagagt
1801 gagatgtaga aagttgtaaa atagaaaaag tggagttggt
gaatcggttg ttctttcctc
1861 acatttggat gattgtcata aggtttttag catgttcctc
cttttcttca ccctcccctt
1921 tgttcttcta ttaatcaaga gaaacttcaa agttaatggg
atggtcggat ctcacaggct
1981 gagaactcgt tcacctccaa gcatttcatg aaaaagctgc
ttcttattaa tcatacaaac
2041 tctcaccatg atgtgaagag tttcacaaat ctttcaaaat
aaaaagtaat gacttagaaa
2101 ctgaaaaaaa aaaaaaaaaa aaaaaaaaaa aaa
**SEQ ID NO: 30 (NM_003118.1) (TAG-33)** 
1
MRAWIFFLLCLAGRALAAPQQEALPDETEVVEETVAEVTEVSVGANPVQVEVGEFDDG
AE
 
61
ETEEEVVAENPCQNHHCKHGKVCELDENNTPMCVCQDPTSCPAPIGEFEKVCSNDNKT
FD
121
SSCHFFATKCTLEGTKKGHKLHLDYIGPCKYIPPCLDSELTEFPLRMRDWLKNVLVTL
YE
181
RDEDNNLLTEKQKLRVKKIHENEKRLEAGDHPVELLARDFEKNYNMYIFPVHWQFGQL
DQ
241
HPIDGYLSHTELAPLRAPLIPMEHCTTRFFETCDLDNDKYIALDEWAGCFGIKQKDID
KD
301 LVI
**SEQ ID NO: 31 (AF077200) (TAG-38)** 
1 gccagccctc ggaaacgcga agtgagcggc gggtcgact
gacggtaacg gggcagagag
61 gctgttcgca gagctgcgga agatgaatgc cagaggactt
ggatctgagc taaaggacag
121 tattccagtt actgaacttt cagcaagtgg acctttgaa
agtcatgatc ttcttcggaa
181 aggtttttct tgtgtgaaaa atgaactttt gcctagtcat
ccccttgaat tatcagaaaa
241 aaatttccag ctcaaccaag ataaaatgaa tttttccaca
ctgagaaaca ttcagggtct
301 atttgctccg ctaaaattac agatggaatt caaggcagtg
cagcaggttc agcgtcttcc
361 atttctttca agctcaaatc tttcactgga tgttttgagg
ggtaatgatg agactattgg
421 atttgaggat attcttaatg atccatcaca aagcgaagtc
atgggagagc cacacttgat
481 ggtggaatat aaacttggtt tactgtaata gtgtgctgtt
catggaaacc gagggctgca
 
541 tcttgtttat agtcatcttt gtactgtaat ttgatgtaca
caacattaaa agtactgaca
601 cctgaaaaaa aaaaaaaaaa aaaaaaaaaa aaaa
**SEQ ID NO: 32 (AF077200) (TAG-38)** 
1
MNARGLGSELKDSIPVTELSASGPFESHDLLRKGFSCVKNELLPSHPLELSEKNFQLN
QD
61
KMNFSTLRNIQGLFAPLKLQMEFKAVQQVQRLPFLSSSNLSLDVLRGNDETIGFEDIL
ND
121 PSQSEVMGEPHLMVEYKLGLL
**SEQ ID NO: 33 (BC030573.1) (TAG-39)** 
1 gtccccgcgc cagagacgca gccgcgctcc caccacccac
acccaccgcg ccctcgttcg
61 cctcttctcc gggagccagt ccgcgccacc gccgccgccc
aggccatcgc caccctccgc
121 agccatgtcc accaggtccg tgtcctcgtc ctcctaccgc
aggatgttcg gcggcccggg
181 caccgcgagc cggccgagct ccagccggag ctacgtgact
acgtccaccc gcacctacag
241 cctgggcagc gcgctgcgcc ccagcaccag ccgcagcctc
tacgcctcgt ccccgggcgg
**Figure 9A - continued** 
301 cgtgtatgcc acgcgctcct ctgccgtgcg cctgcggagc
agcgtgcccg gggtgcggct
361 cctgcaggac tcggtggact tctcgctggc cgacgccatc
aacaccgagt tcaagaacac
421 ccgcaccaac gagaaggtgg agctgcagga gctgaatgac
cgcttcgcca actacatcga
 
481 caaggtgcgc ttcctggagc agcagaataa gatcctgctg
gccgagctcg agcagctcaa
541 gggccaaggc aagtcgcgcc tgggggacct ctacgaggag
gagatgcggg agctgcgccg
601 gcaggtggac cagctaacca acgacaaagc ccgcgtcgag
gtggagcgcg acaacctggc
661 cgaggacatc atgcgcctcc gggagaaatt gcaggaggag
atgcttcaga gagaggaagc
721 cgaaaacacc ctgcaatctt tcagacagga tgttgacaat
gcgtctctgg cacgtcttga
781 ccttgaacgc aaagtggaat ctttgcaaga agagattgcc
tttttgaaga aactccacga
841 agaggaaatc caggagctgc aggctcagat tcaggaacag
catgtccaaa tcgatgtgga
901 tgtttccaag cctgacctca cggctgccct gcgtgacgta
cgtcagcaat atgaaagtgt
961 ggctgccaag aacctgcagg aggcagaaga atggtacaaa
tccaagtttg ctgacctctc
1021 tgaggctgcc aaccggaaca atgacgccct gcgccaggca
aagcaggagt ccactgagta
1081 ccggagacag gtgcagtccc tcacctgtga agtggatgcc
cttaaaggaa ccaatgagtc
1141 cctggaacgc cagatgcgtg aaatggaaga gaactttgcc
gttgaagctg ctaactacca
1201 agacactatt ggccgcctgc aggatgagat tcagaatatg
aaggaggaaa tggctcgtca
1261 ccttcgtgaa taccaagacc tgctcaatgt taagatggcc
cttgacattg agattgccac
1321 ctacaggaag ctgctggaag gcgaggagag caggatttct
ctgcctcttc caaacttttc
1381 ctccctgaac ctgagggaaa ctaatctgga ttcactccct
ctggttgata cccactcaaa
1441 aaggacactt ctgattaaga cggttgaaac tagagatgga
caggttatca acgaaacttc
 
1501 tcagcatcac gatgaccttg aataaaaatt gcacacactc
agtgcagcaa tatattacca
1561 gcaaaaaaaa aaaaaaaaaa aaaaaaaaaa a
**SEQ ID NO: 34 (BC030573.1) (TAG-39)**

1


61
YATRSSAVRLRSSVPGVRLLQDSVDFSLADAINTEFKNTRTNEKVELQELNDRFANYI
DK
121
VRFLEQQNKILLAELEQLKGQGKSRLGDLYEEEMRELRRQVDQLTNDKARVEVERDNL
AE
181
DIMRLREKLQEEMLQREEAENTLQSFRQDVDNASLARLDLERKVESLQEEIAFLKKLH
EE
241
EIQELQAQIQEQHVQIDVDVSKPDLTAALRDVRQQYESVAAKNLQEAEEWYKSKFADL
SE
301
AANRNNDALRQAKQESTEYRRQVQSLTCEVDALKGTNESLERQMREMEENFAVEAANY
QD
361
TIGRLQDEIQNMKEEMARHLREYQDLLNVKMALDIEIATYRKLLEGEESRISLPLPNF
SS
421 LNLRETNLDSLPLVDTHSKRTLLIKTVETRDGQVINETSQHHDDLE

### Figure 9B

**SEQ ID NO: 35 (see SEQ ID NO:s 1 and 2)** 
TAG ID	TAG-1
Clone ID	TN9H3
Accession No	NM_152862.1
Sequence
GTACGGGAGTTTCTTGGTAAATCCAGAATCAGGATACAATGTCTCTTTGCTATATGAC
CTTGAAAATCTTCCGGCATCCAAGGATTCCATTGTGCATCAAGCTGGCATGTTGAAGC
GAAATTGTTTTGCCTCTGTCTTTGAAAAATACTTCCAATTCCAAGAAGAGGGCAAGGA
AGGAGAGAACAGGGCAGTTATCCATTATAGGGATGATGAGACCATGTATGTTGAGTCT
AAAAAGGACAGAGTCACAGTAGTCTTCAGCACAGTGTTTAAGGATGACGACGATGTGG
TCATTGGAAAGGTGTTCATGCAGGAGTTCAAAGAAGGACGCAGAGCCAGCCACACAGC
CCCACAGGTCCTCTTTAGCCACAGGGAACCTCCTCTGGAGCTGAAAGACACAGACGCC
GCTGTGGGTGACAACATTGGCTACATTNACCTTTGNGCTGTTCCNTNGTACACCANTG
CCAGNGCTNGA
**SEQ ID NO: 36 (see SEQ ID NO:s 1 and 2)** 
TAG ID TAG-2
Clone ID TP8F4
Accession No NM_152862.1
Sequence
GTACAAAATGGAACCAAAACGGAGAATCCCTTAAGAACCTGAAGAGGCGCAACATTAA
AAGCTACGATTATCCAGTAGCAAGTGTTCCAGCCTTCAGTTGCCAGCCGCTTCCTCCT
CTTATTCCCAAGATTAGCGGGATGAAAACGTCTTCCCCGTGATTGTTTTCATTTCTTT
TTTCTCGGCATCTGGGCGTGCGCGGTTCAGCACCTTGAGGAAGTCAGACGTTTTCGCC
CGCATACGTGTGTGAATATAGGCCTTAGAGCACTTGATGTGGTAGTGCAGGTAGTCCC
GGAACGTGTGGATCAGGTTGATGGTGTTGTCTCGAGCACTGGCATTGGTGTGACGAGG
GAACAGCACAAAGGTAATGTAGCCAATGTTGTCACCCACAGCGGCGTCTGTGTCTTTC
AGCTCCAGAGGAGGTTCCCAGTGGCTAAAGANGACCTGTGGGGCTGTGTGGCTGGCTC
TGCGTCCTTCTTTGAACTCCTGCATGAACACCTTTCCATGACCACATCGTCGTCATCC
TTAACACTG
**SEQ ID NO: 37 (see SEQ ID NO:s 3 and 4)** 
TAG ID | TAG-3
Clone ID | TP4G12
Accession No | NM_000611.2
Sequence
AGGGTNGGCCNACAGAGTATAATCAAGGGNTTTTTTTCAACATTAATCAANAAATTCC
TCCATNTGCAAAAGTCAGCCTATGCCATTAAATACAGCCNAGATCATAAAATACAAAN
GNCATTAGGGCTACTGAATCTTGANATTCATTCACTAAAATACCCNGGGAAAAGGAAA
AAGTTTGCCCTTGNGAAAGGGGGNATCATTTTTTAAAAAANCTTTTGAAAGCCNCAA
ACATCTGGGAGNGGGCTAAAAGCCGNTACAAAGGNATTTTATACAATCTATGCTGAGT
CAGCTAGGAGTTAACAGGAGGCTGGATGCNNATGGNAAAANATACCCCCGCGTAC
**SEQ ID NO: 38 (see SEQ ID NO:s 5 and 6)** 
TAG ID TAG-4
Clone ID TP4D4
Accession No NM_004642.2
Sequence
GTACGCGGGGAGCAAGAGTGCCATGGAGAGGCTGAAGCGCGGCATCATTCACGCTAGA
GGACTGGTTCGGGAGTGCTTGGCAGAAACGGAACGGAATGCCAGATCCTAGCTGCCTT
GTTGGTTTTGAAGGATTTCCATCTTTTTACAAGATGAGAAGTTACAGTTCATCTCCCC
TGTTCAGATGAAACCCTTGTTTTCAAAATGGTTACAGTTTCGTTTTTCCTCCCATGGT
TCACTTGGCTCTGAACCTACAGTCTCAAAGATTGAGAAAAGATTTTGCAGTTAATTAG
GATTTGCATTTTAAGTAGTTAGGAACTGCCCAGGTTTTTTTTGTTTTTTAAGCATTGA
TTTAAAAGATGCACGGAAAGTTATCTTACAGCAAACTGTAGTTTGCCTCCAAGACACC
ATTGTCTCCCTTTAATCTTCTCTTTTGNATACATTTGGTAC
**Figure 9B - continued**
**SEQ ID NO: 39 (see SEQ ID NO:s 7 and 8)** 
TAG ID | TAG-5
Clone ID | TN4C4
 
Accession No NM_000088.2

Sequence

AGAGTCTTTTGCTTCCTCCCACCCCTAGGGGGAAAAACTGCTTTGTGCTTTGGGAAGT

TGTCTCTGAAACCCGGGGACAGAGGACGCAGGACAGACTAGGAGGGAGCCGGGAGGAT

GGGCTGCAGCTGTGGAGGAGGGTTTCAGAGGAGAGAGGTCGGAGAGCAGAGGCCTGAG

AAGCCAGAGGCAGGTGGAGAGAGGGTGGAAAGTGAGCAGCGGGCTGGGCTGGAGCCGC

ACACGCTCTCCTCCCATGTTAAATAGCACCTTTAGAAAAATTCACAAGTCCCCATCC
**SEQ ID NO: 40 (see SEQ ID NO:s 7 and 8)** 
TAG ID | TAG-6
Clone ID | TN2D7
Accession No | NM_000088.2
Sequence
ACAGCTATGACCTGATTACGCCAAGCTTGGTACCGAGCTCGGATCCACTAGTAACGGC
CGCCAGTGTGCTGGAATTCGCCCTTAGCGGCCGCCCGGGCAGGTACAGAGTCTTTTGC
TTCCTCCCACCCCTAGGGGGAAAAACTGCTTTGTGCTTTGGGAAGTTGTCTCTGAAAC
CCGGGGACAGAGGACGCAGGACAGACTAGGAGGGAGCCGGGAGGATGGGCTGCAGCTG
TGGAGGAGGGTTTCAGAGGAGAGAGGTCGGAGAGCAGAGGCCTGAGAAGCCAGAGGCA
GGTGGAGAGAGGGTGGAAAGTGAGCAGCGGGCTGGGCTGGAGCCGCACACGCTCTCCT
CCCATGTTAAATAGCACCTTTAGAAAAATTCACAAGTCCCCATCC
**SEQ ID NO: 41 (see SEQ ID NO:s 7 and 8)** 
TAG ID TAG-24
Clone ID TN4B8
Accession No NM_000088.2
AGANTCTTTTGCTTCCTCCCACCCCTANGGGAAAAACTGCTTTGTGCTTTGGGAAGT
TGTCTCTGAAACCCGGGGACAGAGGACGCAGGACAGACTAGGAGGGAGCCGGGAGGAT
GGGCTGCAGCTGTGGAGGAGGGTTTCAGAGGAGAGAGGTCGGAGAGCAGAGGCCTGAG
AAGCCAGAGGCAGGTGGAGAGANGGTGGAAAGTGANCANCGGGCTGG
**SEQ ID NO: 42 (see SEQ ID NO:s 7 and 8)** 
TAG ID TAG-26
Clone ID TN7F10
Accession No NM_000088.2
AGANTCTTTTGCTTCCTCCCACCCCTANGGGAAAAACTGCTTTGTGCTTTGGGAAGT
TGTCTCTGAAACCCGGGGACAGANGACGCAGGACAGACTAGGANGGAGCCGGGANGAT
GGGCTGCAGCTGTGGAGGANGGTTTCAGANGAGANANGTCGGAGANCAGAGGCCTGAG
AAGCCAGANGCAGGTGGAGAGANGGTGGAAAGTGAGCAGCGGGCTGGGCTGGAGCCGC
ACACGCTCTCCTNCCATGTTAAATAGCACCTTTANAAAA
**SEQ ID NO: 43 (see SEQ ID NO:s 9 and 10)** 
TAG ID TAG-7
Clone ID TP6B10
Accession No AL390755.5 (cf NM_001845.2
?)
Sequence
GCGGGAGGGCAGATTCGGACCACTAGGCCTGAAATGACATTTCACTAAAAGTCTCCAA
AACATTTCTAAGACTACTAAGGCCTTTTATGTAATTTCTTTAAATGTGTATTTCTTAA
GAATTCAAATTTGTAATAAAACTATTTGTATAAAAATTAAGCTTTTATTAATTTGTTG
CTAGTATTGCCACAGACGCATTAAAAGAAACTTACTGCACAAGCTGCTAATAAATTTG
TAAGCTTTGCATACCTTAGATTA
**Figure 9B - continued**
**SEQ ID NO: 44 (see SEQ ID NO:s 9 and 10)** 
TAG ID TAG-8
Clone ID TN3H4
Accession No NM_001845.2
GCGGGAGGGCAGATTCGGACCACTAGGCCTGAAATGACATTTCACTAAAAGTCTCCAA
AACATTTCTAAGACTACTAAGGCCTTTTATGTAATTTCTTTAAATGTGTATTTCTTAA
GAATTCAAATTTGTAATAAAACTATTTGTAT
**SEQ ID NO: 45 (see SEQ ID NO:s 9 and 10)** 
TAG ID TAG-9
Clone ID TN9G4
Accession No NM_001845.2
GGAGGGCAGATTCGGACCACTAGGCCTGAAATGACATTTCACTAAAAGTCTCCAAAAC
ATTTCTAAGACTACTAAGGCCTTTTATGTAATTTCTTTAAATGTGTATTTCTTAAGAA
TTCAAATTTGTAATAAAACTATTTGTAT
**SEQ ID NO: 46 (see SEQ ID NO:s 9 and 10)** 
TAG ID TAG-10
Clone ID TP11A11
Accession No NM_001845.2
AAGGTATGCAAAGCTTACAAATTTATTAGCAGCTTGTGCAGTAAGTTTCTTTTAATGC
GTCTGTGGCAATACTAGCAACAAATTAATAAAAGCTTAATTTTTATACAAATAGTTTT
ATTACAAATTTGAATTCTTAAGAAATACACATTTAAAGAAATTACATAAAAGGCCTTA
GTAGTCTTAGAAATGTTTTGGAGACTTTTAGTGAAATGTCATTTCAGGCCTAGTGGTC
CGAATCTGCCCTCC
**SEQ ID NO: 47 (see SEQ ID NO:s 9 and 10)** 
TAG ID TAG-11
Clone ID R2A4
Accession No NM_001845.2
GCAAAGCTTACAAATTTATTANCAGCTTGNGCAGTAAGTTTCTTTTAATGCGTCTGNG
GCAATACTAGCAACAAATTAATAAAAGCTTAATTTTTATACAAATAGTTTTATTACAA
ATTTGAATTCTTAAGAAATACNCATTTAAAGAAATTACATAAAAGGCCTTANTAGTCT
TAAAAATGNTTTGGAGACTTTTANTGAAATGNCATTTCAGGCCTAGTGGNCCGAATCT
GCCCTCCTGCGGNCCATGCGATGCCCTGCTGAGGNCTGTGAACACAGCTCATGANAAA
CCACGGAAATGGCCCGAATGNGCTTACGTGNGAAAATACTGATACTGGGATTCAACAG
AGCTGTTTTTCAAGCCAGGATGCAGAATGAGGAATACTAATGAAATGACGGCCTTTAA
GGGTGTTGCTTTTGAAGTCAAGTCATTCAGTTTGNGATTAGTGNTTAAAACCCTGAAA
ATATTTAATACNGAATAAAA
**SEQ ID NO: 109 (see SEQ ID NO:s 9 and 10)** 
TAG ID TAG-12
Clone ID TN2G6
Accession No
GGAGGGCAGATTCGGACCACTAGGCCTGAAATGACATTTCACTAAAAGTCTCCAAAAC
ATTTCTAAGACTACTAAGGCCTTTTATGTAATTCCTTTAAATGTGTATTTCTTAAGAA
TTCAAATTTGTAATAAAACTATTTGTATAAAAATTAAGCTTTTATTAATTTGTTGCTA
GTATTGCCACAGACGCATTAAAAGAAACTTACTGCACAAGCTGCT
**SEQ ID NO: 48 (see SEQ ID NO:s 9 and 10)**
**Figure 9B - continued** 
GGAGGGCAGATTCGGACCACTAGGCCTGAAATGACATTTCACTAAAAGTCTCCAAAAC
ATTTCTAAGACTACTAAGGCCTTTTATGTAATTTCTTTAAATGTGTATTTCTTAAGAA
TTCAAATTTGTAATAAAACTATTTGTATAAAAATTAAGCTTTTATTAATTTGTTGCTA
GTATTGCCACAGACGCATTAAAAGAAACTTACTGCACAAGCTGCTAATAAATTTGTAA
GCTTTGCATACCTT
**SEQ ID NO: 49 (see SEQ ID NO:s 9 and 10)** 
TAG ID TAG-14
Clone ID TN7H6
Accession No NM_001845.2
GCAAAGCTTACAAATTTATTAACAGCTTGGGCAGTAAGTTTCTTTTAATGCGTCTGNG
GNAATACTAGCAACAAATTAATAAAAGCTTAATTTTTATACNCATAGTTTTATTACAA
ATTTGAATTCTTAANAAATACNCATTTAAAGAAATTACNTAAAAGGNCTTANTAGTCT
 
TAG ID | TAG-13
Clone ID | TN7B4
Accession No | NM_001845.2
 
TAAAAATGNTTTGGANACTTTTANTGAAATGNCATTTCAGGCCTANNGGNCCGAATCT
GCCCTCC
**SEQ ID NO: 50 (see SEQ ID NO:s 9 and 10)** 
TAG ID TAG-15
Clone ID TP4E5
Accession No NM_001845.2
GGAGGGCAGATTCGGACCACTAGGCCTGAAATGACATTTCACTAAAAGTCTCCAAAAC
ATTTCTAAGACTACTAAGGCCTTTTATGTAATTTCTTTAAATGTGTATTTCTTAAGAA
TTCAAATTTGTAATAAAACTATTTGTATAAAAATTAAGCTTTTATTAATTTGTTGCTA
GTATTGCCACAGACGCATTAAAAGAAACTTACTGCACAAGCTGCTAATAAATTTGTAA
GCTTTGCATACCTT
**SEQ ID NO: 51 (see SEQ ID NO:s 9 and 10)** 
TAG ID TAG-16
Clone ID TP4F12
Accession No NM_001845.2
GCGGGAGGGCAGATTCGGACCACTAGGCCTGAAATGACATTTCACTAAAAGTCTCCAA
AACATTTCTAAGACTACTAAGGCCTTTTATGTAATTTCTTTAAATGTGTATTTCTTAA
GAATTCAAATTTGTAATAAAACTATTTGTATAAAAATTAAGCTTTTATTAATTTGTTG
CTAGTATTGCCACAGACGCATTAAAAGAAACTTACTGCACAAGCTGCTAATAAATTTG
TAAGCTTTGC
**SEQ ID NO: 52 (see SEQ ID NO:s 9 and 10)** 
TAG ID TAG-17
Clone ID TP7C9
Accession No NM_001845.2
GTACGCGGGAGGGCAGATTCGGACCACTAGGCCTGAAATGACATTTCACTAAAAGTCT
CCAAAACATTTCTAAGACTACTAAGGCCTTTTATGTAATTTCTTTAAATGTGTATTTC
TTAAGAATTCAAATTTGTAATAAAACTATTTGTATAAAAATTAAGCTTTTATTAATTT
 
GTTGCTAGTATTGCCACAGACGCATTAAAAGAAACTTACTGCACAAGCTGCTAATAAA
TTTGTAAGCTTTGCATACCTTA
**SEQ ID NO: 53 (see SEQ ID NO:s 9 and 10)** 
TAG ID | TAG-18
Clone ID | TP11B8
Accession No | NM_001845.2
GCGGGAGGGCAGATTCGGACCACTAGGCCTGAAATGACATTTCACTAAAAGTCTCCAA
AACATTTCTAAGACTACTAAGGCCTTTTATGTAATTTCTTTAAATGTGTATTTCTTAA
GAATTCAAATTTGTAATAAAACTATTTGTATAAAAA
**Figure 9B - continued** 
TTAAGCTTTTATTAATTTGTTGCTAGTATTGCCACAGACGCATTAAAAGAAACTTACT
GCACAAGCTGCTAATAAATTTGTAAGCTTTGCATA
**SEQ ID NO: 54 (see SEQ ID NO:s 9 and 10)** 
TAG ID TAG-19
Clone ID TP11A4
Accession No NM_001845.2
GCGGGAGGGCAGATTCGGACCACTAGGCCTGAAATGACATTTCACTAAAAGTCTCCAA
AACATTTCTAAGACTACTAAGGCCTTTTATGTAATCTCTTTAAATGTGTATTTCTTAA
GAATTCAAATTTGTAATAAAACTATTTGTATAAAAATTAAGCTTTTATTAATTTGTTG
CTAGTATTGCCACAGACGCATTAAAAGAAACTTACTGCACAAGCTGCTAATAAATTTG
TAAGCTTTGCATACCTT
**SEQ ID NO: 55 (see SEQ ID NO:s 9 and 10)** 
TAG ID | TAG-20
Clone ID | TN7A4
Accession No | NM_001845.2
 
GGAGGGCAGATTCGGACCACTAGGCCTGAAATGACATTTCACTAAAAGTCTCCAAAAC
ATTTCTAAGACTACTAAGGCCTTTTATGTAATTTCTTTAAATGTGTATTTCTTAAGAA
TTCAAATTTGTAATAAAACTATTTGTATAAAAATTAAGCTTTTATTAATTTGTTGCTA
GTATTGCCACAGACGCATTAAAAGAAACTTACTGCACAAGCTGCTAATAAATTTGTAA
GCTTTGCATACCTT
**SEQ ID NO: 56 (see SEQ ID NO:s 9 and 10)** 
TAG ID TAG-22
Clone ID TP5D8
Accession No NM_001845.2
GGAGGGCAGATTCGGACCACTAGGCCTGAAATGACATTTCACTAAAAGTCTCCAAAAC
ATTTCTAAGACTACTAAGGCCTTTTATGTAATTTCTTTAAATGTGTATTTCTTAAGAA
TTCAAATTTGTAATAAAACTATTTGTATAAAAATTAAGCTTTTATTAATTTGTTGCTA
GTATTGCCACAGACGCATTAAAAGAAACTTACTGCACAAGCTGCTAATAAATTTGTAA
GCTTTGC
**SEQ ID NO: 57 (see SEQ ID NO:s 11 and 12)** 
TAG ID TAG-21
Clone ID TP8G5
Accession No BC003378
Sequence
GGCACTGGGCGACTCTGTGGCTCGCTGAGGAAAAAAAGAACTAAACATGGGCAAAGGA
GATCTAGGGAAGCTGAGAGGTAAAATGTGATATGCATTCTTTGTCCACACTTGTTGGG
AGGAGCACAAGAAGAAGCACCCAGATGCTCAGTCAGCTCCTCAGAGTTTTCTAAGAAA
TGCTCAGAGAGGTGCAAGACCATGTCTGCTAAAGAGAAAGTGAGATTTGAAGACATGT
CAAAGATGGACAAGACCCATTATGAAAGAGAAATGAAAACCTATATCCTTCCTAAATG
GGAGACAAAAAAGAAGTTCGAGGATCCCAATGCACCCAAGAGGCCTCCTTCGGCCTTC
TTCCTCTTCTGCTCTGAGTATCGCCCAAAAATCAAAGGAGAACATCCTGGCCTGTCCA
TTGGTGATGTTGCGAAAAACTGGGAGAGATGTGGAATAACACTGCTGCAGACGACAGC
AGCCTTATGAAA
**SEQ ID NO: 58 (see SEQ ID NO:s 13 and ??)**
**Figure 9B - continued** 
GTACAAATGATTTTATATCTTAACTGGTAATATATTAAATATGTTATAAAATTAGTTC
AAGAAGCATAAAACACCCTAACTCTCATGAACACAGAAATTCAAGCAAAAGAATATGC
TTAAACAAAAACTAAAGCACACAGCAAACGTAAATTGTTGGTCAAATCTTTTCATAGT
GTTATAAGTTACATTTGTGAAATTTAGGGTAGTTGAAGAGCTTCCTCAATAACAGCAA
GAGGTTTTACATTTATTACCTTTATAGAGTAGGTA
**SEQ ID NO: 59 (see SEQ ID NO:s 15 and 16)** 
TAG ID TAG-25
Clone ID TP8H11
Accession No NM_014571
Sequence
TCGTTGGTAAATACCTACTCCCCCAAGTGACTCCAGGTGCCCCCACCGCCTGGCACT
TCCCCCAGGACTCCTACGATCTGGTTACTGCCTGGCCGATCCAAGGCTGTGGAGTCCC
AGAGCCAGCAGTTCACTGGTGCTCATTCCACACTGGTTAGATACTTCAGTTGTCACCC
CTGGGAAGATTCTCCCACCTCCTCCCTTTGATGGAACCACCCTCCCCAGAGGCTGCAT
TGAGGAGACTCCACAGACTGAAAAGTGAGTTTGCAGAAACCTTGGGGAAAAGGGCCCT
TTCAAAGAAGTGGATAAGAGGGAGGAGATCATTGAGTGACCCAGAAAGCTCTTTTGAA
AAGACAGACTCCTCAAGGAGAGATAAAGAGGAAAGCACCTCTTTCATTTTTTAGTGTG
AGCTAATTCCATCAGACTGCTGTCCTCCTGGACCCATCTGAGATGTGCAGTANCANGG
AGAGGGGGATCA
SEQ ID NO: 60 (see SEQ ID NO:s 17 and 18) 
TAG ID | TAG-27
Clone ID | TN3H10
Accession No | NM_017994.1
 
TAG ID | TAG-23
Clone ID | R1C6
Accession No | BC041913
Sequence
 
Sequence

CCAAGATTACAATCCTTTCTGGTGTTATAAGGGGCCATTGGAAAAGTCTATCATGCT

TTAAATCCCAAGCTTACAGTGATTGTTCCAGATGATGACCGTTCATTAATAAATTTGC

ATCTCATGCACACCAGTTACTTCCTCTTTGTGATGGTGATAACAATGTTTTGCTATGC

TGTTATCAAGGGCAGACCTAGCAAATTGCGTCAGAGCAATCCTGAATTTTGTCCCGAG

AAGGTGGCTTTGGCTGAAGCCTAATTCCACAGCTCCTTGTTTTTGAGAGAGACTGAG

AGAACCATAATCCTTGCCTGCTGAACCCAGCCTGGGCCTGGATGCTCTGTGAATACAT

TATCTTGCGATGTTGGGTTATTCCAGCCAAAGACATTTCAGGNGCCTGTAACTGATT
**SEQ ID NO: 61 (see SEQ ID NO:s 19 and 20)** 
TAG ID TAG-28
Clone ID TP6H4
Accession No X02160
Sequence
CCTANCTTGGTTTGGTCTTGAAAANATTCATAATCACTCCAAATGAAATGCTCCTCCC
TTGGCCACCAATGNGAAGGGAGGGTAAAAACCTGAGGCTANACTTNTGACACAANAAA
AATNTGTCGANAGCACAGTCTCCCAGTCAATAAAAAGGAAGGANANAGGGGGATGANC
TCNCACCCTTGAAAAAAACCTTNATGAGCCAATTCCCAAAGCATCAACTCCGCATGGA
TACTTTGCACACACATCAGCCGNGTNTAANGGACACACACACGTGCATACNCACGTGA
GCACACNCCG
**SEQ ID NO: 62 (see SEQ ID NO:s 21 and 22)** 
TAG ID TAG-29
Clone ID R5D7
Accession No BC017201
Sequence
GATAAAAAGTATTTTATTTGNTTAATGATATGCATGCTTTTCTTCTGTAAATATATAA
TAAATTTTTGTAGATAGTCTTGATGNGNGATCTTTATTTTGTATTTCTCTGGGNAAAA
CCAGNGAATATAACTAAAGNGNTAGTGGATTGGATTAAAAGAAACTTATTAGGCAAGA
ACAGGNAATGNAGTTATCCATGACTACTTTTAACCATGCAGACTAATAATATTCTGGA
GGNTTATAGCTCGGCACCTTCACCTTTTTTCACTGGNATTTCATGNAAGGCATCAACC
 
ACTGNAATTTTTGCTGATGCTGAAGCCTGNCCTTGGGAATTGGATGCATGGCACTCAT
ATTCTCCAGCATCTTCCTTACTTAGAGGAGAT
**Figure 9B - continued** 
ACCAGCACCCAGCCAGTTACTTCATGCTTTTCTGGGCCACCCCGGGTCTGAATGGGCC
AGGTTGTCCCGGNCCCANGCAGGAGTTCTGN
**SEQ ID NO: 63 (see SEQ ID NO:s 23 and 24)** 
TAG ID TAG-30
Clone ID H10F7
Accession No NM_002300
Sequence
ATGGATGAAAACTAAAGGCTCGAGTTAATCACATTGTAGTTTTTAAATTTCTACAGCC
TAGAGCTCACTAGTCACAGGTCTTTTAGGTCCTTCTGGATGTCCCACAGGGTATCTGC
ACTTTTCTTGAGCTGAGCAACCTCATCATCCTTTAGCTTCTGGTTGATAACGCTGGTT
AATCCCCGGGCATTGAGGATACATGGAAGGCTCAGGAAGACTTCATTCTCAATGCCAT
ACATCCCCTTTACCATTGTTGACACGGGATGAATCCTGGATAGATTTTTCAACATGGA
TTCAATAAGATCAGCCACACTTAATCCAATAGCCCAGTTGGTATATCCTTTTAGCTTG
ATGACTTCATAGGCACTTTCAACCACCATCTTATGCACTTCCTTCCAATTTTCACTAT
CATTGNCAGTTCCCATTTCTGGACTCAATTCCTGGAGAGAAACACCTGCCNCATTCC
**SEQ ID NO: 64 (see SEQ ID NO: 25 and ??)** 
TAG ID TAG-31
Clone ID TN2F2
Accession No CV337076
Sequence
GTTCATGCAGCCTGTGATTCATAGCTTCCCTGGGGTGTTGGGGAGAATCACATTTGGG
TCAGCCAGGTTTAGCACTGACAGTTTTGTCTTTAGAATCAAGCAGATGTGGAATCAAA
TCTGGCTGTATCCATGACCAGCTCTGAAGCCATGAGTGGGTTACATAGCTTTAGGGCC
TCAGCATACTCATCTGGAAAGTGGAAGTGATCATGTCTATTTTGCAGAGTTGTTGCCA
CTTTTCCTCTCTGGACCCCACTTTCCCCATCTGTCAGATGAAAGAGTGGGATGAGATG
GCCTGTCTATTTATCTCTCAATCACAATGGCTCTATTTGAAAAAGTTTGAACTGCCC
 
TAAGTGTTAAAAAGGAAGATGGGGAGCCATCAAGACAAACTTAGGCCTACATTACCAT
CGAGTTCAGAGAATGGCAGACCGGAAGCACCAGCCA
**SEQ ID NO: 65 (see SEQ ID NO:s 27 and 28)** 
TAG ID TAG-32
Clone ID TP5E6
Accession No HSA320486(AJ320486)
Sequence
GCGGGAAAGAGAACGTGAGCTTCGGGATGTGAGAATTCGGAAAATGCCTGAGAACAGT
GACTTGCTACCAGTTGCACAAACAGAGCCATCTATATGGACAGTTGATGATGTCTGGG
CCTTCATCCATTCTTTGCCTGGCTGCCAGGATATCGCAGATGAATTCAGAGCACAGGA
GATTGATGGACAGGCCCTTCTCTTGCTGAAAGAAGACCATCTCATGAGTGCAATGAAT
ATCAAGCTAGGCCCAGCCCTGAAGATCTGTGCACGCATCAACTCTCTGAAGGAATCTT
AACAGGAACATGAAGCCTTGATAAAACGGCAGTTTTACTTTTCTCACAAAAACTTGTA
AGGTAAAGGCCTAACTTGGTCTAGAATATGACACTTATTGTGGTGGATAGCCAAGCAC
ATTGGGATCTCCACATCAAATACTGACATTTCTTCTACAGGTATAATAATTCATC
**SEQ ID NO: 66 (see SEQ ID NO:s 29 and 30)** 
TAG ID TAG-33
Clone ID TP4E12
Accession No NM_003118.1
Sequence
CGGATTCTCTCTTTAACCCTCCCCTTCGTGTTTCCCCCAATGTTTAAAATGTTTGGAT
GGTTTGTTGTTCTGCCTGGAGACAAGGTGCTAACATAGATTTAAGTGAATACATTAAC
GGTGCTAAAAATGAAAATTCTAACCCAAGACATGACATTCTTAGCTATAACTTAACTA
TTAAGGCCTTTTCCACACGCATTAATAGTCCCATTTTTCTCTTGCCATTTGTAG
**Figure 9B - continued** 
CTTTGCCCATTGTCCTATTGGCACATGGGTGGACACGGATCTGCTGGGCTCTGCCTTA
AACACACATTGCAGCTTCAACTTTTCTCTTTAGTGTTCTGTTTGAAACTAATACTTAC
CGAGTCAGACTTTGTGTTCATTTCATTTCANGGTCTTGGCTGCCTGTGGGCTTCCCCA
 
GGTGGCCTGGAGGTGGGCAAAGGGAAGTAACAGACACACGATGTTGTCAAGGATGGTT
TTGGGACTAGANGCTCAGTGGTGGGAGAGATCCCTGCAGAANCCACC
**SEQ ID NO: 67 (see SEQ ID NO:s 29 and 30)** 
TAG ID | TAG-34
Clone ID | TP6C9
Accession No | NM_003118.1
Sequence
GTACATGTTATAGTTCTTCTCGAAGTCCCGGGCCAGCAGCTCCACGGGGTGGTCTCCT
GCCTCCAGGCGCTTCTCATTCTCATGGATCTTCTTCACCCGCAGCTTCTGCTTCTCAG
TCAGAAGGTTGTTGTCCTCATCCCTCTCATACAGGGTGACCAGGACGTTCTTGAGCCA
GTCCCGCATGCGCAGGGGGAATTCGGTCAGCTCAGAGTCCAGGCAAGGGGGGATGTAT
TTGCAAGGCCCGATGTAGTCCAGGTGGAGCTTGTGGCCCTTCTTGGTGCCCTCCAGGG
TACACTTTGTGGCAAAGAAGTGGCAGGAAGAGTCGAAGGTCTTGTTGTCATTGCTGCA
CACCTTCTCAAACTCGCCAATGGGGGCTGGGCAGCTGGTGGGGTCCTGGCACACGCAC
ATGGGGGTGTTGTTCTCATCCAGCTCGCACACCTTGCCGTGTTTGCAGTGGTGGTTCT
GGCAGGGATTTTCCGCCACCACCTCCTNTTNGGTTTCCTCTGCACATNATNAAATTNT
NCTANTTNCACCTGGANAGGA
**SEQ ID NO: 68 (see SEQ ID NO:s 29 and 30)** 
TAG ID | TAG-35
Clone ID | TP9C10
Accession No | NM_003118.1
Sequence
GGGCTTCTCCTCCTCTGTCTTTTGGAGAACCAGGGCTCTTCTCAGGGGCTCTAGGGAC
TGTCAGGCTGTTTCAGCCAGGAAGGCCAAAATCAAGAGTGAGATGTAGAAAGTTGTAA
AATAGAAAAAGTGGAGTTGGTGAATCGGTTGTTCTTTCCTCACATTTGGATGATTGTC
ATAAGGTTTTTAGCATGTTCCTCCTTTTCTTCACCCTCCCCTTTTTTCTTCTATTAAT
CAAGAGAAACTTCAAAGTTAATGGGATGGTCGGATCTCACAGGCTGAGGACTCGTTCA
CCTCCAAGCATTTCATGAAAAAGCTGCTTCTTATTAATCATACAAACTCTCACCATGA
TGTGAAGAGTTTCACAAATCTTTCAAAATAAAAAGTAATGACTTAG
**SEQ ID NO: 69 (see SEQ ID NO:s 29 and 30)** 
TAG ID TAG-36
Clone ID TN2F7
Accession No NM_003118.1
Sequence
CTGTCTTATGAAAATATAGACATTCTCACATAAGCCCAGTTCATCACCATTTCCTCCT
TTACCTTTCAGTGCAGTTTCTTTTCACATTAGGCTGTTGGTTCAAACTTTTGGGAGCA
CGGACTGTCAGTTCTCTGGGAAGTGGTCAGCGCATCCTGCAGGGCTTCTCCTCCTCTG
TCTTTTGGAGAACCAGGGCTCTTCTCAGGGGCTCTAGGGACTGCCAGGCTGTTTCAGC
CAGGAAGGCCAAAATCAAGAGTGAGATGTAGAAAGTTGTAAAATAGAAAAAGTGGAGT
TGGTGAATCGGTTGTTCTTTCCTCACATTTGGATGATTGTCATAAGGTTTTTAGCATG
TTCCTCCTTTTCTTCACCCTCCCCTTTTTTCTTCTATTAATCAAGAGAAACTTCAAAG
TTAATGGGATGGTCGGATCTCACAGGCTGAGAACTCGTTCACCTCCAAGCATTTCATG
AAAAAGCTGCTTCTTATTAATCATACAACTCTCACCATGATG
**SEQ ID NO: 70 (see SEQ ID NO:s 29 and 30)** 
TAG ID | TAG-37
Clone ID | TP8E11
Accession No | NM_003118.1
Sequence
CGGATTCTCTCTTTAACCCTCCCCTTCGTGTTTCCCCCAATGTTTAAAATGTTTGGAT
GGTTTGTTGTTCTGCCTGGAGACAAGGTGCTAACATAGATTTAAGTGAATACATTAAC
GGTGCTAAAAATGAAAATTCTAACCCAAGACATGAC
**Figure 9B - continued** 
ATTCTTAGCTGTAACTTAACTATTAAGGCCTTTTCCACACGCATTAATAGTCCCATTT
TTCTCTTGCCATTTGTAGCTTTGCCCATTGTCTTATTGGCACATGGGTGGACACGGAT
CTGCTGGGCTCTGCCTTAAACACACATTGCAGCTTCAACTTTTCTCTTTAGTGTTCTG
TTTGAAACTAATACTTACCGAGTCAGACTTTGNGTTCATTTCATTTNAGGGTCTGGCT
GNCTGGGGGCTTCCCCAGGGGGCCTGGAGG
**SEQ ID NO: 71 (see SEQ ID NO:s 31 and 32)** 
TAG ID TAG-38
Clone ID H10E4
Accession No AF077200
Sequence
AAAGATGACTATAAACAAGATGCAGCCCTCGGTTTCCATGAACAGCACACTATTACAG
TAAACCAAGTTTATATTCCACCATCAAGTGTGGCTCTCCCATGACTTCGCTTTGTGAT
GGATCATTAAGAATATCCTCAAATCCAATAGTCTCATCATTACCCCTCAAAACATCCA
GTGAAAGATTTGAGCTTGAAAGAAATGGAAGACGCTGAACCTGCTGCACTGCCTTGAA
TTCCATCTGTAATTTTAGCGGAGCAAATAGACCCTGAATGTTTCTCAGTGTGGAAAAA
TTCATTTTATCTTGGTTGAGCTGGAAATTTTTTTCTGATAATTCAAGGGGATGACTAG
GCAAAAGTTCATTTTTCACACAAGAAAAACCTTTCCGAAGAAGATCATGACTTTCAAA
AGGTCCACTTGCTGAAAGTTCAGTAACTGGAATACTGTCCTTTANCTCAGATCCAAGT
CCTCTGGCATTCATCTCCCGC
**SEQ ID NO: 72 (see SEQ ID NO:s 33 and 34)** 
TAG ID	TAG-39
Clone ID	TN1A12
Accession No	BC030573.1
Sequence
TTGCNGGNAATATATTGCTGNACTGNGNGTGNGCNANNNTTATTCAAGGNCATCGTGA
TGCTGAGAAGTTNCCNTGATAACCTGNCCATCTCTAGTTTCAACCGTCTTAATCAAAA
GTGTCCTTTTTGAGNGGGTATCAACCAAAGGAGTGAATCCANATTANTTTCCCTCAG
GTTCAGGGAGGAAAAGTTTGGAAGAGGCAGAAAAATCCTGGTCTCCTCGCCTTCCANC
AGCTTCCTGNAGGNGGCAATCTCAATGNCAAGGGCCATCCTTAACATNGANCAGGTCT
TGGTATTCACGAAGGNGACGAGCCATTTCCTCCTTCATATTCTGAANCTCATCCTGCA
GGCNGNCAATAGTG
**SEQ ID NO: 73 (see SEQ ID NO:s 33 and 34)** 
TAG ID | TAG-40
Clone ID | H6C5
Accession No | BC030573.1
 
Sequence

AATATATTGCTGNACTGNGNGTGNGCNANTTTTATTCAAGGNCATCGTGATGCTGAGA

AGTTNCCNTGATAACCTGNCCATCTCTAGTTTCAACCGTCTTAATCAAAAGTGTCCTT

TTTGAGNGGGTATCAACCAAAGGGAGTGAATCCANATTANTTTCCCTCAGGTTCAGGG

AGGAAAAGTTTGGAAGAGGCAGAAAAATCCTGGTCTCCTCGCCTTCCANCAGCTTCCT

GNAGGNGGCAATCTCAATGNCAAGGGCCATCCTTAACATNGANCAGGTCTTGGTATTC

ACGAAGGNGACGAGCCATTTCCTCCTTCATATTCTGAANCTCATCCTGCAGGCNGNCA

ATAGTGNCTTGGNAGATACCAGATTCTACGGGAAAGTTCTNTTTCATTNNACNCATNN

GGNGGNCCAGGGACTCATNGGTTNCNTTAANGGCATCC
**SEQ ID NO: 74 (see SEQ ID NO:s 33 and 34)** 
TAG ID TAG-41
Clone ID R3C5
Accession No BC030573.1
Sequence
TGCGTGAAATGGAAGAGAACTTTGCCGTTGAAGCTGCTAACTACCAAGACACTATTGG
CCGCCTGCAGGATGAGATTCAGAATATGAAGGAGGAAATGGCTCGTCACCTTCGTGAA
TACCAAGACCTGCTCAATGTTAAGATGGCCCTTGACATTGAGATTGCCACCTACAGGA
AGCTGCTGGAAGGCGAGGAGAGCAGGATTTCTCTGCCTCTTCCAAACTTTTCCT
**Figure 9B - continued**

CCCTGAACCTGAGGGAAACTAATCTGGATTCACTCCCTCTGGTTGATACCCACTCAAA
AAGGACACTTCTGATTAAGACGGNTGAAACTAGAGATGGACAGGNTATCAACAAAACT
TCTCAGCATCACGATGACCTTGAATAAAATTGCCACACTCAGTGCAGCATATATTACC
AGCAAGAATAAAAAGAAATCCCTATCTTAAGAACNGCTTTCAGNGCCTTTCTGCAGTT
TTCAGNACNCAAGANAAATTTGAATAGGANAAGC

### EXAMPLES

### Example 1 Experimental procedures

### 1.1 Isolation of endothelial cells from fresh tissues

Fresh colorectal tumors (Dukes C) (n=5) and distant normal colon tissues of the same patient (n=5) were obtained from excision surgery at the department of Pathology (University Hospital Maastricht). Fresh placenta tissues (n=5) were obtained from the department of Obstetrics (University Hospital Maastricht). Endothelial cells were isolated as previously described (St Croix et al., 2000), with minor modifications. Tissues were minced with surgical blades, digested for 30 minutes with 1 mg/ml collagenase (Life Technologies, Breda, The Netherlands) and 2.5 U/ml dispase (Life Technologies) at 37°C with continuous agitation. DNAse I (Sigma, Zwijndrecht, The Netherlands) was added to a final concentration of 100µg/ml and the cell suspension was incubated for another 30 minutes prior to Ficoll Paque gradient density centrifugation (Amersham Biosciences, Uppsala, Sweden).

Endothelial cells were stained with anti-CD31 (clones JC/70A, DAKO, Glostrup, Sweden; and EN4, Monosan, Uden, The Netherlands) and anti-CD34 antibodies (clone Qbend10, Novocastra, Newcastle upon Tyne, United Kingdom) and isolated by positive selection using goat anti-mouse IgG coated paramagnetic beads (Dynal, Oslo, Norway). Hence, the present invention also provides for the selection of endothelial cells from human tissues for the purpose of gene expression by using the combination of CD31 and CD34. The purity of the isolated endothelial cell fraction was assessed by immunofluorescence staining for the endothelium specific von Willebrand Factor (vWF) (DAKO), and was estimated to be over 97% (Figure 1).

### 1.2 Cell culture

Human umbilical vein endothelial cells (HUVEC) were isolated and cultured as previously described (van der *Schaft et al.,* 2000). HUVEC between passages 1 and 3 were used for all experiments. For experiments that required 'tumor-activated' endothelial cells, HUVEC were seeded in 75 cm² tissue flasks coated with 1 mg/ml fibronectin at a density of 1 _{*} 10⁵ cells per flask. The cells were cultured in RPMI 1640 (Life Technologies) supplemented with 20% human serum (HS), 10% filter-sterile conditioned medium from LS174T colorectal tumor cell line, 10% filter-sterile conditioned medium from Caco-2 colorectal tumor cell line, 2 mM L-glutamine (Life Technologies), 50 ng/ml streptomycin (MP Biomedicals, Amsterdam, The Netherlands), 50 U/ml penicillin (MP Biomedicals), 1 ng/ml bFGF (Reliatech, Braunschweig, Germany) and 10 ng/ml VEGF (Reliatech) until 80% confluence was reached. 'Quiescent' endothelial cells were obtained by growing HUVEC for 72 hrs in fibronectin coated 75 cm² tissue flasks seeded at a density of 7*10⁵ cells per flask in RPMI 1640 supplemented with 2% HS, 2mM L-glutamine, 50 ng/ml streptomycin, and 50 U/ml penicillin.

Alternatively, established HUVEC cell lines were used, such as the EC line EVLC2, which is a cell line derived from human umbilical vein ECs by immortalization with simian virus 40 large T antigen (Leeuwen *et al.,* 2001).

### 1.3 RNA isolation and cDNA synthesis

RNA was isolated using RNeasy Mini reagents (Qiagen, Venlo, The Netherlands) according to the manufacturer's instructions. RNA samples were pooled for 5 colorectal tumor endothelial cell fractions (TEC), 5 normal colon endothelial cell fractions (NEC) and 5 placenta endothelial cell fractions (PLEC) and SMART^{™}cDNA (BD Biosciences, Alphen aan den Rijn, The Netherlands) was synthesized from the RNA and amplified to be used for SSH. The number of PCR cycles performed was optimised to maintain the original representation of transcripts in each sample. Input RNA in the cDNA synthesis reactions varied from 100ng (isolated endothelial cells) to 1µg (HUVEC) (Figure 1).

### 1.4 Suppression subtractive hybridisation (SSH)

SSH was performed with the PCR-Select^{™} cDNA subtraction kit (BD Biosciences) according to the manufacturers' instructions. Subtractions were performed to create cDNA repertoires enriched for genes overexpressed in TEC and for genes differentially expressed in activated and quiescent HUVEC. Subtracted cDNA repertoires were T/A cloned in pCR2.1(Invitrogen, La Jolla, CA) and introduced in TOP10 cells, according to the manufacturers' instructions. Individual colonies were picked and grown overnight at 37°C in 2xTY bacterial medium (BD Biosciences) supplemented with 10µg/ml ampicilin (Roche Applied Science, Almere, The Netherlands) and subsequently stored at -80°C in 15% glycerol.

### 1.5 Differential screening

Inserts were amplified using the adaptor specific primers Nested 1 and Nested 2R (BD Biosciences) using HotGoldstar Taq polymerase (Eurogentec, Liege, Belgium). PCR products were spotted in duplicate onto nylon membranes (Eurogentec) and hybridised to radioactively labelled cDNA probes derived from TEC, NEC, PLEC, activated and quiescent HUVEC. Approximately 100ng of SMART^{™} cDNA was labeled using High Prime labelling mix (Roche) in the presence of 25µCi ³³P-dCTP (Amersham). Membranes were pretreated with 0.6M NaCl/0.4M NaOH and subsequently prehybridised for at least 3 hours at 65°C in 5xSSPE, 10x Denhardts solution 0.5% SDS (Roche) and 100µg/ml salmon testes DNA (Sigma). Labelled probe was added to the hybridisation solution to an activity of 2-5 * 10⁶ cpm/ml and hybridised overnight at 65°C in a roller bottle hybridization oven (Techne; Jepsons Bolton, Watford Herts, UK). Membranes were washed with increasing stringency in SSPE/SDS solutions, wrapped in saran wrap and exposed to phosphor screens (Kodak, Rochester, NY) for 16-40 hours. Images were acquired using the Personal FX phosphorimager (Bio-Rad, Veenendaal, The Netherlands) at a resolution of 50µm and analysed as Tiff files using Quantity One software (Bio-Rad). All experiments were performed two times.

Data was processed in MS Excel to identify differentially expressed transcripts. Pair-wise comparisons were performed between duplicate filters hybridised with different probes. Duplicate spots showed excellent concordance (R²>0.99, data not shown) and were averaged. Average spot intensities were included in the analysis when expression was at least 2.5 times background in any experiment. Spot intensities were normalized for total intensity of the filters under comparison. Gene expression ratios were calculated using the average normalized intensities for each spotted insert cDNA. Hierarchical clustering analysis was performed with Cluster 3.0 (de *Hoon et al.,* 2004) and visualized using TreeView (Michael Eisen, University of California at Berkeley, CA).

### 1.6 Sequencing and database searching

Plasmid DNA was isolated using the GenElute Plasmid Miniprep kit (Sigma-Aldrich, St Louis, MO) and used as template for cycle sequencing. Reactions were performed using 300ng plasmid DNA in BigDye^{™} Terminator Cycle Sequencing mix (Applied Biosystems, Foster City, CA) using M13 universal primers (Sigma Genosys, The Woodlands, TX) and analysed on a 3100 Genetic Analyzer (Applied Biosystems; Genome Center Maastricht, Maastricht University, The Netherlands). Homology searches were performed using NCBI nucleotide-nucleotide Blast (blastn) algorithm on the combined GenBank/EMBL/DDBJ non-redundant (nr) and expressed sequence tags (est) databases (http://www.ncbi.nlm.nih.gov/blast/).

### 1.7 Real-time quantitative PCR (RTQ-PCR)

SYBR green assays were performed using 10ng cDNA template per reaction, consisting of 1x SYBR Green Master Mix (Applied Biosystems) and 200µM of each primer (Sigma Genosys) (Supplementary Table 1). Reactions were run and analysed on the AB17700 (Applied Biosystems) using the following cycling conditions: 50°C for 2 minutes, 95°C for 10 minutes and 40 cycles of 95°C for 15 seconds and 60°C for 1 minute. All reactions were performed in triplicate, analysed using SDS software (Applied Biosystems) and further processed in MS Excel. All experiments were normalized for cyclophilin A transcript expression to account for variations in template input.

### 1.8 FACS analysis

Single cell suspensions of fresh colorectal tumor and normal colon tissues were obtained as described above and fixed in 1% paraformaldehyde (Merck). Endothelial cells were stained with a PE-labelled anti-CD31 antibody (DAKO) and separated from other cells by cell sorting (BD FACSAria, BD Biosciences). CD31 positive cells were subsequently stained using the following antibodies, diluted in PBS, 0.5% BSA: rabbit anti-vimentin, rabbit anti-IGFBPrP1 and rabbit anti-HMGB1 followed by biotinylated swine anti-rabbit IgG (DAKO) and streptavidin-FITC (DAKO).

### 1.9 Immunohistochemistry

Formalin-fixed, paraffin embedded or snap frozen colorectal tumor and normal colon tissues were obtained from the department of Pathology (University Hospital Maastricht) and 5µm sections were mounted onto microscope slides (Knittel, Braunschweig, Germany). Sections were deparaffinized and rehydrated in a series of xylol and ethanol where applicable and fixed with 1% paraformaldehyde (Merck, Darmstadt, Germany). Endogenous peroxidase activity was blocked using 0.3% H₂O₂ (Merck) in PBS and non-specific binding was blocked with 1% BSA (Sigma) in PBS. Antibodies were diluted in 0.5% BSA in PBS.

Colon tumor and normal colon tissue sections were stained with the following antibodies: mouse anti-human CD31 (clone JC70/A, DAKO), mouse anti-human vimentin (clone V9, DAKO), mouse anti-human CD59 (clone MEM-43, Chemicon, Temecula, CA), rabbit anti-human HMGB1 (kind gift of Dr. R.G. Roeder, The Rockefeller University, New York, NY), and rabbit anti-human IGFBP1-rP1/IGFBP7 (kind gift of Dr. R. Rosenfeld, Oregon Health and Sciences University, Portland, OR). Primary antibodies were detected with peroxidase conjugated rabbit-anti-mouse IgG (DAKO) or goat-anti-rabbit IgG (DAKO). Color was developed using DAB according to standard protocols.

### 1.10 In vitro sprouting

Sprouting and tube formation of ECs were studied with the use of cytodex-3 beads overgrown with ECs in a 3-dimensional gel, as described previously (van der Schaft *et al.,* 2000). Antibodies dialyzed to PBS were added to the collagen gel and overlay medium in the described concentrations. Cells were incubated 48 hrs, after which photographs were taken of the beads. Five concentric rings were projected over the photographs, and the number of intersections of rings and sprouting endothelial cells was determined and used as a measure *of in vitro* tube formation.

Alternatively, tumor blood vessels from fresh colon tumors were prepared free from the surrounding tissue and sections of 1-2mm in length were embedded in the collagen gel. Sprouting was allowed to proceed for 5-7 days.

### 1.11 Proliferation assay

5*10³ cells were seeded in 96-well cell culture plates coated with 0.2% gelatin (Merck) and allowed to adhere for 2 hours. Antibodies, dialysed to PBS to remove traces of azide, were added to the culture medium in the indicated concentrations. Cells were cultured for 72 hours. During the last 6 hours of the assay, the culture was pulsed with 0.5 µCi [methyl-³H]-thymidine (Amersham) per well. Activity was measured using liquid scintillation counting (Wallac LSC; PerkinElmer, Boston, MA).

### 1.12 Endothelial cell migration

5*10³ cells were seeded in individual wells of gelatin coated 96-well cell culture plates and grown to confluence. Using a blunt pipette tip, a cross-shaped wound of approximately 750 µm wide was made in the well. Cells were washed with PBS, and fresh medium was added. Where appropriate, dialysed antibody was added to the medium in the indicated concentrations. Wound width was measured in 4 predefined locations per well at T= 0, 2, 4, 6 and 8 hours.

### 1.13 Cell cycle analysis

Cells, including floating cells, were harvested and fixed in 70% EtOH. Cells were resuspended in DNA extraction buffer (45 mM Na₂HPO₄•2H₂O, 2.5 mM citric acid, 0.1% Triton-X100, pH7.4) (Merck) and incubated at 37°C for 20 minutes. Propidium iodide (Merck) was then added to a final concentration of 2 µg/ml. Based on DNA content and scatter, cells were classified as dividing (G2/M phase), resting (G1/G0), apoptotic or necrotic.

### 1.14 RNA Interference

SiRNA duplexes were obtained from Eurogentec (Liege, Belgium), targeting the TAG at issue as well as a negative control. Cells were transfected using JetSi-ENDO (Eurogentec) according to the manufacturers' instructions. Briefly, 2500 HUVEC were seeded in a gelatin coated 96-well cell culture plate and allowed to adhere overnight. Medium was replaced with DMEM (Life Technologies) containing L-glutamine (Life Technologies). SiRNA-JetSi-ENDO complexes were made by first combining 0.2 µl JetSi-ENDO with 10 µl RPMI 1640 (Life Technologies) per well; this was incubated 20 minutes at room temperature to generate mix A. SiRNA duplexes were added to 10 µl RPMI-1640 to form mix B. Mix A was added to mix B and incubated at room temperature for 30 minutes. Complex AB (20 µl) was added drop-wise to the cells and incubated 4 hours. Transfection medium was then replaced with normal medium and cells were grown for 72 hours prior to assaying. BCE were first grown on the cytodex beads as described above, treated with siRNA duplexes and grown for 48 hours prior to being embedded in the collagen gel.

### 1.15 Transfection of endothelial cells

EVLC2 cells were transfected using Nucleofector technology (Amaxa, Cologne, Germany). Briefly, 5x10⁵ cells were harvested and resuspended in 100 µl Nucleofector solution R. The cell suspension was mixed with 1 µg DNA and transferred to an electroporation cuvette. Following transfection using program T20, 500 µl filtered HUVEC medium was added and cells were transferred to 2 wells of a gelatin coated 24-wells cell culture plate. Successfully transfected cells were selected based on hygromycin resistance using 25 µg/ml hygromycin (Life Technologies).

### 1.16 Chick chorioallantoic membrane (CAM) assay

Fertilized white leghorn chicken eggs were used to monitor vessel development in the CAM as described previously (van der Schaft *et al.,* 2000). Antibodies were dialysed to 0.9% NaCl and administered in the indicated concentrations in a volume of 65 µl for four consecutive days. On day 14, the CAMs were photographed. Five concentric rings were projected on the image. The number of intersections of rings and blood vessels was determined and used as a measure of vessel density. In some experiments, LS 174T tumor tissues were placed on the chorioallantoic membrane (CAM) within the silicone ring. LS174T were seeded in non-adherent cell culture plates at a density of 10⁵ cells/ml for 10 days to allow spheroids to form. A small surface area (approximately 3x3mm) in the silicone ring placed on the CAM was denudated using lens paper and uniformly looking spheroids of 0.5-1 mm in diameter were applied on the CAM. Antibodies were dialysed to 0.9% NaCl and administered in a volume of 65 µl for four consecutive days. At day 14, the CAMs were photographed.

### 1.17 Mouse tumor models

Female athymic nude mice were used and randomly split in four groups. All experiments were approved by the University of Minnesota Research Animal Resources ethical committee. Mice (n = 6 per group) were inoculated with 1*10⁶ LS174T colorectal carcinoma cells in 100 µl RPMI subcutaneously in the right flank. Four days post-inoculation treatment was started. Mice were treated by i.p. injections every third day with a commercially available anti-TAG antibody, a commercially available isotype control antibody or saline. Tumor volume was determined daily by measuring the diameters of tumors using callipers and calculated as follows: width² × length × 0.52.

Cryosections (5 µm) of the tumors were stained for CD31 and microvessel density was evaluated as described previously *(Dings et al.,* 2003).

To assess the extent of total cell apoptosis, tissue sections are stained by using the TUNEL (terminal deoxyribo-nucleotidyl transferase-mediated dUTP-nick-end labelling) assay, which is performed according to the manufacturer's instructions *(in situ* cell death detection kit, fluorescein; TUNEL, Roche Applied Science).

### Example 2 Identification of tumor endothelial markers by SSH

A suppression subtractive hybridization (SSH) was performed in combination with cDNA array screening to identify novel tumor specific endothelial markers in an unbiased manner. Tumor endothelial cells (TEC) were successfully isolated from colon tumors (n = 5) and patient-matched normal endothelial cells (NEC) from normal colon tissue samples (n = 5), as well as from placenta tissues (PLEC, n = 5) (Figure 1). RNA was isolated (Figure 1) and used to create subtraction repertoires of genes overexpressed in TEC. In addition, HUVEC were stimulated *in vitro* with tumor cell conditioned medium and used to create additional subtraction repertoires. A total of 2746 inserts, 1781 derived from the TEC subtractions and 965 derived from the HUVEC subtractions were amplified and spotted onto duplicate arrays that were probed with ³³P-dCTP labeled cDNA derived from TEC, NEC, PLEC and HUVEC. Phospho-imaging and pair-wise comparisons of spot intensities were performed to identify differentially expressed spots (Figure 1). Insert identity was determined by sequencing analysis.

Transcripts showing overexpression in TEC vs NEC were further subdivided based on their expression in the other EC populations. By comparing expression profiles of TEC with NEC, PLEC, and HUVEC, it was possible to distinguish between genes associated with angiogenesis in general (general angiogenesis genes, GAGs) and genes specific for tumor endothelium (tumor angiogenesis genes, TAGs) (Figure 2A, Table 1). Forty-one transcripts classified as TAGs (Table 1, Figure 2B & C, Table 1) and showed overexpression in both TEC compared to NEC and in TEC compared to PLEC. Eighty-five transcripts were found to be upregulated in TEC compared to NEC as well as in PLEC compared to NEC (GAG/A) (Figure 2B & D, Table 1). Finally, the 24 upregulated transcripts in activated HUVEC vs quiescent HUVEC as well as in TEC vs NEC are named GAG/B (Figure 2B & E, Table 1).

Sequence analysis revealed that the 41 TAG transcripts represented 17 different genes (Table 2). Five of these have previously been described to be overexpressed on tumor endothelium, validating our approach. The identification of the highly abundant collagens 4A1 and 1A1 in different tumor types points towards the possible existence of pan-tumor endothelium specific transcripts *(Madden et al.,* 2004; *Parker et al.,* 2004; St Croix *et al.,* 2000). SPARC and IGFBP7 have also previously been associated with angiogenesis (Akaogi *et al.,* 1996; Porter *et al.,* 1995) and were classified as pan-endothelial markers (St Croix *et al.,* 2000). HEYL, a basic helix-loop-helix transcription factor has recently been associated with breast tumor vasculature (Parker *et al.,* 2004). PPAP2B has been described as a gene that is upregulated *during in vitro* tube formation of endothelial cells under the influence of VEGF (Humtsoe *et al.,* 2003). Very recently, the cytokine HMGB1 was recognized for its role in promoting angiogenesis *in vitro* (Schlueter *et al.,* 2005). The 10 remaining TAG markers have no reported functional contribution to tumors and/or angiogenesis.

### Example 3 Gene expression of tumor endothelial cells is closely related to gene expression during physiological angiogenesis

It emerged that the majority of TEC overexpressed transcripts (85/142 = 60%) are also associated with angiogenesis under physiological conditions *in vivo,* and are therefore not specific for tumor angiogenesis *in vivo* (Figure 2B). These 85 GAG/A transcripts represent 46 different genes, including genes that have been associated with angiogenesis such as matrix metalloproteinases (MMPs) (Pepper, 2001), integrin β1 (Senger *et al.,* 2002) and endothelial cell specific molecule-1 (Aitkenhead *et al.,* 2002) (Table 3).

### Example 4 In vitro endothelial cell activation is a limited substitute for studying tumor angiogenesis

From the gene expression analysis, it is obvious that only a limited number of genes upregulated in TEC vs NEC overlapped with genes overexpressed in tumor-conditioned *HUVEC in vitro* (GAG/B, Figure 2B, Table 1). This suggested that this *in vitro* model may be of only limited value for studying tumor angiogenesis. Most of the GAG/B markers overlap with genes associated with angiogenesis in general (GAG/A) (Figure 2B, Table 3). Hierarchical clustering analysis suggested that the expression pattern in the HUVEC model relates most to that emerging from physiological angiogenesis (i.e. the comparison between PLEC and NEC; Figure 8).

### Example 5 TAG markers are functionally classified as associated in late events of angiogenesis

A functional annotation was assigned to every gene. The distribution of genes was analyzed into different functional classes. TAG markers are predominantly biased towards genes associated with cytoskeletal and extracellular matrix remodelling, indicative of late events in the process of tumor angiogenesis, whereas protein turnover and transcription associated genes are underrepresented within TAG (Figure 2C). The GAG/A class represents genes associated to cell and protein turnover (Figure 2D). Hallmarks of GAG/B molecules are active transcription and protein turnover (Figure 2E). Functional clustering indicates that both GAG/A and GAG/B represent genes important in early events in the angiogenesis process.

Indeed, genes that showed overlapping expression profiles in activated HUVEC and tumor endothelium, GAG/B markers, were also highly biased towards protein turnover and transcription (Table 3). The fact that culture conditions highly influence gene expression was exemplified by the expression profile of HUVEC co-cultured with glioma cells (Khodarev *et al.,* 2003). Encouragingly, genes related to ECM remodelling and cytoskeletal functions, suggestive of advanced stages of angiogenesis, were significantly upregulated in HUVEC co-cultured with tumor cells compared to monoculture of HUVEC. The direct physical contact of endothelial cells with the tumor cells seems necessary to direct this induction of gene expression.

### Example 6 Validation of TAGs

Overexpression of TAGs was confirmed using real-time quantitative PCR (RTQ-PCR) as a second independent technique. For 16 different genes (94%) overexpression in TEC vs NEC was confirmed, also for 16 genes (94%) overexpression in TEC vs PLEC was confirmed (Figure 3A). Taken together, 15 out of 17 (88%) genes validated by RTQ-PCR were positively confirmed TAG markers.

Of the 10 TAGs with no previous association with angiogenesis, 4 have no known function as yet.

For subsequent studies of TAG markers at the protein level, 4 different membrane associated or secreted molecules were selected: (i) CD59, a GPI membrane-anchored inhibitor of complement activation (Gelderman *et al.,* 2004), (ii) insulin-like growth factor binding protein-7 (IGFBP7), a secreted molecule with growth factor modulating function (Akaogi *et al.,* 1996), (iii) HMGB1, a secreted cytokine as well as a non-histone DNA binding protein (Goodwin *et al.,* 1973; Treutiger *et al.,* 2003), and (iv) vimentin, an intermediate filament protein that was recently demonstrated to be actively secreted (Mor-Vaknin *et al.,* 2003; Xu *et al.,* 2004). Immunohistochemical analysis in colorectal carcinoma and normal colon epithelium indicated that all 4 proteins were overexpressed on the tumor vasculature. While vimentin, IGFBP7 and CD59 were predominantly expressed in the endothelial compartment, HMGB1 was found to be expressed in stromal and epithelial cells as well (Figure 3C). Vimentin expression was detected in endothelial cells of both tumor and normal colon tissue, though heavily overexpressed on tumor endothelium. IGFBP7 expression in normal colon tissue is hardly detected, whereas tumor blood vessels show abundant expression of IGFBP7. CD59 expression was mainly localized to vasculature, in particular to the luminal cell membrane (Figure 3C). This is in line with its reported expression as membrane protein with a role in protecting endothelial cells from complement-mediated lysis by binding complement proteins C8 and C9 to prevent the formation of the membrane attack complex (Gelderman *et al.,* 2004). HMGB1 staining was detected in endothelial cells, as cytoplasmic protein, but also in epithelial cells, where the localization was predominantly nuclear. In addition, diffuse stromal staining was observed. Protein expression was much more abundant in colorectal tumor tissue compared to normal colon tissue, predominantly in the stromal compartment, consistent with a secretion product (Figure 3C) (Huttunen and Rauvala, 2004).

Since immunohistochemical analysis is a qualitative rather than a quantitative technique, the expression of our TAGs on freshly isolated endothelial cells of tumor and normal tissues was determined by flow cytometry. The overexpression of vimentin (TAG-39), IGFBP7 (TAG-29) and HMGB1 (TAG-21) protein on colon tumor endothelium compared to normal colon endothelium was quantitatively confirmed. In addition, the expression of CD31 did not differ between TEC and NEC (Figure 3B). These observations further support the value of these proteins as tumor EC signature markers.

### Example 7 Interference with TAG proteins inhibits angiogenesis in vitro and in vivo

### 7.1 Interference with TAG proteins inhibits angiogenesis in in vitro sprout-formation assay

To investigate whether the overexpression of the selected TAG markers is causally related to the process of angiogenesis, *in vitro* bioassays were performed. Antibodies directed against CD59, IGFBP7, vimentin and HMGB1 were tested for their effect on endothelial tube formation in *an in vitro* collagen-gel-based sprout-formation assay. Antibodies directed against the latter three showed inhibitory effects on BCE sprout formation *in vitro,* whereas CD59 and a control antibody were less effective (Figure 4A). Capillary sprouting from isolated tumor vessels in an *ex vivo* set-up was inhibited by antibodies targeting HMGB1 and vimentin, and to a lesser extent CD59 (Figure 4C). These observations suggest that the targeted proteins are actively involved in the process of capillary tube formation.

### 7.2 Interference with TAG proteins inhibits angiogenesis in in vivo CAM assay

To investigate whether these TAGs are involved in angiogenesis *in vivo,* the antibodies were tested in the developing chorioallantoic membrane (CAM) of the chick embryo. A similar result as in the sprouting assay was found for angiogenesis inhibition in the *CAM in vivo* (Figure 4B). Antibodies against CD59, HMGB1 and vimentin inhibited angiogenesis by 27%, 45% and 40%, respectively, while a control antibody did not show any activity (as compared to CAMs treated with saline alone).

These results strongly suggest a role for these molecules in the process of angiogenesis and together with the overexpression on tumor endothelium support their potential for use in targeting of tumor vasculature as therapy against cancer.

### 7.3 Overexpression of TAG proteins promotes angiogenesis in in vivo wound assay

To further investigate the contribution of HMGB1 to tumor angiogenesis, we used expression constructs encoding HMGB 1 in both the sense and the antisense orientation to induce or repress HMGB1 expression, respectively. Overexpression of HMGB1 clearly increased the migration speed of the endothelial cells in a wounding assay (Figure 5A). Also, MMP9 expression was induced in HMGB1 sense expressing cells. In addition, response to growth factor activation was impaired in HMGB1 antisense expressing cells (Figure 5C).

### 7.4 Downregulation of TAG proteins by RNA interference inhibits angiogenesis

RNA interference was employed to investigate the effect of downregulated TAG levels in endothelial cells. Different concentrations siRNA duplex specific for vimentin were capable of inhibiting migration of the cells (Figure 5D), as well as sprouting (Figure 5E). Only at higher concentrations of siRNA duplex, the proliferation of the cells is impaired, suggesting toxicity is non-existing at 50nM siRNA duplex (Figure 5E). Similar results are obtained by downregulation of HMGB1 by different concentrations siRNA duplex specific for HMGB1.

### 7.5 Use of TAGs

Although HMGB1/amphoterin was originally identified as a non-histone DNA binding molecule (Goodwin *et al.,* 1973), more recently focus has shifted to its role as a secreted cytokine. As an extracellular protein, it has been involved in the regulation of cell migration (Fages *et al.,* 2000), tumorigenesis (Taguchi *et al.,* 2000), cell activation (Treutiger *et al.,* 2003) and inflammation (Fiuza *et al.,* 2003). It can act as a paracrine or autocrine factor creating feedback loops for the secretion of TNF-α and IL-1β in monocytes and macrophages. It also acts on endothelial cells to upregulate ICAM-1, VCAM-1 and TNF-α expression (Fiuza *et al.,* 2003; Treutiger *et al.,* 2003) and stimulates sprouting (Schlueter *et al.,* 2005). We have demonstrated that an antibody directed at HMGB1 was effective at inhibiting endothelial cell sprouting *in vitro* as well as angiogenesis *in vivo.* This finding can be exploited for therapeutic modulation of angiogenesis, e.g. inhibiting tumor angiogenesis as shown in Figure 4, or stimulation of angiogenesis in ischemic diseases.

Vimentin is an extensively studied intermediate filament protein (reviewed by Hendrix *et al.,* 1996) which has also been described as a target gene of HIF-1α, a major inducer of VEGF (Krishnamachary *et al.,* 2003). We have shown quantitative data on the overexpression of vimentin on endothelial cells in colon tumor samples compared to normal colon samples, both at the transcriptional level (Figure 3A) and the protein level (Figure 3B). This result suggests a contribution of this protein to the tumor endothelial phenotype. We present evidence that targeting of vimentin by means of antibodies clearly inhibited angiogenesis *both in vitro* and *in vivo.*

CD59 is a GPI anchored membrane protein and an inhibitor of complement activation (Gelderman *et al.,* 2004). CD59 function is dependent upon complement activation. Complement activation does not *apply in vitro,* which explains our result that antibodies directed against CD59 were not readily effective in *our in vitro* assays. Targeting of *CD59 in vivo* is more successful. Indeed, *in vivo* in the CAM assay, a significant effect of anti-CD59 antibodies on vessel formation was demonstrated. IGFBP7 is a secreted protein that accumulates in the basement membrane (Ahmed *et al.,* 2003; Akaogi *et al.,* 1996), where it can bind collagens type 2, 4 and 5, heparan sulfates and different cytokines (Akaogi *et al.,* 1996; Nagakubo *et al.,* 2003). By binding collagens it supports the organization of endothelial cells into tube-like structures (Akaogi *et al.,* 1996). In summary, it is known that IGFBP7 functions in blood vessels. The present study demonstrated that overexpression of IGFBP7 in tumor endothelium was evident both at the transcriptional level and at the protein level. In addition, targeting IGFBP7 with an antibody clearly inhibited endothelial sprouting in vitro, possibly caused by inhibition of the interaction between IGFBP7 and collagens present in the three-dimensional culture matrix.

From the series of 17 TAGs, several different genes encoded membrane-bound or secreted proteins. Four of these were selected to investigate for a role in angiogenesis and to serve as tumor endothelial target for therapeutic applications. The present invention demonstrated that all four genes (i) are necessary in the process of angiogenesis and (ii) can be used for intervention in angiogenesis using antibodies as a treatment opportunity.

### Example 8 Interference with TAG proteins inhibits tumor associated angiogenesis

To further investigate the relation between the selected TAG markers and the process of angiogenesis, the following experiments were conducted.

### 8.1 Tumor angiogenesis is inhibited in CAM assay

An experimental model of tumor angiogenesis was set up that employs the growth of LS174T colon carcinoma cell spheroids transplanted onto the CAM. Growth of these spheroids induces the growth of vasculature and induced aberrant morphology in the chick vasculature (Figure 6). Treatment of the CAMs with commercially available antibodies against HMGB1 (Figure 6A) and vimentin (Figure 6B) shows a reduction in vessel density on the CAM as compared to untreated tumors.

To provide proof of principle that targeting of TAGs inhibits tumor angiogenesis and tumor growth, antibodies against CD59, HMGB1 and vimentin were tested in the model of a transplanted tumor onto the CAM. In this model a lump of 1 mm³ LS174T human colon tumor tissue is put on a 10-day CAM. Antibody treatment, performed as described above, resulted in significant inhibition of vessel growth and in reduction of tumor growth (Figure 7).

### 8.2 Tumor angiogenesis is inhibited in nude mice assay

To provide an absolute proof of the feasibility of the invention to identify endothelial targets for cancer treatment, LS 174T colon carcinoma model in nude mice is used. The nude mice are treated with the mouse-reactive anti-HMGB-1 antibody (clone HAP46.5). Treatment of tumor-bearing mice with HAP46.5 shows a dose-dependent inhibition of tumor growth with no apparent toxic effects. Microvessel density of the HAP46.5 treated tumors is markedly reduced, whereas tumors treated with the control antibody show no inhibition of tumor growth or inhibition of microvessel density. Furthermore, there is no apparent toxicity associated with the treatment as the body weight of the mice does not differ between the treatment groups (Figure 7D), suggesting no or only limited effects of the TAG antibody on normal body physiology. The above proof of the feasibility of the invention to identify endothelial targets for cancer treatment was corroborated by experiments with anti-vimentin antibodies. In particular, nude mice bearing LS174T tumors are treated with the mouse-reactive anti-vimentin antibody. It is verified that the tumor cells do not express vimentin (Figure 7B, panels a and b) to ascertain that effects of the treatment would be the result of targeting the vasculature.

Treatment of tumor-bearing mice with antibody shows a dose-dependent inhibition of tumor growth with no apparent toxic effects. Microvessel density of the antibody treated tumors is markedly reduced, whereas tumors treated with the control antibody shows no inhibition of tumor growth or inhibition of microvessel density. Furthermore, there is no apparent toxicity associated with the treatment as the body weight of the mice did not differ between the treatment groups, suggesting no or only limited effects of the vimentin antibody on normal body physiology (Van Beijnum *et al.* Blood, in press, June 2006, incorporated herein explicitly by reference).

Further substantiation of the feasibility of the invention identifying endothelial targets for cancer treatment comes from the LS174T colon carcinoma model in nude mice as follows. The nude mice are treated with commercially available antibodies against ARPC2, CDK2AP1, CollA1, HEYL, LDHB, PPAP2B, SPARC. Treatment of tumor-bearing mice with these antibodies shows a dose-dependent inhibition of tumor growth with no apparent toxic effects. Microvessel density of the treated tumors is markedly reduced, whereas tumors treated with the control antibody show no inhibition of tumor growth or inhibition of microvessel density. Furthermore, there is no apparent toxicity associated with the treatment as the body weight of the mice does not differ between the treatment groups, suggesting no effects of the antibodies on normal body physiology.

For Co14A1, TAG-23, TAG-27, HSIRPR, PHC3 and HSPC014 antibodies are produced and tested similarly in mouse tumor models as described above for the other TAGs.

### Example 9 Targeting TAG proteins does not cause side effects associated with impaired physiological angiogenesis

TAG proteins are selected on their differential expression pattern in endothelial cells, i. e. higher expression in angiogenic tumor endothelial cells compared to normal resting or normal angiogenic endothelial cells. Therefore, targeting TAGs as a means of therapeutic inhibition of endothelial cells results in an expression-dependent inhibition of angiogenesis. Targeting is most effective in endothelial cells with the highest expression of the TAG. This creates a certain degree of specificity of tumor directed anti-angiogenic therapy and reduces side effects associated with inhibition of physiological angiogenesis.

To determine the magnitude of effects of targeting TAG proteins on physiological angiogenesis, wound healing in mice is studied. Circular wounds of 6mm diameter are made through the skin at the back of the mouse, according to the method described by Eckes *et al.* (Eckes et al., J Cell Sci 113, 2000). Mice are treated with vehicle alone (Control group A), treated with different concentrations of a TAG-specific antibody (Experimental group B), and treated with a non-relevant antibody (Control group C). The size of the wound is measured daily and closure of the wound is a representative measure of physiological angiogenesis. No significant differences in wound healing are observed between the different treatment groups.

It is concluded that treatment of tumors using TAG-specific antibodies causes no side effects associated with impaired physiological angiogenesis.

### REFERENCES

Abe, M., and Sato, Y. (2001). cDNA microarray analysis of the gene expression profile of VEGF-activated human umbilical vein endothelial cells. Angiogenesis 4, 289-298.
Ahmed, S., Yamamoto, K., Sato, Y., Ogawa, T., Herrmann, A., Higashi, S., and Miyazaki, K. (2003). Proteolytic processing of IGFBP-related protein-1 (TAF/angiomodulin/mac25) modulates its biological activity. Biochem Biophys Res Commun 310, 612-618.
Aitkenhead, M., Wang, S. J., Nakatsu, M. N., Mestas, J., Heard, C., and Hughes, C. C. (2002). Identification of endothelial cell genes expressed in an in vitro model of angiogenesis: induction of ESM-1, (beta)ig-h3, and NrCAM. Microvasc Res 63, 159-171.
Akaogi, K., Okabe, Y., Sato, J., Nagashima, Y., Yasumitsu, H., Sugahara, K., and Miyazaki, K. (1996). Specific accumulation of tumor-derived adhesion factor in tumor blood vessels and in capillary tube-like structures of cultured vascular endothelial cells. Proc Natl Acad Sci U S A 93, 8384-8389.
Altschul et al. (1997) Gapped BLAST and PSI-BLAST: a new generation of protein database search programs. Nucleic Acids Res. 1997 Sep 1;25(17):3389-402.
de Hoon, M. J., Imoto, S., Nolan, J., and Miyano, S. (2004). Open source clustering software. Bioinformatics 20, 1453-1454.
Dell'Era, P., Coco, L., Ronca, R., Sennino, B., and Presta, M. (2002). Gene expression profile in fibroblast growth factor 2-transformed endothelial cells. Oncogene 21, 2433-2440.
Dings et al. (2003) β-Sheet is the bioactive conformation of the anti-angiogenic anginex peptide. Biochem. J. 373: 281-288.
Fages, C., Nolo, R., Huttunen, H. J., Eskelinen, E., and Rauvala, H. (2000). Regulation of cell migration by amphoterin. J Cell Sci 113 (Pt 4), 611-620.
Fernandes and Gregoriadis, 1997Polysialylated asparaginase: preparation, activity and pharmacokinetics. Biochim Biophys Acta. 1997 Aug 15;13341(1):26-34.
Fiuza, C., Bustin, M., Talwar, S., Tropea, M., Gerstenberger, E., Shelhamer, J. H., and Suffredini, A. F. (2003). Inflammation-promoting activity of HMGB1 on human microvascular endothelial cells. Blood 101, 2652-2660.
Gelderman, K. A., Tomlinson, S., Ross, G. D., and Gorter, A. (2004). Complement function in mAb-mediated cancer immunotherapy. Trends Immunol 25, 158-164.
Gerritsen, M. E., Tomlinson, J. E., Zlot, C., Ziman, M., and Hwang, S. (2003b). Using gene expression profiling to identify the molecular basis of the synergistic actions of hepatocyte growth factor and vascular endothelial growth factor in human endothelial cells. Br J Pharmacol 140, 595-610.
Goodwin, G. H., Sanders, C., and Johns, E. W. (1973). A new group of chromatin-associated proteins with a high content of acidic and basic amino acids. Eur J Biochem 38, 14-19.
Hendrix, M. J., Seftor, E. A., Chu, Y. W., Trevor, K. T., and Seftor, R. E. (1996). Role of intermediate filaments in migration, invasion and metastasis. Cancer Metastasis Rev 15, 507-525.
Humtsoe, J. O., Feng, S., Thakker, G. D., Yang, J., Hong, J., and Wary, K. K. (2003). Regulation of cell-cell interactions by phosphatidic acid phosphatase 2b/VCIP. Embo J 22, 1539-1554.
Huttunen, H. J., and Rauvala, H. (2004). Amphoterin as an extracellular regulator of cell motility: from discovery to disease. J Intern Med 255, 351-366.
Jayasena (1999) Aptamers: an emerging class of molecules that rival antibodies in diagnostics. Clin Chem. 1999 Sep;45(9):1628-50.
Khodarev, N. N., Yu, J., Labay, E., Darga, T., Brown, C. K., Mauceri, H. J., Yassari, R., Gupta, N., and Weichselbaum, R. R. (2003). Tumor-endothelium interactions in co-culture: coordinated changes of gene expression profiles and phenotypic properties of endothelial cells. J Cell Sci 116, 1013-1022.
Krishnamachary, B., Berg-Dixon, S., Kelly, B., Agani, F., Feldser, D., Ferreira, G., Iyer, N., LaRusch, J., Pak, B., Taghavi, P., and Semenza, G. L. (2003). Regulation of colon carcinoma cell invasion by hypoxia-inducible factor 1. Cancer Res 63, 1138-1143.
Madden, S. L., Cook, B. P., Nacht, M., Weber, W. D., Callahan, M. R., Jiang, Y., Dufault, M. R., Zhang, X., Zhang, W., Walter-Yohrling, J., et al. (2004). Vascular gene expression in nonneoplastic and malignant brain. Am J Pathol 165, 601-608.
Mor-Vaknin, N., Punturieri, A., Sitwala, K., and Markovitz, D. M. (2003). Vimentin is secreted by activated macrophages. Nat Cell Biol 5, 59-63.
Nagakubo, D., Murai, T., Tanaka, T., Usui, T., Matsumoto, M., Sekiguchi, K., and Miyasaka, M. (2003). A high endothelial venule secretory protein, mac25/angiomodulin, interacts with multiple high endothelial venule-associated molecules including chemokines. J Immunol 171, 553-561.
Parker, B. S., Argani, P., Cook, B. P., Liangfeng, H., Chartrand, S. D., Zhang, M., Saha, S., Bardelli, A., Jiang, Y., St Martin, T. B., et al. (2004). Alterations in vascular gene expression in invasive breast carcinoma. Cancer Res 64, 7857-7866.
Pepper, M. S. (2001). Role of the matrix metalloproteinase and plasminogen activator-plasmin systems in angiogenesis. Arterioscler Thromb Vasc Biol 21, 1104-1117.
Porter, P. L., Sage, E. H., Lane, T. F., Funk, S. E., and Gown, A. M. (1995). Distribution of SPARC in normal and neoplastic human tissue. J Histochem Cytochem 43, 791-800.
Schlueter, C., Weber, H., Meyer, B., Rogalla, P., Roser, K., Hauke, S., and Bullerdiek, J. (2005). Angiogenetic signaling through hypoxia: HMGB1: an angiogenetic switch molecule. Am J Pathol 166, 1259-1263.
Senger, D. R., Perruzzi, C. A., Streit, M., Koteliansky, V. E., de Fougerolles, A. R., and Detmar, M. (2002). The alpha(1)beta(1) and alpha(2)beta(1) integrins provide critical support for vascular endothelial growth factor signaling, endothelial cell migration, and tumor angiogenesis. Am J Pathol 160, 195-204.
St Croix, B., Rago, C., Velculescu, V., Traverso, G., Romans, K. E., Montgomery, E., Lal, A., Riggins, G. J., Lengauer, C., Vogelstein, B., and Kinzler, K. W. (2000). Genes expressed in human tumor endothelium. Science 289, 1197-1202.
Taguchi, A., Blood, D. C., del Toro, G., Canet, A., Lee, D. C., Qu, W., Tanji, N., Lu, Y., Lalla, E., Fu, C., et al. (2000). Blockade of RAGE-amphoterin signalling suppresses tumor growth and metastases. Nature 405, 354-360.
Treutiger, C. J., Mullins, G. E., Johansson, A. S., Rouhiainen, A., Rauvala, H. M., Erlandsson-Harris, H., Andersson, U., Yang, H., Tracey, K. J., Andersson, J., and Palmblad, J. E. (2003). High mobility group 1 B-box mediates activation of human endothelium. J Intern Med 254, 375-385.
Van Beijnum, J. R., and Griffioen, A. W. (2005). In silico analysis of angiogenesis associated gene expression identifies angiogenic stage related profiles. Biochim Biophys Acta In press*.*
van der Schaft, D. W., Toebes, E. A., Haseman, J. R., Mayo, K. H., and Griffioen, A. W. (2000). Bactericidal/permeability-increasing protein (BPI) inhibits angiogenesis via induction of apoptosis in vascular endothelial cells. Blood 96, 176-181.
van Leeuwen EB, Wisman GB, Tervaert JW, Palmans LL, van Wijk RT, Veenstra R, Molema G, van der Zee AG, van der MJ, Ruiters MH. An SV40 large T-antigen immortalized human umbilical vein endothelial cell line for anti-endothelial cell antibody detection. Clin Exp Rheumatol. 2001; 19: 283-290
Wang, B., Xiao, Y., Ding, B. B., Zhang, N., Yuan, X., Gui, L., Qian, K. X., Duan, S., Chen, Z., Rao, Y., and Geng, J. G. (2003). Induction of tumor angiogenesis by Slit-Robo signaling and inhibition of cancer growth by blocking Robo activity. Cancer Cell 4, 19-29.
Xu, B., DeWaal, R. M., Mor-Vaknin, N., Hibbard, C., Markovitz, D. M., and Kahn, M. L. (2004). The Endothelial Cell-Specific Antibody PAL-E Identifies a Secreted Form of Vimentin in the Blood Vasculature. Mol Cell Biol 24, 9198-9206.
Zhang, H.-T., Gorn, M., Smith, K., Graham, A. P., Lau, K. K. W., and Bicknell, R. (1999). Transcriptional profiling of human microvascular endothelial cells in the proliferative and quiescent state using cDNA arrays. Angiogenesis 3, 211-219.

## Claims

1. A method to identify candidate drugs for treating a patient having a pathological condition or a susceptibility to a pathological condition, such as bearing tumors or for treating wounds, comprising:
- contacting a test agent with recombinant host cells transfected with an expression construct which encodes for SEQ ID NO: 18, 2, 4, 6, 8, 10, 14, 16, 20, 22, 24, 26, 28, 30, 32 or 34 including parts thereof;
- determining the amount of proliferation of said cells;
- identifying a test agent as a candidate drug for treating patients having a pathological condition or a susceptibility to a pathological condition if it modulates the proliferation of said cells.

2. A method according to claim 1, wherein said pathological condition is a tumor, and wherein said test agent is identified as a candidate drug for treating said tumor if it affects cell growth.

3. A method according to claim 2, wherein said pathological condition is impaired wound healing, and wherein said test agent is identified as a candidate drug for treating said impaired wound healing if it stimulates proliferation of said cells.

4. A method of diagnosing a pathological condition or a susceptibility to a pathological condition, said method comprising:
(a) contacting a tissue sample with an antibody specific for SEQ ID NO: 18, 2, 4, 6, 8, 10, 14, 16, 20, 22, 24, 26, 28, 30, 32 or 34 or a part thereof or with a probe specific for any of SEQ ID NO: 17, 1, 3, 5, 7, 9, 13, 15, 19, 21, 23, 25, 27, 29, 31, or 33 or a part thereof;
(b) detecting binding of said antibody or probe to said tissue sample;
(c) comparing the binding detected in step (b) with a standard,
wherein a difference in binding relative to the standard is diagnostic of a pathological condition or a susceptibility to a pathological condition.

5. A method of screening for neo-angiogenesis in a patient, comprising:
- contacting a biological sample with a molecule comprising an antibody variable region which specifically binds to a protein, wherein said protein is **characterized by** SEQ ID NO: 18, 2, 4, 6, 8, 10, 14, 16, 20, 22, 24, 26, 28, 30, 32 or 34 including parts thereof;
- detecting material in said biological sample that is cross-reactive with said molecule, and
wherein detection of cross-reactive material indicates neo-angiogenesis in the patient, or comprising:
- detecting an expression product of at least one gene in a first tissue sample of a patient, wherein said at least one gene is **characterized by** SEQ ID NO: 17, 1,3, 5, 7, 9, 13, 15, 19, 21, 23, 25, 27, 29, 31 or 33 including parts thereof; and
- comparing expression of the expression product of at least one gene in the first tissue sample with expression of the expression product of the at least one gene in a second tissue sample which is normal,
wherein an increased expression of the expression product of the at least one gene in the first tissue sample relative to the second tissue sample identifies the first tissue sample as likely to be neo-angiogenic.

6. A nucleic acid or a protein for stimulating or inhibiting angiogenesis in a patient, wherein said nucleic acid encodes for a protein comprising an amino acid sequence, which is at least 65% identical to SEQ ID NO: 18, 2, 4, 6, 8, 10, 14, 16, 20, 22, 24, 26, 28, 30, 32 or 34, and wherein protein comprises an amino acid sequence, which is at least 65% identical to SEQ ID NO: 18, 2, 4, 6, 8, 10, 14, 16, 20, 22, 24, 26, 28, 30, 32 or 34.

7. Use of a nucleic acid, polypeptide or antibody for the preparation of a medicament for preventing, treating and/or alleviating proliferative disorders, or for stimulating or inhibiting angiogenesis in a patient,
wherein said nucleic acid is selected from a group consisting of:
(a) a nucleic acid comprising a DNA sequence as given in SEQ ID NO: 17, 1,3,5, 7, 9, 13, 15, 19, 21, 23, 25, 27, 29, 31 or 33 or a part thereof, or the complement thereof,
(b) a nucleic acid comprising the RNA sequences corresponding to SEQ ID NO: 17, 1, 3, 5, 7, 9, 13, 15, 19, 21, 23, 25, 27, 29, 31 or 33 or a part thereof, or the complement thereof,
(c) a nucleic acid specifically hybridizing to the nucleotide sequence as defined in (a) or (b),
(d) a nucleic acid comprising of a nucleotide sequence, which is at least 65% identical to the sequence defined in (a),
(e) a nucleic acid encoding a protein with an amino acid sequence, which is at least 65% identical to the amino acid sequence as given in SEQ ID NO: 18, 2, 4, 6, 8, 10, 14, 16, 20, 22, 24, 26, 28, 30, 32 or 34 or a part thereof,
(f) a nucleic acid encoding a protein comprising the amino acid sequence as given in SEQ ID NO: 18, 2, 4, 6, 8, 10, 14, 16, 20, 22, 24, 26, 28, 30, 32 or 34 or a part thereof,
(g) a nucleic acid which is degenerated as a result of the genetic code to a nucleotide sequence of a nucleic acid as given in SEQ ID NO: 17, 1, 3, 5, 7, 9, 13, 15, 19, 21, 23, 25, 27, 29, 31 or 33 or a part thereof or as defined in (a) to (f),
(h) a nucleic acid which is diverging due to the differences in codon usage between the organisms to a nucleotide sequence encoding a protein as given in SEQ ID NO: 18, 2, 4, 6, 8, 10, 14, 16, 20, 22, 24, 26, 28, 30, 32 or 34 or as defined in (a) to (g),
(i) a nucleic acid which is diverging due to the differences between alleles encoding a protein as given in SEQ ID NO: 18, 2, 4, 6, 8, 10, 14, 16, 20, 22, 24, 26, 28, 30, 32 or 34 or as defined in (a) to (h),
(j) a nucleic acid encoding an immunologically active and/or functional fragment of a protein encoded by a DNA sequence as given in SEQ ID NO: 17, 1, 3, 5, 7, 9, 13, 15, 19, 21, 23, 25, 27, 29, 31 or 33,
(k) a nucleic acid encoding a gene family member of the nucleic acid as given in SEQ ID NO: 17, 1,3, 5, 7, 9, 13, 15, 19, 21, 23, 25, 27, 29, 31 or 33 and,
(l) a nucleic acid encoding a protein as defined in SEQ ID NO: 18, 2, 4, 6, 8, 10, 14, 16, 20, 22, 24, 26, 28, 30, 32 or 34 or a nucleic acid as defined in any one of (a) to (k) **characterized in that** said sequence is DNA, cDNA, genomic DNA or synthetic DNA;
wherein said isolated polypeptide is encodable by a nucleic acid according to (a) to (1), or a variant or a derivative thereof, or an immunologically active and/or functional fragment thereof, preferably said polypeptide comprising an amino acid sequence as given in SEQ ID NO: 18, 2, 4, 6, 8, 10, 14, 16, 20, 22, 24, 26, 28, 30, 32 or 34 or a variant or a derivative thereof, or an immunologically active and/or functional fragment thereof; and
wherein said antibody specifically recognizes said polypeptide or a specific epitope of said polypeptide.


SEQUENCE LISTING

<110>  UNIVERSITEIT MAASTRICHT

<120>  TUMOR ENDOTHELIAL CELL MARKERS AND USES THEREOF

<130>  P6022831PCT

<140>  PCT/EP2006/009496
<141>  2006-09-29

<150>  05447220.4
<151>  2005-09-30

<160>  109

<170>  PatentIn version 3.3

<210>  1
<211>  1462
<212>  DNA
<213>  Homo sapiens

<400>  1
   ggccggctag agccgggggc tgggcgggga ccgggcttgt cggtgaagcg gcagtggcgg      60
   cggcggcggc ggctcggcag gcgggttcag gcttcggggg ccagccgccg ccatgatcct     120
   gctggaggtg aacaaccgca tcatcgagga gacgctcgcg ctcaagttcg agaacgcggc     180
   cgccggaaac aaaccggaag cagtagaagt aacatttgca gatttcgatg gggtcctcta      240
   tcatatttca aatcctaatg gagacaaaac aaaagtgatg gtcagtattt ctttgaaatt     300
   ctacaaggaa cttcaggcac atggtgctga tgagttatta aagagggtgt acgggagttt     360
   cttggtaaat ccagaatcag gatacaatgt ctctttgcta tatgaccttg aaaatcttcc     420
   ggcatccaag gattccattg tgcatcaagc tggcatgttg aagcgaaatt gttttgcctc     480
   tgtctttgaa aaatacttcc aattccaaga agagggcaag gaaggagaga acagggcagt     540
   tatccattat agggatgatg agaccatgta tgttgagtct aaaaaggaca gagtcacagt     600
   agtcttcagc acagtgttta aggatgacga cgatgtggtc attggaaagg tgttcatgca     660
   ggagttcaaa gaaggacgca gagccagcca cacagcccca caggtcctct ttagccacag     720
   ggaacctcct ctggagctga aagacacaga cgccgctgtg ggtgacaaca ttggctacat     780
   tacctttgtg ctgttccctc gtcacaccaa tgccagtgct cgagacaaca ccatcaacct     840
   gatccacacg ttccgggact acctgcacta ccacatcaag tgctctaagg cctatattca     900
   cacacgtatg cgggcgaaaa cgtctgactt cctcaaggtg ctgaaccgcg cacgcccaga     960
   tgccgagaaa aaagaaatga aaacaatcac ggggaagacg ttttcatccc gctaatcttg    1020
   ggaataagag gaggaagcgg ctggcaactg aaggctggaa cacttgctac tggataatcg    1080
   tagcttttaa tgttgcgcct cttcaggttc ttaagggatt ctccgttttg gttccatttt    1140
   gtacacgttt ggaaaataat ctgcagaaac gagctgtgct tgcaaagact tcatagttcc    1200
   caagaattaa aaaaaaaaaa aaaagaattc cacttgatca acttaattcc ttttctttat    1260
   cttccctccc tcacttccct tttctcccac cctcttttcc aagctgtttc gctttgcaat    1320
   atattactgg taatgagttg caggataatg cagtcataac ttgttttctc ctaagtattt    1380
   gagttcaaaa ctcctgtatc taaagaaata cggttggggt cattaataaa gaaaatcttt    1440
   ctatcttaaa aaaaaaaaaa aa                                              1462

<210>  2
<211>  300
<212>  PRT
<213>  Homo sapiens

<400>  2

Met Ile Leu Leu Glu Val Asn Asn Arg Ile Ile Glu Glu Thr Leu Ala
1               5                   10                  15

Leu Lys Phe Glu Asn Ala Ala Ala Gly Asn Lys Pro Glu Ala Val Glu
            20                  25                  30

Val Thr Phe Ala Asp Phe Asp Gly Val Leu Tyr His Ile Ser Asn Pro
        35                  40                  45

Asn Gly Asp Lys Thr Lys Val Met Val Ser Ile Ser Leu Lys Phe Tyr
    50                  55                  60

Lys Glu Leu Gln Ala His Gly Ala Asp Glu Leu Leu Lys Arg Val Tyr
65                  70                  75                  80

Gly Ser Phe Leu Val Asn Pro Glu Ser Gly Tyr Asn Val Ser Leu Leu
                85                  90                  95

Tyr Asp Leu Glu Asn Leu Pro Ala Ser Lys Asp Ser Ile Val His Gln
            100                 105                 110

Ala Gly Met Leu Lys Arg Asn Cys Phe Ala Ser Val Phe Glu Lys Tyr
        115                 120                 125

Phe Gln Phe Gln Glu Glu Gly Lys Glu Gly Glu Asn Arg Ala Val Ile
    130                 135                 140

His Tyr Arg Asp Asp Glu Thr Met Tyr Val Glu Ser Lys Lys Asp Arg
145                 150                 155                 160

Val Thr Val Val Phe Ser Thr Val Phe Lys Asp Asp Asp Asp Val Val
                165                 170                 175

Ile Gly Lys Val Phe Met Gln Glu Phe Lys Glu Gly Arg Arg Ala Ser
            180                 185                 190

His Thr Ala Pro Gln Val Leu Phe Ser His Arg Glu Pro Pro Leu Glu
        195                 200                 205

Leu Lys Asp Thr Asp Ala Ala Val Gly Asp Asn Ile Gly Tyr Ile Thr
    210                 215                 220

Phe Val Leu Phe Pro Arg His Thr Asn Ala Ser Ala Arg Asp Asn Thr
225                 230                 235                 240

Ile Asn Leu Ile His Thr Phe Arg Asp Tyr Leu His Tyr His Ile Lys
                245                 250                 255

Cys Ser Lys Ala Tyr Ile His Thr Arg Met Arg Ala Lys Thr Ser Asp
            260                 265                 270

Phe Leu Lys Val Leu Asn Arg Ala Arg Pro Asp Ala Glu Lys Lys Glu
        275                 280                 285

Met Lys Thr Ile Thr Gly Lys Thr Phe Ser Ser Arg
    290                 295                 300

<210>  3
<211>  7633
<212>  DNA
<213>  Homo sapiens

<400>  3
gggccggggg gcggagcctt gcgggctgga gcgaaagaat gcgggggctg agcgcagaag      60

cggctcgagg ctggaagagg atcttgggcg ccgccaggtt ctgtggacaa tcacaatggg     120

aatccaagga gggtctgtcc tgttcgggct gctgctcgtc ctggctgtct tctgccattc     180

aggtcatagc ctgcagtgct acaactgtcc taacccaact gctgactgca aaacagccgt     240

caattgttca tctgattttg atgcgtgtct cattaccaaa gctgggttac aagtgtataa     300

caagtgttgg aagtttgagc attgcaattt caacgacgtc acaacccgct tgagggaaaa     360

tgagctaacg tactactgct gcaagaagga cctgtgtaac tttaacgaac agcttgaaaa     420

tggtgggaca tccttatcag agaaaacagt tcttctgctg gtgactccat ttctggcagc     480

agcctggagc cttcatccct aagtcaacac caggagagct tctcccaaac tccccgttcc     540

tgcgtagtcc gctttctctt gctgccacat tctaaaggct tgatattttc caaatggatc     600

ctgttgggaa agaataaaat tagcttgagc aacctggcta agatagaggg gctctgggag     660

actttgaaga ccagtcctgt ttgcagggaa gccccacttg aaggaagaag tctaagagtg     720

aagtaggtgt gacttgaact agattgcatg cttcctcctt tgctcttggg aagaccagct     780

ttgcagtgac agcttgagtg ggttctctgc agccctcaga ttatttttcc tctggctcct     840

tggatgtagt cagttagcat cattagtaca tctttggagg gtggggcagg agtatatgag     900

catcctctct cacatggaac gctttcataa acttcaggga tcccgtgttg ccatggaggc     960

atgccaaatg ttccatatgt gggtgtcagt cagggacaac aagatcctta atgcagagct    1020

agaggacttc tggcagggaa gtggggaagt gttccagata gcagggcatg aaaacttaga    1080

gaggtacaag tggctgaaaa tcgagttttt cctctgtctt taaattttat atgggctttg    1140

ttatcttcca ctggaaaagt gtaatagcat acatcaatgg tgtgttaaag ctatttcctt    1200

gccttttttt tattggaatg gtaggatatc ttggctttgc cacacacagt tacagagtga    1260

acactctact acatgtgact ggcagtatta agtgtgctta ttttaaatgt tactggtaga    1320

aaggcagttc aggtatgtgt gtatatagta tgaatgcagt ggggacaccc tttgtggtta 1380
cagtttgaga cttccaaagg tcatccttaa taacaacaga tctgcagggg tatgttttac 1440
catctgcatc cagcctcctg ctaactccta gctgactcag catagattgt ataaaatacc 1500
tttgtaacgg ctcttagcac actcacagat gtttgaggct ttcagaagct cttctaaaaa 1560
atgatacaca cctttcacaa gggcaaactt tttccttttc cctgtgtatt ctagtgaatg 1620
aatctcaaga ttcagtagac ctaatgacat ttgtatttta tgatcttggc tgtatttaat 1680
ggcataggct gacttttgca gatggaggaa tttcttgatt aatgttgaaa aaaaaccctt 1740
gattatactc tgttggacaa accgagtgca atgaatgatg cttttctgaa aatgaaatat 1800
aacaagtggg tgaatgtggt tatggccgaa aaggatatgc agtatgctta atggtagcaa 1860
ctgaaagaag acatcctgag cagtgccagc tttcttctgt tgatgccgtt ccctgaacat 1920
aggaaaatag aaacttgctt atcaaaactt agcattacct tggtgctctg tgttctctgt 1980
tagctcagtg tctttcctta catcaatagg tttttttttt tttttttggc ctgaggaagt 2040
actgaccatg cccacagcca ccggctgagc aaagaagctc atttcatgtg agttctaagg 2100
aatgagaaac aattttgatg aatttaagca gaaaatgaat ttctgggaac tttttggggg 2160
gcggggggtt ggggaattca gccacactcc agaaagccag gagtcgacag ttttggaagc 2220
ctctctcagg attgagattc taggatgaga ttggcttact gctatcttgt gtcatgtacc 2280
cactttttgg ccagactaca ctgggaagaa ggtagtcctc taaagcaaaa tctgagtgcc 2340
actaaatggg gagatggggc tgttaagctg tccaaatcaa caagggtcat ataaatggcc 2400
ttaaactttg gggttgcttt ctgcaaaaag ttgctgtgac tcatgccata gacaaggttg 2460
agtgcctgga cccaaaggca atactgtaat gtaaagacat ttatagtact aggcaaacag 2520
caccccaggt actccaggcc ctcctggctg gagagggctg tggcaataga aaattagtgc 2580
caactgcagt gagtcagcct aggttaaata gagagtgtaa gagtgctgga caggaacctc 2640
caccctcatg tcacatttct tcaatgtgac ccttctggcc cctctcctcc tgacagcgga 2700
acaatgactg ccccgatagg tgaggctgga ggaagaatca gtcctgtcct tggcaagctc 2760
ttcactatga cagtaaaggc tctctgcctg ctgccaaggc ctgtgacttt ctaacctggc 2820
ctcacgctgg gtaagcttaa ggtagaggtg caggattagc aagcccacct ggctaccagg 2880
ccgacagcta catcctccaa ctgaccctga tcaacgaaga gggattcatg tgtctgtctc 2940
agttggttcc aaatgaaacc agggagcagg ggagttagga atcgaacacc agtcatgcct 3000
actggctctc tgctcgagag ccaataccct gtgccctcca ctcatctgga tttacaggaa 3060
ctgtcatagt gttcagtatt gggtggtgat aagcccattg gattgtcccc ttggggggat 3120
gagctagggg tgcaaggaac acctgatgag tagataagtg gagctcatgg tatttcctga 3180
aagatgctaa tctatttgcc aaacttggtc ttgaatgtac tgggggcttc aaggtatggg 3240
tatatttttc ttgtgtcctt gcagttagcc cccatgtctt atgtgtgtcc tgaaaaaata 3300
agagcctgcc caagactttg ggcctcttga cagaattaac cacttttata catctgagtt 3360

ctcttggtaa gttctttagc agtgttcaaa gtctactagc tcgcattagt ttctgttgct 3420
gccaacagat ctgaactaat gctaacagat ccccctgagg gattcttgat gggctgagca 3480
gctggctgga gctagtactg actgacattc attgtgatga gggcagcttt ctggtacagg 3540
attctaagct ctatgtttta tatacatttt catctgtact tgcacctcac tttacacaag 3600
aggaaactat gcaaagttag ctggatcgct caaggtcact taggtaagtt ggcaagtcca 3660
tgcttcccac tcagctcctc aggtcagcaa gtctacttct ctgcctattt tgtatactct 3720
ctttaatatg tgcctagctt tggaaagtct agaatgggtc cctggtgcct ttttactttg 3780
aagaaatcag tttctgcctc tttttggaaa agaaaacaaa gtgcaattgt tttttactgg 3840
aaagttaccc aatagcatga ggtgaacagg acgtagttag gccttcctgt aaacagaaaa 3900
tcatatcaaa acactatctt cccatctgtt tctcaatgcc tgctacttct tgtagatatt 3960
tcatttcagg agagcagcag ttaaacccgt ggattttgta gttaggaacc tgggttcaaa 4020
ccctcttcca ctaattggct atgtctctgg acaagttttt tttttttttt ttttttaaac 4080
cctttctgaa ctttcacttt ctatgtctac ctcaaagaat tgttgtgagg cttgagataa 4140
tgcatttgta aagggtctgc cagataggaa gatgctagtt atggatttac aaggttgtta 4200
aggctgtaag agtctaaaac ctacagtgaa tcacaatgca tttaccccca ctgacttgga 4260
cataagtgaa aactagccag aagtctcttt ttcaaattac ttacaggtta ttcaatataa 4320
aatttttgta atggataatc ttatttatct aaactaaagc ttcctgttta tacacactcc 4380
tgttattctg ggataagata aatgaccaca gtaccttaat ttctaggtgg gtgcctgtga 4440
tggttcattg taggtaagga cattttctct ttttcagcag ctgtgtaggt ccagagcctc 4500
tgggagagga ggggggtagc atgcacccag caggggactg aactgggaaa ctcaaggttc 4560
tttttactgt ggggtagtga gctgcctttc tgtgatcggt ttccctaggg atgttgctgt 4620
tcccctcctt gctattcgca gctacataca acgtggccaa ccccagtagg ctgatcctat 4680
atatgatcag tgctggtgct gactctcaat agccccaccc aagctggcta taggtttaca 4740
gatacattaa ttaggcaacc taaaatattg atgctggtgt tggtgtgaca taatgctatg 4800
gccagaactg aaacttagag ttataattca tgtattaggg ttctccagag ggacagaatt 4860
agtaggatat atgtatatat gaaagggagg ttattaggga gaactggctc ccacagttag 4920
aaggcgaagt cgcacaatag gccgtctgca agctgggtta gagagaagcc agtagtggct 4980
cagcctgagt tcaaaaacct caaaactggg gaagctgaca gtgcagccag ccttcagtct 5040
gtggccaaag gcccaagagc ccctggcaac caacccactg gtgcaagtcc tagattccaa 5100
aggctgaaga acctggagtc tgatgtccaa gagcaggaag agtggaagaa agccagaaga 5160
ctcagcaaac aaggtagaca gtgtctacca ccatagtggc cataccaaag aggctaccga 5220
ttccttcctg ctacctggat ccctgaagtt gccctggtct ctgcaccttc taaacctagt 5280
tcttaagagc tttccattac atgagctgtc tcaaagccct ccaataaatt ctcagtgtaa 5340
gcttctgttg cttgtggaca gaaaattctg acagacctac cctataagtg ttactgtcag 5400
gataacatga gaacgcacaa cagtaagtgg tcactaagtg ttagctacgg ttattttgcc 5460

caaggtagca tggctagttg atgccggttg atggggctta aacccagctc cctcatcttc 5520
caggcctctg tactccctat tccactaaac tacctctcag gtttattttt ttaaattctt 5580
actctgcaag tacataggac cacatttacc tgggaaaaca agaataaagg ctgctctgca 5640
ttttttagaa acttttttga aagggagatg ggaatgcctg cacccccaag tccagaccaa 5700
cacaatggtt aattgagatg aataataaag gaaagactgt tctgggcttc ccagaatagc 5760
ttggtcctta aattgtggca caaacaacct cctgtcagag ccagcctcct gccaggaaga 5820
ggggtaggag actagaggcc gtgtgtgcag ccttgccctg aaggctaggg tgacaatttg 5880
gaggctgtcc aaacaccctg gcctctagag ctggcctgtc tatttgaaat gccggctctg 5940
atgctaatcg gcgaccctca ggcaagttac ttaaccttac atgcctcagt tttctcatct 6000
ggaaaatgag aaccctaggt ttagggttgt tagaaaagtt aaatgagtta agacaagtgc 6060
ctgggacaca gtagcctctt gtgtgtgttt atcattatgt cctcagcagg tcgtagaagc 6120
agcttctcag gtgtgaggct ggcgcattat ctggagtggg ttgggttttc taggatggac 6180
cccctgctgc attttcctca ttcatccacc agggcttaat ggggaatcaa ggaatccatg 6240
tgtaactgta taataactgt agccacactc caatgaccac ctactagttg tccctggcac 6300
tgcttataca tatgtccatc aaatcaatcc tatgaagtag atactgtctt cattttatag 6360
atcagagaca attggggttc agagagctga tgtgattttc ccagggtcac agagagtccc 6420
agattcaggc acaactcttg tattccaaga cacaaccact acatgtccaa aggctgccca 6480
gagccaccgg gcacggcaaa ttgtgacata tccctaaaga ggctgagcac ctggtcagga 6540
tctgatggct gacagtgtgt ccagatgcag agctggagtg ggggagggga aggggggctc 6600
cttgggacag agaaggcttt ctgtgctttc tctgaaggga gcagtctgag gaccaaggga 6660
acccggcaaa cagcacctca ggtactccag gccctcctgg ctggagaggg ctgtggcaat 6720
ggaaaattag tgccaactgc aatgagtcag cctcggttaa atagagagtg aagaatgctg 6780
gacaggaacc tccaccctca tgtcacattt cttcagtgtg acccttctgg cccctctcct 6840
cctgacagcg gaacaatgac tgccccgata ggtgaggctg gaggaagaat cagtcctgtc 6900
cttggcaagc tcttcactat gacagtaaag gctctctgcc tgctgccaag gcctgtgact 6960
ttctaacctg gcctcacgct gggtaagctt aaggtagagg tgcaggatta gcaagcccac 7020
ctggctacca ggccgacagc tacatctttc aactgaccct gatcaacgaa gagggacttg 7080
tgtctctcag ttggttccaa atgaaaccag ggagcagggg cgttaggaag ctccaacagg 7140
atggtactta atggggcatt tgagtggaga ggtaggtgac atagtgcttt ggagcccagg 7200
gagggaaagg ttctgctgaa gttgaattca agactgttct ttcatcacaa acttgagttt 7260
cctggacatt tgtttgcaga aacaaccgta gggttttgcc ttaacctcgt gggtttatta 7320
ttacctcata gggactttgc ctcctgacag cagtttatgg gtgttcattg tggcacttga 7380
gttttcttgc atacttgtta gagaaaccaa gtttgtcatc aacttcttat ttaaccccct 7440
ggctataact tcatggatta tgttataatt aagccatcca gagtaaaatc tgtttagatt 7500

atcttggagt aagggggaaa aaatctgtaa ttttttctcc tcaactagat atatacataa 7560

aaaatgattg tattgcttca tttaaaaaat ataacgcaaa atctcttttc cttctaaaaa 7620

aaaaaaaaaa aaa 7633

<210> 4
<211> 128
<212> PRT
<213> Homo sapiens

<400> 4

Met Gly Ile Gln Gly Gly Ser Val Leu Phe Gly Leu Leu Leu Val Leu
1               5                   10                  15

Ala Val Phe Cys His Ser Gly His Ser Leu Gln Cys Tyr Asn Cys Pro
            20                  25                  30

Asn Pro Thr Ala Asp Cys Lys Thr Ala Val Asn Cys Ser Ser Asp Phe
        35                  40                  45

Asp Ala Cys Leu Ile Thr Lys Ala Gly Leu Gln Val Tyr Asn Lys Cys
    50                  55                  60

Trp Lys Phe Glu His Cys Asn Phe Asn Asp Val Thr Thr Arg Leu Arg
65                  70                  75                  80

Glu Asn Glu Leu Thr Tyr Tyr Cys Cys Lys Lys Asp Leu Cys Asn Phe
                85                  90                  95

Asn Glu Gln Leu Glu Asn Gly Gly Thr Ser Leu Ser Glu Lys Thr Val
            100                 105                 110

Leu Leu Leu Val Thr Pro Phe Leu Ala Ala Ala Trp Ser Leu His Pro
        115                 120                 125

<210> 5
<211> 1627
<212> DNA
<213> Homo sapiens

<400> 5
gcccccacca tcaaggggaa gaaagtgctc ttcggattcc ggttcgccct ggcctcccgc 60

agccgccgcg ggaccggccc ccagcacacc cccggggcgc cgggcgcggg gcagccgccc 120

ggacgcgcgc gggcctcagg cgccgccggg accccagccc cccaaacttt ggcaagttgc 180

gggcgccgag cgcacccgga ggcgcggggc gcggccgcag gcggagccgc cccctgacgc 240

cgggccgccc cctcccggcc ccggccgccc cgccggctcc gcggaaagtt tgcggccgcc 300

cctgcgccgc acccggggcc tgggtgagac tgcggcggcg gcagggcgcg gacggccata 360

tttgccggcg cggcccgagc cgccgacaac aaaaagtgcg cgggcgctcg gcgggcgctc 420

ggacgggcgc ggggctgcag cgctaccgcc cggcctcgcc gccgccgccg ccgccctcgc 480

ggcctggccc cgccgcgccc ggcgcgcccg ccgcccgggg ggatgtctta caaaccgaac 540

ttggccgcgc acatgcccgc cgccgccctc aacgccgctg ggagtgtcca ctcgccttcc 600

accagcatgg caacgtcttc acagtaccgc cagctgctca gtgactacgg gccaccgtcc 660

ctaggctaca cccagggaac tgggaacagc caggtgcccc aaagcaaata cgcggagctg 720

ctggccatca ttgaagagct ggggaaggag atcagaccca cgtacgcagg gagcaagagt 780

gccatggaga ggctgaagcg cggcatcatt cacgctagag gactggttcg ggagtgcttg 840

gcagaaacgg aacggaatgc cagatcctag ctgccttgtt ggttttgaag gatttccatc 900

tttttacaag atgagaagtt acagttcatc tcccctgttc agatgaaacc cttgttttca 960

aaatggttac agtttcgttt ttcctcccat ggttcacttg gctctgaacc tacagtctca 1020

aagattgaga aaagattttg cagttaatta ggatttgcat tttaagtagt taggaactgc 1080

ccaggttttt tttgtttttt aagcattgat ttaaaagatg cacggaaagt tatcttacag 1140

caaactgtag tttgcctcca agacaccatt gtctcccttt aatcttctct tttgtataca 1200

tttgttaccc atggtgttct ttgttccttt tcataagcta ataccactgt agggattttg 1260

ttttgaacgc atattgacag cacgctttac ttagtagccg gttcccattt gccatacaat 1320

gtaggttctg cttaatgtaa cttctttttt gcttaagcat ttgcatgact attagtgctt 1380

caaagtcaat tttaaaaaat gcacaagtta taaatacaga agaaagagca acccaccaaa 1440

cctaacaagg acccccgaac actttcatac taagactgta agtagatctc agttctgcgt 1500

ttattgtaag ttgataaaaa catctggaag aaaatgacta aaactgtttg catctttgta 1560

tgtatttatt acttgatgta ataaagctta ttttcattaa caatttgtat taaaaaaaaa 1620

aaaaaaa 1627

<210> 6
<211> 115
<212> PRT
<213> Homo sapiens

<400> 6

Met Ser Tyr Lys Pro Asn Leu Ala Ala His Met Pro Ala Ala Ala Leu
1               5                   10                  15

Asn Ala Ala Gly Ser Val His Ser Pro Ser Thr Ser Met Ala Thr Ser
            20                  25                  30

Ser Gln Tyr Arg Gln Leu Leu Ser Asp Tyr Gly Pro Pro Ser Leu Gly
        35                  40                  45

Tyr Thr Gln Gly Thr Gly Asn Ser Gln Val Pro Gln Ser Lys Tyr Ala
    50                  55                  60

Glu Leu Leu Ala Ile Ile Glu Glu Leu Gly Lys Glu Ile Arg Pro Thr
65                  70                  75                  80

Tyr Ala Gly Ser Lys Ser Ala Met Glu Arg Leu Lys Arg Gly Ile Ile
                85                  90                  95

His Ala Arg Gly Leu Val Arg Glu Cys Leu Ala Glu Thr Glu Arg Asn
            100                 105                 110

Ala Arg Ser
        115

<210>  7
<211>  5921
<212>  DNA
<213>  Homo sapiens

<400>  7
   agcagacggg agtttctcct cggggtcgga gcaggaggca cgcggagtgt gaggccacgc      60
   atgagcggac gctaaccccc tccccagcca caaagagtct acatgtctag ggtctagaca     120
   tgttcagctt tgtggacctc cggctcctgc tcctcttagc ggccaccgcc ctcctgacgc     180
   acggccaaga ggaaggccaa gtcgagggcc aagacgaaga catcccacca atcacctgcg     240
   tacagaacgg cctcaggtac catgaccgag acgtgtggaa acccgagccc tgccggatct     300
   gcgtctgcga caacggcaag gtgttgtgcg atgacgtgat ctgtgacgag accaagaact     360
   gccccggcgc cgaagtcccc gagggcgagt gctgtcccgt ctgccccgac ggctcagagt     420
   cacccaccga ccaagaaacc accggcgtcg agggacccaa gggagacact ggcccccgag     480
   gcccaagggg acccgcaggc ccccctggcc gagatggcat ccctggacag cctggacttc     540
   ccggaccccc cggacccccc ggacctcccg gaccccctgg cctcggagga aactttgctc     600
   cccagctgtc ttatggctat gatgagaaat caaccggagg aatttccgtg cctggcccca     660
   tgggtccctc tggtcctcgt ggtctccctg gcccccctgg tgcacctggt ccccaaggct     720
   tccaaggtcc ccctggtgag cctggcgagc ctggagcttc aggtcccatg ggtccccgag     780
   gtcccccagg tccccctgga aagaatggag atgatgggga agctggaaaa cctggtcgtc     840
   ctggtgagcg tgggcctcct gggcctcagg gtgctcgagg attgcccgga acagctggcc     900
   tccctggaat gaagggacac agaggtttca gtggtttgga tggtgccaag ggagatgctg     960
   gtcctgctgg tcctaagggt gagcctggca gccctggtga aaatggagct cctggtcaga    1020
   tgggcccccg tggcctgcct ggtgagagag gtcgccctgg agcccctggc cctgctggtg    1080
   ctcgtggaaa tgatggtgct actggtgctg ccgggccccc tggtcccacc ggccccgctg    1140
   gtcctcctgg cttccctggt gctgttggtg ctaagggtga agctggtccc caagggcccc    1200
   gaggctctga aggtccccag ggtgtgcgtg gtgagcctgg cccccctggc cctgctggtg    1260
   ctgctggccc tgctggaaac cctggtgctg atggacagcc tggtgctaaa ggtgccaatg    1320
   gtgctcctgg tattgctggt gctcctggct tccctggtgc ccgaggcccc tctggacccc    1380
   agggccccgg cggccctcct ggtcccaagg gtaacagcgg tgaacctggt gctcctggca    1440
   gcaaaggaga cactggtgct aagggagagc ctggccctgt tggtgttcaa ggaccccctg    1500
   gccctgctgg agaggaagga aagcgaggag ctcgaggtga acccggaccc actggcctgc    1560
   ccggaccccc tggcgagcgt ggtggacctg gtagccgtgg tttccctggc gcagatggtg    1620
   ttgctggtcc caagggtccc gctggtgaac gtggttctcc tggccccgct ggccccaaag 1680
   gatctcctgg tgaagctggt cgtcccggtg aagctggtct gcctggtgcc aagggtctga 1740
   ctggaagccc tggcagccct ggtcctgatg gcaaaactgg cccccctggt cccgccggtc 1800
   aagatggtcg ccccggaccc ccaggcccac ctggtgcccg tggtcaggct ggtgtgatgg 1860
   gattccctgg acctaaaggt gctgctggag agcccggcaa ggctggagag cgaggtgttc 1920
   ccggaccccc tggcgctgtc ggtcctgctg gcaaagatgg agaggctgga gctcagggac 1980
   cccctggccc tgctggtccc gctggcgaga gaggtgaaca aggccctgct ggctcccccg 2040
   gattccaggg tctccctggt cctgctggtc ctccaggtga agcaggcaaa cctggtgaac 2100
   agggtgttcc tggagacctt ggcgcccctg gcccctctgg agcaagaggc gagagaggtt 2160
   tccctggcga gcgtggtgtg caaggtcccc ctggtcctgc tggaccccga ggggccaacg 2220
   gtgctcccgg caacgatggt gctaagggtg atgctggtgc ccctggagct cccggtagcc 2280
   agggcgcccc tggccttcag ggaatgcctg gtgaacgtgg tgcagctggt cttccagggc 2340
   ctaagggtga cagaggtgat gctggtccca aaggtgctga tggctctcct ggcaaagatg 2400
   gcgtccgtgg tctgaccggc cccattggtc ctcctggccc tgctggtgcc cctggtgaca 2460
   agggtgaaag tggtcccagc ggccctgctg gtcccactgg agctcgtggt gcccccggag 2520
   accgtggtga gcctggtccc cccggccctg ctggctttgc tggccccctt ggtgctgacg 2580
   gccaacctgg tgctaaaggc gaacctggtg atgctggtgc caaaggcgat gctggtcccc 2640
   ctgggcctgc cggacccgct ggaccccctg gccccattgg taatgttggt gctcctggag 2700
   ccaaaggtgc tcgcggcagc gctggtcccc ctggtgctac tggtttccct ggtgctgctg 2760
   gccgagtcgg tcctcctggc ccctctggaa atgctggacc ccctggccct cctggtcctg 2820
   ctggcaaaga aggcggcaaa ggtccccgtg gtgagactgg ccctgctgga cgtcctggtg 2880
   aagttggtcc ccctggtccc cctggccctg ctggcgagaa aggatcccct ggtgctgatg 2940
   gtcctgctgg tgctcctggt actcccgggc ctcaaggtat tgctggacag cgtggtgtgg 3000
   tcggcctgcc tggtcagaga ggagagagag gcttccctgg tcttcctggc ccctctggtg 3060
   aacctggcaa acaaggtccc tctggagcaa gtggtgaacg tggtcccccc ggtcccatgg 3120
   gcccccctgg attggctgga ccccctggtg aatctggacg tgagggggct cctgctgccg 3180
   aaggttcccc tggacgagac ggttctcctg gcgccaaggg tgaccgtggt gagaccggcc 3240
   ccgctggacc ccctggtgct cctggtgctc ctggtgcccc tggccccgtt ggccctgctg 3300
   gcaagagtgg tgatcgtggt gagactggtc ctgctggtcc cgccggtccc gtcggccccg 3360
   tcggcgcccg tggccccgcc ggaccccaag gcccccgtgg tgacaagggt gagacaggcg 3420
   aacagggcga cagaggcata aagggtcacc gtggcttctc tggcctccag ggtccccctg 3480
   gccctcctgg ctctcctggt gaacaaggtc cctctggagc ctctggtcct gctggtcccc 3540
   gaggtccccc tggctctgct ggtgctcctg gcaaagatgg actcaacggt ctccctggcc 3600
   ccattgggcc ccctggtcct cgcggtcgca ctggtgatgc tggtcctgtt ggtccccccg 3660
   gccctcctgg acctcctggt ccccctggtc ctcccagcgc tggtttcgac ttcagcttcc 3720
   tgccccagcc acctcaagag aaggctcacg atggtggccg ctactaccgg gctgatgatg 3780
   ccaatgtggt tcgtgaccgt gacctcgagg tggacaccac cctcaagagc ctgagccagc 3840
   agatcgagaa catccggagc ccagagggaa gccgcaagaa ccccgcccgc acctgccgtg 3900
   acctcaagat gtgccactct gactggaaga gtggagagta ctggattgac cccaaccaag 3960
   gctgcaacct ggatgccatc aaagtcttct gcaacatgga gactggtgag acctgcgtgt 4020
   accccactca gcccagtgtg gcccagaaga actggtacat cagcaagaac cccaaggaca 4080
   agaggcatgt ctggttcggc gagagcatga ccgatggatt ccagttcgag tatggcggcc 4140
   agggctccga ccctgccgat gtggccatcc agctgacctt cctgcgcctg atgtccaccg 4200
   aggcctccca gaacatcacc taccactgca agaacagcgt ggcctacatg gaccagcaga 4260
   ctggcaacct caagaaggcc ctgctcctca agggctccaa cgagatcgag atccgcgccg 4320
   agggcaacag ccgcttcacc tacagcgtca ctgtcgatgg ctgcacgagt cacaccggag 4380
   cctggggcaa gacagtgatt gaatacaaaa ccaccaagtc ctcccgcctg cccatcatcg 4440
   atgtggcccc cttggacgtt ggtgccccag accaggaatt cggcttcgac gttggccctg 4500
   tctgcttcct gtaaactccc tccatcccaa cctggctccc tcccacccaa ccaactttcc 4560
   ccccaacccg gaaacagaca agcaacccaa actgaacccc cccaaaagcc aaaaaatggg 4620
   agacaatttc acatggactt tggaaaatat ttttttcctt tgcattcatc tctcaaactt 4680
   agtttttatc tttgaccaac cgaacatgac caaaaaccaa aagtgcattc aaccttacca 4740
   aaaaaaaaaa aaaaaaaaaa agaataaata aataagtttt taaaaaagga agcttggtcc 4800
   acttgcttga agacccatgc gggggtaagt ccctttctgc ccgttgggtt atgaaacccc 4860
   aatgctgccc tttctgctcc tttctccaca ccccccttgg cctcccctcc actccttccc 4920
   aaatctgtct ccccagaaga cacaggaaac aatgtattgt ctgcccagca atcaaaggca 4980
   atgctcaaac acccaagtgg cccccaccct cagcccgctc ctgcccgccc agcaccccca 5040
   ggccctgggg acctggggtt ctcagactgc caaagaagcc ttgccatctg gcgctcccat 5100
   ggctcttgca acatctcccc ttcgtttttg agggggtcat gccgggggag ccaccagccc 5160
   ctcactgggt tcggaggaga gtcaggaagg gccacgacaa agcagaaaca tcggatttgg 5220
   ggaacgcgtg tcatcccttg tgccgcaggc tgggcgggag agactgttct gttctgttcc 5280
   ttgtgtaact gtgttgctga aagactacct cgttcttgtc ttgatgtgtc accggggcaa 5340
   ctgcctgggg gcggggatgg gggcagggtg gaagcggctc cccattttta taccaaaggt 5400
   gctacatcta tgtgatgggt ggggtgggga gggaatcact ggtgctatag aaattgagat 5460
   gcccccccag gccagcaaat gttccttttt gttcaaagtc tatttttatt ccttgatatt 5520
   ttttctttct tttttttttt ttttgtggat ggggacttgt gaatttttct aaaggtgcta 5580
   tttaacatgg gaggagagcg tgtgcgctcc agcccagccc gctgctcact ttccaccctc 5640
   tctccacctg cctctggctt ctcaggcctc tgctctccga cctctctcct ctgaaaccct 5700
   cctccacagc tgcagcccat cctcccggct ccctcctagt ctgtcctgcg tcctctgtcc 5760
   ccgggtttca gagacaactt cccaaagcac aaagcagttt ttccctaggg gtgggaggaa 5820
   gcaaaagact ctgtacctat tttgtatgtg tataataatt tgagatgttt ttaattattt 5880
   tgattgctgg aataaagcat gtggaaatga cccaaacata a 5921

<210> 8
<211> 1464
<212> PRT
<213> Homo sapiens

<400> 8

Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125

Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

Pro Gln Leu Ser Tyr Gly Tyr Asp Glu Lys Ser Thr Gly Gly Ile Ser
                165                 170                 175

Val Pro Gly Pro Met Gly Pro Ser Gly Pro Arg Gly Leu Pro Gly Pro
            180                 185                 190

Pro Gly Ala Pro Gly Pro Gln Gly Phe Gln Gly Pro Pro Gly Glu Pro
        195                 200                 205

Gly Glu Pro Gly Ala Ser Gly Pro Met Gly Pro Arg Gly Pro Pro Gly
    210                 215                 220

Pro Pro Gly Lys Asn Gly Asp Asp Gly Glu Ala Gly Lys Pro Gly Arg
225                 230                 235                 240

Pro Gly Glu Arg Gly Pro Pro Gly Pro Gln Gly Ala Arg Gly Leu Pro
                245                 250                 255

Gly Thr Ala Gly Leu Pro Gly Met Lys Gly His Arg Gly Phe Ser Gly
            260                 265                 270

Leu Asp Gly Ala Lys Gly Asp Ala Gly Pro Ala Gly Pro Lys Gly Glu
        275                 280                 285

Pro Gly Ser Pro Gly Glu Asn Gly Ala Pro Gly Gln Met Gly Pro Arg
    290                 295                 300

Gly Leu Pro Gly Glu Arg Gly Arg Pro Gly Ala Pro Gly Pro Ala Gly
305                 310                 315                 320

Ala Arg Gly Asn Asp Gly Ala Thr Gly Ala Ala Gly Pro Pro Gly Pro
                325                 330                 335

Thr Gly Pro Ala Gly Pro Pro Gly Phe Pro Gly Ala Val Gly Ala Lys
            340                 345                 350

Gly Glu Ala Gly Pro Gln Gly Pro Arg Gly Ser Glu Gly Pro Gln Gly
        355                 360                 365

Val Arg Gly Glu Pro Gly Pro Pro Gly Pro Ala Gly Ala Ala Gly Pro
    370                 375                 380

Ala Gly Asn Pro Gly Ala Asp Gly Gln Pro Gly Ala Lys Gly Ala Asn
385                 390                 395                 400

Gly Ala Pro Gly Ile Ala Gly Ala Pro Gly Phe Pro Gly Ala Arg Gly
                405                 410                 415

Pro Ser Gly Pro Gln Gly Pro Gly Gly Pro Pro Gly Pro Lys Gly Asn
            420                 425                 430

Ser Gly Glu Pro Gly Ala Pro Gly Ser Lys Gly Asp Thr Gly Ala Lys
        435                 440                 445

Gly Glu Pro Gly Pro Val Gly Val Gln Gly Pro Pro Gly Pro Ala Gly
    450                 455                 460

Glu Glu Gly Lys Arg Gly Ala Arg Gly Glu Pro Gly Pro Thr Gly Leu
465                 470                 475                 480

Pro Gly Pro Pro Gly Glu Arg Gly Gly Pro Gly Ser Arg Gly Phe Pro
                485                 490                 495

Gly Ala Asp Gly Val Ala Gly Pro Lys Gly Pro Ala Gly Glu Arg Gly
            500                 505                 510

Ser Pro Gly Pro Ala Gly Pro Lys Gly Ser Pro Gly Glu Ala Gly Arg
        515                 520                 525

Pro Gly Glu Ala Gly Leu Pro Gly Ala Lys Gly Leu Thr Gly Ser Pro
    530                 535                 540

Gly Ser Pro Gly Pro Asp Gly Lys Thr Gly Pro Pro Gly Pro Ala Gly
545                 550                 555                 560

Gln Asp Gly Arg Pro Gly Pro Pro Gly Pro Pro Gly Ala Arg Gly Gln
                565                 570                 575

Ala Gly Val Met Gly Phe Pro Gly Pro Lys Gly Ala Ala Gly Glu Pro
            580                 585                 590

Gly Lys Ala Gly Glu Arg Gly Val Pro Gly Pro Pro Gly Ala Val Gly
        595                 600                 605

Pro Ala Gly Lys Asp Gly Glu Ala Gly Ala Gln Gly Pro Pro Gly Pro
    610                 615                 620

Ala Gly Pro Ala Gly Glu Arg Gly Glu Gln Gly Pro Ala Gly Ser Pro
625                 630                 635                 640

Gly Phe Gln Gly Leu Pro Gly Pro Ala Gly Pro Pro Gly Glu Ala Gly
                645                 650                 655

Lys Pro Gly Glu Gln Gly Val Pro Gly Asp Leu Gly Ala Pro Gly Pro
            660                 665                 670

Ser Gly Ala Arg Gly Glu Arg Gly Phe Pro Gly Glu Arg Gly Val Gln
        675                 680                 685

Gly Pro Pro Gly Pro Ala Gly Pro Arg Gly Ala Asn Gly Ala Pro Gly
    690                 695                 700

Asn Asp Gly Ala Lys Gly Asp Ala Gly Ala Pro Gly Ala Pro Gly Ser
705                 710                 715                 720

Gln Gly Ala Pro Gly Leu Gln Gly Met Pro Gly Glu Arg Gly Ala Ala
                725                 730                 735

Gly Leu Pro Gly Pro Lys Gly Asp Arg Gly Asp Ala Gly Pro Lys Gly
            740                 745                 750

Ala Asp Gly Ser Pro Gly Lys Asp Gly Val Arg Gly Leu Thr Gly Pro
        755                 760                 765

Ile Gly Pro Pro Gly Pro Ala Gly Ala Pro Gly Asp Lys Gly Glu Ser
    770                 775                 780

Gly Pro Ser Gly Pro Ala Gly Pro Thr Gly Ala Arg Gly Ala Pro Gly
785                 790                 795                 800

Asp Arg Gly Glu Pro Gly Pro Pro Gly Pro Ala Gly Phe Ala Gly Pro
                805                 810                 815

Pro Gly Ala Asp Gly Gln Pro Gly Ala Lys Gly Glu Pro Gly Asp Ala
            820                 825                 830

Gly Ala Lys Gly Asp Ala Gly Pro Pro Gly Pro Ala Gly Pro Ala Gly
        835                 840                 845

Pro Pro Gly Pro Ile Gly Asn Val Gly Ala Pro Gly Ala Lys Gly Ala
    850                 855                 860

Arg Gly Ser Ala Gly Pro Pro Gly Ala Thr Gly Phe Pro Gly Ala Ala
865                 870                 875                 880

Gly Arg Val Gly Pro Pro Gly Pro Ser Gly Asn Ala Gly Pro Pro Gly
                885                 890                 895

Pro Pro Gly Pro Ala Gly Lys Glu Gly Gly Lys Gly Pro Arg Gly Glu
            900                 905                 910

Thr Gly Pro Ala Gly Arg Pro Gly Glu Val Gly Pro Pro Gly Pro Pro
        915                 920                 925

Gly Pro Ala Gly Glu Lys Gly Ser Pro Gly Ala Asp Gly Pro Ala Gly
    930                 935                 940

Ala Pro Gly Thr Pro Gly Pro Gln Gly Ile Ala Gly Gln Arg Gly Val
945                 950                 955                 960

Val Gly Leu Pro Gly Gln Arg Gly Glu Arg Gly Phe Pro Gly Leu Pro
                965                 970                 975

Gly Pro Ser Gly Glu Pro Gly Lys Gln Gly Pro Ser Gly Ala Ser Gly
            980                 985                 990

Glu Arg Gly Pro Pro Gly Pro Met  Gly Pro Pro Gly Leu  Ala Gly Pro
        995                 1000                 1005

Pro Gly  Glu Ser Gly Arg Glu  Gly Ala Pro Ala Ala  Glu Gly Ser
    1010                 1015                 1020

Pro Gly  Arg Asp Gly Ser Pro  Gly Ala Lys Gly Asp  Arg Gly Glu
    1025                 1030                 1035

Thr Gly  Pro Ala Gly Pro Pro  Gly Ala Pro Gly Ala  Pro Gly Ala

1040                    1045                    1050

Pro Gly Pro Val Gly Pro Ala Gly Lys Ser Gly Asp Arg Gly Glu
    1055                1060                1065

Thr Gly Pro Ala Gly Pro Ala Gly Pro Val Gly Pro Val Gly Ala
    1070                1075                1080

Arg Gly Pro Ala Gly Pro Gln Gly Pro Arg Gly Asp Lys Gly Glu
    1085                1090                1095

Thr Gly Glu Gln Gly Asp Arg Gly Ile Lys Gly His Arg Gly Phe
    1100                1105                1110

Ser Gly Leu Gln Gly Pro Pro Gly Pro Pro Gly Ser Pro Gly Glu
    1115                1120                1125

Gln Gly Pro Ser Gly Ala Ser Gly Pro Ala Gly Pro Arg Gly Pro
    1130                1135                1140

Pro Gly Ser Ala Gly Ala Pro Gly Lys Asp Gly Leu Asn Gly Leu
    1145                1150                1155

Pro Gly Pro Ile Gly Pro Pro Gly Pro Arg Gly Arg Thr Gly Asp
    1160                1165                1170

Ala Gly Pro Val Gly Pro Pro Gly Pro Pro Gly Pro Pro Gly Pro
    1175                1180                1185

Pro Gly Pro Pro Ser Ala Gly Phe Asp Phe Ser Phe Leu Pro Gln
    1190                1195                1200

Pro Pro Gln Glu Lys Ala His Asp Gly Gly Arg Tyr Tyr Arg Ala
    1205                1210                1215

Asp Asp Ala Asn Val Val Arg Asp Arg Asp Leu Glu Val Asp Thr
    1220                1225                1230

Thr Leu Lys Ser Leu Ser Gln Gln Ile Glu Asn Ile Arg Ser Pro
    1235                1240                1245

Glu Gly Ser Arg Lys Asn Pro Ala Arg Thr Cys Arg Asp Leu Lys
    1250                1255                1260

Met Cys His Ser Asp Trp Lys Ser Gly Glu Tyr Trp Ile Asp Pro
    1265                1270                1275

Asn Gln Gly Cys Asn Leu Asp Ala Ile Lys Val Phe Cys Asn Met
    1280                1285                1290

Glu Thr Gly Glu Thr Cys Val Tyr Pro Thr Gln Pro Ser Val Ala
    1295                1300                1305

Gln Lys Asn Trp Tyr Ile Ser Lys Asn Pro Lys Asp Lys Arg His
    1310                1315                1320

Val Trp Phe Gly Glu Ser Met Thr Asp Gly Phe Gln Phe Glu Tyr
    1325                1330                1335

Gly Gly Gln Gly Ser Asp Pro Ala Asp Val Ala Ile Gln Leu Thr
    1340                1345                1350

Phe Leu Arg Leu Met Ser Thr Glu Ala Ser Gln Asn Ile Thr Tyr
    1355                1360                1365

His Cys Lys Asn Ser Val Ala Tyr Met Asp Gln Gln Thr Gly Asn
    1370                1375                1380

Leu Lys Lys Ala Leu Leu Leu Lys Gly Ser Asn Glu Ile Glu Ile
    1385                1390                1395

Arg Ala Glu Gly Asn Ser Arg Phe Thr Tyr Ser Val Thr Val Asp
    1400                1405                1410

Gly Cys Thr Ser His Thr Gly Ala Trp Gly Lys Thr Val Ile Glu
    1415                1420                1425

Tyr Lys Thr Thr Lys Ser Ser Arg Leu Pro Ile Ile Asp Val Ala
    1430                1435                1440

Pro Leu Asp Val Gly Ala Pro Asp Gln Glu Phe Gly Phe Asp Val
    1445                1450                1455

Gly Pro Val Cys Phe Leu
    1460

<210> 9
<211> 6447
<212> DNA
<213> Homo sapiens

<400> 9
aggtctccgc ttggagccgc cgcacccggg acggtgcgta tcgctggaag tccggccttc 60

cgagagctag ctgtccgccg cggcccccgc acgccgggca gccgtccctc gcgcctcggg 120

cgcgccacca tggggccccg gctcagcgtc tggctgctgc tgctgcccgc cgcccttctg 180

ctccacgagg agcacagccg ggccgctgcg aagggtggct gtgctggctc tggctgtggc 240

aaatgtgact gccatggagt gaagggacaa aagggtgaaa gaggcctccc ggggttacaa 300

ggtgtcattg ggtttcctgg aatgcaagga cctgaggggc cacagggacc accaggacaa 360

aagggtgata ctggagaacc aggactacct ggaacaaaag ggacaagagg acctccggga 420

gcatctggct accctggaaa cccaggactt cccggaattc ctggccaaga cggcccgcca 480

ggccccccag gtattccagg atgcaatggc acaaaggggg agagagggcc gctcgggcct 540

cctggcttgc ctggtttcgc aggaaatccc ggaccaccag gcttaccagg gatgaagggt 600
gatccaggtg agatacttgg ccatgtgccc gggatgctgt tgaaaggtga aagaggattt 660
cccggaatcc cagggactcc aggcccacca ggactgccag ggcttcaagg tcctgttggg 720
cctccaggat ttaccggacc accaggtccc ccaggccctc ccggccctcc aggtgaaaag 780
ggacaaatgg gcttaagttt tcaaggacca aaaggtgaca agggtgacca aggggtcagt 840
gggcctccag gagtaccagg acaagctcaa gttcaagaaa aaggagactt cgccaccaag 900
ggagaaaagg gccaaaaagg tgaacctgga tttcagggga tgccaggggt cggagagaaa 960
ggtgaacccg gaaaaccagg acccagaggc aaacccggaa aagatggtga caaaggggaa 1020
aaagggagtc ccggttttcc tggtgaaccc gggtacccag gactcatagg ccgccagggc 1080
ccgcagggag aaaagggtga agcaggtcct cctggcccac ctggaattgt tataggcaca 1140
ggacctttgg gagaaaaagg agagaggggc taccctggaa ctccggggcc aagaggagag 1200
ccaggcccaa aaggtttccc aggactacca ggccaacccg gacctccagg cctccctgta 1260
cctgggcagg ctggtgcccc tggcttccct ggtgaaagag gagaaaaagg tgaccgagga 1320
tttcctggta catctctgcc aggaccaagt ggaagagatg ggctcccggg tcctcctggt 1380
tcccccgggc cccctgggca gcctggctac acaaatggaa ttgtggaatg tcagcccgga 1440
cctccaggtg accagggtcc tcctggaatt ccagggcagc caggatttat aggcgaaatt 1500
ggagagaaag gtcaaaaagg agagagttgc ctcatctgtg atatagacgg atatcggggg 1560
cctcccgggc cacagggacc cccgggagaa ataggtttcc cagggcagcc aggggccaag 1620
ggcgacagag gtttgcctgg cagagatggt gttgcaggag tgccaggccc tcaaggtaca 1680
ccagggctga taggccagcc aggagccaag ggggagcctg gtgagtttta tttcgacttg 1740
cggctcaaag gtgacaaagg agacccaggc tttccaggac agcccggcat gccagggaga 1800
gcgggttctc ctggaagaga tggccatccg ggtcttcctg gccccaaggg ctcgccgggt 1860
tctgtaggat tgaaaggaga gcgtggcccc cctggaggag ttggattccc aggcagtcgt 1920
ggtgacaccg gcccccctgg gcctccagga tatggtcctg ctggtcccat tggtgacaaa 1980
ggacaagcag gctttcctgg aggccctgga tccccaggcc tgccaggtcc aaagggtgaa 2040
ccaggaaaaa ttgttccttt accaggcccc cctggagcag aaggactgcc ggggtcccca 2100
ggcttcccag gtccccaagg agaccgaggc tttcccggaa ccccaggaag gccaggcctg 2160
ccaggagaga agggcgctgt gggccagcca ggcattggat ttccagggcc ccccggcccc 2220
aaaggtgttg acggcttacc tggagacatg ggcccaccgg ggactccagg tcgcccggga 2280
tttaatggct tacctgggaa cccaggtgtg cagggccaga agggagagcc tggagttggt 2340
ctaccgggac tcaaaggttt gccaggtctt cccggcattc ctggcacacc cggggagaag 2400
gggagcattg ggtaccaggg cgttcctgga gaacatggag cgatcggacc ccctgggctt 2460
caggggatca gaggtgaacc gggacctcct ggattgccag gctccgtggg gtctccagga 2520
gttccaggaa taggcccccc tggagctagg ggtccccctg gaggacaggg accaccgggg 2580

ttgtcaggcc ctcctggaat aaaaggagag aagggtttcc ccggattccc tggactggac 2640
atgccgggcc ctaaaggaga taaaggggct caaggactcc ctggcataac gggacagtcg 2700
gggctccctg gccttcctgg acagcagggg gctcctggga ttcctgggtt tccaggttcc 2760
aagggagaaa tgggcgtcat ggggaccccc gggcagccgg gctcaccagg accagtgggt 2820
gctcctggat taccgggtga aaaaggggac catggctttc cgggctcctc aggacccagg 2880
ggagaccctg gcttgaaagg tgataagggg gatgtcggtc tccctggcaa gcctggctcc 2940
atggataagg tggacatggg cagcatgaag ggccagaaag gagaccaagg agagaaagga 3000
caaattggac caattggtga gaagggatcc cgaggagacc ctgggacccc aggagtgcct 3060
ggaaaggacg ggcaggcagg acagcctggg cagccaggac ctaaaggtga tccaggtata 3120
agtggaaccc caggtgctcc aggacttccg ggaccaaaag gatctgttgg tggaatgggc 3180
ttgccaggaa cacctggaga gaaaggtgtg cctggcatcc ctggcccaca aggttcacct 3240
ggcttacctg gagacaaagg tgcaaaagga gagaaagggc aggcaggccc acctggcata 3300
ggcatcccag gactgcgtgg tgaaaaggga gatcaaggga tagcgggttt cccaggaagc 3360
cctggagaga agggagaaaa aggaagcatt gggatcccag gaatgccagg gtccccaggc 3420
cttaaagggt ctcccgggag tgttggctat ccaggaagtc ctgggctacc tggagaaaaa 3480
ggtgacaaag gcctcccagg attggatggc atccctggtg tcaaaggaga agcaggtctt 3540
cctgggactc ctggccccac aggcccagct ggccagaaag gggagccagg cagtgatgga 3600
atcccggggt cagcaggaga gaagggtgaa ccaggtctac caggaagagg attcccaggg 3660
tttccagggg ccaaaggaga caaaggttca aagggtgagg tgggtttccc aggattagcc 3720
gggagcccag gaattcctgg atccaaagga gagcaaggat tcatgggtcc tccggggccc 3780
cagggacagc cggggttacc gggatcccca ggccatgcca cggaggggcc caaaggagac 3840
cgcggacctc agggccagcc tggcctgcca ggacttccgg gacccatggg gcctccaggg 3900
cttcctggga ttgatggagt taaaggtgac aaaggaaatc caggctggcc aggagcaccc 3960
ggtgtcccag ggcccaaggg agaccctgga ttccagggca tgcctggtat tggtggctct 4020
ccaggaatca caggctctaa gggtgatatg gggcctccag gagttccagg atttcaaggt 4080
ccaaaaggtc ttcctggcct ccagggaatt aaaggtgatc aaggcgatca aggcgtcccg 4140
ggagctaaag gtctcccggg tcctcctggc cccccaggtc cttacgacat catcaaaggg 4200
gagcccgggc tccctggtcc tgagggcccc ccagggctga aagggcttca gggactgcca 4260
ggcccgaaag gccagcaagg tgttacagga ttggtgggta tacctggacc tccaggtatt 4320
cctgggtttg acggtgcccc tggccagaaa ggagagatgg gacctgccgg gcctactggt 4380
ccaagaggat ttccaggtcc accaggcccc gatgggttgc caggatccat ggggccccca 4440
ggcaccccat ctgttgatca cggcttcctt gtgaccaggc atagtcaaac aatagatgac 4500
ccacagtgtc cttctgggac caaaattctt taccacgggt actctttgct ctacgtgcaa 4560
ggcaatgaac gggcccatgg acaggacttg ggcacggccg gcagctgcct gcgcaagttc 4620
agcacaatgc ccttcctgtt ctgcaatatt aacaacgtgt gcaactttgc atcacgaaat 4680

gactactcgt actggctgtc cacccctgag cccatgccca tgtcaatggc acccatcacg 4740

ggggaaaaca taagaccatt tattagtagg tgtgctgtgt gtgaggcgcc tgccatggtg 4800

atggccgtgc acagccagac cattcagatc ccaccgtgcc ccagcgggtg gtcctcgctg 4860

tggatcggct actcttttgt gatgcacacc agcgctggtg cagaaggctc tggccaagcc 4920

ctggcgtccc ccggctcctg cctggaggag tttagaagtg cgccattcat cgagtgtcac 4980

ggccgtggga cctgcaatta ctacgcaaac gcttacagct tttggctcgc caccatagag 5040

aggagcgaga tgttcaagaa gcctacgccg tccaccttga aggcagggga gctgcgcacg 5100

cacgtcagcc gctgccaagt ctgtatgaga agaacataag aagcctgact cagctaatgt 5160

cacaacatgg tgctacttct tcttcttttt gttaacagca acgaacccta gaaatatatc 5220

ctgtgtacct cactgtccaa tatgaaaacc gtaaagtgcc ttataggaat ttgcgtaact 5280

aacacaccct gcttcattga cctctacttg ctgaaggaga aaaagacagc gataagcttc 5340

aatagtggca taccaaatgg cacttttgat gaaataaaat atcaatattt tctgcaatcc 5400

aatgcactga tgtgtgaagt gagaactcca tcagaaaacc aaagggtgct aggaggtgtg 5460

ggtgccttcc atactgtttg cccattttca ttcttgtatt ataattaatt ttctaccccc 5520

agagataaat gtttgtttat atcactgtct agctgtttca aaatttaggt cccttggtct 5580

gtacaaataa tagcaatgta aaaatggttt tttgaacctc caaatggaat tacagactca 5640

gtagccatat cttccaaccc cccagtataa atttctgtct ttctgctatg tgtggtactt 5700

tgcagctgct tttgcagaaa tcacaatttt cctgtggaat aaagatggtc caaaaatagt 5760

caaaaattaa atatatatat atattagtaa tttatataga tgtcagcaat taggcagatc 5820

aaggtttagt ttaacttcca ctgttaaaat aaagcttaca tagttttctt cctttgaaag 5880

actgtgctgt cctttaacat aggtttttaa agactaggat attgaatgtg aaacatccgt 5940

tttcattgtt cacttctaaa ccaaaaatta tgtgttgcca aaaccaaacc caggttcatg 6000

aatatggtgt ctattatagt gaaacatgta ctttgagctt attgttttta ttctgtatta 6060

aatattttca gggttttaaa cactaatcac aaactgaatg acttgacttc aaaagcaaca 6120

accttaaagg ccgtcatttc attagtattc ctcattctgc atcctggctt gaaaaacagc 6180

tctgttgaat cacagtatca gtattttcac acgtaagcac attcgggcca tttccgtggt 6240

ttctcatgag ctgtgttcac agacctcagc agggcatcgc atggaccgca ggagggcaga 6300

ttcggaccac taggcctgaa atgacatttc actaaaagtc tccaaaacat ttctaagact 6360

actaaggcct tttatgtaat ttctttaaat gtgtatttct taagaattca aatttgtaat 6420

aaaactattt gtataaaaat taagctt 6447

<210> 10
<211> 1669
<212> PRT
<213> Homo sapiens

<400> 10

Met Gly Pro Arg Leu Ser Val Trp Leu Leu Leu Leu Pro Ala Ala Leu
1               5                   10                  15

Leu Leu His Glu Glu His Ser Arg Ala Ala Ala Lys Gly Gly Cys Ala
            20                  25                  30

Gly Ser Gly Cys Gly Lys Cys Asp Cys His Gly Val Lys Gly Gln Lys
        35                  40                  45

Gly Glu Arg Gly Leu Pro Gly Leu Gln Gly Val Ile Gly Phe Pro Gly
    50                  55                  60

Met Gln Gly Pro Glu Gly Pro Gln Gly Pro Pro Gly Gln Lys Gly Asp
65                  70                  75                  80

Thr Gly Glu Pro Gly Leu Pro Gly Thr Lys Gly Thr Arg Gly Pro Pro
                85                  90                  95

Gly Ala Ser Gly Tyr Pro Gly Asn Pro Gly Leu Pro Gly Ile Pro Gly
            100                 105                 110

Gln Asp Gly Pro Pro Gly Pro Pro Gly Ile Pro Gly Cys Asn Gly Thr
        115                 120                 125

Lys Gly Glu Arg Gly Pro Leu Gly Pro Pro Gly Leu Pro Gly Phe Ala
    130                 135                 140

Gly Asn Pro Gly Pro Pro Gly Leu Pro Gly Met Lys Gly Asp Pro Gly
145                 150                 155                 160

Glu Ile Leu Gly His Val Pro Gly Met Leu Leu Lys Gly Glu Arg Gly
                165                 170                 175

Phe Pro Gly Ile Pro Gly Thr Pro Gly Pro Pro Gly Leu Pro Gly Leu
            180                 185                 190

Gln Gly Pro Val Gly Pro Pro Gly Phe Thr Gly Pro Pro Gly Pro Pro
        195                 200                 205

Gly Pro Pro Gly Pro Pro Gly Glu Lys Gly Gln Met Gly Leu Ser Phe
    210                 215                 220

Gln Gly Pro Lys Gly Asp Lys Gly Asp Gln Gly Val Ser Gly Pro Pro
225                 230                 235                 240

Gly Val Pro Gly Gln Ala Gln Val Gln Glu Lys Gly Asp Phe Ala Thr
                245                 250                 255

Lys Gly Glu Lys Gly Gln Lys Gly Glu Pro Gly Phe Gln Gly Met Pro
            260                 265                 270

Gly Val Gly Glu Lys Gly Glu Pro Gly Lys Pro Gly Pro Arg Gly Lys

275                 280                 285

Pro Gly Lys Asp Gly Asp Lys Gly Glu Lys Gly Ser Pro Gly Phe Pro
    290                 295                 300

Gly Glu Pro Gly Tyr Pro Gly Leu Ile Gly Arg Gln Gly Pro Gln Gly
305                 310                 315                 320

Glu Lys Gly Glu Ala Gly Pro Pro Gly Pro Pro Gly Ile Val Ile Gly
                325                 330                 335

Thr Gly Pro Leu Gly Glu Lys Gly Glu Arg Gly Tyr Pro Gly Thr Pro
            340                 345                 350

Gly Pro Arg Gly Glu Pro Gly Pro Lys Gly Phe Pro Gly Leu Pro Gly
        355                 360                 365

Gln Pro Gly Pro Pro Gly Leu Pro Val Pro Gly Gln Ala Gly Ala Pro
    370                 375                 380

Gly Phe Pro Gly Glu Arg Gly Glu Lys Gly Asp Arg Gly Phe Pro Gly
385                 390                 395                 400

Thr Ser Leu Pro Gly Pro Ser Gly Arg Asp Gly Leu Pro Gly Pro Pro
                405                 410                 415

Gly Ser Pro Gly Pro Pro Gly Gln Pro Gly Tyr Thr Asn Gly Ile Val
            420                 425                 430

Glu Cys Gln Pro Gly Pro Pro Gly Asp Gln Gly Pro Pro Gly Ile Pro
        435                 440                 445

Gly Gln Pro Gly Phe Ile Gly Glu Ile Gly Glu Lys Gly Gln Lys Gly
    450                 455                 460

Glu Ser Cys Leu Ile Cys Asp Ile Asp Gly Tyr Arg Gly Pro Pro Gly
465                 470                 475                 480

Pro Gln Gly Pro Pro Gly Glu Ile Gly Phe Pro Gly Gln Pro Gly Ala
                485                 490                 495

Lys Gly Asp Arg Gly Leu Pro Gly Arg Asp Gly Val Ala Gly Val Pro
            500                 505                 510

Gly Pro Gln Gly Thr Pro Gly Leu Ile Gly Gln Pro Gly Ala Lys Gly
        515                 520                 525

Glu Pro Gly Glu Phe Tyr Phe Asp Leu Arg Leu Lys Gly Asp Lys Gly
    530                 535                 540

Asp Pro Gly Phe Pro Gly Gln Pro Gly Met Pro Gly Arg Ala Gly Ser
545                 550                 555                 560

Pro Gly Arg Asp Gly His Pro Gly Leu Pro Gly Pro Lys Gly Ser Pro
                565                 570                 575

Gly Ser Val Gly Leu Lys Gly Glu Arg Gly Pro Pro Gly Gly Val Gly
            580                 585                 590

Phe Pro Gly Ser Arg Gly Asp Thr Gly Pro Pro Gly Pro Pro Gly Tyr
        595                 600                 605

Gly Pro Ala Gly Pro Ile Gly Asp Lys Gly Gln Ala Gly Phe Pro Gly
    610                 615                 620

Gly Pro Gly Ser Pro Gly Leu Pro Gly Pro Lys Gly Glu Pro Gly Lys
625                 630                 635                 640

Ile Val Pro Leu Pro Gly Pro Pro Gly Ala Glu Gly Leu Pro Gly Ser
                645                 650                 655

Pro Gly Phe Pro Gly Pro Gln Gly Asp Arg Gly Phe Pro Gly Thr Pro
            660                 665                 670

Gly Arg Pro Gly Leu Pro Gly Glu Lys Gly Ala Val Gly Gln Pro Gly
        675                 680                 685

Ile Gly Phe Pro Gly Pro Pro Gly Pro Lys Gly Val Asp Gly Leu Pro
    690                 695                 700

Gly Asp Met Gly Pro Pro Gly Thr Pro Gly Arg Pro Gly Phe Asn Gly
705                 710                 715                 720

Leu Pro Gly Asn Pro Gly Val Gln Gly Gln Lys Gly Glu Pro Gly Val
                725                 730                 735

Gly Leu Pro Gly Leu Lys Gly Leu Pro Gly Leu Pro Gly Ile Pro Gly
            740                 745                 750

Thr Pro Gly Glu Lys Gly Ser Ile Gly Val Pro Gly Val Pro Gly Glu
        755                 760                 765

His Gly Ala Ile Gly Pro Pro Gly Leu Gln Gly Ile Arg Gly Glu Pro
    770                 775                 780

Gly Pro Pro Gly Leu Pro Gly Ser Val Gly Ser Pro Gly Val Pro Gly
785                 790                 795                 800

Ile Gly Pro Pro Gly Ala Arg Gly Pro Pro Gly Gly Gln Gly Pro Pro
                805                 810                 815

Gly Leu Ser Gly Pro Pro Gly Ile Lys Gly Glu Lys Gly Phe Pro Gly
            820                 825                 830

Phe Pro Gly Leu Asp Met Pro Gly Pro Lys Gly Asp Lys Gly Ala Gln
        835                 840                 845

Gly Leu Pro Gly Ile Thr Gly Gln Ser Gly Leu Pro Gly Leu Pro Gly
    850                 855                 860

Gln Gln Gly Ala Pro Gly Ile Pro Gly Phe Pro Gly Ser Lys Gly Glu
865                 870                 875                 880

Met Gly Val Met Gly Thr Pro Gly Gln Pro Gly Ser Pro Gly Pro Val
                885                 890                 895

Gly Ala Pro Gly Leu Pro Gly Glu Lys Gly Asp His Gly Phe Pro Gly
            900                 905                 910

Ser Ser Gly Pro Arg Gly Asp Pro Gly Leu Lys Gly Asp Lys Gly Asp
        915                 920                 925

Val Gly Leu Pro Gly Lys Pro Gly Ser Met Asp Lys Val Asp Met Gly
    930                 935                 940

Ser Met Lys Gly Gln Lys Gly Asp Gln Gly Glu Lys Gly Gln Ile Gly
945                 950                 955                 960

Pro Ile Gly Glu Lys Gly Ser Arg Gly Asp Pro Gly Thr Pro Gly Val
                965                 970                 975

Pro Gly Lys Asp Gly Gln Ala Gly Gln Pro Gly Gln Pro Gly Pro Lys
            980                 985                 990

Gly Asp Pro Gly Ile Ser Gly Thr  Pro Gly Ala Pro Gly  Leu Pro Gly
        995                 1000                 1005

Pro Lys  Gly Ser Val Gly Gly  Met Gly Leu Pro Gly  Thr Pro Gly
    1010                 1015                 1020

Glu Lys  Gly Val Pro Gly Ile  Pro Gly Pro Gln Gly  Ser Pro Gly
    1025                 1030                 1035

Leu Pro  Gly Asp Lys Gly Ala  Lys Gly Glu Lys Gly  Gln Ala Gly
    1040                 1045                 1050

Pro Pro  Gly Ile Gly Ile Pro  Gly Leu Arg Gly Glu  Lys Gly Asp
    1055                 1060                 1065

Gln Gly  Ile Ala Gly Phe Pro  Gly Ser Pro Gly Glu  Lys Gly Glu
    1070                 1075                 1080

Lys Gly  Ser Ile Gly Ile Pro  Gly Met Pro Gly Ser  Pro Gly Leu
    1085                 1090                 1095

Lys Gly Ser Pro Gly Ser Val Gly Tyr Pro Gly Ser Pro Gly Leu
    1100                1105                1110

Pro Gly Glu Lys Gly Asp Lys Gly Leu Pro Gly Leu Asp Gly Ile
    1115                1120                1125

Pro Gly Val Lys Gly Glu Ala Gly Leu Pro Gly Thr Pro Gly Pro
    1130                1135                1140

Thr Gly Pro Ala Gly Gln Lys Gly Glu Pro Gly Ser Asp Gly Ile
    1145                1150                1155

Pro Gly Ser Ala Gly Glu Lys Gly Glu Pro Gly Leu Pro Gly Arg
    1160                1165                1170

Gly Phe Pro Gly Phe Pro Gly Ala Lys Gly Asp Lys Gly Ser Lys
    1175                1180                1185

Gly Glu Val Gly Phe Pro Gly Leu Ala Gly Ser Pro Gly Ile Pro
    1190                1195                1200

Gly Ser Lys Gly Glu Gln Gly Phe Met Gly Pro Pro Gly Pro Gln
    1205                1210                1215

Gly Gln Pro Gly Leu Pro Gly Ser Pro Gly His Ala Thr Glu Gly
    1220                1225                1230

Pro Lys Gly Asp Arg Gly Pro Gln Gly Gln Pro Gly Leu Pro Gly
    1235                1240                1245

Leu Pro Gly Pro Met Gly Pro Pro Gly Leu Pro Gly Ile Asp Gly
    1250                1255                1260

Val Lys Gly Asp Lys Gly Asn Pro Gly Trp Pro Gly Ala Pro Gly
    1265                1270                1275

Val Pro Gly Pro Lys Gly Asp Pro Gly Phe Gln Gly Met Pro Gly
    1280                1285                1290

Ile Gly Gly Ser Pro Gly Ile Thr Gly Ser Lys Gly Asp Met Gly
    1295                1300                1305

Pro Pro Gly Val Pro Gly Phe Gln Gly Pro Lys Gly Leu Pro Gly
    1310                1315                1320

Leu Gln Gly Ile Lys Gly Asp Gln Gly Asp Gln Gly Val Pro Gly
    1325                1330                1335

Ala Lys Gly Leu Pro Gly Pro Pro Gly Pro Pro Gly Pro Tyr Asp
    1340                1345                1350

Ile Ile Lys Gly Glu Pro Gly Leu Pro Gly Pro Glu Gly Pro Pro

1355                    1360                    1365

Gly Leu  Lys Gly Leu Gln Gly  Leu Pro Gly Pro Lys  Gly Gln Gln
    1370                    1375                    1380

Gly Val  Thr Gly Leu Val Gly  Ile Pro Gly Pro Pro  Gly Ile Pro
    1385                    1390                    1395

Gly Phe  Asp Gly Ala Pro Gly  Gln Lys Gly Glu Met  Gly Pro Ala
    1400                    1405                    1410

Gly Pro  Thr Gly Pro Arg Gly  Phe Pro Gly Pro Pro  Gly Pro Asp
    1415                    1420                    1425

Gly Leu  Pro Gly Ser Met Gly  Pro Pro Gly Thr Pro  Ser Val Asp
    1430                    1435                    1440

His Gly  Phe Leu Val Thr Arg  His Ser Gln Thr Ile  Asp Asp Pro
    1445                    1450                    1455

Gln Cys  Pro Ser Gly Thr Lys  Ile Leu Tyr His Gly  Tyr Ser Leu
    1460                    1465                    1470

Leu Tyr  Val Gln Gly Asn Glu  Arg Ala His Gly Gln  Asp Leu Gly
    1475                    1480                    1485

Thr Ala  Gly Ser Cys Leu Arg  Lys Phe Ser Thr Met  Pro Phe Leu
    1490                    1495                    1500

Phe Cys  Asn Ile Asn Asn Val  Cys Asn Phe Ala Ser  Arg Asn Asp
    1505                    1510                    1515

Tyr Ser  Tyr Trp Leu Ser Thr  Pro Glu Pro Met Pro  Met Ser Met
    1520                    1525                    1530

Ala Pro  Ile Thr Gly Glu Asn  Ile Arg Pro Phe Ile  Ser Arg Cys
    1535                    1540                    1545

Ala Val  Cys Glu Ala Pro Ala  Met Val Met Ala Val  His Ser Gln
    1550                    1555                    1560

Thr Ile  Gln Ile Pro Pro Cys  Pro Ser Gly Trp Ser  Ser Leu Trp
    1565                    1570                    1575

Ile Gly  Tyr Ser Phe Val Met  His Thr Ser Ala Gly  Ala Glu Gly
    1580                    1585                    1590

Ser Gly  Gln Ala Leu Ala Ser  Pro Gly Ser Cys Leu  Glu Glu Phe
    1595                    1600                    1605

Arg Ser  Ala Pro Phe Ile Glu  Cys His Gly Arg Gly  Thr Cys Asn
    1610                    1615                    1620

Tyr Tyr Ala Asn Ala Tyr Ser Phe Trp Leu Ala Thr Ile Glu Arg
    1625                1630                1635

Ser Glu Met Phe Lys Lys Pro Thr Pro Ser Thr Leu Lys Ala Gly
    1640                1645                1650

Glu Leu Arg Thr His Val Ser Arg Cys Gln Val Cys Met Arg Arg
    1655                1660                1665

Thr

<210> 11
<211> 1207
<212> DNA
<213> Homo sapiens

<400> 11
gagacagcgc cggggcaagt gagagccgga cgggcactgg gcgactctgt gcctcgctga      60

ggaaaaataa ctaaacatgg gcaaaggaga tcctaagaag ccgagaggca aaatgtcatc     120

atatgcattt tttgtgcaaa cttgtcggga ggagcataag aagaagcacc cagatgcttc     180

agtcaacttc tcagagtttt ctaagaagtg ctcagagagg tggaagacca tgtctgctaa     240

agagaaagga aaatttgaag atatggcaaa ggcggacaag gcccgttatg aaagagaaat     300

gaaaacctat atccctccca aaggggagac aaaaaagaag ttcaaggatc ccaatgcacc     360

caagaggcct ccttcggcct tcttcctctt ctgctctgag tatcgcccaa aaatcaaagg     420

agaacatcct ggcctgtcca ttggtgatgt tgcgaagaaa ctgggagaga tgtggaataa     480

cactgctgca gatgacaagc agccttatga aaagaaggct gcgaagctga aggaaaaata     540

cgaaaaggat attgctgcat atcgagctaa aggaaagcct gatgcagcaa aaaagggagt     600

tgtcaaggct gaaaaaagca agaaaaagaa ggaagaggag gaagatgagg aagatgaaga     660

ggatgaggag gaggaggaag atgaagaaga tgaagatgaa gaagaagatg atgatgatga     720

ataagttggt tctagcgcag tttttttttc ttgtctataa agcatttaac ccccctgtac     780

acaactcact ccttttaaag aaaaaaattg aaatgtaagg ctgtgtaaga tttgttttta     840

aactgtacag tgtctttttt tgtatagtta acacactacc gaatgtgtct ttagatagcc     900

ctgtcctggt ggtattttca atagccacta accttgcctg gtacagtatg ggggttgtaa     960

attggcatgg aaatttaaag caggttcttg ttggtgcaca gcacaaatta gttatatatg    1020

gggatggtag ttttttcatc ttcagttgtc tctgatgcag cttatacgaa ataattgttg    1080

ttctgttaac tgaataccac tctgtaattg caaaaaaaaa aaaagttgca gctgttttgt    1140

tgacattctg aatgcttcta agtaaataca atttttttta ttaaaaaaaa aaaaaaaaaa    1200

aaaaaaa                                                              1207

<210> 12
<211> 215

<212>  PRT
<213>  Homo sapiens

<400>  12

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys Cys Ser Glu Arg
        35                  40                  45

Trp Lys Thr Met Ser Ala Lys Glu Lys Gly Lys Phe Glu Asp Met Ala
    50                  55                  60

Lys Ala Asp Lys Ala Arg Tyr Glu Arg Glu Met Lys Thr Tyr Ile Pro
65                  70                  75                  80

Pro Lys Gly Glu Thr Lys Lys Lys Phe Lys Asp Pro Asn Ala Pro Lys
                85                  90                  95

Arg Pro Pro Ser Ala Phe Phe Leu Phe Cys Ser Glu Tyr Arg Pro Lys
            100                 105                 110

Ile Lys Gly Glu His Pro Gly Leu Ser Ile Gly Asp Val Ala Lys Lys
        115                 120                 125

Leu Gly Glu Met Trp Asn Asn Thr Ala Ala Asp Asp Lys Gln Pro Tyr
    130                 135                 140

Glu Lys Lys Ala Ala Lys Leu Lys Glu Lys Tyr Glu Lys Asp Ile Ala
145                 150                 155                 160

Ala Tyr Arg Ala Lys Gly Lys Pro Asp Ala Ala Lys Lys Gly Val Val
                165                 170                 175

Lys Ala Glu Lys Ser Lys Lys Lys Lys Glu Glu Glu Glu Asp Glu Glu
            180                 185                 190

Asp Glu Glu Asp Glu Glu Glu Glu Glu Asp Glu Glu Asp Glu Asp Glu
        195                 200                 205

Glu Glu Asp Asp Asp Asp Glu
    210                 215


<210>  13
<211>  2227
<212>  DNA
<213>  Homo sapiens

<400>  13
taattcagaa ttgagtaaag aaatattttt tctagtcctt catatattga aaacttgcca      60

catgacattg tatcgtcttc attttccaga agatgcgttg gtgtgccata ggtttctaac 120
ttccttgaaa atagtttttt aagtcaattg taaatatacg tattattgtt aaaagtaact 180
ttaaactgca acacatagct tcaaaacaat atagagattt tgtaatacct tataagtgga 240
gttggctaaa ataccttatc catataaaac ttattctatt ctttgcatgc ttattttgtg 300
tgttggttgc tagcttaaag tttgatttgt tgttactctt tgtgtgccaa attcactagg 360
caagcggatt tttcctcaga cttcaaaaaa taattctttt aagaaaaaat gtaaaaatgt 420
ttattctaaa aagctgcatt aaagggacaa cctataaaaa gttttgctag ctcatcttta 480
gaaggaagaa agaatattag cttgggtgat gtttaatttg ggtggcgata gtttctgtag 540
gctaaacttt atgagaaaag tgtacctact ctataaaggt aataaatgta aaacctcttg 600
ctgttattga ggaagctctt caactaccct aaatttcaca aatgtaactt ataacactat 660
gaaaagattt gaccaacaat ttacgtttgc tgtgtgcttt agtttttgtt taagcatatt 720
cttttgcttg aatttctgtg ttcatgagag ttagggtgtt ttatgcttct tgaactaatt 780
ttataacata tttaatatat taccagttaa gatataaaat catttgtaca tagcgaattg 840
taaagcagct attaaagtag gtgaaataaa gtatatattt gccggttatc catatctttt 900
agaagtcctg acagaacaac cagtttattt gcacataggt agcttctgtt tgaaggaagg 960
taaagttata aggaaactca aatactataa gatgtgtcaa ggtatttctc cagaattaat 1020
tgcaaagcta gtgctgaagg attttaatca gcttctaaaa ttttcttctc aataagacat 1080
atgttttgat tacttaggga agattcctca tttttatttg ccctttatgc atttaatcca 1140
catgatagga cattaaaaat taatataaag aaaaatcgtg ctcatactgt acatctattt 1200
ctgtgcttgg aactacttgt taatagtttt tatcgaagct gtcagcaata agggacataa 1260
aactgctgta ttatacattg tggaattgaa taaacagcct aatttttttt tttctagtat 1320
agggtactta agcatttcca cttttggaag aaaagtgtat tagtatttta tattgcattt 1380
catttaaaag gacagttttt tttttttttg taaatccatt cattgaaatg gtttctaaac 1440
tgtataatgt aatttggagc ctatttagta atagaattaa atgtcctatg tagtgctaca 1500
atttttgaat tagaaagtga tcaaatgtaa gaaaaaaatt taaaaattca gcccagaaaa 1560
caaaatagtg tattaaatta gtttaatgta aaaggaattt ataagatttt tttcctcaat 1620
atagatacct cacttgaaaa gaaagcacag catacttaaa gtagttctag taaacatgtc 1680
ctagaaaaca gttgctaaat gtaggacatc ttttgaggaa ttagtttatg agaaataaaa 1740
ttttacttgt ttttactatc ctgttagaag tatttgttta tcctgataat tttaagccaa 1800
catagtagtc ttaaattact tttgaatttc taatctgtga aggcagtaaa tgaaatatct 1860
gttctgcaac tgttgaaaca aataattggc tacattgacc ataattaaag ttaaaatttt 1920
gccaatgatg tacagtttta tggttaaagt tgctgtggtt ggttgcatta catgacacag 1980
aaaactgtcc tctacctcac gtgaaataaa tattttatat ggttttacta aaaataagac 2040
tcatgtatct ggtcacctag tttacaaatt ttgaattata tttattgaaa catgacatac 2100
tgtgctctga gcttatacct caattgtatt ttgtgctgtt ttccattttc atgccttgta 2160

aataacttgt atagattgtg gatcaaatac taaataaaaa cttttaatgc caaaaaaaaa 2220

aaaaaaa 2227

<210> 14
<211> 2227
<212> DNA
<213> Homo sapiens

<400> 14
taattcagaa ttgagtaaag aaatattttt tctagtcctt catatattga aaacttgcca 60

catgacattg tatcgtcttc attttccaga agatgcgttg gtgtgccata ggtttctaac 120

ttccttgaaa atagtttttt aagtcaattg taaatatacg tattattgtt aaaagtaact 180

ttaaactgca acacatagct tcaaaacaat atagagattt tgtaatacct tataagtgga 240

gttggctaaa ataccttatc catataaaac ttattctatt ctttgcatgc ttattttgtg 300

tgttggttgc tagcttaaag tttgatttgt tgttactctt tgtgtgccaa attcactagg 360

caagcggatt tttcctcaga cttcaaaaaa taattctttt aagaaaaaat gtaaaaatgt 420

ttattctaaa aagctgcatt aaagggacaa cctataaaaa gttttgctag ctcatcttta 480

gaaggaagaa agaatattag cttgggtgat gtttaatttg ggtggcgata gtttctgtag 540

gctaaacttt atgagaaaag tgtacctact ctataaaggt aataaatgta aaacctcttg 600

ctgttattga ggaagctctt caactaccct aaatttcaca aatgtaactt ataacactat 660

gaaaagattt gaccaacaat ttacgtttgc tgtgtgcttt agtttttgtt taagcatatt 720

cttttgcttg aatttctgtg ttcatgagag ttagggtgtt ttatgcttct tgaactaatt 780

ttataacata tttaatatat taccagttaa gatataaaat catttgtaca tagcgaattg 840

taaagcagct attaaagtag gtgaaataaa gtatatattt gccggttatc catatctttt 900

agaagtcctg acagaacaac cagtttattt gcacataggt agcttctgtt tgaaggaagg 960

taaagttata aggaaactca aatactataa gatgtgtcaa ggtatttctc cagaattaat 1020

tgcaaagcta gtgctgaagg attttaatca gcttctaaaa ttttcttctc aataagacat 1080

atgttttgat tacttaggga agattcctca tttttatttg ccctttatgc atttaatcca 1140

catgatagga cattaaaaat taatataaag aaaaatcgtg ctcatactgt acatctattt 1200

ctgtgcttgg aactacttgt taatagtttt tatcgaagct gtcagcaata agggacataa 1260

aactgctgta ttatacattg tggaattgaa taaacagcct aatttttttt tttctagtat 1320

agggtactta agcatttcca cttttggaag aaaagtgtat tagtatttta tattgcattt 1380

catttaaaag gacagttttt tttttttttg taaatccatt cattgaaatg gtttctaaac 1440

tgtataatgt aatttggagc ctatttagta atagaattaa atgtcctatg tagtgctaca 1500

atttttgaat tagaaagtga tcaaatgtaa gaaaaaaatt taaaaattca gcccagaaaa 1560

caaaatagtg tattaaatta gtttaatgta aaaggaattt ataagatttt tttcctcaat 1620

atagatacct cacttgaaaa gaaagcacag catacttaaa gtagttctag taaacatgtc 1680

ctagaaaaca gttgctaaat gtaggacatc ttttgaggaa ttagtttatg agaaataaaa 1740

ttttacttgt ttttactatc ctgttagaag tatttgttta tcctgataat tttaagccaa 1800

catagtagtc ttaaattact tttgaatttc taatctgtga aggcagtaaa tgaaatatct 1860

gttctgcaac tgttgaaaca aataattggc tacattgacc ataattaaag ttaaattttt 1920

gccaatgatg tacagtttta tggttaaagt tgctgtggtt ggttgcatta catgacacag 1980

aaaactgtcc tctacctcac gtgaaataaa tattttatat ggttttacta aaaataagac 2040

tcatgtatct ggtcacctag tttacaaatt ttgaattata tttattgaaa catgacatac 2100

tgtgctctga gcttatacct caattgtatt ttgtgctgtt ttccattttc atgccttgta 2160

aataacttgt atagattgtg gatcaaatac taaataaaaa cttttaatgc caaaaaaaaa 2220

aaaaaaa 2227

<210> 15
<211> 3416
<212> DNA
<213> Homo sapiens

<400> 15
accaggcagc ctgcgttcgc catgaagcga cccaaggagc cgagcggctc cgacggggag 60

tccgacggac ccatcgacgt gggccaagag ggccagctga gccagatggc caggccgctg 120

tccaccccca gctcttcgca gatgcaagcc aggaagaaac gcagagggat catagagaaa 180

cggcgtcgag accgcatcaa cagtagcctt tctgaattgc gacgcttggt ccccactgcc 240

tttgagaaac agggctcttc caagctggag aaagccgagg tcttgcagat gacggtggat 300

cacttgaaaa tgctccatgc cactggtggg acaggattct ttgatgcccg agccctggca 360

gttgacttcc ggagcattgg ttttcgggag tgcctcactg aggtcatcag gtacctgggg 420

gtccttgaag ggcccagcag ccgtgcagac cccgtccgga ttcgccttct ctcccacctc 480

aacagctacg cagccgagat ggagccttcg cccacgccca ctggcccttt ggccttccct 540

gcctggccct ggtctttctt ccatagctgt ccagggctgc cagccctgag caaccagctc 600

gccatcctgg gaagagtgcc cagccctgtc ctccccggtg tctcctctcc tgcttacccc 660

atcccagccc tccgaaccgc tccccttcgc agagccacag gcatcatcct gccagcccgg 720

aggaatgtgc tgcccagtcg aggggcatct tccacccgga gggcccgccc cctagagagg 780

ccagcgaccc ctgtgcctgt cgcccccagc agcagggctg ccaggagcag ccacatcgct 840

cccctcctgc agtcttcctc cccaacaccc cctggtccta cagggtcggc tgcttacgtg 900

gctgttccca cccccaactc atcctcccca gggccagctg ggaggccagc gggagccatg 960

ctctaccact cctgggtctc tgaaatcact gaaatcgggg ctttctgagc tgccccttca 1020

ccaccccgcc ccaaggaata aggaaggttc ttttaccagg agcccaaaaa agggcactgc 1080

cttttctgct ttgcttcgtg gactggctca tatgtgaagg cacgttctcc agccatcaga 1140

ggccccctcc tcctccaacc catctctcct tctcactgtt atcccagctt atccacccag 1200

ctctcctgga gctgttctgg tctcagaggc ttggttccat ttctcacctg aacagatgag 1260

tcctgggaga gaccctcaga gatccgccca gacccctctc ctgccctctg cacaccagca 1320

gcaggcatga accttgggtc tgggaaaaag ctttaacctg cagggcacca ggacccaagg 1380
caggctgttc cttggggcgg tcagacccca gtcaggagca atgactgact ggctgcagcc 1440
ttcccacgcc aagaggctgg aacatagtgt ctgcctcgct tcctggagat agtaactgag 1500
caggggctac aaagaggtct cctgggaacc ctgtctgccc cttcccacct gtccttgggc 1560
cacaccatca cactgaacca caggacagac cctttctcca ccacagccaa ggcctggaga 1620
ctgggggccc agcagagcct gctcccaccc tcctcccagc agcagacacc caccctctca 1680
ctgactaaca ggtccctgca cacagctggc ctggtaaacc cagctgggag gtttctaggc 1740
agcagcaaaa ctctgtgaca gggtgtcctc acaccaggcc ttggacagct ctcccagaca 1800
ggagccaggg ttgagcaatg gagagcccag cccccacgtc ttacagtcgc catcctccag 1860
gcgtgtggtc cctccccatt gggtgcacag tgcagagggg ccgtggcccc atgtgatggt 1920
gcgcagagag gaacctcttg ggattcagca ccagacgtct gtgctgcctg gtttgcatcc 1980
ggctcacaga gcccagactg ctggaacagc caaggactgt caggctggac aaaaataact 2040
gcaaggaggg gcaagagaaa ggatgattcg aggcaccttg gcccttcaag gtcatgcagt 2100
gggtcgagcg cctgagatcc tgttcaccag gactccacag agctggctct gctcagaagc 2160
catttcattc cccggctcca ccctaggcca ctttttctaa cagaggaaac aaatggtcca 2220
gcagtcgttc ccagcagaac agcggagcct ggactgacac ccagtgggac cagtgttgcc 2280
acaccagttg ataaaatgca gaaacccttc tgtactcgtt ggtaaatatc tactccccca 2340
agtgactcca ggtgcccccc accgcctggc acttccccca ggactcctac gatctggtta 2400
ctgcctggcc gatccaaggc tgtggagtcc cagagccagc agttcactgg tgctcattcc 2460
acactggtta gatacttcag ttgtcacccc tgggaagatt ctcccacctc ctccctttga 2520
tggaaccacc ctccccagag gctgcattga ggagactcca cagactgaaa agtgagtttg 2580
cagaaacctt ggggaaaagg gccctttcaa agaagtggat aagagggagg agatcattga 2640
gtgacccaga aagctctttt gaaaagacag actcctcaag gagagataaa gaggaaagca 2700
cctctttcat tttttagtgt gagctaattc catcagactg ctgtcctcct ggacccatct 2760
gagatgtgca gtagcaagga gaggggggat cattttagag agtgggtcat tggcagggag 2820
tgctccggag ggaggcagag gggagactgt ggtagaagga agacagaact cacacatgct 2880
cccaggattg gggacaggga cagaggaggt aacagaaggc aaaggccagt ttccccgtta 2940
tcatgaaggg gcccactcag gacaggaaca aggacaactc ctcctcctcc tcctcctctc 3000
ctgctgctcc tgggatacca ggtcagtgat gtagtcttgc agtttggcaa cttcctagcc 3060
tgagaatccc tagtggggct gtgggaaaca catttccacg ttgcaagcat gcaactccaa 3120
agaatctgtg atgccactga aatgagatgg gaatgatcca gctctttcag catcttggtt 3180
gaacttgctt tcattgtccc tgggatattg tggaaggaaa ggtgactgtg tgatctgatt 3240
ctgtggtcaa ggacttgcat cttgtgtttc tatccccaag ccttcctggt gtctccaact 3300
cctaccccat tgcatgggtt gttgcggaca tccaataaag atttttttag tgcttctgga 3360

aaaaaaaaaa aaaaaaaaaa aaaaaaaaaa aaaaaaaaaa aaaaaaaaaa aaaacc 3416

<210> 16
<211> 328
<212> PRT
<213> Homo sapiens

<400> 16

Met Lys Arg Pro Lys Glu Pro Ser Gly Ser Asp Gly Glu Ser Asp Gly
1               5                   10                  15

Pro Ile Asp Val Gly Gln Glu Gly Gln Leu Ser Gln Met Ala Arg Pro
            20                  25                  30

Leu Ser Thr Pro Ser Ser Ser Gln Met Gln Ala Arg Lys Lys Arg Arg
        35                  40                  45

Gly Ile Ile Glu Lys Arg Arg Arg Asp Arg Ile Asn Ser Ser Leu Ser
    50                  55                  60

Glu Leu Arg Arg Leu Val Pro Thr Ala Phe Glu Lys Gln Gly Ser Ser
65                  70                  75                  80

Lys Leu Glu Lys Ala Glu Val Leu Gln Met Thr Val Asp His Leu Lys
                85                  90                  95

Met Leu His Ala Thr Gly Gly Thr Gly Phe Phe Asp Ala Arg Ala Leu
            100                 105                 110

Ala Val Asp Phe Arg Ser Ile Gly Phe Arg Glu Cys Leu Thr Glu Val
        115                 120                 125

Ile Arg Tyr Leu Gly Val Leu Glu Gly Pro Ser Ser Arg Ala Asp Pro
    130                 135                 140

Val Arg Ile Arg Leu Leu Ser His Leu Asn Ser Tyr Ala Ala Glu Met
145                 150                 155                 160

Glu Pro Ser Pro Thr Pro Thr Gly Pro Leu Ala Phe Pro Ala Trp Pro
                165                 170                 175

Trp Ser Phe Phe His Ser Cys Pro Gly Leu Pro Ala Leu Ser Asn Gln
            180                 185                 190

Leu Ala Ile Leu Gly Arg Val Pro Ser Pro Val Leu Pro Gly Val Ser
        195                 200                 205

Ser Pro Ala Tyr Pro Ile Pro Ala Leu Arg Thr Ala Pro Leu Arg Arg
    210                 215                 220

Ala Thr Gly Ile Ile Leu Pro Ala Arg Arg Asn Val Leu Pro Ser Arg
225                 230                 235                 240

Gly Ala Ser Ser Thr Arg Arg Ala Arg Pro Leu Glu Arg Pro Ala Thr
                245                 250                 255

Pro Val Pro Val Ala Pro Ser Ser Arg Ala Ala Arg Ser Ser His Ile
            260                 265                 270

Ala Pro Leu Leu Gln Ser Ser Ser Pro Thr Pro Pro Gly Pro Thr Gly
        275                 280                 285

Ser Ala Ala Tyr Val Ala Val Pro Thr Pro Asn Ser Ser Ser Pro Gly
    290                 295                 300

Pro Ala Gly Arg Pro Ala Gly Ala Met Leu Tyr His Ser Trp Val Ser
305                 310                 315                 320

Glu Ile Thr Glu Ile Gly Ala Phe
                325

<210>  17
<211>  1793
<212>  DNA
<213>  Homo sapiens

<400>  17
aaaaccgcgg ttgccggagc ccgaactgag gcggcggcgg gagcccggtt ggcgtctggt      60

cttcgcgtcg gccccgcgga gccagacgct gcccccggcg cggggagaag atggtgccta     120

gcggcctcgg gcccgccacg cgccgccacg agtgagccca gcgcgaccgc gggcgtccgc     180

cgagcagctg gcccggctgg gcccggggcg cgcagctgcc cgccggggcg gggtggagct     240

gatcagaata atgttcagca tcaaccccct ggagaacctg aaggtgtaca tcagcagtcg     300

gcctcccctg gtggtcttca tgatcagcgt aagcgccatg gccatagctt tcctgaccct     360

gggctacttc ttcaaaatca aggagattaa atccccagaa atggcagagg attggaatac     420

ttttctgcta cggttcaatg atttggactt gtgtgtatca gagaatgaaa ccctcaagca     480

tctcacaaac gacaccacaa ctccggaaag tacaatgacc agcgggcagg cccgagcttc     540

cacccagtcc ccccaggccc tggaggactc gggcccggtg aatatctcag tctcaatcac     600

cctaaccctg gacccactga aacccttcgg agggtattcc cgcaacgtca cccatctgta     660

ctcaaccatc ttagggcatc agattggact ttcaggcagg gaagcccacg aggagataaa     720

catcaccttc accctgccta cagcgtggag ctcagatgac tgcgccctcc acggtcactg     780

tgagcaggtg gtattcacag cctgcatgac cctcacggcc agccctgggg tgttccccgt     840

cactgtacag ccaccgcact gtgttcctga cacgtacagc aacgccacgc tctggtacaa     900

gatcttcaca actgccagag atgccaacac aaaatacgcc caagattaca atcctttctg     960

gtgttataag ggggccattg gaaaagtcta tcatgcttta aatcccaagc ttacagtgat    1020

tgttccagat gatgaccgtt cattaataaa tttgcatctc atgcacacca gttacttcct    1080

ctttgtgatg gtgataacaa tgttttgcta tgctgttatc aagggcagac ctagcaaatt    1140

gcgtcagagc aatcctgaat tttgtcccga gaaggtggct ttggctgaag cctaattcca 1200

cagctccttg ttttttgaga gagactgaga gaaccataat ccttgcctgc tgaacccagc 1260

ctgggcctgg atgctctgtg aatacattat cttgcgatgt tgggttattc cagccaaaga 1320

catttcaagt gcctgtaact gatttgtaca tatttataaa aatctattca gaaattggtc 1380

caataatgca cgtgctttgc cctgggtaca gccagagccc ttcaacccca ccttggactt 1440

gaggacctac ctgatgggac gtttccacgt gtctctagag aaggattcct ggatctagct 1500

ggtcacgacg atgttttcac caaggtcaca ggagcattgc gtcgctgatg gggttgaagt 1560

ttggtttggt tcttgtttca gcccaatatg tagagaacat ttgaaacagt ctgcaccttt 1620

gatacggtat tgcatttcca aagccaccaa tccattttgt ggattttatg tgtctgtggc 1680

ttaataatca tagtaacaac aataatacct tttcctccat tttgcttgca ggaaacatac 1740

cttaagtttt ttttgttttg tttttgtttt tttgtttttt gttttccttt atg 1793

<210> 18
<211> 314
<212> PRT
<213> Homo sapiens

<400> 18

Met Phe Ser Ile Asn Pro Leu Glu Asn Leu Lys Val Tyr Ile Ser Ser
1               5                   10                  15

Arg Pro Pro Leu Val Val Phe Met Ile Ser Val Ser Ala Met Ala Ile
            20                  25                  30

Ala Phe Leu Thr Leu Gly Tyr Phe Phe Lys Ile Lys Glu Ile Lys Ser
        35                  40                  45

Pro Glu Met Ala Glu Asp Trp Asn Thr Phe Leu Leu Arg Phe Asn Asp
    50                  55                  60

Leu Asp Leu Cys Val Ser Glu Asn Glu Thr Leu Lys His Leu Thr Asn
65                  70                  75                  80

Asp Thr Thr Thr Pro Glu Ser Thr Met Thr Ser Gly Gln Ala Arg Ala
                85                  90                  95

Ser Thr Gln Ser Pro Gln Ala Leu Glu Asp Ser Gly Pro Val Asn Ile
            100                 105                 110

Ser Val Ser Ile Thr Leu Thr Leu Asp Pro Leu Lys Pro Phe Gly Gly
        115                 120                 125

Tyr Ser Arg Asn Val Thr His Leu Tyr Ser Thr Ile Leu Gly His Gln
    130                 135                 140

Ile Gly Leu Ser Gly Arg Glu Ala His Glu Glu Ile Asn Ile Thr Phe
145                 150                 155                 160

Thr Leu Pro Thr Ala Trp Ser Ser Asp Asp Cys Ala Leu His Gly His
                165                 170                 175

Cys Glu Gln Val Val Phe Thr Ala Cys Met Thr Leu Thr Ala Ser Pro
            180                 185                 190

Gly Val Phe Pro Val Thr Val Gln Pro Pro His Cys Val Pro Asp Thr
        195                 200                 205

Tyr Ser Asn Ala Thr Leu Trp Tyr Lys Ile Phe Thr Thr Ala Arg Asp
    210                 215                 220

Ala Asn Thr Lys Tyr Ala Gln Asp Tyr Asn Pro Phe Trp Cys Tyr Lys
225                 230                 235                 240

Gly Ala Ile Gly Lys Val Tyr His Ala Leu Asn Pro Lys Leu Thr Val
                245                 250                 255

Ile Val Pro Asp Asp Asp Arg Ser Leu Ile Asn Leu His Leu Met His
            260                 265                 270

Thr Ser Tyr Phe Leu Phe Val Met Val Ile Thr Met Phe Cys Tyr Ala
        275                 280                 285

Val Ile Lys Gly Arg Pro Ser Lys Leu Arg Gln Ser Asn Pro Glu Phe
    290                 295                 300

Cys Pro Glu Lys Val Ala Leu Ala Glu Ala
305                 310

<210>  19
<211>  5180
<212>  DNA
<213>  Homo sapiens

<400>  19
accgggagcg cgcgctctga tccgaggaga ccccgcgctc ccgcagccat gggcaccggg      60

ggccggcggg gggcggcggc cgcgccgctg ctggtggcgg tggccgcgct gctactgggc     120

gccgcgggcc acctgtaccc cggagaggtg tgtcccggca tggatatccg gaacaacctc     180

actaggttgc atgagctgga gaattgctct gtcatcgaag gacacttgca gatactcttg     240

atgttcaaaa cgaggcccga agatttccga gacctcagtt tccccaaact catcatgatc     300

actgattact tgctgctctt ccgggtctat gggctcgaga gcctgaagga cctgttcccc     360

aacctcacgg tcatccgggg atcacgactg ttctttaact acgcgctggt catcttcgag     420

atggttcacc tcaaggaact cggcctctac aacctgatga acatcacccg gggttctgtc     480

cgcatcgaga agaacaatga gctctgttac ttggccacta tcgactggtc ccgtatcctg     540

gattccgtgg aggataatta catcgtgttg aacaaagatg acaacgagga gtgtggagac     600

atctgtccgg gtaccgcgaa gggcaagacc aactgccccg ccaccgtcat caacgggcag     660

tttgtcgaac gatgttggac tcatagtcac tgccagaaag tttgcccgac catctgtaag 720
tcacacggct gcaccgccga aggcctctgt tgccacagcg agtgcctggg caactgttct 780
cagcccgacg accccaccaa gtgcgtggcc tgccgcaact tctacctgga cggcaggtgt 840
gtggagacct gcccgccccc gtactaccac ttccaggact ggcgctgtgt gaacttcagc 900
ttctgccagg acctgcacca caaatgcaag aactcgcgga ggcagggctg ccaccagtac 960
gtcattcaca acaacaagtg catccctgag tgtccctccg ggtacacgat gaattccagc 1020
aacttgctgt gcaccccatg cctgggtccc tgtcccaagg tgtgccacct cctagaaggc 1080
gagaagacca tcgactcggt gacgtctgcc caggagctcc gaggatgcac cgtcatcaac 1140
gggagtctga tcatcaacat tcgaggaggc aacaatctgg cagctgagct agaagccaac 1200
ctcggcctca ttgaagaaat ttcagggtat ctaaaaatcc gccgatccta cgctctggtg 1260
tcactttcct tcttccggaa gttacgtctg attcgaggag agaccttgga aattgggaac 1320
tactccttct atgccttgga caaccagaac ctaaggcagc tctgggactg gagcaaacac 1380
aacctcacca tcactcaggg gaaactcttc ttccactata accccaaact ctgcttgtca 1440
gaaatccaca agatggaaga agtttcagga accaaggggc gccaggagag aaacgacatt 1500
gccctgaaga ccaatgggga ccaggcatcc tgtgaaaatg agttacttaa attttcttac 1560
attcggacat cttttgacaa gatcttgctg agatgggagc cgtactggcc ccccgacttc 1620
cgagacctct tggggttcat gctgttctac aaagaggccc cttatcagaa tgtgacggag 1680
ttcgacgggc aggatgcatg tggttccaac agttggacgg tggtagacat tgacccaccc 1740
ctgaggtcca acgaccccaa atcacagaac cacccagggt ggctgatgcg gggtctcaag 1800
ccctggaccc agtatgccat ctttgtgaag accctggtca ccttttcgga tgaacgccgg 1860
acctatgggg ccaagagtga catcatttat gtccagacag atgccaccaa cccctctgtg 1920
cccctggatc caatctcagt gtctaactca tcatcccaga ttattctgaa gtggaaacca 1980
ccctccgacc ccaatggcaa catcacccac tacctggttt tctgggagag gcaggcggaa 2040
gacagtgagc tgttcgagct ggattattgc ctcaaagggc tgaagctgcc ctcgaggacc 2100
tggtctccac cattcgagtc tgaagattct cagaagcaca accagagtga gtatgaggat 2160
tcggccggcg aatgctgctc ctgtccaaag acagactctc agatcctgaa ggagctggag 2220
gagtcctcgt ttaggaagac gtttgaggat tacctgcaca acgtggtttt cgtccccagg 2280
ccatctcgga aacgcaggtc ccttggcgat gttgggaatg tgacggtggc cgtgcccacg 2340
gtggcagctt tccccaacac ttcctcgacc agcgtgccca cgagtccgga ggagcacagg 2400
ccttttgaga aggtggtgaa caaggagtcg ctggtcatct ccggcttgcg acacttcacg 2460
ggctatcgca tcgagctgca ggcttgcaac caggacaccc ctgaggaacg gtgcagtgtg 2520
gcagcctacg tcagtgcgag gaccatgcct gaagccaagg ctgatgacat tgttggccct 2580
gtgacgcatg aaatctttga gaacaacgtc gtccacttga tgtggcagga gccgaaggag 2640
cccaatggtc tgatcgtgct gtatgaagtg agttatcggc gatatggtga tgaggagctg 2700
catctctgcg tctcccgcaa gcacttcgct ctggaacggg gctgcaggct gcgtgggctg 2760

tcaccgggga actacagcgt gcgaatccgg gccacctccc ttgcgggcaa cggctcttgg 2820
acggaaccca cctatttcta cgtgacagac tatttagacg tcccgtcaaa tattgcaaaa 2880
attatcatcg gccccctcat ctttgtcttt ctcttcagtg ttgtgattgg aagtatttat 2940
ctattcctga gaaagaggca gccagatggg ccgctgggac cgctttacgc ttcttcaaac 3000
cctgagtatc tcagtgccag tgatgtgttt ccatgctctg tgtacgtgcc ggacgagtgg 3060
gaggtgtctc gagagaagat caccctcctt cgagagctgg ggcagggctc cttcggcatg 3120
gtgtatgagg gcaatgccag ggacatcatc aagggtgagg cagagacccg cgtggcggtg 3180
aagacggtca acgagtcagc cagtctccga gagcggattg agttcctcaa tgaggcctcg 3240
gtcatgaagg gcttcacctg ccatcacgtg gtgcgcctcc tgggagtggt gtccaagggc 3300
cagcccacgc tggtggtgat ggagctgatg gctcacggag acctgaagag ctacctccgt 3360
tctctgcggc cagaggctga gaataatcct ggccgccctc cccctaccct tcaagagatg 3420
attcagatgg cggcagagat tgctgacggg atggcctacc tgaacgccaa gaagtttgtg 3480
catcgggacc tggcagcgag aaactgcatg gtcgcccatg attttactgt caaaattgga 3540
gactttggaa tgaccagaga catctatgaa acggattact accggaaagg gggcaagggt 3600
ctgctccctg tacggtggat ggcaccggag tccctgaagg atggggtctt caccacttct 3660
tctgacatgt ggtcctttgg cgtggtcctt tgggaaatca ccagcttggc agaacagcct 3720
taccaaggcc tgtctaatga acaggtgttg aaatttgtca tggatggagg gtatctggat 3780
caacccgaca actgtccaga gagagtcact gacctcatgc gcatgtgctg gcaattcaac 3840
cccaacatga ggccaacctt cctggagatt gtcaacctgc tcaaggacga cctgcacccc 3900
agctttccag aggtgtcgtt cttccacagc gaggagaaca aggctcccga gagtgaggag 3960
ctggagatgg agtttgagga catggagaat gtgcccctgg accgttcctc gcactgtcag 4020
agggaggagg cggggggccg ggatggaggg tcctcgctgg gtttcaagcg gagctacgag 4080
gaacacatcc cttacacaca catgaacgga ggcaagaaaa acgggcggat tctgaccttg 4140
cctcggtcca atccttccta acagtgccta ccgtggcggg ggcgggcagg ggttcccatt 4200
ttcgctttcc tctggtttga aagcctctgg aaaactcagg attctcacga ctctaccatg 4260
tccaatggag ttcagagatc gttcctatac atttctgttc atcttaaggt ggactcgttt 4320
ggttaccaat ttaactagtc ctgcagagga tttaactgtg aacctggagg gcaaggggtt 4380
tccacagttg ctgctccttt ggggcaacga cggtttcaaa ccaggatttt gtgttttttc 4440
gttcccccca cccgccccca gcagatggaa agaaagcacc tgtttttaca aattcttttt 4500
tttttttttt ttttttgctg gtgtctgagc ttcagtataa aagacaaaac ttcctgtttg 4560
tggaacaaaa gttcgaaaga aaaaacaaaa caaaaacacc cagccctgtt ccaggagaat 4620
ttcaagtttt acaggttgag cttcaagatg gtttttttgg tttttttttt ttctctcatc 4680
caggctgaag gatttttttt ttctttacaa aatgagttcc tcaaattgac caatagctgc 4740
tgctttcata ttttggataa gggtctgtgg tcccggcgtg tgctcacgtg tgtatgcacg 4800

tgtgtgtgtc cattagacac ggctgacgtg tgtgcaaagt atccatgcgg agttgatgct 4860

ttgggaattg gctcatgaag gttcttctca agggtgcgag ctcatccccc tctctccttc 4920

cttcttattg actgggagac tgtgctctcg acagattctt cttgtgtcag aagtctagcc 4980

tcaggtttct accctccctt cacattggtg gccaagggag gagcatttca tttggagtga 5040

ttatgaatct tttcaagacc aaaccaagct aggacattaa aaaaaaaaaa aagaaaaaga 5100

aagaaaaaac aaaatggaaa aaggaaaaaa aaaaagaact gagatgacag agttttgaga 5160

atatatttgt accatattta 5180

<210> 20
<211> 1370
<212> PRT
<213> Homo sapiens

<400> 20

<210>  21
<211>  1155
<212>  DNA
<213>  Homo sapiens

<400>  21
gccgctgcca ccgcaccccg ccatggagcg gccgtcgctg cgcgccctgc tcctcggcgc      60

cgctgggctg ctgctcctgc tcctgcccct ctcctcttcc tcctcttcgg acacctgcgg     120

cccctgcgag ccggcctcct gcccgcccct gcccccgctg ggctgcctgc tgggcgagac     180

ccgcgacgcg tgcggctgct gccctatgtg cgcccgcggc gagggcgagc cgtgcggggg     240

tggcggcgcc ggcagggggt actgcgcgcc gggcatggag tgcgtgaaga gccgcaagag     300

gcggaagggt aaagccgggg cagcagccgg cggtccgggt gtaagcggcg tgtgcgtgtg     360

caagagccgc tacccggtgt gcggcagcga cggcaccacc tacccgagcg gctgccagct     420

gcgcgccgcc agccagaggg ccgagagccg cggggagaag gccatcaccc aggtcagcaa     480

gggcacctgc gagcaaggtc cttccatagt gacgcccccc aaggacatct ggaatgtcac     540

tggtgcccag gtgtacttga gctgtgaggt catcggaatc ccgacacctg tcctcatctg     600

gaacaaggta aaaaggggtc actatggagt tcaaaggaca gaactcctgc ctggtgaccg     660

ggacaacctg gccattcaga cccggggtgg cccagaaaag catgaagtaa ctggctgggt     720

gctggtatct cctctaagta aggaagatgc tggagaatat gagtgccatg catccaattc     780

ccaaggacag gcttcagcat cagcaaaaat tacagtggtt gatgccttac atgaaatacc     840

agtgaaaaaa ggtgaaggtg ccgagctata aacctccaga atattattag tctgcatggt     900

taaaagtagt catggataac tacattacct gttcttgcct aataagtttc ttttaatcca     960

atccactaac actttagtta tattcactgg ttttacacag agaaatacaa aataaagatc    1020

acacatcaag actatctaca aaaatttatt atatatttac agaagaaaag catgcatatc 1080

attaaacaaa taaaatactt tttatcacaa aaaaaaaaaa aaaaaaaaaa aaaaaaaaaa 1140

aaaaaaaaaa aaaaa 1155

<210> 22
<211> 282
<212> PRT
<213> Homo sapiens

<400> 22

Met Glu Arg Pro Ser Leu Arg Ala Leu Leu Leu Gly Ala Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60

Arg Gly Glu Gly Glu Pro Cys Gly Gly Gly Gly Ala Gly Arg Gly Tyr
65                  70                  75                  80

Cys Ala Pro Gly Met Glu Cys Val Lys Ser Arg Lys Arg Arg Lys Gly
                85                  90                  95

Lys Ala Gly Ala Ala Ala Gly Gly Pro Gly Val Ser Gly Val Cys Val
            100                 105                 110

Cys Lys Ser Arg Tyr Pro Val Cys Gly Ser Asp Gly Thr Thr Tyr Pro
        115                 120                 125

Ser Gly Cys Gln Leu Arg Ala Ala Ser Gln Arg Ala Glu Ser Arg Gly
    130                 135                 140

Glu Lys Ala Ile Thr Gln Val Ser Lys Gly Thr Cys Glu Gln Gly Pro
145                 150                 155                 160

Ser Ile Val Thr Pro Pro Lys Asp Ile Trp Asn Val Thr Gly Ala Gln
                165                 170                 175

Val Tyr Leu Ser Cys Glu Val Ile Gly Ile Pro Thr Pro Val Leu Ile
            180                 185                 190

Trp Asn Lys Val Lys Arg Gly His Tyr Gly Val Gln Arg Thr Glu Leu
        195                 200                 205

Leu Pro Gly Asp Arg Asp Asn Leu Ala Ile Gln Thr Arg Gly Gly Pro
    210                 215                 220

Glu Lys His Glu Val Thr Gly Trp Val Leu Val Ser Pro Leu Ser Lys
225                 230                 235                 240

Glu Asp Ala Gly Glu Tyr Glu Cys His Ala Ser Asn Ser Gln Gly Gln
                245                 250                 255

Ala Ser Ala Ser Ala Lys Ile Thr Val Val Asp Ala Leu His Glu Ile
            260                 265                 270

Pro Val Lys Lys Gly Glu Gly Ala Glu Leu
        275                 280

<210>  23
<211>  1336
<212>  DNA
<213>  Homo sapiens

<400>  23
ggggcttgca gagccggcgc cggaggagac gcacgcagct gactttgtct tctccgcacg      60

actgttacag aggtctccag agccttctct ctcctgtgca aaatggcaac tcttaaggaa     120

aaactcattg caccagttgc ggaagaagag gcaacagttc caaacaataa gatcactgta     180

gtgggtgttg gacaagttgg tatggcgtgt gctatcagca ttctgggaaa gtctctggct     240

gatgaacttg ctcttgtgga tgttttggaa gataagctta aaggagaaat gatggatctg     300

cagcatggga gcttatttct tcagacacct aaaattgtgg cagataaaga ttattctgtg     360

accgccaatt ctaagattgt agtggtaact gcaggagtcc gtcagcaaga aggggagagt     420

cggctcaatc tggtgcagag aaatgttaat gtcttcaaat tcattattcc tcagatcgtc     480

aagtacagtc ctgattgcat cataattgtg gtttccaacc cagtggacat tcttacgtat     540

gttacctgga aactaagtgg attacccaaa caccgcgtga ttggaagtgg atgtaatctg     600

gattctgcta gatttcgcta ccttatggct gaaaaacttg gcattcatcc cagcagctgc     660

catggatgga ttttggggga acatggcgac tcaagtgtgg ctgtgtggag tggtgtgaat     720

gtggcaggtg tttctctcca ggaattgaat ccagaaatgg gaactgacaa tgatagtgaa     780

aattggaagg aagtgcataa gatggtggtt gaaagtgcct atgaagtcat caagctaaaa     840

ggatatacca actgggctat tggattaagt gtggctgatc ttattgaatc catgttgaaa     900

aatctatcca ggattcatcc cgtgtcaaca atggtaaagg ggatgtatgg cattgagaat     960

gaagtcttcc tgagccttcc atgtatcctc aatgcccggg gattaaccag cgttatcaac    1020

cagaagctaa aggatgatga ggttgctcag ctcaagaaaa gtgcagatac cctgtgggac    1080

atccagaagg acctaaaaga cctgtgacta gtgagctcta ggctgtagaa atttaaaaac    1140

tacaatgtga ttaactcgag cctttagttt tcatccatgt acatggatca cagtttgctt    1200

tgatcttctt caatatgtga atttgggctc acagaatcaa agcctatgct tggtttaatg    1260

cttgcaatct gagctcttga acaaataaaa ttaactattg tagtgcgaaa aaaaaaaaaa    1320

aaaaaaaaaa aaaaaa                                                    1336

<210>  24
<211>  334
<212>  PRT
<213>  Homo sapiens

<400>  24

Met Ala Thr Leu Lys Glu Lys Leu Ile Ala Pro Val Ala Glu Glu Glu
1               5                   10                  15

Ala Thr Val Pro Asn Asn Lys Ile Thr Val Val Gly Val Gly Gln Val
            20                  25                  30

Gly Met Ala Cys Ala Ile Ser Ile Leu Gly Lys Ser Leu Ala Asp Glu
        35                  40                  45

Leu Ala Leu Val Asp Val Leu Glu Asp Lys Leu Lys Gly Glu Met Met
    50                  55                  60

Asp Leu Gln His Gly Ser Leu Phe Leu Gln Thr Pro Lys Ile Val Ala
65                  70                  75                  80

Asp Lys Asp Tyr Ser Val Thr Ala Asn Ser Lys Ile Val Val Val Thr
                85                  90                  95

Ala Gly Val Arg Gln Gln Glu Gly Glu Ser Arg Leu Asn Leu Val Gln
            100                 105                 110

Arg Asn Val Asn Val Phe Lys Phe Ile Ile Pro Gln Ile Val Lys Tyr
        115                 120                 125

Ser Pro Asp Cys Ile Ile Ile Val Val Ser Asn Pro Val Asp Ile Leu
    130                 135                 140

Thr Tyr Val Thr Trp Lys Leu Ser Gly Leu Pro Lys His Arg Val Ile
145                 150                 155                 160

Gly Ser Gly Cys Asn Leu Asp Ser Ala Arg Phe Arg Tyr Leu Met Ala
                165                 170                 175

Glu Lys Leu Gly Ile His Pro Ser Ser Cys His Gly Trp Ile Leu Gly
            180                 185                 190

Glu His Gly Asp Ser Ser Val Ala Val Trp Ser Gly Val Asn Val Ala
        195                 200                 205

Gly Val Ser Leu Gln Glu Leu Asn Pro Glu Met Gly Thr Asp Asn Asp
    210                 215                 220

Ser Glu Asn Trp Lys Glu Val His Lys Met Val Val Glu Ser Ala Tyr
225                 230                 235                 240

Glu Val Ile Lys Leu Lys Gly Tyr Thr Asn Trp Ala Ile Gly Leu Ser

245                 250                 255

Val Ala Asp Leu Ile Glu Ser Met Leu Lys Asn Leu Ser Arg Ile His
            260                 265                 270

Pro Val Ser Thr Met Val Lys Gly Met Tyr Gly Ile Glu Asn Glu Val
        275                 280                 285

Phe Leu Ser Leu Pro Cys Ile Leu Asn Ala Arg Gly Leu Thr Ser Val
    290                 295                 300

Ile Asn Gln Lys Leu Lys Asp Asp Glu Val Ala Gln Leu Lys Lys Ser
305                 310                 315                 320

Ala Asp Thr Leu Trp Asp Ile Gln Lys Asp Leu Lys Asp Leu
                325                 330

<210>  25
<211>  543
<212>  DNA
<213>  Homo sapiens

<400>  25
ccctccccca gagctgttat gatgagctgc aggaagctct ggagttgggc ggggagactg      60

gactcccggg agtacccagc gctgcttttt tgttcatgca gcctgtgatt catagcttcc     120

ctggggtgtt ggggagaatc acatttgggt cagccaggtt tagcactgac agttttgtct     180

ttagaatcaa gcagatgtgg aatcaaatct ggctgtatcc atgaccaact ctgaagccat     240

gagtgggtta catagcttta gagcctcagc atactcatct ggaaagtgga agtgatcatg     300

tctattttgc agagttgttg ccacttttcc tctctggacc ccactttccc catctgtcag     360

atgaaagagt gggatgagat ggcctgtcta tttatctctc aatcacaatg gctctatttg     420

aaaaaagttt gaactgccct aagtgctaaa aaggaagatg gggagccatc aagacaaact     480

taggcctaca ttaccatcga gttcagagaa tggcagaccg taagcaccag ccagcttcac     540

tcc                                                                   543

<210>  26
<211>  543
<212>  DNA
<213>  Homo sapiens

<400>  26
ccctccccca gagctgttat gatgagctgc aggaagctct ggagttgggc ggggagactg      60

gactcccggg agtacccagc gctgcttttt tgttcatgca gcctgtgatt catagcttcc     120

ctggggtgtt ggggagaatc acatttgggt cagccaggtt tagcactgac agttttgtct     180

ttagaatcaa gcagatgtgg aatcaaatct ggctgtatcc atgaccaact ctgaagccat     240

gagtgggtta catagcttta gagcctcagc atactcatct ggaaagtgga agtgatcatg     300

tctattttgc agagttgttg ccacttttcc tctctggacc ccactttccc catctgtcag     360

atgaaagagt gggatgagat ggcctgtcta tttatctctc aatcacaatg gctctatttg     420

aaaaaagttt gaactgccct aagtgctaaa aaggaagatg gggagccatc aagacaaact 480

taggcctaca ttaccatcga gttcagagaa tggcagaccg taagcaccag ccagcttcac 540

tcc 543

<210> 27
<211> 3350
<212> DNA
<213> Homo sapiens

<400> 27
tggaggagag aagatggcgg aagcggagtg agtgactaga tgatttaagg accatagtac 60

agctatggat actgaaccaa acccgggaac atcttctgtg tcaacaacaa ccagcagtac 120

caccaccacc accatcacca cttcctcctc tcgaatgcag cagccacaga tctctgtcta 180

cagtggttca gaccgacatg ctgtacaggt aattcaacag gcattgcatc ggccccccag 240

ctcagctgct cagtaccttc agcaaatgta tgcagcccaa caacagcact tgatgctgca 300

tactgcagct cttcagcagc agcatttaag cagctcccag cttcagagcc ttgctgctgt 360

tcaggcaagt ttgtccagtg gaagaccatc tacatctccc acaggaagtg tcacacagca 420

gtcaagtatg tcccaaacgt ctatcaacct ctccacttct cctacacctg cacagttaat 480

aagccgttcc caggcttcca gttctaccag cggcagtatt acccaacaga ctatgttact 540

agggagtact tcccctaccc taacggcaag ccaagctcaa atgtatctcc gagctcaaat 600

gctgattttc acacccgcta ccactgtggc tgctgtacag tctgacattc ctgttgtctc 660

gtcgtcatcg tcatcttcct gtcagtctgc agctactcag gttcagaatt taacattacg 720

cagccagaag ttgggtgtat tatctagctc acagaatggt ccaccaaaaa gcactagtca 780

aactcagtca ttgacaattt gtcataacaa aacaacagtg accagttcta aaatcagcca 840

acgagatcct tctccagaaa gtaataagaa aggagagagc ccaagcctgg aatcacgaag 900

cacagctgtc acccggacat caagtattca ccagttaata gcaccagctt catattctcc 960

aattcagcct cattctctaa taaaacatca gcagattcct cttcattcac caccttccaa 1020

agtttcccat catcagctga tattacaaca gcagcaacag caaattcagc caatcacact 1080

tcagaattca actcaagacc cacccccatc ccagcactgt ataccactcc agaaccatgg 1140

ccttcctcca gctcccagta atgcccagtc acagcattgt tcaccgattc agagtcatcc 1200

ctctccttta acagtgtctc ctaatcagtc acagtcagca cagcagtctg tagtggtgtc 1260

tcctccacca cctcattcac caagtcagtc tcctactata attattcatc cacaagcact 1320

tattcagcca caccctcttg tgtcatcagc tctccagcca gggccaaatt tgcagcagtc 1380

cactgctaat caggtgcaag ctacagcaca gttgaatctt ccatcccatc ttccacttcc 1440

agcttcccct gttgtacaca ttggcccagt tcagcagtct gccttggtat ccccaggcca 1500

gcagattgtc tctccatcac accagcaata ttcatccctg cagtcctctc caatcccaat 1560

tgcaagtcct ccacagatgt cgacatctcc tccagctcag attccaccac tgcccttgca 1620

gtctatgcag tctttacaag tgcagcctga aattctgtcc cagggccagg ttttggtgca 1680

gaatgctttg gtgtcagaag aggaacttcc agctgcagaa gctttggtcc agttgccatt 1740

tcagactctt cctcctccac agactgttgc ggtaaaccta caagtgcaac caccagcacc 1800

tgttgatcca ccagtggttt atcaggtaga agatgtgtgt gaagaagaaa tgccagaaga 1860

gtcagatgaa tgtgtccgga tggatagaac cccaccacca cccactttgt ctccagcagc 1920

tataacagtg gggagaggag aagatttgac ttctgaacat cctttgttag agcaagtgga 1980

attacctgct gtggcatcag tcagtgcttc agtaattaaa tctccatcag atccctcaca 2040

tgtttctgtt ccaccacctc cattgttact tccagctgcc accacaagga gtaacagtac 2100

atctatgcac agtagcattc ccagtataga gaacaaacct ccacaggcta ttgttaaacc 2160

acagatccta acccatgtta ttgaaggctt tgtgattcag gagggattgg agccatttcc 2220

tgtgagtcgt tcctctttgc taatagaaca gcctgtgaaa aaacggcctc ttttggataa 2280

tcaggtgata aattcagtgt gtgttcagcc agagctacag aataatacaa aacatgcgga 2340

taattcatct gacacagaga tggaagacat gattgctgaa gagacattag aagaaatgga 2400

cagtgagttg ctcaagtgtg aattctgtgg gaaaatggga tatgctaatg aatttttgcg 2460

gtcaaaacga ttctgcacta tgtcatgtgc caaaaggtac aatgttagct gttctaaaaa 2520

atttgcactt agtcgttgga atcgtaagcc tgataatcaa agtcttgggc atcgtggccg 2580

tcgtccaagt ggccctgatg gggcagcgag agaacatatc cttaggcagc ttccaattac 2640

ttatccatct gcagaagaag acttggcttc tcatgaagat tctgtgccat ctgctatgac 2700

aactcgtctg cgcaggcaga gcgagcggga aagagaacgt gagcttcggg atgtgagaat 2760

tcggaaaatg cctgagaaca gtgacttgct accagttgca caaacagagc catctatatg 2820

gacagttgat gatgtctggg ccttcatcca ttctttgcct ggctgccagg atatcgcaga 2880

tgaattcaga gcacaggaga ttgatggaca ggcccttctc ttgctgaaag aagaccatct 2940

catgagtgca atgaatatca agctaggccc agccctgaag atctgtgcac gcatcaactc 3000

tctgaaggaa tcttaacagg aacatgaagc cttgataaaa cagcagtttt acttttctca 3060

caaaaacttg taaggtaaag gcctaacttg gtctagaata tgacacttat tgtggtggat 3120

agccaagcac attgggatct ccacatcaaa tactgacatt tcttctacag gtataataat 3180

tcatcatgca ttttcataat taataaacat tggtaaaatt aattttacag gttacatgaa 3240

acattgaaag acttgttaca gagggccatg atatttttca aagaaatgtg ttatactaga 3300

taattttttt aaaggtgatg tttatcatta atataaagaa tccttttaaa 3350

<210> 28
<211> 983
<212> PRT
<213> Homo sapiens

<400> 28
   Met Asp Thr Glu Pro Asn Pro Gly Thr Ser Ser Val Ser Thr Thr Thr
   1 5 10 15
   Ser Ser Thr Thr Thr Thr Thr Ile Thr Thr Ser Ser Ser Arg Met Gln
               20                  25                  30
   Gln Pro Gln Ile Ser Val Tyr Ser Gly Ser Asp Arg His Ala Val Gln
           35                  40                  45
   Val Ile Gln Gln Ala Leu His Arg Pro Pro Ser Ser Ala Ala Gln Tyr
       50                  55                  60
   Leu Gln Gln Met Tyr Ala Ala Gln Gln Gln His Leu Met Leu His Thr
   65                  70                  75                  80
   Ala Ala Leu Gln Gln Gln His Leu Ser Ser Ser Gln Leu Gln Ser Leu
                   85                  90                  95
   Ala Ala Val Gln Ala Ser Leu Ser Ser Gly Arg Pro Ser Thr Ser Pro
               100                 105                 110
   Thr Gly Ser Val Thr Gln Gln Ser Ser Met Ser Gln Thr Ser Ile Asn
           115                 120                 125
   Leu Ser Thr Ser Pro Thr Pro Ala Gln Leu Ile Ser Arg Ser Gln Ala
       130                 135                 140
   Ser Ser Ser Thr Ser Gly Ser Ile Thr Gln Gln Thr Met Leu Leu Gly
   145                 150                 155                 160
   Ser Thr Ser Pro Thr Leu Thr Ala Ser Gln Ala Gln Met Tyr Leu Arg
                   165                 170                 175
   Ala Gln Met Leu Ile Phe Thr Pro Ala Thr Thr Val Ala Ala Val Gln
               180                 185                 190
   Ser Asp Ile Pro Val Val Ser Ser Ser Ser Ser Ser Ser Cys Gln Ser
           195                 200                 205
   Ala Ala Thr Gln Val Gln Asn Leu Thr Leu Arg Ser Gln Lys Leu Gly
       210                 215                 220
   Val Leu Ser Ser Ser Gln Asn Gly Pro Pro Lys Ser Thr Ser Gln Thr
   225                 230                 235                 240
   Gln Ser Leu Thr Ile Cys His Asn Lys Thr Thr Val Thr Ser Ser Lys
                   245                 250                 255
   Ile Ser Gln Arg Asp Pro Ser Pro Glu Ser Asn Lys Lys Gly Glu Ser
               260                 265                 270
   Pro Ser Leu Glu Ser Arg Ser Thr Ala Val Thr Arg Thr Ser Ser Ile
           275                 280                 285
   His Gln Leu Ile Ala Pro Ala Ser Tyr Ser Pro Ile Gln Pro His Ser
   290                 295                 300
   Leu Ile Lys His Gln Gln Ile Pro Leu His Ser Pro Pro Ser Lys Val
   305                 310                 315                 320
   Ser His His Gln Leu Ile Leu Gln Gln Gln Gln Gln Gln Ile Gln Pro
                   325                 330                 335
   Ile Thr Leu Gln Asn Ser Thr Gln Asp Pro Pro Pro Ser Gln His Cys
               340                 345                 350
   Ile Pro Leu Gln Asn His Gly Leu Pro Pro Ala Pro Ser Asn Ala Gln
           355                 360                 365
   Ser Gln His Cys Ser Pro Ile Gln Ser His Pro Ser Pro Leu Thr Val
       370                 375                 380
   Ser Pro Asn Gln Ser Gln Ser Ala Gln Gln Ser Val Val Val Ser Pro
   385                 390                 395                 400
   Pro Pro Pro His Ser Pro Ser Gln Ser Pro Thr Ile Ile Ile His Pro
                   405                 410                 415
   Gln Ala Leu Ile Gln Pro His Pro Leu Val Ser Ser Ala Leu Gln Pro
               420                 425                 430
   Gly Pro Asn Leu Gln Gln Ser Thr Ala Asn Gln Val Gln Ala Thr Ala
           435                 440                 445
   Gln Leu Asn Leu Pro Ser His Leu Pro Leu Pro Ala Ser Pro Val Val
       450                 455                 460
   His Ile Gly Pro Val Gln Gln Ser Ala Leu Val Ser Pro Gly Gln Gln
   465                 470                 475                 480
   Ile Val Ser Pro Ser His Gln Gln Tyr Ser Ser Leu Gln Ser Ser Pro
                   485                 490                 495
   Ile Pro Ile Ala Ser Pro Pro Gln Met Ser Thr Ser Pro Pro Ala Gln
               500                 505                 510
   Ile Pro Pro Leu Pro Leu Gln Ser Met Gln Ser Leu Gln Val Gln Pro
           515                 520                 525
   Glu Ile Leu Ser Gln Gly Gln Val Leu Val Gln Asn Ala Leu Val Ser
       530                 535                 540
   Glu Glu Glu Leu Pro Ala Ala Glu Ala Leu Val Gln Leu Pro Phe Gln
   545                 550                 555                 560
   Thr Leu Pro Pro Pro Gln Thr Val Ala Val Asn Leu Gln Val Gln Pro
                   565                 570                 575
   Pro Ala Pro Val Asp Pro Pro Val Val Tyr Gln Val Glu Asp Val Cys
               580                 585                 590
   Glu Glu Glu Met Pro Glu Glu Ser Asp Glu Cys Val Arg Met Asp Arg
           595                 600                 605
   Thr Pro Pro Pro Pro Thr Leu Ser Pro Ala Ala Ile Thr Val Gly Arg
       610                 615                 620
   Gly Glu Asp Leu Thr Ser Glu His Pro Leu Leu Glu Gln Val Glu Leu
   625                 630                 635                 640
   Pro Ala Val Ala Ser Val Ser Ala Ser Val Ile Lys Ser Pro Ser Asp
                   645                 650                 655
   Pro Ser His Val Ser Val Pro Pro Pro Pro Leu Leu Leu Pro Ala Ala
               660                 665                 670
   Thr Thr Arg Ser Asn Ser Thr Ser Met His Ser Ser Ile Pro Ser Ile
           675                 680                 685
   Glu Asn Lys Pro Pro Gln Ala Ile Val Lys Pro Gln Ile Leu Thr His
       690                 695                 700
   Val Ile Glu Gly Phe Val Ile Gln Glu Gly Leu Glu Pro Phe Pro Val
   705                 710                 715                 720
   Ser Arg Ser Ser Leu Leu Ile Glu Gln Pro Val Lys Lys Arg Pro Leu
                   725                 730                 735
   Leu Asp Asn Gln Val Ile Asn Ser Val Cys Val Gln Pro Glu Leu Gln
               740                 745                 750
   Asn Asn Thr Lys His Ala Asp Asn Ser Ser Asp Thr Glu Met Glu Asp
           755                 760                 765
   Met Ile Ala Glu Glu Thr Leu Glu Glu Met Asp Ser Glu Leu Leu Lys
       770                 775                 780
   Cys Glu Phe Cys Gly Lys Met Gly Tyr Ala Asn Glu Phe Leu Arg Ser
   785                 790                 795                 800
   Lys Arg Phe Cys Thr Met Ser Cys Ala Lys Arg Tyr Asn Val Ser Cys
                   805                 810                 815
   Ser Lys Lys Phe Ala Leu Ser Arg Trp Asn Arg Lys Pro Asp Asn Gln
               820                 825                 830
   Ser Leu Gly His Arg Gly Arg Arg Pro Ser Gly Pro Asp Gly Ala Ala
           835                 840                 845
   Arg Glu His Ile Leu Arg Gln Leu Pro Ile Thr Tyr Pro Ser Ala Glu
       850                 855                 860
   Glu Asp Leu Ala Ser His Glu Asp Ser Val Pro Ser Ala Met Thr Thr
   865                 870                 875                 880
   Arg Leu Arg Arg Gln Ser Glu Arg Glu Arg Glu Arg Glu Leu Arg Asp
                   885                 890                 895
   Val Arg Ile Arg Lys Met Pro Glu Asn Ser Asp Leu Leu Pro Val Ala
               900                 905                 910
   Gln Thr Glu Pro Ser Ile Trp Thr Val Asp Asp Val Trp Ala Phe Ile
           915                 920                 925
   His Ser Leu Pro Gly Cys Gln Asp Ile Ala Asp Glu Phe Arg Ala Gln
       930                 935                 940
   Glu Ile Asp Gly Gln Ala Leu Leu Leu Leu Lys Glu Asp His Leu Met
   945                 950                 955                 960
   Ser Ala Met Asn Ile Lys Leu Gly Pro Ala Leu Lys Ile Cys Ala Arg
                   965                 970                 975
   Ile Asn Ser Leu Lys Glu Ser
               980

<210>  29
<211>  2133
<212>  DNA
<213>  Homo sapiens

<400>  29
cgggagagcg cgctctgcct gccgcctgcc tgcctgccac tgagggttcc cagcaccatg      60

agggcctgga tcttctttct cctttgcctg gccgggaggg ccttggcagc ccctcagcaa     120

gaagccctgc ctgatgagac agaggtggtg gaagaaactg tggcagaggt gactgaggta     180

tctgtgggag ctaatcctgt ccaggtggaa gtaggagaat ttgatgatgg tgcagaggaa     240

accgaagagg aggtggtggc ggaaaatccc tgccagaacc accactgcaa acacggcaag     300

gtgtgcgagc tggatgagaa caacaccccc atgtgcgtgt gccaggaccc caccagctgc     360

ccagccccca ttggcgagtt tgagaaggtg tgcagcaatg acaacaagac cttcgactct     420

tcctgccact tctttgccac aaagtgcacc ctggagggca ccaagaaggg ccacaagctc     480

cacctggact acatcgggcc ttgcaaatac atcccccctt gcctggactc tgagctgacc     540

gaattccccc tgcgcatgcg ggactggctc aagaacgtcc tggtcaccct gtatgagagg     600

gatgaggaca acaaccttct gactgagaag cagaagctgc gggtgaagaa gatccatgag     660

aatgagaagc gcctggaggc aggagaccac cccgtggagc tgctggcccg ggacttcgag     720

aagaactata acatgtacat cttccctgta cactggcagt tcggccagct ggaccagcac     780

cccattgacg ggtacctctc ccacaccgag ctggctccac tgcgtgctcc cctcatcccc 840

atggagcatt gcaccacccg ctttttcgag acctgtgacc tggacaatga caagtacatc 900

gccctggatg agtgggccgg ctgcttcggc atcaagcaga aggatatcga caaggatctt 960

gtgatctaaa tccactcctt ccacagtacc ggattctctc tttaaccctc cccttcgtgt 1020

ttcccccaat gtttaaaatg tttggatggt ttgttgttct gcctggagac aaggtgctaa 1080

catagattta agtgaataca ttaacggtgc taaaaatgaa aattctaacc caagacatga 1140

cattcttagc tgtaacttaa ctattaaggc cttttccaca cgcattaata gtcccatttt 1200

tctcttgcca tttgtagctt tgcccattgt cttattggca catgggtgga cacggatctg 1260

ctgggctctg ccttaaacac acattgcagc ttcaactttt ctctttagtg ttctgtttga 1320

aactaatact taccgagtca gactttgtgt tcatttcatt tcagggtctt ggctgcctgt 1380

gggcttcccc aggtggcctg gaggtgggca aagggaagta acagacacac gatgttgtca 1440

aggatggttt tgggactaga ggctcagtgg tgggagagat ccctgcagaa tccaccaacc 1500

agaacgtggt ttgcctgagg ctgtaactga gagaaagatt ctggggctgt cttatgaaaa 1560

tatagacatt ctcacataag cccagttcat caccatttcc tcctttacct ttcagtgcag 1620

tttcttttca cattaggctg ttggttcaaa cttttgggag cacggactgt cagttctctg 1680

ggaagtggtc agcgcatcct gcagggcttc tcctcctctg tcttttggag aaccagggct 1740

cttctcaggg gctctaggga ctgccaggct gtttcagcca ggaaggccaa aatcaagagt 1800

gagatgtaga aagttgtaaa atagaaaaag tggagttggt gaatcggttg ttctttcctc 1860

acatttggat gattgtcata aggtttttag catgttcctc cttttcttca ccctcccctt 1920

tgttcttcta ttaatcaaga gaaacttcaa agttaatggg atggtcggat ctcacaggct 1980

gagaactcgt tcacctccaa gcatttcatg aaaaagctgc ttcttattaa tcatacaaac 2040

tctcaccatg atgtgaagag tttcacaaat ctttcaaaat aaaaagtaat gacttagaaa 2100

ctgaaaaaaa aaaaaaaaaa aaaaaaaaaa aaa 2133

<210> 30
<211> 303
<212> PRT
<213> Homo sapiens

<400> 30

Met Arg Ala Trp Ile Phe Phe Leu Leu Cys Leu Ala Gly Arg Ala Leu
1               5                   10                  15

Ala Ala Pro Gln Gln Glu Ala Leu Pro Asp Glu Thr Glu Val Val Glu
            20                  25                  30

Glu Thr Val Ala Glu Val Thr Glu Val Ser Val Gly Ala Asn Pro Val
        35                  40                  45

Gln Val Glu Val Gly Glu Phe Asp Asp Gly Ala Glu Glu Thr Glu Glu
    50                  55                  60

Glu Val Val Ala Glu Asn Pro Cys Gln Asn His His Cys Lys His Gly
65                  70                  75                  80

Lys Val Cys Glu Leu Asp Glu Asn Asn Thr Pro Met Cys Val Cys Gln
                85                  90                  95

Asp Pro Thr Ser Cys Pro Ala Pro Ile Gly Glu Phe Glu Lys Val Cys
            100                 105                 110

Ser Asn Asp Asn Lys Thr Phe Asp Ser Ser Cys His Phe Phe Ala Thr
        115                 120                 125

Lys Cys Thr Leu Glu Gly Thr Lys Lys Gly His Lys Leu His Leu Asp
    130                 135                 140

Tyr Ile Gly Pro Cys Lys Tyr Ile Pro Pro Cys Leu Asp Ser Glu Leu
145                 150                 155                 160

Thr Glu Phe Pro Leu Arg Met Arg Asp Trp Leu Lys Asn Val Leu Val
                165                 170                 175

Thr Leu Tyr Glu Arg Asp Glu Asp Asn Asn Leu Leu Thr Glu Lys Gln
            180                 185                 190

Lys Leu Arg Val Lys Lys Ile His Glu Asn Glu Lys Arg Leu Glu Ala
        195                 200                 205

Gly Asp His Pro Val Glu Leu Leu Ala Arg Asp Phe Glu Lys Asn Tyr
    210                 215                 220

Asn Met Tyr Ile Phe Pro Val His Trp Gln Phe Gly Gln Leu Asp Gln
225                 230                 235                 240

His Pro Ile Asp Gly Tyr Leu Ser His Thr Glu Leu Ala Pro Leu Arg
                245                 250                 255

Ala Pro Leu Ile Pro Met Glu His Cys Thr Thr Arg Phe Phe Glu Thr
            260                 265                 270

Cys Asp Leu Asp Asn Asp Lys Tyr Ile Ala Leu Asp Glu Trp Ala Gly
        275                 280                 285

Cys Phe Gly Ile Lys Gln Lys Asp Ile Asp Lys Asp Leu Val Ile
    290                 295                 300

<210>  31
<211>  634
<212>  DNA
<213>  Homo sapiens

<400>  31
gccagccctc ggaaacgcga agtgagcggc ggggtcgact gacggtaacg gggcagagag      60

gctgttcgca gagctgcgga agatgaatgc cagaggactt ggatctgagc taaaggacag 120

tattccagtt actgaacttt cagcaagtgg accttttgaa agtcatgatc ttcttcggaa 180

aggtttttct tgtgtgaaaa atgaactttt gcctagtcat ccccttgaat tatcagaaaa 240

aaatttccag ctcaaccaag ataaaatgaa tttttccaca ctgagaaaca ttcagggtct 300

atttgctccg ctaaaattac agatggaatt caaggcagtg cagcaggttc agcgtcttcc 360

atttctttca agctcaaatc tttcactgga tgttttgagg ggtaatgatg agactattgg 420

atttgaggat attcttaatg atccatcaca aagcgaagtc atgggagagc cacacttgat 480

ggtggaatat aaacttggtt tactgtaata gtgtgctgtt catggaaacc gagggctgca 540

tcttgtttat agtcatcttt gtactgtaat ttgatgtaca caacattaaa agtactgaca 600

cctgaaaaaa aaaaaaaaaa aaaaaaaaaa aaaa 634

<210> 32
<211> 141
<212> PRT
<213> Homo sapiens

<400> 32

Met Asn Ala Arg Gly Leu Gly Ser Glu Leu Lys Asp Ser Ile Pro Val
1               5                   10                  15

Thr Glu Leu Ser Ala Ser Gly Pro Phe Glu Ser His Asp Leu Leu Arg
            20                  25                  30

Lys Gly Phe Ser Cys Val Lys Asn Glu Leu Leu Pro Ser His Pro Leu
        35                  40                  45

Glu Leu Ser Glu Lys Asn Phe Gln Leu Asn Gln Asp Lys Met Asn Phe
    50                  55                  60

Ser Thr Leu Arg Asn Ile Gln Gly Leu Phe Ala Pro Leu Lys Leu Gln
65                  70                  75                  80

Met Glu Phe Lys Ala Val Gln Gln Val Gln Arg Leu Pro Phe Leu Ser
                85                  90                  95

Ser Ser Asn Leu Ser Leu Asp Val Leu Arg Gly Asn Asp Glu Thr Ile
            100                 105                 110

Gly Phe Glu Asp Ile Leu Asn Asp Pro Ser Gln Ser Glu Val Met Gly
        115                 120                 125

Glu Pro His Leu Met Val Glu Tyr Lys Leu Gly Leu Leu
    130                 135                 140

<210> 33
<211> 1591
<212> DNA
<213> Homo sapiens

<400> 33
gtccccgcgc cagagacgca gccgcgctcc caccacccac acccaccgcg ccctcgttcg 60

cctcttctcc gggagccagt ccgcgccacc gccgccgccc aggccatcgc caccctccgc 120

agccatgtcc accaggtccg tgtcctcgtc ctcctaccgc aggatgttcg gcggcccggg 180

caccgcgagc cggccgagct ccagccggag ctacgtgact acgtccaccc gcacctacag 240

cctgggcagc gcgctgcgcc ccagcaccag ccgcagcctc tacgcctcgt ccccgggcgg 300

cgtgtatgcc acgcgctcct ctgccgtgcg cctgcggagc agcgtgcccg gggtgcggct 360

cctgcaggac tcggtggact tctcgctggc cgacgccatc aacaccgagt tcaagaacac 420

ccgcaccaac gagaaggtgg agctgcagga gctgaatgac cgcttcgcca actacatcga 480

caaggtgcgc ttcctggagc agcagaataa gatcctgctg gccgagctcg agcagctcaa 540

gggccaaggc aagtcgcgcc tgggggacct ctacgaggag gagatgcggg agctgcgccg 600

gcaggtggac cagctaacca acgacaaagc ccgcgtcgag gtggagcgcg acaacctggc 660

cgaggacatc atgcgcctcc gggagaaatt gcaggaggag atgcttcaga gagaggaagc 720

cgaaaacacc ctgcaatctt tcagacagga tgttgacaat gcgtctctgg cacgtcttga 780

ccttgaacgc aaagtggaat ctttgcaaga agagattgcc tttttgaaga aactccacga 840

agaggaaatc caggagctgc aggctcagat tcaggaacag catgtccaaa tcgatgtgga 900

tgtttccaag cctgacctca cggctgccct gcgtgacgta cgtcagcaat atgaaagtgt 960

ggctgccaag aacctgcagg aggcagaaga atggtacaaa tccaagtttg ctgacctctc 1020

tgaggctgcc aaccggaaca atgacgccct gcgccaggca aagcaggagt ccactgagta 1080

ccggagacag gtgcagtccc tcacctgtga agtggatgcc cttaaaggaa ccaatgagtc 1140

cctggaacgc cagatgcgtg aaatggaaga gaactttgcc gttgaagctg ctaactacca 1200

agacactatt ggccgcctgc aggatgagat tcagaatatg aaggaggaaa tggctcgtca 1260

ccttcgtgaa taccaagacc tgctcaatgt taagatggcc cttgacattg agattgccac 1320

ctacaggaag ctgctggaag gcgaggagag caggatttct ctgcctcttc caaacttttc 1380

ctccctgaac ctgagggaaa ctaatctgga ttcactccct ctggttgata cccactcaaa 1440

aaggacactt ctgattaaga cggttgaaac tagagatgga caggttatca acgaaacttc 1500

tcagcatcac gatgaccttg aataaaaatt gcacacactc agtgcagcaa tatattacca 1560

gcaaaaaaaa aaaaaaaaaa aaaaaaaaaa a 1591

<210> 34
<211> 466
<212> PRT
<213> Homo sapiens

<400> 34

Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
            20                  25                  30

Thr Ser Thr Arg Thr Tyr Ser Leu Gly Ser Ala Leu Arg Pro Ser Thr
        35                  40                  45

Ser Arg Ser Leu Tyr Ala Ser Ser Pro Gly Gly Val Tyr Ala Thr Arg
    50                  55                  60

Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
        115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
            180                 185                 190

Met Leu Gln Arg Glu Glu Ala Glu Asn Thr Leu Gln Ser Phe Arg Gln
        195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile
                245                 250                 255

Asp Val Asp Val Ser Lys Pro Asp Leu Thr Ala Ala Leu Arg Asp Val
            260                 265                 270

Arg Gln Gln Tyr Glu Ser Val Ala Ala Lys Asn Leu Gln Glu Ala Glu
        275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
                325                 330                 335

Asn Glu Ser Leu Glu Arg Gln Met Arg Glu Met Glu Glu Asn Phe Ala
            340                 345                 350

Val Glu Ala Ala Asn Tyr Gln Asp Thr Ile Gly Arg Leu Gln Asp Glu
        355                 360                 365

Ile Gln Asn Met Lys Glu Glu Met Ala Arg His Leu Arg Glu Tyr Gln
    370                 375                 380

Asp Leu Leu Asn Val Lys Met Ala Leu Asp Ile Glu Ile Ala Thr Tyr
385                 390                 395                 400

Arg Lys Leu Leu Glu Gly Glu Glu Ser Arg Ile Ser Leu Pro Leu Pro
                405                 410                 415

Asn Phe Ser Ser Leu Asn Leu Arg Glu Thr Asn Leu Asp Ser Leu Pro
            420                 425                 430

Leu Val Asp Thr His Ser Lys Arg Thr Leu Leu Ile Lys Thr Val Glu
        435                 440                 445

Thr Arg Asp Gly Gln Val Ile Asn Glu Thr Ser Gln His His Asp Asp
    450                 455                 460

Leu Glu
465

<210>  35
<211>  475
<212>  DNA
<213>  Homo sapiens

<220>
<221>  misc_feature
<222>  (434)..(434)
<223>  n is a, c, g, or t

<220>
<221>  misc_feature
<222>  (442)..(442)
<223>  n is a, c, g, or t

<220>
<221>  misc_feature
<222>  (451)..(451)
<223>  n is a, c, g, or t

<220>
<221>  misc_feature
<222>  (453)..(453)

<223>  n is a, c, g, or t

<220>
<221>  misc_feature
<222>  (462)..(462)
<223>  n is a, c, g, or t

<220>
<221>  misc_feature
<222>  (469)..(469)
<223>  n is a, c, g, or t

<220>
<221>  misc_feature
<222>  (473)..(473)
<223>  n is a, c, g, or t

<400>  35
gtacgggagt ttcttggtaa atccagaatc aggatacaat gtctctttgc tatatgacct      60

tgaaaatctt ccggcatcca aggattccat tgtgcatcaa gctggcatgt tgaagcgaaa     120

ttgttttgcc tctgtctttg aaaaatactt ccaattccaa gaagagggca aggaaggaga     180

gaacagggca gttatccatt atagggatga tgagaccatg tatgttgagt ctaaaaagga     240

cagagtcaca gtagtcttca gcacagtgtt taaggatgac gacgatgtgg tcattggaaa     300

ggtgttcatg caggagttca aagaaggacg cagagccagc cacacagccc cacaggtcct     360

ctttagccac agggaacctc ctctggagct gaaagacaca gacgccgctg tgggtgacaa     420

cattggctac attnaccttt gngctgttcc ntngtacacc antgccagng ctnga          475

<210>  36
<211>  531
<212>  DNA
<213>  Homo sapiens


<220>
<221>  misc_feature
<222>  (438)..(438)
<223>  n is a, c, g, or t

<400>  36
gtacaaaatg gaaccaaaac ggagaatccc ttaagaacct gaagaggcgc aacattaaaa      60

gctacgatta tccagtagca agtgttccag ccttcagttg ccagccgctt cctcctctta     120

ttcccaagat tagcgggatg aaaacgtctt ccccgtgatt gttttcattt cttttttctc    180

ggcatctggg cgtgcgcggt tcagcacctt gaggaagtca gacgttttcg cccgcatacg     240

tgtgtgaata taggccttag agcacttgat gtggtagtgc aggtagtccc ggaacgtgtg     300

gatcaggttg atggtgttgt ctcgagcact ggcattggtg tgacgaggga acagcacaaa     360

ggtaatgtag ccaatgttgt cacccacagc ggcgtctgtg tctttcagct ccagaggagg     420

ttcccagtgg ctaaaganga cctgtggggc tgtgtggctg gctctgcgtc cttctttgaa     480

ctcctgcatg aacacctttc catgaccaca tcgtcgtcat ccttaacact g              531

<210>  37
<211>  345
<212>  DNA

<213>  Homo sapiens


<220>
<221>  misc_feature
<222>  (6)..(6)
<223>  n is a, c, g, or t

<220>
<221>  misc_feature
<222>  (11)..(11)
<223>  n is a, c, g, or t

<220>
<221>  misc_feature
<222>  (30)..(30)
<223>  n is a, c, g, or t

<220>
<221>  misc_feature
<222>  (51)..(51)
<223>  n is a, c, g, or t

<220>
<221>  misc_feature
<222>  (64)..(64)
<223>  n is a, c, g, or t

<220>
<221>  misc_feature
<222>  (98)..(98)
<223>  n is a, c, g, or t

<220>
<221>  misc_feature
<222>  (116)..(116)
<223>  n is a, c, g, or t

<220>
<221>  misc_feature
<222>  (118)..(118)
<223>  n is a, c, g, or t

<220>
<221>  misc_feature
<222>  (141)..(141)
<223>  n is a, c, g, or t

<220>
<221>  misc_feature
<222>  (162)..(162)
<223>  n is a, c, g, or t

<220>
<221>  misc_feature
<222>  (188)..(188)
<223>  n is a, c, g, or t

<220>
<221>  misc_feature
<222>  (199)..(199)
<223>  n is a, c, g, or t

<220>
<221>  misc_feature
<222>  (216)..(216)
<223>  n is a, c, g, or t

<220>

<221>  misc_feature
<222>  (229)..(229)
<223>  n is a, c, g, or t

<220>
<221>  misc_feature
<222>  (244)..(244)
<223>  n is a, c, g, or t

<220>
<221>  misc_feature
<222>  (258)..(258)
<223>  n is a, c, g, or t

<220>
<221>  misc_feature
<222>  (267)..(267)
<223>  n is a, c, g, or t

<220>
<221>  misc_feature
<222>  (320)..(321)
<223>  n is a, c, g, or t

<220>
<221>  misc_feature
<222>  (326)..(326)
<223>  n is a, c, g, or t

<220>
<221>  misc_feature
<222>  (331)..(331)
<223>  n is a, c, g, or t

<400>  37
agggtnggcc nacagagtat aatcaagggn tttttttcaa cattaatcaa naaattcctc      60

catntgcaaa agtcagccta tgccattaaa tacagccnag atcataaaat acaaangnca     120

ttagggctac tgaatcttga nattcattca ctaaaatacc cngggaaaag gaaaaagttt     180

gcccttgnga aaggggggna tcatttttta aaaaancttt tgaaagccnc aaacatctgg     240

gagngggcta aaagccgnta caaaggnatt ttatacaatc tatgctgagt cagctaggag     300

ttaacaggag gctggatgcn natggnaaaa natacccccg cgtac                     345


<210>  38
<211>  447
<212>  DNA
<213>  Homo sapiens


<220>
<221>  misc_feature
<222>  (434)..(434)
<223>  n is a, c, g, or t

<400>  38
gtacgcgggg agcaagagtg ccatggagag gctgaagcgc ggcatcattc acgctagagg      60

actggttcgg gagtgcttgg cagaaacgga acggaatgcc agatcctagc tgccttgttg     120

gttttgaagg atttccatct ttttacaaga tgagaagtta cagttcatct cccctgttca     180

gatgaaaccc ttgttttcaa aatggttaca gtttcgtttt tcctcccatg gttcacttgg     240

ctctgaacct acagtctcaa agattgagaa aagattttgc agttaattag gatttgcatt     300

ttaagtagtt aggaactgcc caggtttttt ttgtttttta agcattgatt taaaagatgc 360

acggaaagtt atcttacagc aaactgtagt ttgcctccaa gacaccattg tctcccttta 420

atcttctctt ttgnatacat ttggtac 447

<210> 39
<211> 289
<212> DNA
<213> Homo sapiens

<400> 39
agagtctttt gcttcctccc acccctaggg ggaaaaactg ctttgtgctt tgggaagttg 60

tctctgaaac ccggggacag aggacgcagg acagactagg agggagccgg gaggatgggc 120

tgcagctgtg gaggagggtt tcagaggaga gaggtcggag agcagaggcc tgagaagcca 180

gaggcaggtg gagagagggt ggaaagtgag cagcgggctg ggctggagcc gcacacgctc 240

tcctcccatg ttaaatagca cctttagaaa aattcacaag tccccatcc 289

<210> 40
<211> 393
<212> DNA
<213> Homo sapiens

<400> 40
acagctatga cctgattacg ccaagcttgg taccgagctc ggatccacta gtaacggccg 60

ccagtgtgct ggaattcgcc cttagcggcc gcccgggcag gtacagagtc ttttgcttcc 120

tcccacccct agggggaaaa actgctttgt gctttgggaa gttgtctctg aaacccgggg 180

acagaggacg caggacagac taggagggag ccgggaggat gggctgcagc tgtggaggag 240

ggtttcagag gagagaggtc ggagagcaga ggcctgagaa gccagaggca ggtggagaga 300

gggtggaaag tgagcagcgg gctgggctgg agccgcacac gctctcctcc catgttaaat 360

agcaccttta gaaaaattca caagtcccca tcc 393

<210> 41
<211> 221
<212> DNA
<213> Homo sapiens

<220>
<221> misc_feature
<222> (4)..(4)
<223> n is a, c, g, or t

<220>
<221> misc_feature
<222> (28)..(28)
<223> n is a, c, g, or t

<220>
<221> misc_feature
<222> (197)..(197)
<223> n is a, c, g, or t

<220>
<221> misc_feature

<222>  (210)..(210)
<223>  n is a, c, g, or t

<220>
<221>  misc_feature
<222>  (213)..(213)
<223>  n is a, c, g, or t

<400>  41
agantctttt gcttcctccc acccctangg ggaaaaactg ctttgtgctt tgggaagttg      60

tctctgaaac ccggggacag aggacgcagg acagactagg agggagccgg gaggatgggc     120

tgcagctgtg gaggagggtt tcagaggaga gaggtcggag agcagaggcc tgagaagcca     180

gaggcaggtg gagaganggt ggaaagtgan cancgggctg g                         221


<210>  42
<211>  271
<212>  DNA
<213>  Homo sapiens


<220>
<221>  misc_feature
<222>  (4)..(4)
<223>  n is a, c, g, or t

<220>
<221>  misc_feature
<222>  (28)..(28)
<223>  n is a, c, g, or t

<220>
<221>  misc_feature
<222>  (82)..(82)
<223>  n is a, c, g, or t

<220>
<221>  misc_feature
<222>  (102)..(102)
<223>  n is a, c, g, or t

<220>
<221>  misc_feature
<222>  (113)..(113)
<223>  n is a, c, g, or t

<220>
<221>  misc_feature
<222>  (136)..(136)
<223>  n is a, c, g, or t

<220>
<221>  misc_feature
<222>  (146)..(146)
<223>  n is a, c, g, or t

<220>
<221>  misc_feature
<222>  (151)..(151)
<223>  n is a, c, g, or t

<220>
<221>  misc_feature
<222>  (153)..(153)
<223>  n is a, c, g, or t

<220>
<221> misc_feature
<222> (162)..(162)
<223> n is a, c, g, or t

<220>
<221> misc_feature
<222> (183)..(183)
<223> n is a, c, g, or t

<220>
<221> misc_feature
<222> (197)..(197)
<223> n is a, c, g, or t

<220>
<221> misc_feature
<222> (245)..(245)
<223> n is a, c, g, or t

<220>
<221> misc_feature
<222> (267)..(267)
<223> n is a, c, g, or t

<400> 42
agantctttt gcttcctccc acccctangg ggaaaaactg ctttgtgctt tgggaagttg 60

tctctgaaac ccggggacag angacgcagg acagactagg anggagccgg gangatgggc 120

tgcagctgtg gagganggtt tcagangaga nangtcggag ancagaggcc tgagaagcca 180

gangcaggtg gagaganggt ggaaagtgag cagcgggctg ggctggagcc gcacacgctc 240

tcctnccatg ttaaatagca cctttanaaa a 271


<210> 43
<211> 255
<212> DNA
<213> Homo sapiens

<400> 43
gcgggagggc agattcggac cactaggcct gaaatgacat ttcactaaaa gtctccaaaa 60

catttctaag actactaagg ccttttatgt aatttcttta aatgtgtatt tcttaagaat 120

tcaaatttgt aataaaacta tttgtataaa aattaagctt ttattaattt gttgctagta 180

ttgccacaga cgcattaaaa gaaacttact gcacaagctg ctaataaatt tgtaagcttt 240

gcatacctta gatta 255


<210> 44
<211> 147
<212> DNA
<213> Homo sapiens

<400> 44
gcgggagggc agattcggac cactaggcct gaaatgacat ttcactaaaa gtctccaaaa 60

catttctaag actactaagg ccttttatgt aatttcttta aatgtgtatt tcttaagaat 120

tcaaatttgt aataaaacta tttgtat 147


<210> 45
<211> 144

<212>  DNA
<213>  Homo sapiens

<400>  45
ggagggcaga ttcggaccac taggcctgaa atgacatttc actaaaagtc tccaaaacat      60

ttctaagact actaaggcct tttatgtaat ttctttaaat gtgtatttct taagaattca     120

aatttgtaat aaaactattt gtat                                            144


<210>  46
<211>  246
<212>  DNA
<213>  Homo sapiens

<400>  46
aaggtatgca aagcttacaa atttattagc agcttgtgca gtaagtttct tttaatgcgt      60

ctgtggcaat actagcaaca aattaataaa agcttaattt ttatacaaat agttttatta     120

caaatttgaa ttcttaagaa atacacattt aaagaaatta cataaaaggc cttagtagtc     180

ttagaaatgt tttggagact tttagtgaaa tgtcatttca ggcctagtgg tccgaatctg     240

ccctcc                                                                246


<210>  47
<211>  484
<212>  DNA
<213>  Homo sapiens


<220>
<221>  misc_feature
<222>  (22)..(22)
<223>  n is a, c, g, or t

<220>
<221>  misc_feature
<222>  (30)..(30)
<223>  n is a, c, g, or t

<220>
<221>  misc_feature
<222>  (57)..(57)
<223>  n is a, c, g, or t

<220>
<221>  misc_feature
<222>  (138)..(138)
<223>  n is a, c, g, or t

<220>
<221>  misc_feature
<222>  (168)..(168)
<223>  n is a, c, g, or t

<220>
<221>  misc_feature
<222>  (183)..(183)
<223>  n is a, c, g, or t

<220>
<221>  misc_feature
<222>  (198)..(198)
<223>  n is a, c, g, or t

<220>
<221>  misc_feature
<222>  (206)..(206)
<223>  n is a, c, g, or t

<220>
<221>  misc_feature
<222>  (224)..(224)
<223>  n is a, c, g, or t

<220>
<221>  misc_feature
<222>  (245)..(245)
<223>  n is a, c, g, or t

<220>
<221>  misc_feature
<222>  (267)..(267)
<223>  n is a, c, g, or t

<220>
<221>  misc_feature
<222>  (287)..(287)
<223>  n is a, c, g, or t

<220>
<221>  misc_feature
<222>  (311)..(311)
<223>  n is a, c, g, or t

<220>
<221>  misc_feature
<222>  (321)..(321)
<223>  n is a, c, g, or t

<220>
<221>  misc_feature
<222>  (441)..(441)
<223>  n is a, c, g, or t

<220>
<221>  misc_feature
<222>  (450)..(450)
<223>  n is a, c, g, or t

<220>
<221>  misc_feature
<222>  (476)..(476)
<223>  n is a, c, g, or t

<400>  47
gcaaagctta caaatttatt ancagcttgn gcagtaagtt tcttttaatg cgtctgnggc      60

aatactagca acaaattaat aaaagcttaa tttttataca aatagtttta ttacaaattt     120

gaattcttaa gaaatacnca tttaaagaaa ttacataaaa ggccttanta gtcttaaaaa     180

tgntttggag acttttantg aaatgncatt tcaggcctag tggnccgaat ctgccctcct     240

gcggnccatg cgatgccctg ctgaggnctg tgaacacagc tcatganaaa ccacggaaat     300

ggcccgaatg ngcttacgtg ngaaaatact gatactggga ttcaacagag ctgtttttca     360

agccaggatg cagaatgagg aatactaatg aaatgacggc ctttaagggt gttgcttttg     420

aagtcaagtc attcagtttg ngattagtgn ttaaaaccct gaaaatattt aatacngaat     480

aaaa                                                                  484

<210>  48
<211>  246
<212>  DNA
<213>  Homo sapiens

<400>  48
ggagggcaga ttcggaccac taggcctgaa atgacatttc actaaaagtc tccaaaacat      60

ttctaagact actaaggcct tttatgtaat ttctttaaat gtgtatttct taagaattca     120

aatttgtaat aaaactattt gtataaaaat taagctttta ttaatttgtt gctagtattg     180

ccacagacgc attaaaagaa acttactgca caagctgcta ataaatttgt aagctttgca     240

tacctt                                                                246


<210>  49
<211>  239
<212>  DNA
<213>  Homo sapiens


<220>
<221>  misc_feature
<222>  (57)..(57)
<223>  n is a, c, g, or t

<220>
<221>  misc_feature
<222>  (60)..(60)
<223>  n is a, c, g, or t

<220>
<221>  misc_feature
<222>  (100)..(100)
<223>  n is a, c, g, or t

<220>
<221>  misc_feature
<222>  (131)..(131)
<223>  n is a, c, g, or t

<220>
<221>  misc_feature
<222>  (138)..(138)
<223>  n is a, c, g, or t

<220>
<221>  misc_feature
<222>  (155)..(155)
<223>  n is a, c, g, or t

<220>
<221>  misc_feature
<222>  (163)..(163)
<223>  n is a, c, g, or t

<220>
<221>  misc_feature
<222>  (168)..(168)
<223>  n is a, c, g, or t

<220>
<221>  misc_feature
<222>  (183)..(183)
<223>  n is a, c, g, or t

<220>
<221>  misc_feature
<222>  (190)..(190)
<223>  n is a, c, g, or t

<220>
<221>  misc_feature
<222>  (198)..(198)
<223>  n is a, c, g, or t

<220>
<221>  misc_feature
<222>  (206)..(206)
<223>  n is a, c, g, or t

<220>
<221>  misc_feature
<222>  (220)..(221)
<223>  n is a, c, g, or t

<220>
<221>  misc_feature
<222>  (224)..(224)
<223>  n is a, c, g, or t

<400>  49
gcaaagctta caaatttatt aacagcttgg gcagtaagtt tcttttaatg cgtctgnggn      60

aatactagca acaaattaat aaaagcttaa tttttatacn catagtttta ttacaaattt     120

gaattcttaa naaatacnca tttaaagaaa ttacntaaaa ggncttanta gtcttaaaaa     180

tgntttggan acttttantg aaatgncatt tcaggcctan nggnccgaat ctgccctcc      239


<210>  50
<211>  246
<212>  DNA
<213>  Homo sapiens

<400>  50
ggagggcaga ttcggaccac taggcctgaa atgacatttc actaaaagtc tccaaaacat      60

ttctaagact actaaggcct tttatgtaat ttctttaaat gtgtatttct taagaattca     120

aatttgtaat aaaactattt gtataaaaat taagctttta ttaatttgtt gctagtattg     180

ccacagacgc attaaaagaa acttactgca caagctgcta ataaatttgt aagctttgca     240

tacctt                                                                246


<210>  51
<211>  242
<212>  DNA
<213>  Homo sapiens

<400>  51
gcgggagggc agattcggac cactaggcct gaaatgacat ttcactaaaa gtctccaaaa      60

catttctaag actactaagg ccttttatgt aatttcttta aatgtgtatt tcttaagaat     120

tcaaatttgt aataaaacta tttgtataaa aattaagctt ttattaattt gttgctagta     180

ttgccacaga cgcattaaaa gaaacttact gcacaagctg ctaataaatt tgtaagcttt     240

gc                                                                    242

<210>  52
<211>  254
<212>  DNA
<213>  Homo sapiens

<400>  52
gtacgcggga gggcagattc ggaccactag gcctgaaatg acatttcact aaaagtctcc      60

aaaacatttc taagactact aaggcctttt atgtaatttc tttaaatgtg tatttcttaa     120

gaattcaaat ttgtaataaa actatttgta taaaaattaa gcttttatta atttgttgct     180

agtattgcca cagacgcatt aaaagaaact tactgcacaa gctgctaata aatttgtaag     240

ctttgcatac ctta                                                       254


<210>  53
<211>  245
<212>  DNA
<213>  Homo sapiens

<400>  53
gcgggagggc agattcggac cactaggcct gaaatgacat ttcactaaaa gtctccaaaa      60

catttctaag actactaagg ccttttatgt aatttcttta aatgtgtatt tcttaagaat     120

tcaaatttgt aataaaacta tttgtataaa aattaagctt ttattaattt gttgctagta     180

ttgccacaga cgcattaaaa gaaacttact gcacaagctg ctaataaatt tgtaagcttt     240

gcata                                                                 245


<210>  54
<211>  249
<212>  DNA
<213>  Homo sapiens

<400>  54
gcgggagggc agattcggac cactaggcct gaaatgacat ttcactaaaa gtctccaaaa      60

catttctaag actactaagg ccttttatgt aatctcttta aatgtgtatt tcttaagaat     120

tcaaatttgt aataaaacta tttgtataaa aattaagctt ttattaattt gttgctagta     180

ttgccacaga cgcattaaaa gaaacttact gcacaagctg ctaataaatt tgtaagcttt     240

gcatacctt                                                             249


<210>  55
<211>  246
<212>  DNA
<213>  Homo sapiens

<400>  55
ggagggcaga ttcggaccac taggcctgaa atgacatttc actaaaagtc tccaaaacat      60

ttctaagact actaaggcct tttatgtaat ttctttaaat gtgtatttct taagaattca     120

aatttgtaat aaaactattt gtataaaaat taagctttta ttaatttgtt gctagtattg     180

ccacagacgc attaaaagaa acttactgca caagctgcta ataaatttgt aagctttgca     240

tacctt                                                                246


<210>  56

<211>  239
<212>  DNA
<213>  Homo sapiens

<400>  56
ggagggcaga ttcggaccac taggcctgaa atgacatttc actaaaagtc tccaaaacat      60

ttctaagact actaaggcct tttatgtaat ttctttaaat gtgtatttct taagaattca     120

aatttgtaat aaaactattt gtataaaaat taagctttta ttaatttgtt gctagtattg     180

ccacagacgc attaaaagaa acttactgca caagctgcta ataaatttgt aagctttgc      239


<210>  57
<211>  476
<212>  DNA
<213>  Homo sapiens

<400>  57
ggcactgggc gactctgtgg ctcgctgagg aaaaaaagaa ctaaacatgg gcaaaggaga      60

tctagggaag ctgagaggta aaatgtgata tgcattcttt gtccacactt gttgggagga     120

gcacaagaag aagcacccag atgctcagtc agctcctcag agttttctaa gaaatgctca     180

gagaggtgca agaccatgtc tgctaaagag aaagtgagat ttgaagacat gtcaaagatg     240

gacaagaccc attatgaaag agaaatgaaa acctatatcc ttcctaaatg ggagacaaaa     300

aagaagttcg aggatcccaa tgcacccaag aggcctcctt cggccttctt cctcttctgc     360

tctgagtatc gcccaaaaat caaaggagaa catcctggcc tgtccattgg tgatgttgcg     420

aaaaactggg agagatgtgg aataacactg ctgcagacga cagcagcctt atgaaa         476


<210>  58
<211>  267
<212>  DNA
<213>  Homo sapiens

<400>  58
gtacaaatga ttttatatct taactggtaa tatattaaat atgttataaa attagttcaa      60

gaagcataaa acaccctaac tctcatgaac acagaaattc aagcaaaaga atatgcttaa     120

acaaaaacta aagcacacag caaacgtaaa ttgttggtca aatcttttca tagtgttata     180

agttacattt gtgaaattta gggtagttga agagcttcct caataacagc aagaggtttt     240

acatttatta cctttataga gtaggta                                         267


<210>  59
<211>  477
<212>  DNA
<213>  Homo sapiens


<220>
<221>  misc_feature
<222>  (459)..(459)
<223>  n is a, c, g, or t

<220>
<221>  misc_feature
<222>  (462)..(462)
<223>  n is a, c, g, or t

<400>  59
tcgttggtaa atacctactc ccccaagtga ctccaggtgc cccccaccgc ctggcacttc      60

ccccaggact cctacgatct ggttactgcc tggccgatcc aaggctgtgg agtcccagag     120

ccagcagttc actggtgctc attccacact ggttagatac ttcagttgtc acccctggga     180

agattctccc acctcctccc tttgatggaa ccaccctccc cagaggctgc attgaggaga     240

ctccacagac tgaaaagtga gtttgcagaa accttgggga aaagggccct ttcaaagaag     300

tggataagag ggaggagatc attgagtgac ccagaaagct cttttgaaaa gacagactcc     360

tcaaggagag ataaagagga aagcacctct ttcatttttt agtgtgagct aattccatca     420

gactgctgtc ctcctggacc catctgagat gtgcagtanc anggagaggg gggatca        477


<210>  60
<211>  405
<212>  DNA
<213>  Homo sapiens


<220>
<221>  misc_feature
<222>  (391)..(391)
<223>  n is a, c, g, or t

<400>  60
   ccaagattac aatcctttct ggtgttataa gggggccatt ggaaaagtct atcatgcttt      60
   aaatcccaag cttacagtga ttgttccaga tgatgaccgt tcattaataa atttgcatct     120
   catgcacacc agttacttcc tctttgtgat ggtgataaca atgttttgct atgctgttat     180
   caagggcaga cctagcaaat tgcgtcagag caatcctgaa ttttgtcccg agaaggtggc     240
   tttggctgaa gcctaattcc acagctcctt gttttttgag agagactgag agaaccataa     300
   tccttgcctg ctgaacccag cctgggcctg gatgctctgt gaatacatta tcttgcgatg     360
   ttgggttatt ccagccaaag acatttcagg ngcctgtaac tgatt                     405


<210>  61
<211>  300
<212>  DNA
<213>  Homo sapiens


<220>
<221>  misc_feature
<222>  (5)..(5)
<223>  n is a, c, g, or t

<220>
<221>  misc_feature
<222>  (25)..(25)
<223>  n is a, c, g, or t

<220>
<221>  misc_feature
<222>  (72)..(72)
<223>  n is a, c, g, or t

<220>
<221>  misc_feature

<222>  (99)..(99)
<223>  n is a, c, g, or t

<220>
<221>  misc_feature
<222>  (104)..(104)
<223>  n is a, c, g, or t

<220>
<221>  misc_feature
<222>  (113)..(113)
<223>  n is a, c, g, or t

<220>
<221>  misc_feature
<222>  (120)..(120)
<223>  n is a, c, g, or t

<220>
<221>  misc_feature
<222>  (127)..(127)
<223>  n is a, c, g, or t

<220>
<221>  misc_feature
<222>  (160)..(160)
<223>  n is a, c, g, or t

<220>
<221>  misc_feature
<222>  (162)..(162)
<223>  n is a, c, g, or t

<220>
<221>  misc_feature
<222>  (173)..(173)
<223>  n is a, c, g, or t

<220>
<221>  misc_feature
<222>  (177)..(177)
<223>  n is a, c, g, or t

<220>
<221>  misc_feature
<222>  (197)..(197)
<223>  n is a, c, g, or t

<220>
<221>  misc_feature
<222>  (255)..(255)
<223>  n is a, c, g, or t

<220>
<221>  misc_feature
<222>  (258)..(258)
<223>  n is a, c, g, or t

<220>
<221>  misc_feature
<222>  (262)..(262)
<223>  n is a, c, g, or t

<220>
<221>  misc_feature
<222>  (283)..(283)
<223>  n is a, c, g, or t

<220>

<221>  misc_feature
<222>  (297)..(297)
<223>  n is a, c, g, or t

<400>  61
cctancttgg tttggtcttg aaaanattca taatcactcc aaatgaaatg ctcctccctt      60

ggccaccaat gngaagggag ggtaaaaacc tgaggctana cttntgacac aanaaaaatn     120

tgtcganagc acagtctccc agtcaataaa aaggaaggan anagggggat ganctcncac     180

ccttgaaaaa aaccttnatg agccaattcc caaagcatca actccgcatg gatactttgc     240

acacacatca gccgngtnta anggacacac acacgtgcat acncacgtga gcacacnccg     300


<210>  62
<211>  469
<212>  DNA
<213>  Homo sapiens


<220>
<221>  misc_feature
<222>  (21)..(21)
<223>  n is a, c, g, or t

<220>
<221>  misc_feature
<222>  (84)..(84)
<223>  n is a, c, g, or t

<220>
<221>  misc_feature
<222>  (86)..(86)
<223>  n is a, c, g, or t

<220>
<221>  misc_feature
<222>  (112)..(112)
<223>  n is a, c, g, or t

<220>
<221>  misc_feature
<222>  (121)..(121)
<223>  n is a, c, g, or t

<220>
<221>  misc_feature
<222>  (136)..(136)
<223>  n is a, c, g, or t

<220>
<221>  misc_feature
<222>  (138)..(138)
<223>  n is a, c, g, or t

<220>
<221>  misc_feature
<222>  (180)..(180)
<223>  n is a, c, g, or t

<220>
<221>  misc_feature
<222>  (185)..(185)
<223>  n is a, c, g, or t

<220>
<221>  misc_feature

<222>  (235)..(235)
<223>  n is a, c, g, or t

<220>
<221>  misc_feature
<222>  (269)..(269)
<223>  n is a, c, g, or t

<220>
<221>  misc_feature
<222>  (278)..(278)
<223>  n is a, c, g, or t

<220>
<221>  misc_feature
<222>  (295)..(295)
<223>  n is a, c, g, or t

<220>
<221>  misc_feature
<222>  (320)..(320)
<223>  n is a, c, g, or t

<220>
<221>  misc_feature
<222>  (451)..(451)
<223>  n is a, c, g, or t

<220>
<221>  misc_feature
<222>  (456)..(456)
<223>  n is a, c, g, or t

<220>
<221>  misc_feature
<222>  (469)..(469)
<223>  n is a, c, g, or t

<400>  62
gataaaaagt attttatttg nttaatgata tgcatgcttt tcttctgtaa atatataata      60

aatttttgta gatagtcttg atgngngatc tttattttgt atttctctgg gnaaaaccag     120

ngaatataac taaagngnta gtggattgga ttaaaagaaa cttattaggc aagaacaggn     180

aatgnagtta tccatgacta cttttaacca tgcagactaa taatattctg gaggnttata     240

gctcggcacc ttcacctttt ttcactggna tttcatgnaa ggcatcaacc actgnaattt     300

ttgctgatgc tgaagcctgn ccttgggaat tggatgcatg gcactcatat tctccagcat     360

cttccttact tagaggagat accagcaccc agccagttac ttcatgcttt tctgggccac     420

cccgggtctg aatgggccag gttgtcccgg ncccangcag gagttctgn                 469


<210>  63
<211>  463
<212>  DNA
<213>  Homo sapiens


<220>
<221>  misc_feature
<222>  (412)..(412)
<223>  n is a, c, g, or t

<220>
<221>  misc_feature

<222>  (457)..(457)
<223>  n is a, c, g, or t

<400>  63
atggatgaaa actaaaggct cgagttaatc acattgtagt ttttaaattt ctacagccta      60

gagctcacta gtcacaggtc ttttaggtcc ttctggatgt cccacagggt atctgcactt     120

ttcttgagct gagcaacctc atcatccttt agcttctggt tgataacgct ggttaatccc     180

cgggcattga ggatacatgg aaggctcagg aagacttcat tctcaatgcc atacatcccc     240

tttaccattg ttgacacggg atgaatcctg gatagatttt tcaacatgga ttcaataaga     300

tcagccacac ttaatccaat agcccagttg gtatatcctt ttagcttgat gacttcatag     360

gcactttcaa ccaccatctt atgcacttcc ttccaatttt cactatcatt gncagttccc     420

atttctggac tcaattcctg gagagaaaca cctgccncat tcc                       463


<210>  64
<211>  442
<212>  DNA
<213>  Homo sapiens

<400>  64
gttcatgcag cctgtgattc atagcttccc tggggtgttg gggagaatca catttgggtc      60

agccaggttt agcactgaca gttttgtctt tagaatcaag cagatgtgga atcaaatctg     120

gctgtatcca tgaccagctc tgaagccatg agtgggttac atagctttag ggcctcagca     180

tactcatctg gaaagtggaa gtgatcatgt ctattttgca gagttgttgc cacttttcct     240

ctctggaccc cactttcccc atctgtcaga tgaaagagtg ggatgagatg gcctgtctat     300

ttatctctca atcacaatgg ctctatttga aaaaagtttg aactgcccta agtgttaaaa     360

aggaagatgg ggagccatca agacaaactt aggcctacat taccatcgag ttcagagaat     420

ggcagaccgg aagcaccagc ca                                              442


<210>  65
<211>  461
<212>  DNA
<213>  Homo sapiens

<400>  65
gcgggaaaga gaacgtgagc ttcgggatgt gagaattcgg aaaatgcctg agaacagtga      60

cttgctacca gttgcacaaa cagagccatc tatatggaca gttgatgatg tctgggcctt     120

catccattct ttgcctggct gccaggatat cgcagatgaa ttcagagcac aggagattga     180

tggacaggcc cttctcttgc tgaaagaaga ccatctcatg agtgcaatga atatcaagct     240

aggcccagcc ctgaagatct gtgcacgcat caactctctg aaggaatctt aacaggaaca     300

tgaagccttg ataaaacggc agttttactt ttctcacaaa aacttgtaag gtaaaggcct     360

aacttggtct agaatatgac acttattgtg gtggatagcc aagcacattg ggatctccac     420

atcaaatact gacatttctt ctacaggtat aataattcat c                         461


<210>  66
<211>  507

<212>  DNA
<213>  Homo sapiens


<220>
<221>  misc_feature
<222>  (375)..(375)
<223>  n is a, c, g, or t

<220>
<221>  misc_feature
<222>  (472)..(472)
<223>  n is a, c, g, or t

<220>
<221>  misc_feature
<222>  (502)..(502)
<223>  n is a, c, g, or t

<400>  66
cggattctct ctttaaccct ccccttcgtg tttcccccaa tgtttaaaat gtttggatgg      60

tttgttgttc tgcctggaga caaggtgcta acatagattt aagtgaatac attaacggtg     120

ctaaaaatga aaattctaac ccaagacatg acattcttag ctataactta actattaagg     180

ccttttccac acgcattaat agtcccattt ttctcttgcc atttgtagct ttgcccattg     240

tcctattggc acatgggtgg acacggatct gctgggctct gccttaaaca cacattgcag     300

cttcaacttt tctctttagt gttctgtttg aaactaatac ttaccgagtc agactttgtg     360

ttcatttcat ttcanggtct tggctgcctg tgggcttccc caggtggcct ggaggtgggc     420

aaagggaagt aacagacaca cgatgttgtc aaggatggtt ttgggactag angctcagtg     480

gtgggagaga tccctgcaga anccacc                                         507


<210>  67
<211>  543
<212>  DNA
<213>  Homo sapiens


<220>
<221>  misc_feature
<222>  (492)..(492)
<223>  n is a, c, g, or t

<220>
<221>  misc_feature
<222>  (495)..(495)
<223>  n is a, c, g, or t

<220>
<221>  misc_feature
<222>  (512)..(512)
<223>  n is a, c, g, or t

<220>
<221>  misc_feature
<222>  (515)..(515)
<223>  n is a, c, g, or t

<220>
<221>  misc_feature
<222>  (521)..(521)
<223>  n is a, c, g, or t

<220>
<221>  misc_feature
<222>  (523)..(523)
<223>  n is a, c, g, or t

<220>
<221>  misc_feature
<222>  (527)..(527)
<223>  n is a, c, g, or t

<220>
<221>  misc_feature
<222>  (530)..(530)
<223>  n is a, c, g, or t

<220>
<221>  misc_feature
<222>  (539)..(539)
<223>  n is a, c, g, or t

<400>  67
gtacatgtta tagttcttct cgaagtcccg ggccagcagc tccacggggt ggtctcctgc      60

ctccaggcgc ttctcattct catggatctt cttcacccgc agcttctgct tctcagtcag     120

aaggttgttg tcctcatccc tctcatacag ggtgaccagg acgttcttga gccagtcccg     180

catgcgcagg gggaattcgg tcagctcaga gtccaggcaa ggggggatgt atttgcaagg     240

cccgatgtag tccaggtgga gcttgtggcc cttcttggtg ccctccaggg tacactttgt     300

ggcaaagaag tggcaggaag agtcgaaggt cttgttgtca ttgctgcaca ccttctcaaa     360

ctcgccaatg ggggctgggc agctggtggg gtcctggcac acgcacatgg gggtgttgtt     420

ctcatccagc tcgcacacct tgccgtgttt gcagtggtgg ttctggcagg gattttccgc     480

caccacctcc tnttnggttt cctctgcaca tnatnaaatt ntnctanttn cacctggana     540

gga                                                                   543


<210>  68
<211>  394
<212>  DNA
<213>  Homo sapiens

<400>  68
gggcttctcc tcctctgtct tttggagaac cagggctctt ctcaggggct ctagggactg      60

tcaggctgtt tcagccagga aggccaaaat caagagtgag atgtagaaag ttgtaaaata     120

gaaaaagtgg agttggtgaa tcggttgttc tttcctcaca tttggatgat tgtcataagg     180

tttttagcat gttcctcctt ttcttcaccc tccccttttt tcttctatta atcaagagaa     240

acttcaaagt taatgggatg gtcggatctc acaggctgag gactcgttca cctccaagca     300

tttcatgaaa aagctgcttc ttattaatca tacaaactct caccatgatg tgaagagttt     360

cacaaatctt tcaaaataaa aagtaatgac ttag                                 394


<210>  69
<211>  506
<212>  DNA
<213>  Homo sapiens

<400>  69
ctgtcttatg aaaatataga cattctcaca taagcccagt tcatcaccat ttcctccttt      60

acctttcagt gcagtttctt ttcacattag gctgttggtt caaacttttg ggagcacgga     120

ctgtcagttc tctgggaagt ggtcagcgca tcctgcaggg cttctcctcc tctgtctttt     180

ggagaaccag ggctcttctc aggggctcta gggactgcca ggctgtttca gccaggaagg     240

ccaaaatcaa gagtgagatg tagaaagttg taaaatagaa aaagtggagt tggtgaatcg     300

gttgttcttt cctcacattt ggatgattgt cataaggttt ttagcatgtt cctccttttc     360

ttcaccctcc ccttttttct tctattaatc aagagaaact tcaaagttaa tgggatggtc     420

ggatctcaca ggctgagaac tcgttcacct ccaagcattt catgaaaaag ctgcttctta     480

ttaatcatac aactctcacc atgatg                                          506


<210>  70
<211>  414
<212>  DNA
<213>  Homo sapiens


<220>
<221>  misc_feature
<222>  (359)..(359)
<223>  n is a, c, g, or t

<220>
<221>  misc_feature
<222>  (373)..(373)
<223>  n is a, c, g, or t

<220>
<221>  misc_feature
<222>  (386)..(386)
<223>  n is a, c, g, or t

<400>  70
cggattctct ctttaaccct ccccttcgtg tttcccccaa tgtttaaaat gtttggatgg      60

tttgttgttc tgcctggaga caaggtgcta acatagattt aagtgaatac attaacggtg     120

ctaaaaatga aaattctaac ccaagacatg acattcttag ctgtaactta actattaagg     180

ccttttccac acgcattaat agtcccattt ttctcttgcc atttgtagct ttgcccattg     240

tcttattggc acatgggtgg acacggatct gctgggctct gccttaaaca cacattgcag     300

cttcaacttt tctctttagt gttctgtttg aaactaatac ttaccgagtc agactttgng     360

ttcatttcat ttnagggtct ggctgnctgg gggcttcccc agggggcctg gagg           414


<210>  71
<211>  485
<212>  DNA
<213>  Homo sapiens


<220>
<221>  misc_feature
<222>  (451)..(451)
<223>  n is a, c, g, or t

<400>  71

aaagatgact ataaacaaga tgcagccctc ggtttccatg aacagcacac tattacagta 60

aaccaagttt atattccacc atcaagtgtg gctctcccat gacttcgctt tgtgatggat 120

cattaagaat atcctcaaat ccaatagtct catcattacc cctcaaaaca tccagtgaaa 180

gatttgagct tgaaagaaat ggaagacgct gaacctgctg cactgccttg aattccatct 240

gtaattttag cggagcaaat agaccctgaa tgtttctcag tgtggaaaaa ttcattttat 300

cttggttgag ctggaaattt ttttctgata attcaagggg atgactaggc aaaagttcat 360

ttttcacaca agaaaaacct ttccgaagaa gatcatgact ttcaaaaggt ccacttgctg 420

aaagttcagt aactggaata ctgtccttta nctcagatcc aagtcctctg gcattcatct 480

cccgc 485

<210> 72
<211> 362
<212> DNA
<213> Homo sapiens

<220>
<221> misc_feature
<222> (5)..(5)
<223> n is a, c, g, or t

<220>
<221> misc_feature
<222> (8)..(8)
<223> n is a, c, g, or t

<220>
<221> misc_feature
<222> (21)..(21)
<223> n is a, c, g, or t

<220>
<221> misc_feature
<222> (26)..(26)
<223> n is a, c, g, or t

<220>
<221> misc_feature
<222> (28)..(28)
<223> n is a, c, g, or t

<220>
<221> misc_feature
<222> (32)..(32)
<223> n is a, c, g, or t

<220>
<221> misc_feature
<222> (35)..(35)
<223> n is a, c, g, or t

<220>
<221> misc_feature
<222> (37)..(39)
<223> n is a, c, g, or t

<220>
<221> misc_feature
<222> (50)..(50)
<223> n is a, c, g, or t

<220>
<221>  misc_feature
<222>  (71)..(71)
<223>  n is a, c, g, or t

<220>
<221>  misc_feature
<222>  (74)..(74)
<223>  n is a, c, g, or t

<220>
<221>  misc_feature
<222>  (85)..(85)
<223>  n is a, c, g, or t

<220>
<221>  misc_feature
<222>  (131)..(131)
<223>  n is a, c, g, or t

<220>
<221>  misc_feature
<222>  (159)..(159)
<223>  n is a, c, g, or t

<220>
<221>  misc_feature
<222>  (164)..(164)
<223>  n is a, c, g, or t

<220>
<221>  misc_feature
<222>  (231)..(231)
<223>  n is a, c, g, or t

<220>
<221>  misc_feature
<222>  (242)..(242)
<223>  n is a, c, g, or t

<220>
<221>  misc_feature
<222>  (246)..(246)
<223>  n is a, c, g, or t

<220>
<221>  misc_feature
<222>  (260)..(260)
<223>  n is a, c, g, or t

<220>
<221>  misc_feature
<222>  (280)..(280)
<223>  n is a, c, g, or t

<220>
<221>  misc_feature
<222>  (283)..(283)
<223>  n is a, c, g, or t

<220>
<221>  misc_feature
<222>  (306)..(306)
<223>  n is a, c, g, or t

<220>
<221>  misc_feature
<222>  (337)..(337)

<223>  n is a, c, g, or t

<220>
<221>  misc_feature
<222>  (352)..(352)
<223>  n is a, c, g, or t

<220>
<221>  misc_feature
<222>  (354)..(354)
<223>  n is a, c, g, or t

<400>  72
ttgcnggnaa tatattgctg nactgngngt gngcnannnt tattcaaggn catcgtgatg      60

ctgagaagtt nccntgataa cctgnccatc tctagtttca accgtcttaa tcaaaagtgt     120

cctttttgag ngggtatcaa ccaaagggag tgaatccana ttantttccc tcaggttcag     180

ggaggaaaag tttggaagag gcagaaaaat cctggtctcc tcgccttcca ncagcttcct     240

gnaggnggca atctcaatgn caagggccat ccttaacatn gancaggtct tggtattcac     300

gaaggngacg agccatttcc tccttcatat tctgaanctc atcctgcagg cngncaatag     360

tg                                                                    362


<210>  73
<211>  444
<212>  DNA
<213>  Homo sapiens


<220>
<221>  misc_feature
<222>  (13)..(13)
<223>  n is a, c, g, or t

<220>
<221>  misc_feature
<222>  (18)..(18)
<223>  n is a, c, g, or t

<220>
<221>  misc_feature
<222>  (20)..(20)
<223>  n is a, c, g, or t

<220>
<221>  misc_feature
<222>  (24)..(24)
<223>  n is a, c, g, or t

<220>
<221>  misc_feature
<222>  (27)..(27)
<223>  n is a, c, g, or t

<220>
<221>  misc_feature
<222>  (29)..(29)
<223>  n is a, c, g, or t

<220>
<221>  misc_feature
<222>  (42)..(42)
<223>  n is a, c, g, or t

<220>
<221>  misc_feature
<222>  (63)..(63)
<223>  n is a, c, g, or t

<220>
<221>  misc_feature
<222>  (66)..(66)
<223>  n is a, c, g, or t

<220>
<221>  misc_feature
<222>  (77)..(77)
<223>  n is a, c, g, or t

<220>
<221>  misc_feature
<222>  (123)..(123)
<223>  n is a, c, g, or t

<220>
<221>  misc_feature
<222>  (151)..(151)
<223>  n is a, c, g, or t

<220>
<221>  misc_feature
<222>  (156)..(156)
<223>  n is a, c, g, or t

<220>
<221>  misc_feature
<222>  (223)..(223)
<223>  n is a, c, g, or t

<220>
<221>  misc_feature
<222>  (234)..(234)
<223>  n is a, c, g, or t

<220>
<221>  misc_feature
<222>  (238)..(238)
<223>  n is a, c, g, or t

<220>
<221>  misc_feature
<222>  (252)..(252)
<223>  n is a, c, g, or t

<220>
<221>  misc_feature
<222>  (272)..(272)
<223>  n is a, c, g, or t

<220>
<221>  misc_feature
<222>  (275)..(275)
<223>  n is a, c, g, or t

<220>
<221>  misc_feature
<222>  (298)..(298)
<223>  n is a, c, g, or t

<220>
<221>  misc_feature
<222>  (329)..(329)
<223>  n is a, c, g, or t

<220>
<221>  misc_feature
<222>  (344)..(344)
<223>  n is a, c, g, or t

<220>
<221>  misc_feature
<222>  (346)..(346)
<223>  n is a, c, g, or t

<220>
<221>  misc_feature
<222>  (355)..(355)
<223>  n is a, c, g, or t

<220>
<221>  misc_feature
<222>  (361)..(361)
<223>  n is a, c, g, or t

<220>
<221>  misc_feature
<222>  (389)..(389)
<223>  n is a, c, g, or t

<220>
<221>  misc_feature
<222>  (397)..(398)
<223>  n is a, c, g, or t

<220>
<221>  misc_feature
<222>  (401)..(401)
<223>  n is a, c, g, or t

<220>
<221>  misc_feature
<222>  (405)..(406)
<223>  n is a, c, g, or t

<220>
<221>  misc_feature
<222>  (409)..(409)
<223>  n is a, c, g, or t

<220>
<221>  misc_feature
<222>  (412)..(412)
<223>  n is a, c, g, or t

<220>
<221>  misc_feature
<222>  (425)..(425)
<223>  n is a, c, g, or t

<220>
<221>  misc_feature
<222>  (430)..(430)
<223>  n is a, c, g, or t

<220>
<221>  misc_feature
<222>  (432)..(432)
<223>  n is a, c, g, or t

<220>
<221>  misc_feature
<222>  (437)..(437)

<223>  n is a, c, g, or t

<400>  73
aatatattgc tgnactgngn gtgngcnant tttattcaag gncatcgtga tgctgagaag      60

ttnccntgat aacctgncca tctctagttt caaccgtctt aatcaaaagt gtcctttttg     120

agngggtatc aaccaaaggg agtgaatcca nattantttc cctcaggttc agggaggaaa     180

agtttggaag aggcagaaaa atcctggtct cctcgccttc cancagcttc ctgnaggngg     240

caatctcaat gncaagggcc atccttaaca tngancaggt cttggtattc acgaaggnga     300

cgagccattt cctccttcat attctgaanc tcatcctgca ggcngncaat agtgncttgg     360

nagataccag attctacggg aaagttctnt ttcattnnac ncatnnggng gnccagggac     420

tcatnggttn cnttaanggc atcc                                            444


<210>  74
<211>  494
<212>  DNA
<213>  Homo sapiens


<220>
<221>  misc_feature
<222>  (310)..(310)
<223>  n is a, c, g, or t

<220>
<221>  misc_feature
<222>  (331)..(331)
<223>  n is a, c, g, or t

<220>
<221>  misc_feature
<222>  (437)..(437)
<223>  n is a, c, g, or t

<220>
<221>  misc_feature
<222>  (446)..(446)
<223>  n is a, c, g, or t

<220>
<221>  misc_feature
<222>  (466)..(466)
<223>  n is a, c, g, or t

<220>
<221>  misc_feature
<222>  (469)..(469)
<223>  n is a, c, g, or t

<220>
<221>  misc_feature
<222>  (475)..(475)
<223>  n is a, c, g, or t

<220>
<221>  misc_feature
<222>  (490)..(490)
<223>  n is a, c, g, or t

<400>  74
tgcgtgaaat ggaagagaac tttgccgttg aagctgctaa ctaccaagac actattggcc      60

gcctgcagga tgagattcag aatatgaagg aggaaatggc tcgtcacctt cgtgaatacc    120

aagacctgct caatgttaag atggcccttg acattgagat tgccacctac aggaagctgc    180

tggaaggcga ggagagcagg atttctctgc ctcttccaaa cttttcctcc ctgaacctga    240

gggaaactaa tctggattca ctccctctgg ttgataccca ctcaaaaagg acacttctga    300

ttaagacggn tgaaactaga gatggacagg ntatcaacaa aacttctcag catcacgatg    360

accttgaata aaattgccac actcagtgca gcatatatta ccagcaagaa taaaaagaaa    420

tccctatctt aagaacngct ttcagngcct ttctgcagtt ttcagnacnc aaganaaatt    480

tgaataggan aagc                                                      494

<210>  75
<211>  19
<212>  DNA
<213>  Homo sapiens

<400>  75
gcaactgaag gctggaaca                                                  19

<210>  76
<211>  20
<212>  DNA
<213>  Homo sapiens

<400>  76
tgaagaggcg caacattaaa                                                 20

<210>  77
<211>  23
<212>  DNA
<213>  Homo sapiens

<400>  77
agtgggtgaa tgtggttatg gcc                                             23

<210>  78
<211>  23
<212>  DNA
<213>  Homo sapiens

<400>  78
ctggcactgc tcaggatgtc ttc                                             23

<210>  79
<211>  21
<212>  DNA
<213>  Homo sapiens

<400>  79
gctggccatc attgaagagc t                                               21

<210>  80
<211>  21
<212>  DNA
<213>  Homo sapiens

<400>  80
tctccatggc actcttgctc c                                               21

<210>  81
<211>  17
<212>  DNA
<213>  Homo sapiens

<400>  81
gagatcgaga tccgcgc                                                    17


<210>  82
<211>  19
<212>  DNA
<213>  Homo sapiens

<400>  82
tgcagccatc gacagtgac                                                  19


<210>  83
<211>  21
<212>  DNA
<213>  Homo sapiens

<400>  83
ggcaccccat ctgttgatca c                                               21


<210>  84
<211>  23
<212>  DNA
<213>  Homo sapiens

<400>  84
ggtaaagaat tttggtccca gaa                                             23


<210>  85
<211>  23
<212>  DNA
<213>  Homo sapiens

<400>  85
tctaagaagt gctcagagag gtg                                             23


<210>  86
<211>  22
<212>  DNA
<213>  Homo sapiens

<400>  86
ttcatttctc tttcataacg gg                                              22


<210>  87
<211>  21
<212>  DNA
<213>  Homo sapiens

<400>  87
caaattcact aggcaagcgg a                                               21


<210>  88
<211>  22
<212>  DNA
<213>  Homo sapiens

<400>  88
ggttgtccct ttaatgcagc tt                                                22


<210>  89
<211>  21
<212>  DNA
<213>  Homo sapiens

<400>  89
tctgagctgc cccttcacca c                                                 21


<210>  90
<211>  23
<212>  DNA
<213>  Homo sapiens

<400>  90
acgtgccttc acatatgagc cag                                               23


<210>  91
<211>  21
<212>  DNA
<213>  Homo sapiens

<400>  91
attttgtccc gagaaggtgg c                                                 21


<210>  92
<211>  23
<212>  DNA
<213>  Homo sapiens

<400>  92
agcaggcaag gattatggtt ctc                                               23


<210>  93
<211>  22
<212>  DNA
<213>  Homo sapiens

<400>  93
ttctcaaggg tgcgagctca tc                                                22


<210>  94
<211>  20
<212>  DNA
<213>  Homo sapiens

<400>  94
tcctcccttg gccaccaatg                                                   20


<210>  95
<211>  23
<212>  DNA
<213>  Homo sapiens

<400>  95
aaggggtcac tatggagttc aaa                                               23


<210>  96

<211>  23
<212>  DNA
<213>  Homo sapiens

<400>  96
ggcactcata ttctccagca tct                                              23


<210>  97
<211>  23
<212>  DNA
<213>  Homo sapiens

<400>  97
tgggctattg gattaagtgt ggc                                              23


<210>  98
<211>  21
<212>  DNA
<213>  Homo sapiens

<400>  98
ttgacacggg atgaatcctg g                                                21


<210>  99
<211>  22
<212>  DNA
<213>  Homo sapiens

<400>  99
tggggagaat cacatttggg tc                                               22


<210>  100
<211>  22
<212>  DNA
<213>  Homo sapiens

<400>  100
atggcttcag agctggtcat gg                                               22


<210>  101
<211>  23
<212>  DNA
<213>  Homo sapiens

<400>  101
tcgcagatga attcagagca cag                                              23


<210>  102
<211>  23
<212>  DNA
<213>  Homo sapiens

<400>  102
ttgatgcgtg cacagatctt cag                                              23


<210>  103
<211>  17
<212>  DNA
<213>  Homo sapiens

<400>  103
gcaccacccg ctttttc                                                     17

<210>  104
<211>  22
<212>  DNA
<213>  Homo sapiens

<400>  104
gatccttgtc gatatccttc tg                                                22


<210>  105
<211>  22
<212>  DNA
<213>  Homo sapiens

<400>  105
cattcagggt ctatttgctc cg                                                22


<210>  106
<211>  20
<212>  DNA
<213>  Homo sapiens

<400>  106
gaagacgctg aacctgctgc                                                   20


<210>  107
<211>  23
<212>  DNA
<213>  Homo sapiens

<400>  107
acacactcag tgcagcaata tat                                               23


<210>  108
<211>  23
<212>  DNA
<213>  Homo sapiens

<400>  108
ggagtgtcgg ttgttaagaa cta                                               23


<210>  109
<211>  219
<212>  DNA
<213>  Homo sapiens

<400>  109
ggagggcaga ttcggaccac taggcctgaa atgacatttc actaaaagtc tccaaaacat      60

ttctaagact actaaggcct tttatgtaat tcctttaaat gtgtatttct taagaattca     120

aatttgtaat aaaactattt gtataaaaat taagctttta ttaatttgtt gctagtattg     180

ccacagacgc attaaaagaa acttactgca caagctgct                            219